# EUROPEAN PATENT APPLICATION

(11) **EP 1 790 636 A1**
(43) Date of publication of application: **30.05.2007**
(21) Application number: 05785870.6
(22) Date of filing: 16.09.2005
(51) Int. Cl.: C07D 211/46

(54) **PIPERIDINE DERIVATIVES AND USE THEREOF**

(30) Priority: 17.09.2004 JP 2004272639
(71) Applicant: Takeda Pharmaceutical Company Limited, Osaka-shi, Osaka 541-0045 (JP)
(72) Inventor: IKEURA, Yoshinori, Takeda Pharmaceutical Comp. Ltd, Osaka-shi, Osaka 5328686 (JP); HASHIMOTO, T., Takeda Pharmaceutical Company Ltd., Osaka-shi Osaka 5328686 (JP); NISHIDA, Haruyuki, Takeda Pharmaceutical Co. Ltd., Osaka-shi Osaka 5328686 (JP); SHIRAI, Junya, Takeda Pharmaceutical Company Ltd., Osaka-shi, Osaka 5328686 (JP); SAKAUCHI, N., Takeda Pharmaceutical CompanyLtd., Osaka-shi, Osaka 5328686 (JP)
(74) Representative: Rickard, Timothy Mark Adrian
(86) International application number: PCT/JP2005/017538
(87) International publication number: WO 2006/030975

(57) **Abstract**

The present invention provides a compound represented by the formula: 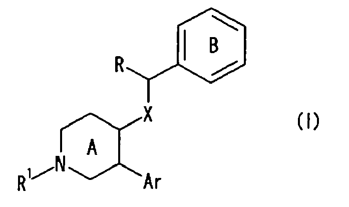 wherein Ar is an aryl group optionally having substituents, R is a C₁₋₆ alkyl group, R¹ is a hydrogen atom, a hydrocarbon group optionally having substituents, an acyl group or a heterocyclic group optionally having substituents, X is an oxygen atom or an imino group optionally having substituents, ring A is a piperidine ring optionally further having substituents, and ring B is a benzene ring having substituents, or a salt thereof, and an agent for the prophylaxis or treatment of lower urinary tract abnormality and the like, which contains the compound.

## Description

### TECHNICAL FIELD

The present invention relates to a novel piperidine derivative having excellent antagonistic action for a tachykinin receptor, and use thereof.

### BACKGROUND ART

Tachykinin is a generic term for a group of neuropeptides. Substance P (SP), neurokinin-A and neurokinin-B are known in mammals, and these peptides are known to bind to the corresponding receptors (neurokinin-1, neurokinin-2 and neurokinin-3) that exist in a living body and thereby to exhibit various biological activities.

Of such neuropeptides, SP has the longest history and has been studied in detail. In 1931, the existence of SP in the extract from equine intestines was confirmed, and in 1971, its structure was determined. SP is a peptide consisting of 11 amino acids.

SP is broadly distributed over the central and peripheral nervous systems, and has various physiological activities such as vasodilation, enhancement of vascular extravasation, contraction of smooth muscles, excitation of neurons, salivation, enhancement of diuresis, immunological enhancement and the like, in addition to the function as a transmitter substance for primary sensory neurons. In particular, it is known that SP released from the terminal in the spinal (dorsal) horn due to a pain impulse transmits the information of pain to secondary neurons, and that SP released from the peripheral terminal induces an inflammatory response in the receptor thereof. Thus, it is considered that SP is involved in various disorders (e.g., pain, headache, particularly migraine, Alzheimer's disease, multiple sclerosis, cardiovascular modulation, chronic inflammatory diseases such as chronic rheumatic arthritis, respiratory diseases including asthma or allergic rhinitis, intestinal inflammatory diseases including ulcerative colitis and Crohn's disease, ocular damage and ocular inflammatory diseases, proliferative vitreous retinopathy, irritable bowel syndrome, urinary frequency, psychosis, vomiting etc.) [see a review article: Physiological Reviews, Vol. 73, pp. 229-308 (1993); Journal of Autonomic Pharmacology, Vol. 13, pp. 23-93 (1993)].

At present, the following compounds have been known as those having antagonistic actions for SP receptors.

In EP-A-436,334, disclosed are the compound of the formula: 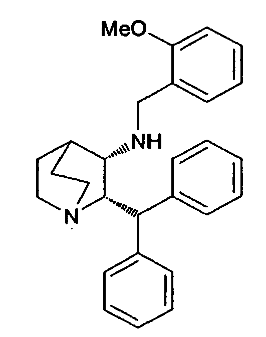 , and the like, in WO 92/17449, disclosed are the compound of the formula: 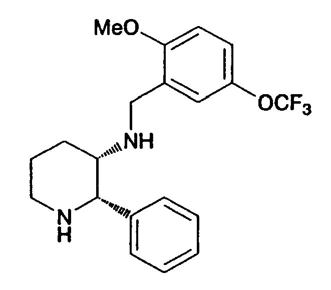 , and the like, in WO 95/16679, disclosed are the compound of the formula: 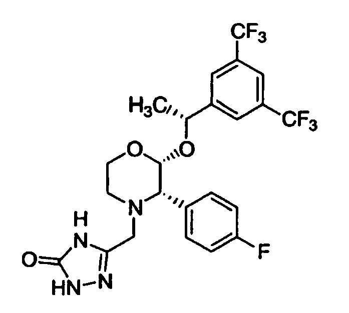 , and the like, and in JP-A-9-263585, disclosed are the heterocyclic compounds represented by the formula: 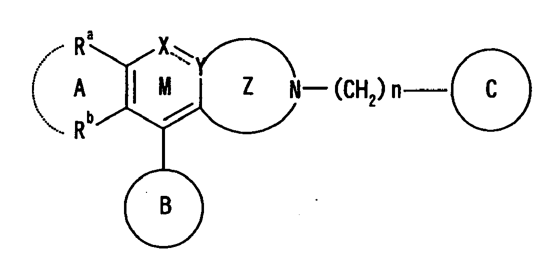 wherein Ring M is a heterocycle having -N=C<, -CO-N< or -CS-N< as a partial structure of 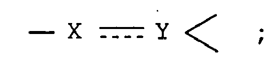 R^{a} and R^{b} are bonded to each other to form Ring A, or they are the same or different and represent a hydrogen atom or a substituent in Ring M; Ring A and Ring B are homocyclic or heterocyclic rings optionally having substituents, respectively and at least one of them is a heterocyclic ring optionally having substituents; Ring C is a homocyclic or heterocyclic ring optionally having substituents; Ring Z is a nitrogen-containing heterocyclic ring optionally having substituents; and n is an integer of 1 to 6, or salts thereof, and the like.

WO03/101964 describes a compound having a tachykinin receptor antagonistic action, which is represented by the formula: 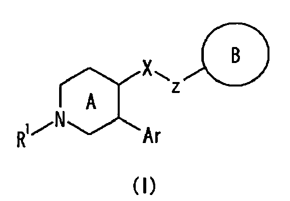 wherein Ar is an aryl group, an aralkyl group or an aromatic heterocyclic group, each of which optionally having substituents, R¹ is a hydrogen atom, a hydrocarbon group optionally having substituents, an acyl group or a heterocyclic group optionally having substituents, X is an oxygen atom or an imino group optionally having a substituent, Z is a methylene group optionally having substituents, ring A is a piperidine ring optionally further having substituents, ring B is an aromatic ring optionally having substituents, provided when Z is a methylene group substituted by an oxo group, then R¹ is not a methyl group and when Z is a methylene group substituted by a methyl group, then ring B is an aromatic ring having substituents] or a salt thereof.

An object of the present invention is to provide a piperidine derivative having antagonistic action for a tachykinin receptor etc. with a different chemical structure from the known compounds including the above-mentioned compounds, an agent for the prophylaxis or treatment of lower urinary tract abnormality comprising the compound, and the like.

### Disclosure of the Invention

The present inventors have made extensive studies in consideration of the above-mentioned situation and, as a result, have found unexpectedly that piperidine derivatives represented by the formula (I) below or a salt thereof have excellent antagonistic action for a tachykinin receptor (particularly antagonistic action for a SP receptor) as based on their peculiar chemical structures and are sufficiently satisfactory as pharmaceutical compositions. On the basis of these findings, the present inventors have completed the present invention.

Specifically, the present invention provides:
[1] a compound represented by the formula: 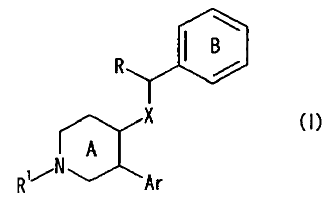 wherein Ar is an aryl group optionally having substituents, R is a C₁₋₆ alkyl group, R¹ is a hydrogen atom, a hydrocarbon group optionally having substituents, an acyl group or a heterocyclic group optionally having substituents, X is an oxygen atom or an imino group optionally having a substituent, ring A is a piperidine ring optionally further having substituents, and ring B is a benzene ring having substituents (hereinafter sometimes to be simply referred to as compound (I)) or a salt thereof;
[2] the compound of the above-mentioned [1], wherein Ar is a C₆₋₁₄ aryl group optionally having halogen atoms;
[3] the compound of the above-mentioned [1], wherein R is a methyl group;
[4] the compound of the above-mentioned [1], wherein R¹ is a hydrogen atom, a C₆₋₁₄ aryl-C₁₋₆ alkyl group or an acyl group;
[5] the compound of the above-mentioned [1], wherein X is an oxygen atom or NH;
[6] the compound of the above-mentioned [1], wherein Ar has a (R) configuration, and 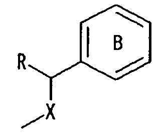 has an (S) configuration;
[7] the compound of the above-mentioned [1], which is an optically active compound represented by the formula: 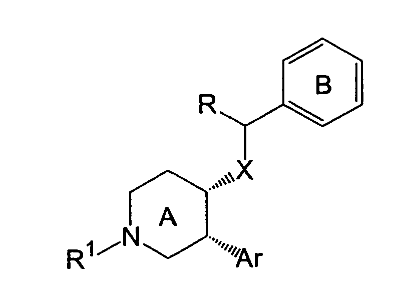 wherein each symbol is as defined in the above-mentioned [1];
[8] the compound of the above-mentioned [1], wherein X is an oxygen atom;
[9] the compound of the above-mentioned [1], wherein R has an (R) configuration and X is an oxygen atom;
[10] the compound of the above-mentioned [1], wherein ring B is a benzene ring having substituents selected from the group consisting of an optionally halogenated C₁₋₆ alkyl group and a halogen atom;
[11] the compound of the above-mentioned [1], wherein Ar is a C₆₋₁₄ aryl group optionally having halogen atoms,
   R is a methyl group, and R¹ is a hydrogen atom, a C₆₋₁₄ aryl-C₁₋₆ alkyl group or an acyl group;
[12] the compound of the above-mentioned [1], wherein Ar is a C₆₋₁₄ aryl group optionally having halogen atoms,
   R is a methyl group,
   R¹ is
   (1) a hydrogen atom,
   (2) a formyl group,
   (3) a group represented by the formula -(C=O)-NRb¹Rb²
      wherein Rb¹ and Rb² are each a hydrogen atom or a C₁₋₆ alkyl group,
   (4) a group represented by the formula -(C=O)-(CH₂)m-Rb³
      wherein m is an integer of 1 to 3, and Rb³ is a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which is optionally substituted by substituents selected from the group consisting of C₁₋₆ alkyl, formyl, C₁₋₆ alkyl-carbonyl, hydroxy, C₁₋₆ alkoxy-carbonyl, formylamino and C₁₋₆ alkyl-carbonylamino, and optionally has one or two oxos,
   (5) a group represented by the formula -(C=O)-(CH₂)n-NRb⁴Rb⁵
      wherein n is an integer of 1 to 4, Rb⁴ and Rb⁵ are each (a) a hydrogen atom, (b) a formyl group, (c) a C₁₋₆ alkyl-carbonyl group optionally having halogen atoms, (d) a C₁₋₆ alkoxy-carbonyl group, (e) a C₁₋₆ alkylsulfonyl group or (f) a mono- or di-C₁₋₆ alkyl-carbamoyl group,
   (6) a group represented by the formula: 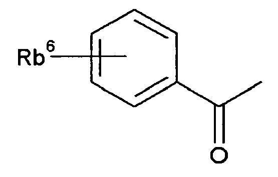 wherein Rb⁶ is formylamino or a C₁₋₆ alkyl-carbonylamino group,
   (7) a group represented by the formula: 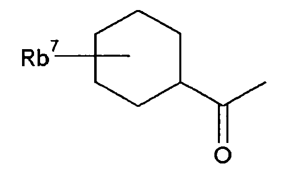 wherein Rb⁷ is
      (i) an amino group,
      (ii) a formylamino group,
      (iii) a C₁₋₆ alkyl-carbonylamino group optionally substituted by substituents selected from the group consisting of (a) C₁₋₆ alkyl, (b) hydroxy, (c) C₁₋₆ alkoxy, (d) a halogen atom, (e) formylamino, (f) C₁₋₆ alkyl-carbonylamino, and (g) a 5- or 6-membered non-aromatic or aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups and one or two C₁₋₆ alkyl groups,
      (iv) a C₃₋₇ cycloalkyl-carbonylamino group,
      (v) a C₁₋₆ alkoxy-carbonylamino group,
      (vi) a 5- or 6-membered aromatic heterocycle-carbonyl-amino group, wherein the heterocycle contains, besides carbon atoms, 1 to 4 of one or two kinds of hetero atoms selected from the group consisting of a nitrogen atom, an oxygen atom and a sulfur atom,
      (vii) an ureido group optionally substituted by one or two C₁₋₆ alkyl groups,
      (viii) a C₁₋₆ alkoxy-carbonyl group,
      (ix) a carbamoyl group optionally having C₁₋₆ alkoxy groups,
      (x) a mono- or di-C₁₋₆ alkyl-carbamoyl group,
      (xi) a C₁₋₆ alkylsulfonylamino group,
      (xii) a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups, or
      (xiii) a 5- or 6-membered aromatic or non-aromatic heterocycle-carbonyl group, wherein the heterocyclic group contains, besides carbon atoms, 1 to 4 of one or two kinds of hetero atoms selected from the group consisting of a nitrogen atom, an oxygen atom and a sulfur atom,
   (8) a group represented by the formula: 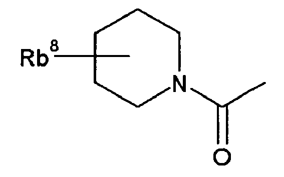 wherein Rb⁸ is (i) a formylamino group, (ii) a C₁₋₆ alkyl-carbonylamino group optionally substituted by hydroxyl groups, (iii) a C₁₋₆ alkylsulfonylamino group, or (iv) a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups,
   (9) a group represented by the formula: 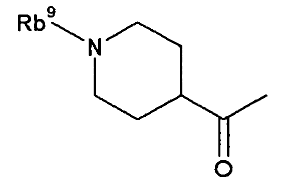 wherein Rb⁹ is
      (i) a hydrogen atom,
      (ii) a C₁₋₆ alkyl group optionally substituted by substituents selected from the group consisting of formyl, C₁₋₆ alkyl-carbonyl, C₁₋₆ alkoxy-carbonyl, carbamoyl and mono or di-C₁₋₆ alkyl-carbamoyl,
      (iii) a formyl group,
      (iv) a C₁₋₆ alkyl-carbonyl group optionally substituted by substituents selected from the group consisting of (a) formylamino, (b) C₁₋₆ alkyl-carbonylamino, and (c) a 5- or 6-membered aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups,
      (v) a 5- or 6-membered aromatic or non-aromatic heterocycle-carbonyl group, wherein the heterocycle comprises, besides carbon atoms, 1 to 4 of one or two kinds of hetero atoms selected from the group consisting of a nitrogen atom, an oxygen atom and a sulfur atom, which optionally has one or two oxo groups,
      (vi) a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups,
      (vii) a formylamino group,
      (viii) a C₁₋₆ alkyl-carbonylamino group,
      (ix) a C₁₋₆ alkylsulfonyl group,
      (x) a C₁₋₆ alkoxy-carbonyl group, or
      (xi) a mono- or di-C₁₋₆ alkyl-carbamoyl group,
   (10) a group represented by the formula: 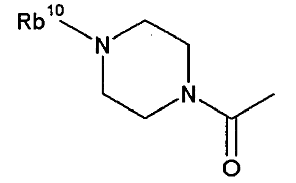 wherein Rb¹⁰ is (i) a formyl group, (ii) a C₁₋₆ alkyl-carbonyl group or (iii) a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups,
   (11) a group represented by the formula: 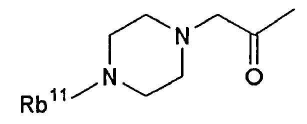 wherein Rb¹¹ is a C₁₋₆ alkyl group, a formyl group, or a C₁₋₆ alkyl-carbonyl group,
   (12) a group represented by the formula: 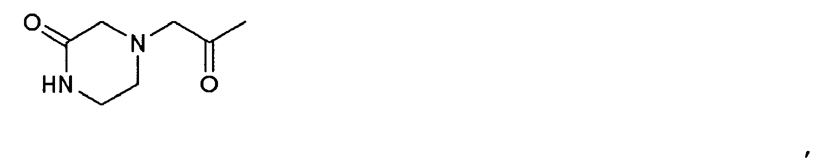
   (13) a group represented by the formula: 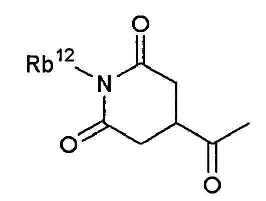 wherein Rb¹² is a hydrogen atom or a C₁₋₆ alkyl group,
   (14) a group represented by the formula: 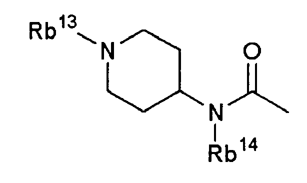 wherein Rb¹³ is
      (i) a hydrogen atom,
      (ii) a formyl group,
      (iii) a C₁₋₆ alkyl-carbonyl group optionally substituted by hydroxyl groups,
      (iv) a C₁₋₆ alkoxy-carbonyl group, or
      (v) a C₁₋₆ alkylsulfonyl group, and
         Rb¹⁴ is a hydrogen atom or a C₁₋₆ alkyl group,
   (15) a group represented by the formula: 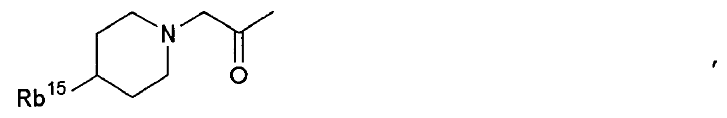 wherein Rb¹⁵ is a hydroxy group, formylamino or a C₁₋₆ alkyl-carbonylamino group,
   (16) a group represented by the formula: 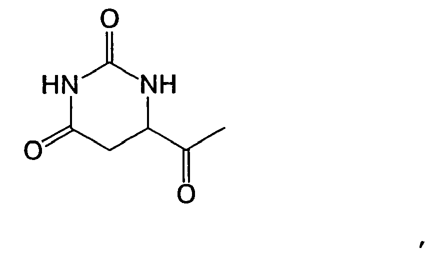
   (17) a group represented by the formula: 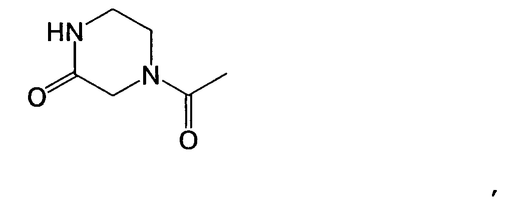
   (18) a group represented by the formula: 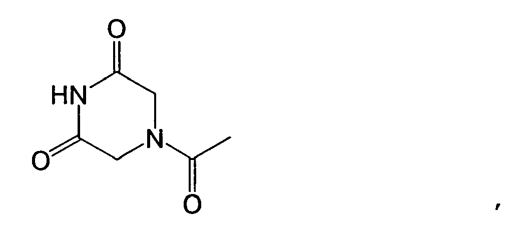
   (19) a C₁₋₆ alkyl-carbonyl group optionally substituted by substituents selected from the group consisting of (i) cyano, (ii) hydroxy, (iii) carboxy, (iv) C₁₋₆ alkoxy-carbonyl, (v) carbamoyl, (vi) C₁₋₆ alkyl-carbamoyl and (vii) a 5- or 6-membered aromatic or non-aromatic heterocycle-carbonyl, wherein the heterocycle comprises, besides carbon atoms, 1 to 4 of one or two kinds of hetero atoms selected from the group consisting of a nitrogen atom, an oxygen atom and a sulfur atom,
   (20) a C₃₋₆ cycloalkyl-carbonyl group optionally substituted by carbamoyl,
   (21) a C₁₋₆ alkenyl-carbonyl group substituted by a 5- or 6-membered aromatic or non-aromatic heterocyclic groups containing, besides carbon atoms, 1 to 4 of one or two kinds of hetero atoms selected from the group consisting of a nitrogen atom, an oxygen atom and a sulfur atom, which is optionally substituted by C₁₋₆ alkyl groups,
   (22) a group represented by the formula-(C=O)-(CRb¹⁶Rb¹⁷)-NHRb¹⁸
      wherein Rb¹⁶ is a hydrogen atom or a C₁₋₆ alkyl group, Rb¹⁷ is a C₁₋₆ alkyl group optionally substituted by substituents selected from the group consisting of a carbamoyl group and a 5- or 6-membered aromatic heterocyclic group containing, besides carbon atoms, 1 to 4 of one or two kinds of hetero atoms selected from the group consisting of a nitrogen atom, an oxygen atom and a sulfur atom, and Rb¹⁸ is a hydrogen atom, a C₁₋₆ alkyl-carbonyl group or a C₁₋₆ alkoxy-carbonyl group, or
   (23) a C₆₋₁₄ aryl-C₁₋₆ alkyl group,
      X is an oxygen atom or NH, and
      ring B is a benzene ring having substituents selected from the group consisting of an optionally halogenated C₁₋₆ alkyl group and a halogen atom;
[13] (3R, 4S)-4-[(1R)-1-[3,5-Bis(trifluoromethyl)phenyl]ethoxy] - 3-phenyl-1-[[1-(1H-tetrazol-1-ylacetyl)piperidin-4-yl]carbonyl]piperidine, N-[trans-4-[[(3R,4S)-4-[(1R)-1-[3,5-bis(trifluoromethyl)phenyl]ethoxy]-3-phenylpiperidin-1-yl]carbonyl]cyclohexyl]propanamide, 4-[[(3R,4S)-4-[(1R)-1-[3,5-bis(trifluoromethyl)phenyl]ethoxy]-3-phenylpiperidin-1-yl]carbonyl]piperidine-2,6-dione, (3R,4S)-4-{(1R)-1-[3,5-bis(trifluoromethyl)phenyl]ethoxy}-3-phenyl-1-{[1-(1H-1,2,4-triazol-1-ylacetyl)piperidin-4-yl]carbonyl}piperidine, (3R,4S)-4-{(1R)-1-[3,5-bis(trifluoromethyl)phenyl]ethoxy}-3-phenyl-1-{[1-(1H-pyrazol-1-ylacetyl)piperidin-4-yl]carbonyl}piperidine, or a salt thereof;
[14] a prodrug of the compound of the above-mentioned [1];
[15] a pharmaceutical composition comprising the compound of the above-mentioned [1] or a prodrug thereof;
[16] the pharmaceutical composition of the above-mentioned [15], which is a tachykinin receptor antagonist;
[17] the pharmaceutical composition of the above-mentioned [15], which is an agent for the prophylaxis or treatment of lower urinary tract abnormality, gastrointestinal disease or central nervous system disease;
[18] the pharmaceutical composition of the above-mentioned [15], which is an agent for the prophylaxis or treatment of overactive bladder, irritable bowel syndrome, inflammatory bowel disease, vomiting, nausea, depression, anxiety neurosis, anxiety, pelvic visceral pain or interstitial cystitis;
[19] a method for the prophylaxis or treatment of lower urinary tract abnormality, gastrointestinal disease or central nervous system disease in a mammal, which comprises administering an effective amount of the compound of the above-mentioned [1] or a prodrug thereof; and
[20] use of the compound of the above-mentioned [1] or a prodrug thereof for the production of an agent for the prophylaxis or treatment of lower urinary tract abnormality, gastrointestinal disease or central nervous system disease.

### Best Mode for carrying out the Invention

The present invention is described in detail in the following.

### (Explanation of Ar)

In the above-mentioned formula (I), Ar is an aryl group optionally having substituents.

The "aryl group" includes, for example, a C₆₋₁₄ aryl group such as phenyl, naphthyl, anthryl, phenanthryl etc., preferably, a phenyl group.

The substituent for the "aryl group" includes, for example, 1 to 3 substituents selected from the group consisting of (1) a halogen atom (e.g., fluorine, chlorine, bromine, iodine etc.), (2) C₁₋₃ alkylenedioxy (e.g., methylenedioxy, ethylenedioxy etc.), (3) nitro, (4) cyano, (5) optionally halogenated C₁₋₆ alkyl , (6) optionally halogenated C₂₋₆ alkenyl, (7) optionally halogenated C₂₋₆ alkynyl , (8) optionally halogenated C₃₋₆ cycloalkyl, (9) C₆₋₁₄ aryl (e.g., phenyl, 1-naphthyl, 2-naphthyl, biphenylyl, 2-anthryl etc.), (10) optionally halogenated C₁₋₆ alkoxy, (11) optionally halogenated C₁₋₆ alkylthio or mercapto, (12) hydroxy, (13) amino, (14) mono-C₁₋₆ alkylamino (e.g., methylamino, ethylamino etc.), (15) mono-C₆₋₁₄ arylamino (e.g., phenylamino, 1-naphthylamino, 2-naphthylamino etc.), (16) di-C₁₋₆ alkylamino (e.g., dimethylamino, diethylamino etc.), (17) di-C₆₋₁₄ arylamino (e.g., diphenylamino etc.), (18) acyl, (19) acylamino, (20) acyloxy, (21) 5- to 7-membered cyclic amino optionally having substituents, (22) a 5- to 10-membered aromatic heterocyclic group (e.g., 2- or 3-thienyl, 2-, 3- or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[b]furanyl etc.), (23) sulfo, (24) C₆₋₁₄ aryloxy (e.g., phenyloxy, naphthyloxy etc.), (25) a group obtained by combining 1 to 3 groups of the above-mentioned (1)-(24) and the like.

The "optionally halogenated C₁₋₆ alkyl" includes, for example, C₁₋₆ alkyl (e.g., methyl, ethyl, propyl, isopropyl, butyl, isobutyl, sec-butyl, tert-butyl, pentyl, hexyl etc.), optionally having 1 to 5, preferably 1 to 3 halogen atoms (e.g., fluorine, chlorine, bromine, iodine etc.) and the like, specifically, methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl etc.

The "optionally halogenated C₂₋₆ alkenyl" includes, for example, C₂₋₆ alkenyl (e.g., ethenyl (vinyl), allyl, isopropenyl, butenyl, isobutenyl, sec-butenyl etc.) optionally having 1 to 5, preferably 1 to 3 halogen atoms (e.g., fluorine, chlorine, bromine, iodine etc.) and the like, specifically, ethenyl (vinyl), allyl, isopropenyl, butenyl, isobutenyl, sec-butenyl, 3,3,3-trifluoro-1-propenyl, 4,4,4-trifluoro-l-butenyl etc.

The "optionally halogenated C₂₋₆ alkynyl" includes, for example, C₂₋₆ alkynyl (e.g., ethynyl, propargyl, butynyl, 1-hexynyl etc.) optionally having 1 to 5, preferably 1 to 3 halogen atoms (e.g., fluorine, chlorine, bromine, iodine etc.) and the like, specifically, ethynyl, propargyl, butynyl, 1-hexynyl, 3,3,3-trifluoro-1-propynyl, 4,4,4-trifluoro-1-butynyl etc.

The "optionally halogenated C₃₋₆ cycloalkyl" includes, for example, C₃₋₆ cycloalkyl (e.g., cyclopropyl, cyclobutyl, cyclopentyl, cyclohexyl etc.) optionally having 1 to 5, preferably 1 to 3 halogen atoms (e.g., fluorine, chlorine, bromine, iodine etc.) and the like, specifically, cyclopropyl, cyclobutyl, cyclopentyl, cyclohexyl, 4,4-dichlorocyclohexyl, 2,2,3,3-tetrafluorocyclopentyl, 4-chlorocyclohexyl etc.

The "optionally halogenated C₁₋₆ alkoxy" includes, for example, C₁₋₆ alkoxy (e.g., methoxy, ethoxy, propoxy, isopropoxy, butoxy, isobutoxy, sec-butoxy, pentyloxy, hexyloxy etc.) optionally having 1 to 5, preferably 1 to 3 halogen atoms (e.g., fluorine, chlorine, bromine, iodine etc.) and the like, specifically, methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy etc.

The "optionally halogenated C₁₋₆ alkylthio" includes, for example, C₁₋₆ alkylthio (e.g., methylthio, ethylthio, propylthio, isopropylthio, butylthio, sec-butylthio, tert-butylthio etc.) optionally having 1 to 5, preferably 1 to 3 halogen atoms (e.g., fluorine, chlorine, bromine, iodine etc.) and the like, specifically, methylthio, difluoromethylthio, trifluoromethylthio, ethylthio, propylthio, isopropylthio, butylthio, 4,4,4-trifluorobutylthio, pentylthio, hexylthio etc.

The "acyl" includes, for example, -(C=O)-R³, -(C=S)-R³, -SO₂-R³, -SO-R³, -(P=O) (OR⁴) (OR⁴') (R³ is a hydrogen atom, a hydrocarbon group optionally having substituents, an amino group optionally having substituents, a hydroxy group optionally having a substituent or a heterocyclic group optionally having substituents, and R⁴ and R⁴' are the same or different and represents a hydrogen atom or a hydrocarbon group optionally having substituents) and the like.

The "hydrocarbon group optionally having substituents" represented by R³, R⁴ or R⁴, includes, for example, the same group as those referred to herein for the "hydrocarbon group optionally having substituents" represented by R¹ which will be described below.

The "substituent" for the "amino group optionally having substituents" represented by R³ includes, for example, a hydrocarbon group optionally having substituents, a heterocyclic group optionally having substituents, a hydroxy group optionally having a substituent, an acyl group and the like.

The "hydrocarbon group optionally having substituents" as the "substituent" for the "amino group optionally having substituents" represented by R³ includes, for example, the same group as those referred to herein for the "hydrocarbon group optionally having substituents" represented by R¹ which will be described below.

The "heterocyclic group optionally having substituents" as the "substituent" in the "amino group optionally having substituents" represented by R³ includes, for example, the same group as those referred to herein for the "heterocyclic group optionally having substituents" represented by R¹ which will be described below.

The "hydroxy group optionally having a substituent" as the "substituent" for the "amino group optionally having substituents" represented by R³ includes, for example, (i) a hydroxy group, (ii) a C₁₋₆ alkoxy group (e.g., a methoxy group, an ethoxy group, a propoxy group, an isopropoxy group, a butoxy group, a tert-butoxy group etc.), (iii) a C₆₋₁₄ aryloxy group (e.g., a phenyloxy group, a naphthyloxy group etc.), (iv) a formyloxy group or a C₁₋₆ alkyl-carbonyloxy group (e.g., an acetoxy group, a propionyloxy group etc.) and (v) a C₆₋₁₄ aryl-carbonyloxy group (e.g., a benzoyloxy group, a naphthylcarbonyloxy group etc.) and the like, and preferably, a hydroxy group and a C₁₋₆ alkoxy group (e.g., a methoxy group, an ethoxy group, a propoxy group, an isopropoxy group etc.).

The "acyl group" as the "substituent" for the "amino group optionally having substituents" represented by R³ includes, for example, -(C=O)-R", -(C=S)-R", -SO₂-R", -SO-R", -(C=O)NR"R'", -(C=O)O-R", -(C=S)O-R", -(C=S)NR"R'" (R" is a hydrogen atom or a hydrocarbon group optionally having substituents, R"' is a hydrogen atom or a lower alkyl group (e.g., a C₁₋₆ alkyl group such as methyl, ethyl, propyl, isopropyl, butyl, isobutyl, sec-butyl, tert-butyl, pentyl, hexyl etc., and particularly preferably a C₁₋₃ alkyl group such as methyl, ethyl, propyl, isopropyl etc.) and the like.

The "hydrocarbon group optionally having substituents" represented by R" includes, for example, the same group as those referred to herein for the "hydrocarbon group optionally having substituents" represented by R¹ which will be described below.

The "C₁₋₆ alkoxy group", the "C₆₋₁₄ aryloxy group", the "formyloxy group", the "C₁₋₆ alkyl-carbonyloxy group" and the "C₆₋₁₄ aryl-carbonyloxy group" exemplified as the "hydroxy group optionally having a substituent" as the "substituent" for the "amino group optionally having substituents" represented by R³, may be optionally further substituted with the same groups as those referred to herein for the "substituent" for the "hydrocarbon group optionally having substituents" represented by R¹ which will be described below and the like, and such a substituent preferably includes a halogen atom (e.g., fluorine, chlorine, bromine etc.) and the like.

The "amino group optionally having substituents" represented by R³ may form a cyclic amino group (e.g., a 5- to 9-membered cyclic amino group having 1 to 3 hetero atoms such as an oxygen atom, a sulfur atom etc. in addition to a nitrogen atom (e.g., a pyrrolidino group, a piperidinyl group, a piperazinyl group, a morpholino group etc.) and the like.

The "hydroxy group optionally having a substituent" represented by R³ includes, for example, the same group as those referred to herein for the "hydroxy group optionally having a substituent" as the "substituent" for the "amino group optionally having substituents" represented by R³, and the like.

The "heterocyclic group optionally having substituents" represented by R³ includes, for example, the same group as those referred to herein for the "heterocyclic group optionally having substituents" represented by R¹ which will be described below.

The "acylamino" as described above includes, for example, formylamino, C₁₋₆ alkyl-carbonylamino (e.g., acetylamino etc.), heterocycle-C₁₋₆ alkyl-carbonylamino (e.g., optionally oxidized piperidino-acetylamino etc.), C₃₋₇ cycloalkyl-carbonylamino (e.g., cyclopropylcarbonylamino etc.), C₆₋₁₄ aryl-carbonylamino (e.g., phenylcarbonylamino, naphthylcarbonylamino etc.), heterocycle-carbonylamino (e.g., thienylcarbonylamino, furylcarbonylamino, pyrrolylcarbonylamino etc.), C₁₋₆ alkoxy-carbonylamino (e.g., methoxycarbonylamino, ethoxycarbonylamino, propoxycarbonylamino, butoxycarbonylamino etc.), C₆₋₁₄ aryloxy-carbonylamino (e.g., phenoxycarbonylamino, naphthoxycarbonylamino etc.), heterocycleoxy-carbonylamino, C₁₋₆ alkylsulfonylamino (e.g., methylsulfonylamino, ethylsulfonylamino etc.), C₆₋₁₄ arylsulfonylamino (e.g., phenylsulfonylamino, 2-naphthylsulfonylamino, 1-naphthylsulfonylamino etc.), heterocycle-sulfonylamino, ureido, mono- or di-C₁₋₆ alkyl-ureido (e.g., methylureido, dimethylureido etc.), mono- or di-C₆₋₁₄ aryl-ureido (e.g., phenylureido, diphenylureido etc.) and the like.

The "acyloxy" as described above includes, for example, formyloxy, C₁₋₆ alkyl-carbonyloxy (e.g., acetoxy, propionyloxy etc.), heterocycle-C₁₋₆ alkyl-carbonyloxy, C₃₋₇ cycloalkyl-carbonyloxy (e.g., cyclopropylcarbonyloxy etc.), C₆₋₁₄ aryl-carbonyloxy (e.g., benzoyloxy, naphthylcarbonyloxy etc.), heterocycle-carbonyloxy, C₁₋₆ alkoxy-carbonyloxy (e.g., methoxycarbonyloxy, ethoxycarbonyloxy, propoxycarbonyloxy, butoxycarbonyloxy etc.), C₆₋₁₄ aryloxy-carbonyloxy, heterocycleoxy-carbonyloxy, mono-C₁₋₆ alkyl-carbamoyloxy (e.g., methylcarbamoyloxy, ethylcarbamoyloxy etc.), di-C₁₋₆ alkyl-carbamoyloxy (e.g., dimethylcarbamoyloxy, diethylcarbamoyloxy etc.), C₆₋₁₄ aryl-carbamoyloxy (e.g., phenylcarbamoyloxy, naphthylcarbamoyloxy etc.), nicotinoyloxy etc.

Here, as the heterocycle of heterocycle-C₁₋₆ alkyl-carbonylamino, heterocycle-carbonylamino, heterocycleoxy-carbonylamino, heterocycle-sulfonylamino, heterocycle-C₁₋₆ alkyl-carbonyloxy, heterocycle-carbonyloxy and heterocycleoxy-carbonyloxy, for example, a 5- to 14-membered (preferably 5- to 9-membered, more preferably 5- or 6-membered) non-aromatic heterocycle containing, besides carbon atoms, 1 to 4 of one or two kinds of hetero atoms selected from the group consisting of a nitrogen atom, an oxygen atom and a sulfur atom (e.g., pyrrolidinyl, tetrahydrofuryl, tetrahydrothienyl, piperidyl, tetrahydropyranyl, morpholinyl, thiomorpholinyl, piperazinyl), or an aromatic heterocyclic group containing, besides carbon atoms, 1 to 4 of one or two kinds of hetero atoms selected from the group consisting of a nitrogen atom, an oxygen atom and a sulfur atom (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl), each of which optionally has one or two oxo groups, and the like are used.

The "5- to 7-membered saturated cyclic amino" for the "5-to 7-membered saturated cyclic amino optionally having substituents" includes, for example, morpholino, thiomorpholino, piperazin-1-yl, piperidino, pyrrolidin-1-yl etc. The "substituent" in the "5- to 7-membered saturated cyclic amino optionally having substituents" includes, for example, C₁₋₆ alkyl (e.g., methyl, ethyl, propyl, isopropyl, butyl, isobutyl, sec-butyl, tert-butyl, pentyl, hexyl etc.), C₆₋₁₄ aryl (e.g., phenyl, 1-naphthyl, 2-naphthyl, biphenylyl, 2-anthryl etc.), a 5- to 10-membered aromatic heterocyclic group (e.g., 2- or 3-thienyl, 2-, 3- or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[b]furanyl etc.) and the like. The number of the substituents is 1 to 3.

Ar is preferably a C₆₋₁₄ aryl group optionally having halogen atoms (e.g., fluorine atom), more preferably a phenyl group optionally having halogen atoms (e.g., fluorine atom) (particularly, a phenyl group having a halogen atom at the para-position).

Ar is most preferably a phenyl group optionally substituted by a fluorine atom at the para-position and the like.

### (Explanation of R)

In the above-mentioned the formula (I), R is a C₁₋₆ alkyl group.

The C₁₋₆ alkyl group represented by R includes, for example, methyl, ethyl, propyl, isopropyl, butyl, isobutyl, sec-butyl and tert-butyl, pentyl, hexyl etc., preferably a C₁₋₃ alkyl group such as methyl, ethyl etc., particularly preferably a methyl group.

### (Explanation of R¹)

R¹ is a hydrogen atom, a hydrocarbon group optionally having substituents, an acyl group or a heterocyclic group optionally having substituents in the above-mentioned formula (I).

The "hydrocarbon group" for the "hydrocarbon group optionally having substituents" represented by R¹ includes, for example, an aliphatic hydrocarbon group, a monocyclic saturated hydrocarbon group, an aromatic hydrocarbon group etc., preferably having 1 to 16 carbons, and specifically, for example, an alkyl group, an alkenyl group, an alkynyl group, a cycloalkyl group, an aryl group etc.

The "alkyl group" is preferably, for example, a lower alkyl group etc., for example, a C₁₋₆ alkyl group such as methyl, ethyl, propyl, isopropyl, butyl, isobutyl, sec-butyl, tert-butyl, pentyl, hexyl etc.

The "alkenyl group" is preferably, for example, a lower alkenyl group etc., for example, a C₂₋₆ alkenyl group such as ethenyl (vinyl), 1-propenyl, allyl, isopropenyl, butenyl, isobutenyl etc.

The "alkynyl group" is preferably, for example, a lower alkynyl group etc., for example, a C₂₋₆ alkynyl group such as ethynyl, propargyl, 1-propynyl etc.

The "cycloalkyl group" is preferably, for example, a lower cycloalkyl group etc., for example, a C₃₋₆ cycloalkyl group such as cyclopropyl, cyclobutyl, cyclopentyl, cyclohexyl etc.

The "aryl group" is preferably, for example, a C₆₋₁₄ aryl group such as phenyl, 1-naphthyl, 2-naphthyl, biphenylyl, 2-anthryl etc., specifically, a phenyl group etc.

The substituent that the "hydrocarbon group" for the "hydrocarbon group optionally having substituents" represented by R¹ may have includes, for example, (1) a halogen atom (e.g., a fluorine atom, a chlorine atom, a bromine atom, an iodine atom etc.), (2) a nitro group, (3) a cyano group, (4) a hydroxy group , (5) an optionally halogenated lower alkyl group (e.g., an optionally halogenated C₁₋₆ alkyl group such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, isobutyl, sec-butyl, tert-butyl, 4,4,4-trifluorobutyl, pentyl, isopentyl, neopentyl, 5,5,5-trifluoropentyl, hexyl, 6,6,6-trifluorohexyl etc.), (6) optionally halogenated C₂₋₆ alkenyl, (7) optionally halogenated C₂₋₆ alkynyl, (8) optionally halogenated C₃₋₆ cycloalkyl, (9) optionally halogenated a lower alkoxy group (e.g., a C₁₋₆ alkoxy group such as methoxy, ethoxy, propoxy, isopropoxy, butoxy, isobutoxy, pentyloxy and hexyloxy etc.), (10) acyloxy, (11) optionally halogenated C₁₋₆ alkylthio or mercapto, (12) acyl, (13) an amino group, (14) a mono-lower alkylamino group (e.g., a mono-C₁₋₆ alkylamino group such as methylamino and ethylamino etc.), (15) a di-lower alkylamino group (e.g., a di-C₁₋₆ alkylamino group such as dimethylamino and diethylamino etc.), (16) mono-C₆₋₁₄ arylamino (e.g., phenylamino, 1-naphthylamino, 2-naphthylamino etc.), (17) di-C₆₋₁₄ arylamino (e.g., diphenylamino etc.), (18) acylamino, (19) a carboxyl group, (20) an aryl group (e.g., a C₆₋₁₄ aryl group such as phenyl, naphthyl, biphenyl, 2-anthryl etc.), (21) an aryloxy group (e.g., a C₆₋₁₄ aryloxy group such as phenyloxy and naphthyloxy etc.), (22) an optionally halogenated lower alkylcarbonylamino group (e.g., an optionally halogenated C₁₋₆ alkyl-carbonylamino group such as acetylamino and trifluoroacetylamino etc.), (23) an oxo group, (24) 5- to 7-membered cyclic amino optionally having substituents, (25) a heterocyclic group, (26) C₁₋₃ alkylenedioxy (e.g., methylenedioxy, ethylenedioxy etc.), (27) mono- or di-C₁₋₆ alkylureido (e.g., methylureido, ethylureido, isopropylureido, dimethylureido etc.), (28) a group obtained by combining 1 to 3 groups of the above-mentioned (1)-(27) and the like.

The "hydrocarbon group" for the "hydrocarbon group optionally having substituents" optionally has 1 to 5, preferably 1 to 3 substituents mentioned above at the substitutable positions on the hydrocarbon group. If the number of the substituents is 2 or more, the substituents may be the same or different.

The "acyl" as the "substituent" for the "hydrocarbon group optionally having substituents" represented by R¹ includes, for example, formyl, C₁₋₆ alkyl-carbonyl (e.g., acetyl, propionyl etc.), heterocycle-C₁₋₆ alkyl-carbonyl, C₃₋₇ cycloalkyl-carbonyl (e.g., cyclopropylcarbonyl etc.), C₆₋₁₄ aryl-carbonyl (e.g., phenylcarbonyl, naphthylcarbonyl etc.), heterocycle-carbonyl, C₁₋₆ alkoxy-carbonyl (e.g., methoxycarbonyl, ethoxycarbonyl, propoxycarbonyl, butoxycarbonyl etc.), C₆₋₁₄ aryloxy-carbonyl (e.g., phenoxycarbonyl, naphthoxycarbonyl etc.), heterocycleoxy-carbonyl, C₁₋₆ alkylsulfonyl (e.g., methylsulfonyl, ethylsulfonyl etc.), C₆₋₁₄ arylsulfonyl (e.g., phenylsulfonyl, 2-naphthylsulfonyl, 1-naphthylsulfonyl etc.), heterocycle-sulfonyl, C₁₋₆ alkylsulfinyl (e.g., methylsulfinyl, ethylsulfinyl, propylsulfinyl, butylsulfinyl etc.), C₆₋₁₄ arylsulfinyl (e.g., phenylsulfinyl, naphthylsulfinyl etc.), carbamoyl, thiocarbamoyl, mono-C₁₋₆ alkyl-carbamoyl (e.g., methylcarbamoyl, ethylcarbamoyl etc.), di-C₁₋₆ alkyl-carbamoyl (e.g., dimethylcarbamoyl, diethylcarbamoyl etc.), C₆₋₁₄ aryl-carbamoyl (e.g., phenylcarbamoyl, naphthylcarbamoyl etc.), nicotinoyl and the like. The heterocycle of the heterocycle-C₁₋₆ alkyl-carbonyl, heterocycle-carbonyl, heterocycleoxy-carbonyl and heterocycle-sulfonyl includes, for example, a 5- to 14-membered (preferably 5- to 9-membered, more preferably 5- or 6-membered) non-aromatic heterocycle containing, besides carbon atoms, 1 to 4 of one or two kinds of hetero atoms selected from the group consisting of a nitrogen atom, an oxygen atom and a sulfur atom (e.g., pyrrolidinyl, tetrahydrofuryl, tetrahydrothienyl, piperidyl, tetrahydropyranyl, morpholinyl, thiomorpholinyl, piperazinyl) or an aromatic heterocyclic group containing, besides carbon atoms, 1 to 4 of one or two kinds of hetero atoms selected from the group consisting of a nitrogen atom, an oxygen atom and a sulfur atom (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl), each of which optionally has substituents such as a halogen atom, optionally halogenated C₁₋₆ alkyl, C₁₋₆ alkoxy, oxo and the like, and the like.

The "acyloxy" and "acylamino" as the "substituent" for the "hydrocarbon group optionally having substituents" represented by R¹ includes the same group as those referred to herein for the aforementioned "acyloxy" and "acylamino" as the substituent of the "aryl group" represented by Ar.

The "5- to 7-membered cyclic amino optionally having substituents" as the "substituent" for the "hydrocarbon group optionally having substituents" represented by R¹ includes the same group as those referred to herein for the aforementioned "5- to 7-membered cyclic amino optionally having substituents" as the substituent of the "aryl group" represented by Ar.

The "heterocyclic group" as the "substituent" for the "hydrocarbon group optionally having substituents" represented by R¹ includes, for example, a 5- to 14-membered (preferably 5-to 9-membered, more preferably 5- or 6-membered) aromatic heterocyclic group containing, besides carbon atoms, 1 to 4 of one or two kinds of hetero atoms selected from the group consisting of a nitrogen atom, an oxygen atom and a sulfur atom (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl) or a non-aromatic heterocyclic group containing, besides carbon atoms, 1 to 4 of one or two kinds of hetero atoms selected from the group consisting of a nitrogen atom, an oxygen atom and a sulfur atom (e.g., pyrrolidinyl, tetrahydrofuryl, tetrahydrothienyl, piperidyl, tetrahydropyranyl, morpholinyl, thiomorpholinyl, piperazinyl) and the like. These non-aromatic heterocyclic group is optionally further condensed with other aromatic or non-aromatic homocyclic ring or heterocycle. The "heterocyclic group" may also have one or two oxo groups, and may have substituents such as a halogen atom, optionally halogenated C₁₋₆ alkyl, C₁₋₆ alkoxy, hydroxy, formyl, C₁₋₆ alkyl-carbonyl, C₁₋₆ alkoxy-carbonyl, formylamino, C₁₋₆ alkyl-carbonylamino and the like.

The "acyl group" represented by R¹ includes, for example, the same group as those referred to herein above for the foregoing "acyl" as the substituent of the "aryl group" represented by Ar.

The "heterocyclic group" for the "heterocyclic group optionally having substituents" represented by R¹ includes, for example, a 5- to 14-membered (preferably 5- to 10-membered) (monocyclic to tricyclic, preferably, monocyclic or dicyclic) heterocyclic group containing 1 to 4 (preferably 1 to 3) hetero atoms of one or two kinds selected from the group consisting of a nitrogen atom, an oxygen atom and a sulfur atom in addition to carbon atoms etc. For example, it includes a 5-membered cyclic group containing, besides carbon atoms, 1 to 4 hetero atoms selected from the group consisting of an oxygen atom, a sulfur atom and a nitrogen atom, such as 2- or 3-thienyl, 2- or 3-furyl, 1-, 2- or 3-pyrrolyl, 1-, 2- or 3-pyrrolidinyl, 2-, 4- or 5-oxazolyl, 3-, 4- or 5-isoxazolyl, 2-, 4- or 5-thiazolyl, 3-, 4-or 5-isothiazolyl, 3-, 4- or 5-pyrazolyl, 2-, 3- or 4-pyrazolidinyl, 2-, 4- or 5-imidazolyl, 1,2,3-triazolyl, 1,2,4-triazolyl and 1H- or 2H-tetrazolyl, a 6-membered cyclic group containing, besides carbon atoms, 1 to 4 hetero atoms selected from the group consisting of an oxygen atom, a sulfur atom and a nitrogen atom, such as 2-, 3- or 4-pyridyl, 2-, 3- or 4-pyridyl N-oxide, 2-, 4- or 5-pyrimidinyl, 2-, 4- or 5-pyrimidinyl N-oxide, thiomorpholinyl, morpholinyl, piperidino, 2-, 3- or 4-piperidyl, thiopyranyl, 1,4-oxazinyl, 1,4-thiazinyl, 1,3-thiazinyl, piperazinyl, triazinyl, 3- or 4-pyridazinyl, pyrazinyl and 3- or 4-pyridazinyl N-oxide, a bicyclic or a tricyclic fused cyclic group containing, besides carbon atoms, 1 to 4 hetero atoms selected from the group consisting of an oxygen atom, a sulfur atom and a nitrogen atom, such as indolyl, benzofuryl, benzothiazolyl, benzoxazolyl, benzimidazolyl, quinolyl, isoquinolyl, phthalazinyl, quinazolinyl, quinoxalinyl, indolizinyl, quinolizinyl, 1,8-naphthyridinyl, dibenzofuranyl, carbazolyl, acridinyl, phenanthridinyl, chromanyl, phenothiazinyl and phenoxazinyl (preferably, a group formed by the fusion of the above-mentioned 5- or 6-membered ring to one or two of 5- or 6-membered cyclic groups optionally containing, besides carbon atoms, 1 to 4 hetero atoms selected from the group consisting of an oxygen atom, a sulfur atom and a nitrogen atom). Among these, a 5- to 7-membered (preferably 5- or 6-membered) heterocyclic group containing 1 to 3 hetero atoms selected from the group consisting of an oxygen atom, a sulfur atom and a nitrogen atom in addition to carbon atoms is preferred.

The substituent that the "heterocyclic group" for the "heterocyclic group optionally having substituents" may have includes the same group as those referred to herein for the aforementioned substituent that the "hydrocarbon group" for the "hydrocarbon group optionally having substituents" may have, for example, (1) halogen atom (e.g., fluorine, chlorine, bromine, iodine etc.), (2) a lower alkyl group (e.g., a C₁₋₆ alkyl group such as methyl, ethyl, propyl, isopropyl, butyl, isobutyl, sec-butyl, tert-butyl, pentyl, hexyl etc.), (3) a cycloalkyl group (e.g., a C₃₋₆ cycloalkyl group such as cyclopropyl, cyclobutyl, cyclopentyl, cyclohexyl, etc.), (4) a lower alkynyl group (e.g., a C₂₋₆ alkynyl group such as ethynyl, 1-propynyl, propargyl etc.), (5) a lower alkenyl group (e.g., a C₂₋₆ alkenyl group such as ethenyl (vinyl), allyl, isopropenyl, butenyl, isobutenyl etc.), (6) an aralkyl group (e.g., a C₇₋₁₁ aralkyl group such as benzyl, α-methylbenzyl, phenethyl, etc.), (7) an aryl group (e.g., a C₆₋₁₀ aryl group such as phenyl, naphthyl, etc., preferably a phenyl group etc.), (8) a lower alkoxy group (e.g., a C₁₋₆ alkoxy group such as methoxy, ethoxy, propoxy, isopropoxy, butoxy, isobutoxy, sec-butoxy, tert-butoxy etc.), (9) an aryloxy group (e.g., a C₆₋₁₀ aryloxy group such as phenoxy etc.), (10) an acyl (e.g., a formyl group, a lower alkyl-carbonyl group (e.g., a C₁₋₆ alkyl-carbonyl group such as acetyl, propionyl, butyryl, isobutyryl etc.), an arylcarbonyl (e.g., a C₆₋₁₄ aryl-carbonyl group such as benzoyl group, naphthoyl group etc.), a carbamoyl group, a sulfo group, a sulfino group, a phosphono group, a sulfamoyl group, a lower alkylsulfinyl group (e.g., a C₁₋₆ alkylsulfinyl group such as methylsulfinyl, ethylsulfinyl, propylsulfinyl, butylsulfinyl etc.), an arylsulfinyl group (e.g., a C₆₋₁₄ arylsulfinyl group such as phenylsulfinyl, naphthylsulfinyl etc.), a lower alkylsulfonyl group (e.g., a C₁₋₆ alkylsulfonyl group such as methylsulfonyl, ethylsulfonyl, propylsulfonyl, butylsulfonyl etc.), an arylsulfonyl group (e.g., a C₆₋₁₄ arylsulfonyl group such as phenylsulfonyl, naphthylsulfonyl etc.), a monoalkylsulfamoyl group (e.g., a mono-C₁₋₆ alkylsulfamoyl group such as N-methylsulfamoyl, N-ethylsulfamoyl, N-propylsulfamoyl, N-isopropylsulfamoyl, N-butylsulfamoyl etc.), a dialkylsulfamoyl group (e.g., a di-C₁₋₆ alkylsulfamoyl group such as N,N-dimethylsulfamoyl, N,N-diethylsulfamoyl, N,N-dipropylsulfamoyl, N,N-dibutylsulfamoyl etc.) etc.), (11) a carboxyl group, (12) acyloxy (e.g., formyloxy, a lower alkyl-carbonyloxy group (e.g., a C₁₋₆ alkyl-carbonyloxy group such as acetyloxy, propionyloxy, butyryloxy, isobutyryloxy etc.), an arylcarbonyloxy group (e.g., a C₆₋₁₄ aryl-carbonyloxy group such as benzoyloxy, naphthoyloxy etc.), a lower alkoxycarbonyl group (e.g., a C₁₋₆ alkoxy-carbonyl group such as methoxycarbonyl, ethoxycarbonyl, propoxycarbonyl, isopropoxycarbonyl, butoxycarbonyl, isobutoxycarbonyl, tert-butoxycarbonyl etc.), an aralkyloxycarbonyl (e.g., a C₇₋₁₅ aralkyloxycarbonyl group such as benzyloxycarbonyl etc.), (13) a mono-, di- or tri-halogeno-lower alkyl group (e.g., a mono-, di-or tri-halogeno-C₁₋₆ alkyl group such as chloromethyl, dichloromethyl, trifluoromethyl, 2,2,2-trifluoroethyl etc.), (14) an oxo group, (15) an amidino group, (16) an imino group, (17) an amino group, (18) a mono-lower alkylamino group (e.g., a mono-C₁₋₆ alkylamino group such as methylamino, ethylamino, propylamino, isopropylamino, butylamino etc.), (19) a di-lower alkylamino group (e.g., a di-C₁₋₄ alkylamino group such as dimethylamino, diethylamino, dipropylamino, diisopropylamino, dibutylamino, methylethylamino etc.), (20) an acylamino, (21) a 3- to 6-membered cyclic amino group optionally containing, besides carbon atoms and one nitrogen atom, 1 to 3 hetero atoms selected from the group consisting of an oxygen atom, a sulfur atom and a nitrogen atom (e.g., a 3- to 6-membered cyclic amino group such as aziridinyl, azetidinyl, pyrrolidinyl, pyrrolinyl, pyrrolyl, imidazolyl, pyrazolyl, imidazolidinyl, piperidyl, morpholinyl, dihydropyridyl, pyridyl, N-methylpiperazinyl, N-ethylpiperazinyl etc.), (22) an alkylenedioxy group (e.g., a C₁₋₃ alkylenedioxy group such as methylenedioxy, ethylenedioxy etc.), (23) a hydroxy group, (24) a nitro group, (25) a cyano group, (26) a mercapto group, (27) an alkylthio group (e.g., a C₁₋₆ alkylthio group such as methylthio, ethylthio, propylthio, isopropylthio, butylthio, sec-butylthio, tert-butylthio etc.), (28) an arylthio group (e.g., a C₆₋₁₄ arylthio group such as phenylthio, naphthylthio etc.), (29) a group obtained by combining 1 to 3 groups of the above-mentioned (1)-(28) and the like. Here, the "acyl", "acyloxy" and "acylamino" include the same group as those referred to herein for the above-mentioned "acyl", "acyloxy" and "acylamino", which are substituents that the "hydrocarbon group" in the "hydrocarbon group optionally having substituents" may have.

The "heterocyclic group" for the "heterocyclic group optionally having substituents" may have 1 to 5, preferably 1 to 3 of the above-mentioned substituents at the substitutable positions of the heterocyclic group. If the number of the substituents is 2 or more, the substituents may be the same or different.

R¹ is preferably a hydrogen atom, a C₆₋₁₄ aryl-C₁₋₆ alkyl group or an acyl group. The acyl group is preferably a group represented by the aforementioned -(C=O)-R³ (R³ is a hydrogen atom, a hydrocarbon group optionally having substituents, an amino group optionally having substituents, a hydroxy group optionally having a substituent or a heterocyclic group optionally having substituents).

R³ is preferably a hydrogen atom, a hydrocarbon group optionally having substituents or a heterocyclic group optionally having substituents.

The "hydrocarbon group" for the "hydrocarbon group optionally having substituents" represented by R³ is preferably includes a C₁₋₆ alkyl group (e.g., a methyl group, an ethyl group, a propyl group, an isopropyl group, a butyl group), a C₂₋₆ alkenyl group (e.g., an ethenyl (vinyl) group), a C₃₋₇ cycloalkyl group (e.g., a cyclohexyl group, a cyclopropyl group), a C₆₋₁₄ aryl group (e.g., a phenyl group) and the like.

The "heterocyclic group" for the "heterocyclic group optionally having substituents" represented by R³ is preferably, for example, a 5- or 6-membered non-aromatic heterocyclic group containing, besides carbon atoms, 1 or 2 nitrogen atoms, and optionally having one or two oxo groups, particularly preferably a 1-piperidyl group, a 4-piperidyl group, a 1,4-piperazinyl group.

When the C₃₋₇ cycloalkyl group represented by R³ is a cyclohexyl group, or when R³ is a 1-piperidyl group, the substituent for these groups is preferably bonded to the para-position. Preferable examples of such a substituent specifically include, substituents which bond via a nitrogen atom, such as
(i) an amino group,
(ii) a formylamino group,
(iii) a C₁₋₆ alkyl-carbonylamino group (e.g., acetylamino, ethylcarbonylamino, propylcarbonylamino, isopropylcarbonylamino, n-butylcarbonylamino) optionally substituted by substituents selected from the group consisting of C₁₋₆ alkyl (e.g., methyl), C₁₋₆ alkoxy (e.g., methoxy), hydroxy, a halogen atom (e.g., chlorine atom), formylamino, C₁₋₆ alkyl-carbonylamino (e.g., acetylamino) and a heterocyclic group optionally having one or two C₁₋₆ alkyl groups (e.g., methyl) and one or two oxo groups (e.g., 5- or 6-membered non-aromatic or aromatic heterocyclic group containing, besides carbon atoms, 1 to 4 of one or two kinds of hetero atoms selected from the group consisting of a nitrogen atom, an oxygen atom and a sulfur atom (e.g., piperidino, imidazolyl, triazolyl, tetrazolyl, oxazolyl), which optionally has one or two oxo groups),
(iv) a C₃₋₇ cycloalkyl-carbonylamino group (e.g., cyclopropylcarbonylamino),
(v) a C₁₋₆ alkoxy-carbonylamino group (e.g., methoxycarbonylamino, ethoxycarbonylamino, tert-butoxycarbonylamino),
(vi) an aromatic heterocycle-carbonylamino group (e.g., a 5- or 6-membered aromatic heterocycle containing, besides carbon atoms, 1 to 4 of one or two kinds of hetero atoms selected from the group consisting of a nitrogen atom, an oxygen atom and a sulfur atom (e.g., thienyl, furyl, pyrrolyl)-carbonylamino group),
(vii) an ureido group optionally substituted by one or two C₁₋₆ alkyl (e.g., methyl, ethyl, propyl, isopropyl),
(viii) a C₁₋₆ alkylsulfonylamino group (e.g., methylsulfonylamino, ethylsulfonylamino),
(ix) a heterocyclic group optionally having one or two oxo groups (e.g., a 5- or 6-membered non-aromatic or aromatic heterocyclic group containing, besides carbon atoms, 1 to 4 hetero atoms selected from the group consisting of a nitrogen atom, an oxygen atom and a sulfur atom, and optionally having one or two oxo groups (e.g., pyrrolidinyl, morpholino, isothiazolyl, oxazolyl, piperidino, pyrrolyl, oxazinyl)), and the like, and
   substituents which bond via a carbon atom, such as
(x) a C₁₋₆ alkoxy-carbonyl group (e.g., methoxycarbonyl),
(xi) a carbamoyl group,
(xii) a mono or di-C₁₋₆ alkyl-carbamoyl group (e.g., methylcarbamoyl, ethylcarbamoyl, dimethylcarbamoyl),
(xiii) mono or di-C₁₋₆ alkoxy-carbamoyl (e.g., methoxycarbamoyl, ethoxycarbamoyl),
(xiv) a heterocycle-carbonyl group (e.g., a 5- or 6-membered non-aromatic or aromatic heterocyclic group containing, besides carbon atoms, 1 to 4 hetero atoms selected from the group consisting of a nitrogen atom, an oxygen atom and a sulfur atom (e.g., pyrrolidinyl, piperidino)-carbonyl group), and the like.

When R³ is a 4-piperidyl group or a 1,4-piperazinyl group, the substituent for the group is preferably bonded to the nitrogen atom at the 1-position. Preferable examples of the substituent specifically include,
(i) a hydrogen atom,
   substituents which bond via a carbon atom, such as
(ii) C₁₋₆ alkyl (e.g., methyl, ethyl, propyl, isopropyl),
(iii) a C₁₋₆ alkyl group (e.g., methyl) optionally substituted by substituents selected from the group consisting of formyl, C₁₋₆ alkyl-carbonyl (e.g., acetyl), C₁₋₆ alkoxy-carbonyl (e.g., methoxycarbonyl, isopropoxycarbonyl), carbamoyl and mono or di-C₁₋₆ alkyl-carbamoyl (e.g., dimethylcarbamoyl),
(iv) a formyl group,
(v) a C₁₋₆ alkyl-carbonyl group (e.g., acetyl) optionally substituted by substituents selected from the group consisting of formylamino, C₁₋₆ alkyl-carbonylamino (e.g., acetylamino), and a heterocyclic group optionally having one or two oxo groups (e.g., a 5- or 6-membered non-aromatic or aromatic heterocyclic group containing, besides carbon atoms, 1 to 4 of one or two kinds of hetero atoms selected from the group consisting of a nitrogen atom, an oxygen atom and a sulfur atom (e.g., tetrazolyl, triazolyl, pyrazolyl, imidazolyl, pyrrolyl), which optionally has one or two oxo groups),
(vi) a C₁₋₆ alkoxy-carbonyl group (e.g., methylcarbonyl),
(vii) a mono- or di-C₁₋₆ alkyl-carbamoyl group (e.g., methylcarbamoyl, ethylcarbamoyl, dimethylcarbamoyl), and
(viii) a heterocycle-carbonyl group optionally having one or two oxo groups (e.g., a 5- or 6-membered aromatic or non-aromatic heterocycle (e.g., imidazolyl)-carbonyl group containing, besides carbon atoms, 1 to 4 of one or two kinds of hetero atoms selected from the group consisting of a nitrogen atom, an oxygen atom and a sulfur atom, which optionally has one or two oxo groups), and the like,
(ix) a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups (e.g., dihydrofuryl (e.g., dihydrofuran-2(3H)-one), pyrrolidinyl, piperidino)), and
(x) a C₁₋₆ alkylsulfonyl group (e.g., methylsulfonyl), and the like.

The substituent for the C₁₋₆ alkyl group (e.g., a methyl group, an ethyl group, a propyl group, an isopropyl group, a butyl group) represented by R³ includes, for example, the same group as those referred to herein for the aforementioned substituent that the "hydrocarbon group" for the "hydrocarbon group optionally having substituents" represented by R¹ may have. Preferable examples specifically include
(i) formylamino,
(ii) a C₁₋₆ alkyl-carbonylamino (e.g., acetylamino, ethylcarbonylamino, tert-butylcarbonylamino) optionally substituted by halogen atoms (e.g., chlorine atom),
(iii) a heterocyclic group optionally having one or two oxo groups (e.g., a 5- or 6-membered aromatic or non-aromatic heterocycle containing, besides carbon atoms, 1 to 4 hetero atoms selected from the group consisting of a nitrogen atom, an oxygen atom and a sulfur atom, which optionally has one or two oxo groups) (e.g., 1,4-piperazinyl, piperazinone, pyrrolidinyl, 1-piperidinyl, imidazolyl, tetrazolyl, triazolyl, oxadiazolyl, thiazolyl, pyridyl, furyl), optionally having substituents selected from the group consisting of C₁₋₆ alkyl (e.g., methyl, ethyl, isopropyl), hydroxy, formyl, C₁₋₆ alkyl-carbonyl (e.g., acetyl), formylamino, C₁₋₆ alkyl-carbonylamino (e.g., acetylamino) and C₁₋₆ alkoxy-carbonyl (e.g., tert-butoxycarbonyl),
(iv) hydroxy,
(v) amino,
(vi) C₁₋₆ alkoxy-carbonylamino (e.g., tert-butoxycarbonylamino),
(vii) C₁₋₆ alkylsulfonylamino (e.g., methylsulfonylamino),
(viii) mono- or di-C₁₋₆ alkylureido (e.g., isopropylureido, dimethylureido),
(ix) C₁₋₆ alkoxy-carbonyl (e.g., methoxycarbonyl),
(x) carboxy,
(xi) carbamoyl,
(xii) mono- or di-C₁₋₆ alkyl-carbamoyl (e.g., methylcarbamoyl, dimethylcarbamoyl),
(xiii) heterocycle-carbonyl optionally having one or two oxo groups (e.g., a 5- or 6-membered aromatic or non-aromatic heterocycle (e.g., pyrrolidinyl)-carbonyl, wherein the heterocycle contains, besides carbon atoms, 1 to 4 hetero atoms selected from the group consisting of a nitrogen atom, an oxygen atom and a sulfur atom, which optionally has one or two oxo groups),
(xiv) cyano, and the like.

The substituent for the C₂₋₆ alkenyl group (e.g., an ethenyl(vinyl) group) represented by R³ includes, for example, the same group as those referred to herein for the aforementioned substituent that the "hydrocarbon group" for the "hydrocarbon group optionally having substituents" represented by R¹ may have and the like, particularly, heterocycle (e.g., a 5- or 6-membered aromatic or non-aromatic heterocycle containing, besides carbon atoms, 1 to 4 hetero atoms selected from the group consisting of a nitrogen atom, an oxygen atom and a sulfur atom (e.g., pyridyl, thienyl, imidazolyl, pyrrolyl, thiazolyl)) optionally having a C₁₋₆ alkyl group (e.g., methyl, ethyl), and the like.

The substituent for the C₆₋₁₄ aryl group (e.g., a phenyl group) represented by R³ includes, for example, the same group as those referred to herein for the aforementioned substituent that the "hydrocarbon group" for the "hydrocarbon group optionally having substituents" represented by R¹ may have, and a formylamino group, a C₁₋₆ alkyl-carbonylamino group (e.g., acetylamino) and the like are particularly preferable.

Of these, R¹ is preferably
(1) a hydrogen atom,
(2) an acyl group represented by the formula: -(C=O)-Ra or -(C=O) NRaRa'
   wherein Ra is
   (I) a hydrogen atom, or
   (II) (a) a hydrocarbon group (e.g., a C₁₋₆ alkyl group (e.g., a methyl group), a C₃₋₆ cycloalkyl group (e.g., a cyclohexyl group, a cyclopropyl group), a C₂₋₆ alkenyl group (e.g., an ethenyl (vinyl) group) or a C₆₋₁₄ aryl group (e.g., a phenyl group)), or
      (b) a non-aromatic heterocyclic group optionally having one or two oxo groups (e.g., a 4- to 6-membered non-aromatic heterocyclic group containing, besides carbon atoms, 1 to 4 hetero atoms selected from the group consisting of a nitrogen atom, an oxygen atom and a sulfur atom (e.g., 1-piperidyl, 4-piperidyl, 1,4-piperazinyl) which optionally has one or two oxo groups),
      each of which optionally having 1 or 2 substituents selected from the group consisting of
      (i) a halogen atom,
      (ii) a formyl group,
      (iii) a C₁₋₆ alkyl group optionally substituted by substituents selected from the group consisting of formyl, C₁₋₆ alkyl-carbonyl (e.g., acetyl), C₁₋₆ alkoxy-carbonyl (e.g., methoxycarbonyl, isopropoxycarbonyl), carbamoyl and mono or di-C₁₋₆ alkyl-carbamoyl (e.g., dimethylcarbamoyl),
      (iv) an amino group,
      (v) a formylamino group,
      (vi) a C₁₋₆ alkyl-carbonylamino group (e.g., acetylamino, ethylcarbonylamino, propylcarbonylamino, isopropylcarbonylamino, tert-butylcarbonylamino) optionally substituted by substituents selected from the group consisting of (i') C₁₋₆ alkyl (e.g., methyl), (ii') hydroxy, (iii') C₁₋₆ alkoxy (e.g., methoxy), (iv') halogen atom (e.g., chlorine atom), (v') formylamino, (vi') C₁₋₆ alkyl-carbonylamino (e.g., acetylamino) and (vii') a heterocyclic group optionally having one or two oxo groups (e.g., a 5- or 6-membered a non-aromatic or aromatic heterocyclic group containing, besides carbon atoms, 1 to 4 of one or two kinds of hetero atoms selected from the group consisting of a nitrogen atom, an oxygen atom and a sulfur atom (e.g., piperidino, imidazolyl, triazolyl, tetrazolyl, oxazolyl), which optionally has one or two oxo groups), optionally having one or two C₁₋₆ alkyl groups (e.g., methyl),
      (vii) a C₃₋₇ cycloalkyl-carbonylamino group (e.g., cyclopropylcarbonylamino),
      (viii) a C₁₋₆ alkoxy-carbonylamino group (e.g., methoxycarbonylamino, ethoxycarbonylamino, tert-butoxycarbonylamino),
      (ix) an aromatic heterocycle-carbonylamino group (e.g., a 5- or 6-membered aromatic heterocycle (e.g., thienyl, furyl, pyrrolyl)-carbonyl-amino group, wherein the heterocycle contains, besides carbon atoms, 1 to 4 of one or two kinds of hetero atoms selected from the group consisting of a nitrogen atom, an oxygen atom and a sulfur atom,
      (x) an ureido group optionally substituted by one or two C₁₋₆ alkyl groups (e.g., methyl, ethyl, propyl, isopropyl),
      (xi) a C₁₋₆ alkoxy-carbonyl group (e.g., methoxycarbonyl, ethoxycarbonyl),
      (xii) a C₁₋₆ alkyl-carbonyl group (e.g., acetyl, ethylcarbonyl) optionally substituted by substituents selected from the group consisting of hydroxy, formylamino, C₁₋₆ alkyl-carbonylamino (e.g., acetylamino) and an aromatic heterocyclic group optionally having one or two oxo groups (e.g., a 5- or 6-membered aromatic heterocyclic group containing, besides carbon atoms, 1 to 4 of one or two kinds of hetero atoms selected from the group consisting of a nitrogen atom, an oxygen atom and a sulfur atom (e.g., triazolyl, tetrazolyl, pyrazolyl, imidazolyl, pyrrolyl), which optionally has one or two oxo groups),
      (xiii) a heterocycle-carbonyl group optionally having one or two oxo groups (e.g., a 5- or 6-membered aromatic or non-aromatic heterocycle (e.g., imidazolyl, pyrrolidinyl, piperidino)-carbonyl group, wherein the heterocycle contains, besides carbon atoms, 1 to 4 of one or two kinds of hetero atoms selected from the group consisting of a nitrogen atom, an oxygen atom and a sulfur atom, which optionally has one or two oxo groups),
      (xiv) a hydroxy group,
      (xv) a carbamoyl group optionally having C₁₋₆ alkoxy groups (e.g., methoxy, ethoxy),
      (xvi) a mono- or di-C₁₋₆ alkyl-carbamoyl group (e.g., methylcarbamoyl, ethylcarbamoyl, dimethylcarbamoyl),
      (xvii) a C₁₋₆ alkylsulfonylamino group (e.g., methylsulfonylamino, ethylsulfonylamino),
      (xviii) one or two oxo groups,
      (xix) a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., triazolyl, tetrazolyl, imidazolyl, thiazolyl, isothiazolidinyl, oxazolidinyl, dihydrofuryl, pyrrolyl, piperidino, morpholino, pyrrolidinyl, piperazinyl, pyridyl, thienyl, furyl, oxazinyl, oxadiazolyl), which optionally has one or two oxo groups), optionally having substituents selected from the group consisting of (i') a hydroxy group, (ii') a C₁₋₆ alkyl group (e.g., a methyl group, an ethyl group, an isopropyl group), (iii') a formyl group, (iv') a C₁₋₆ alkyl-carbonyl group (e.g., acetyl), (v') a formylamino group, (vi') a C₁₋₆ alkyl-carbonylamino group (e.g., acetylamino) and (vii') a C₁₋₆ alkoxy-carbonyl group (e.g., tert-butoxycarbonyl),
      (xx) a C₁₋₆ alkylsulfonyl group (e.g., methylsulfonyl),
      (xxi) a cyano group, and
      (xxii) a carboxy group, and
         Ra' is a hydrogen atom or a C₁₋₆ alkyl group, or
(3) a C₆₋₁₄ aryl-C₁₋₆ alkyl group (e.g., a benzyl group).

Furthermore, examples of the preferable R¹ include
(1) a hydrogen atom,
(2) a formyl group,
(3) a group represented by the formula -(C=O)-NRb¹Rb²
   wherein Rb¹ and Rb² are the same or different and each is a hydrogen atom or a C₁₋₆ alkyl group (e.g., methyl, ethyl),
(4) a group represented by the formula -(C=O)-(CH₂)m-Rb³
   wherein m is an integer of 1 to 3, Rb³ is a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., triazolyl, tetrazolyl, imidazolyl, dihydrofuryl, pyrrolyl, pyrrolidinyl, piperidino, piperazinyl, pyridyl, thienyl, furyl, thiazolyl, oxadiazolyl), preferably an aromatic heterocyclic group (e.g., triazolyl, tetrazolyl, pyrrolyl,-pyridyl, thienyl, furyl, thiazolyl, oxadiazolyl), which optionally has one or two oxo groups), optionally substituted by substituents selected from the group consisting of C₁₋₆ alkyl (e.g., methyl, ethyl), formyl, C₁₋₆ alkyl-carbonyl (e.g., acetyl, ethylcarbonyl), hydroxy, C₁₋₆ alkoxy-carbonyl (e.g., methoxycarbonyl, ethoxycarbonyl, tert-butoxycarbonyl), formylamino and C₁₋₆ alkyl-carbonylamino (e.g., acetylamino, ethylcarbonylamino),
(5) a group represented by the formula -(C=O)-(CH₂)n-NRb⁴Rb⁵
   wherein n is an integer of 1 to 4, Rb⁴ and Rb⁵ are the same and different and each is (a) a hydrogen atom, (b) a formyl group, (c) a C₁₋₆ alkyl-carbonyl group (e.g., acetyl, ethylcarbonyl, propylcarbonyl, isopropylcarbonyl, tert-butylcarbonyl) optionally having halogen atoms (e.g., chlorine atom), (d) a C₁₋₆ alkoxy-carbonyl group (e.g., methoxycarbonyl, ethoxycarbonyl, tert-butoxycarbonyl), (e) a C₁₋₆ alkylsulfonyl group (e.g., methylsulfonyl, ethylsulfonyl) or (f) a mono- or di-C₁₋₆ alkyl-carbamoyl group (e.g., methylcarbamoyl, ethylcarbamoyl, isopropylcarbamoyl, dimethylcarbamoyl, diethylcarbamoyl),
(6) a group represented by the formula: 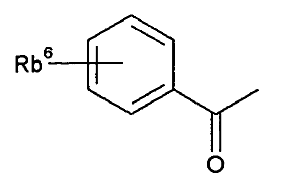 wherein Rb⁶ is a formylamino group or a C₁₋₆ alkyl-carbonylamino group (e.g., acetylamino),
(7) a group represented by the formula: 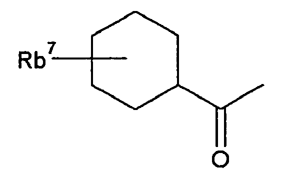 wherein Rb⁷ is
   (i) an amino group,
   (ii) a formylamino group,
   (iii) a C₁₋₆ alkyl-carbonylamino group (e.g., acetylamino, ethylcarbonylamino, propylcarbonylamino, isopropylcarbonylamino) optionally substituted by substituents selected from the group consisting of (a) C₁₋₆ alkyl (e.g., methyl, ethyl), (b) hydroxy, (c) C₁₋₆ alkoxy (e.g., methoxy), (d) a halogen atom (e.g., chlorine atom), (e) formylamino, (f) C₁₋₆ alkyl-carbonylamino (e.g., acetylamino) and (g) a heterocyclic group optionally having one or two oxo groups (e.g., a 5- or 6-membered non-aromatic or aromatic heterocyclic group containing, besides carbon atoms, 1 to 4 of one or two kinds of hetero atoms selected from the group consisting of a nitrogen atom, an oxygen atom and a sulfur atom (e.g., piperidino, imidazolyl, triazolyl, tetrazolyl, oxazolyl), which optionally has one or two oxo groups), optionally having one or two C₁₋₆ alkyl groups (e.g., methyl, ethyl),
   (iv) a C₃₋₇ cycloalkyl-carbonylamino group (e.g., cyclopropylcarbonylamino),
   (v) a C₁₋₆ alkoxy-carbonylamino group (e.g., methoxycarbonylamino, ethoxycarbonylamino, tert-butoxycarbonylamino),
   (vi) a heterocycle-carbonylamino group (e.g., a 5- or 6-membered aromatic heterocycle (e.g., thienyl, furyl, pyrrolyl)-carbonylamino group, wherein the heterocycle contains, besides carbon atoms, 1 to 4 of one or two kinds of hetero atoms selected from the group consisting of a nitrogen atom, an oxygen atom and a sulfur atom,
   (vii) an ureido group optionally substituted by one or two C₁₋₆ alkyl groups (e.g., methyl, ethyl, propyl, isopropyl),
   (viii) a C₁₋₆ alkoxy-carbonyl group (e.g., methoxycarbonyl, ethoxycarbonyl),
   (ix) a carbamoyl group optionally having C₁₋₆ alkoxy groups (e.g., methoxy, ethoxy),
   (x) a mono- or di-C₁₋₆ alkyl-carbamoyl group (e.g., methylcarbamoyl, ethylcarbamoyl, dimethylcarbamoyl),
   (xi) a C₁₋₆ alkylsulfonylamino group (e.g., methylsulfonylamino, ethylsulfonylamino),
   (xii) a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., triazolyl, tetrazolyl, imidazolyl, dihydrofuryl, pyrrolyl, pyrrolidinyl, piperidino), which optionally has one or two oxo groups), or
   (xiii) a heterocycle-carbonyl group (e.g., a 5- or 6-membered aromatic or non-aromatic heterocycle (e.g., pyrrolidinyl, piperidino)-carbonyl group, wherein the heterocycle contains besides carbon atoms, 1 to 4 of one or two kinds of hetero atoms selected from the group consisting of a nitrogen atom, an oxygen atom and a sulfur atom),
(8) a group represented by the formula: 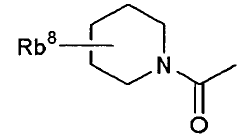 wherein Rb⁸ is (i) a formylamino group, (ii) an alkyl-carbonylamino group (e.g., acetylamino, ethylcarbonylamino) optionally substituted by hydroxy, (iii) a C₁₋₆ alkylsulfonylamino group (e.g., methylsulfonylamino, ethylsulfonylamino) or (iv) a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., pyrrolidinyl, piperidino, morpholino, isothiazolinyl, oxazolinyl, oxazinyl), which optionally has one or two oxo groups),
(9) a group represented by the formula: 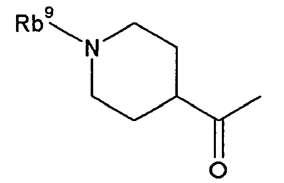 wherein Rb⁹ is
   (i) a hydrogen atom,
   (ii) a C₁₋₆ alkyl group (e.g., methyl) optionally substituted by substituents selected from the group consisting of formyl, C₁₋₆ alkyl-carbonyl (e.g., acetyl), C₁₋₆ alkoxy-carbonyl (e.g., methoxycarbonyl, isopropoxycarbonyl) and mono or di-C₁₋₆ alkyl-carbamoyl (e.g., dimethylcarbamoyl),
   (iii) a formyl group,
   (iv) a C₁₋₆ alkyl-carbonyl group (e.g., acetyl) optionally substituted by substituents selected from the group consisting of (a) formylamino, (b) C₁₋₆ alkyl-carbonylamino (e.g., acetylamino) and (c) a heterocyclic group optionally having one or two oxo groups (e.g., a 5- or 6-membered aromatic heterocyclic group containing, besides carbon atoms, 1 to 4 of one or two kinds of hetero atoms selected from the group consisting of a nitrogen atom, an oxygen atom and a sulfur atom (e.g., triazolyl, tetrazolyl, imidazolyl, pyrazolyl and the like), which optionally has one or two oxo groups),
   (v) a heterocycle-carbonyl group optionally having one or two oxo groups (e.g., a 5- or 6-membered aromatic or non-aromatic heterocycle (e.g., imidazolyl)-carbonyl group, wherein the heterocycle contains besides carbon atoms, 1 to 4 of one or two kinds of hetero atoms selected from the group consisting of a nitrogen atom, an oxygen atom and a sulfur atom, and optionally has one or two oxo groups),
   (vi) a heterocyclic group optionally having one or two oxo (e.g., a 5- or 6-membered aromatic or non-aromatic heterocyclic group containing, besides carbon atom, 1 to 4 of one or two kinds of hetero atoms selected from the group consisting of a nitrogen atom, an oxygen atom and a sulfur atom (e.g., dihydrofuryl), which optionally has one or two oxo groups),
   (vii) a formylamino group,
   (viii) a C₁₋₆ alkyl-carbonylamino group (e.g., acetylamino),
   (ix) a C₁₋₆ alkylsulfonyl group (e.g., methylsulfonyl, ethylsulfonyl),
   (x) a C₁₋₆ alkoxy-carbonyl group (e.g., methoxycarbonyl, ethoxycarbonyl), or
   (xi) a mono- or di-C₁₋₆ alkyl-carbamoyl group (e.g., methylcarbamoyl, ethylcarbamoyl, dimethylcarbamoyl, diethylcarbamoyl),
(10) a group represented by the formula: 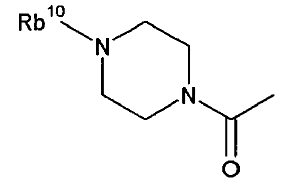 wherein Rb¹⁰ is (i) a formyl group, (ii) a C₁₋₆ alkyl-carbonyl group (e.g., acetyl) or (iii) a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., pyrrolidinyl, piperidino), which optionally has one or two oxo groups),
(11) a group represented by the formula: 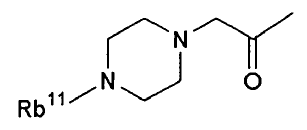 wherein Rb¹¹ is a C₁₋₆ alkyl group (e.g., isopropyl), a formyl group or a C₁₋₆ alkyl-carbonyl group (e.g., acetyl),
(12) a group represented by the formula: 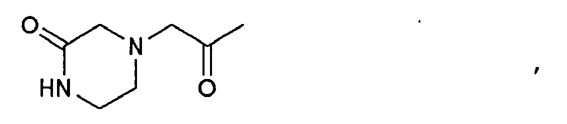
(13) a group represented by the formula: 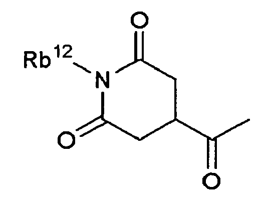 wherein Rb¹² is a hydrogen atom or a C₁₋₆ alkyl group (e.g., methyl),
(14) a group represented by the formula: 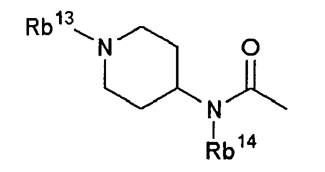 wherein Rb¹³ is
   (i) a hydrogen atom,
   (ii) a formyl group,
   (iii) a C₁₋₆ alkyl-carbonyl group (e.g., acetyl, ethylcarbonyl) optionally substituted by hydroxyl groups,
   (iv) a C₁₋₆ alkoxy-carbonyl group (e.g., methoxycarbonyl, ethoxycarbonyl), or
   (v) a C₁₋₆ alkylsulfonyl group (e.g., methylsulfonyl, ethylsulfonyl), and Rb¹⁴ is a hydrogen atom or a C₁₋₆ alkyl group (e.g., methyl),
(15) a group represented by the formula: 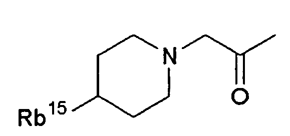 wherein Rb¹⁵ is a hydroxy group, a formylamino group or a C₁₋₆ alkyl-carbonylamino group (e.g., acetylamino),
(16) a group represented by the formula: 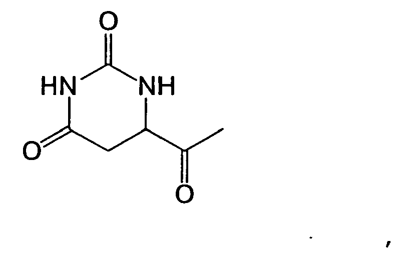
(17) a group represented by the formula: 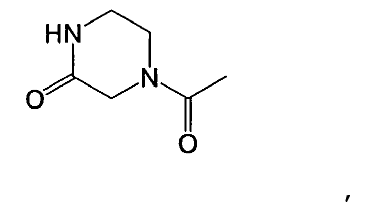
(18) a group represented by the formula: 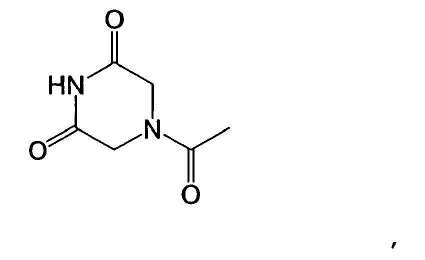
(19) a C₁₋₆ alkyl-carbonyl group optionally substituted by substituents selected from the group consisting of (i) cyano, (ii) hydroxy, (iii) carboxy, (iv) C₁₋₆ alkoxy-carbonyl (e.g., methoxycarbonyl, ethoxycarbonyl), (v) carbamoyl, (vi) C₁-₆ alkyl-carbamoyl (e.g., methylcarbamoyl, ethylcarbamoyl) and (vii) heterocycle-carbonyl (e.g., 5- or 6-membered aromatic or non-aromatic heterocycle (e.g., pyrrolidinyl)-carbonyl, wherein the heterocycle contains, besides carbon atoms, 1 to 4 of one or two kinds of hetero atoms selected from the group consisting of a nitrogen atom, an oxygen atom and a sulfur atom),
(20) C₃₋₆ cycloalkyl-carbonyl group (e.g., cyclopropylcarbonyl) optionally substituted by carbamoyl,
(21) a C₁₋₆ alkenyl-carbonyl group (e.g., ethenyl(vinyl)carbonyl) substituted by heterocycles (e.g., 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., pyridyl, thienyl, imidazolyl, pyrrolyl, thiazolyl) optionally substituted by C₁₋₆ alkyl groups (e.g., methyl, ethyl),
(22) a group represented by the formula -(C=O)-(CRb¹⁶Rb¹⁷)-NHRb¹⁸
   wherein Rb¹⁶ is a hydrogen atom or a C₁₋₆ alkyl group (e.g., methyl, ethyl), Rb¹⁷ is a C₁₋₆ alkyl group (e.g., methyl, ethyl) optionally substituted by substituents selected from the group consisting of a carbamoyl group and an aromatic heterocyclic group (e.g., a 5- or 6-membered aromatic heterocycle containing, besides carbon atoms, 1 to 4 of one or two kinds of hetero atoms selected from the group consisting of a nitrogen atom, an oxygen atom and a sulfur atom (e.g., imidazolyl)), and Rb¹⁸ is a hydrogen atom, a C₁₋₆ alkyl-carbonyl group (e.g., acetyl, ethylcarbonyl) or a C₁₋₆ alkoxy-carbonyl group (e.g., methoxycarbonyl, ethoxycarbonyl, tert-butoxycarbonyl), or
(23) a C₆₋₁₄ aryl-C₁₋₆ alkyl group (e.g., benzyl), and the like.
   m is preferably 1.
   Rb⁶-Rb⁸ are preferably substituted at the para-position.

### (Explanation of X)

X is an oxygen atom or an imino group optionally having a substituent in the above-mentioned formula (I).

The "substituent" for the "an imino group optionally having a substituent" represented by X is a hydrocarbon group optionally having substituents or an acyl group.

The "hydrocarbon group optionally having substituents" includes, for example, the same group as those referred to herein above for the foregoing "hydrocarbon group optionally having substituents" represented by R¹.

The "acyl group" includes, for example, the same group as those referred to herein above for the foregoing "acyl" as the substituent of the "aryl group" represented by Ar.

X is preferably an oxygen atom or NH, more preferably an oxygen atom.

### (Explanation of ring A)

In the above-mentioned formula (I), ring A is a piperidine ring optionally further having substituents. That is, ring A further optionally has 1 to 8 substituents besides R¹, X and Ar.

The "substituent" for the "piperidine ring optionally having substituents" includes those referred to herein for the substituent for "aryl group" represented by Ar.

Ring A is preferably a piperidine ring having no. substituent other than R¹, X and Ar.

### (Explanation of ring B)

Ring B is a benzene ring having substituents in the above-mentioned the formula.

The "substituent" in the "benzene ring having substituents" includes the same group as those referred to herein above for the above-mentioned substituent of the "aryl group" represented by Ar. The number of substituents is 1 to 5.

Ring B is preferably a benzene ring having substituents selected from the group consisting of (i) an optionally halogenated C₁₋₆ alkyl group, (ii) a halogen atom (e.g., fluorine atom), (iii) a C₁₋₆ alkoxy group (e.g., methoxy) and (iv) a 5- or 6-membered aromatic heterocyclic group containing, besides carbon atoms, 1 to 4 hetero atoms selected from the group consisting of a nitrogen atom, an oxygen atom and a sulfur atom, which is optionally substituted by an optionally halogenated C₁₋₆ alkyl group, and particularly preferably a benzene ring having substituents selected from the group consisting of an optionally halogenated C₁₋₆ alkyl group and a halogen atom. The number of the substituents on the benzene ring is 1 to 3, preferably 1 or 2, particularly preferably 2.

The "halogen atom" includes, for example, fluorine atom, chlorine atom, iodine atom, bromine atom and the like, preferably fluorine atom.

The "optionally halogenated C₁₋₆ alkyl group" in the "optionally halogenated C₁₋₆ alkyl group" or "5- or 6-membered aromatic heterocyclic group containing, besides carbon atoms, 1 to 4 hetero atoms selected from the group consisting of a nitrogen atom, an oxygen atom and a sulfur atom, which is optionally substituted by an optionally halogenated C₁₋₆ alkyl group" includes, for example, methyl group, ethyl group, propyl group, isopropyl group and the like, optionally having 1 to 3 halogen atoms such as fluorine atom, chlorine atom, iodine atom and the like, particularly fluorine atom, and particularly preferably trifluoromethyl group.

The "5- or 6-membered aromatic heterocyclic group containing, besides carbon atoms, 1 to 4 hetero atoms selected from the group consisting of a nitrogen atom, an oxygen atom and a sulfur atom" of the "5- or 6-membered aromatic heterocyclic group containing, besides carbon atoms, 1 to 4 hetero atoms selected from the group consisting of a nitrogen atom, an oxygen atom and a sulfur atom, which is optionally substituted by an optionally halogenated C₁₋₆ alkyl group" includes, for example, 2-or 3-thienyl, 2- or 3-furyl, 1-, 2- or 3-pyrrolyl, 1-, 2- or 3-pyrrolidinyl, 2-, 4- or 5-oxazolyl, 3-, 4- or 5-isoxazolyl, 2-, 4- or 5-thiazolyl, 3-, 4- or 5-isothiazolyl, 3-, 4- or 5-pyrazolyl, 2-, 3- or 4-pyrazolidinyl, 2-, 4- or 5-imidazolyl, 1,2,3-triazolyl, 1,2,4-triazolyl, 1H- or 2H-tetrazolyl, 2-, 3-or 4-pyridyl, 2-, 3- or 4-pyridyl N-oxide, 2-, 4- or 5-pyrimidinyl, 2-, 4- or 5-pyrimidinyl N-oxide, thiomorpholinyl, morpholinyl, piperidino, 2-, 3- or 4-piperidyl, thiopyranyl, 1,4-oxazinyl, 1,4-thiazinyl, 1,3-thiazinyl, piperazinyl, triazinyl, 3- or 4-pyridazinyl, pyrazinyl, 3- or 4-pyridazinyl N-oxide and the like, preferably a 5- or 6-membered aromatic nitrogen-containing heterocyclic group containing, besides carbon atoms, 1 to 4 nitrogen atoms, such as 1H- or 2H-tetrazolyl and the like.

When X is an oxygen atom, ring B is preferably a benzene ring having substituents selected from the group consisting of an optionally halogenated C₁₋₆ alkyl group and a halogen atom, and when X is NH, ring B is preferably a benzene ring having substituents selected from the group consisting of (i) C₁₋₆ alkoxy group (e.g., methoxy) and (ii) 5- or 6-membered aromatic heterocyclic group containing, besides carbon atoms, 1 to 4 hetero atoms selected from the group consisting of a nitrogen atom, an oxygen atom and a sulfur atom, which is optionally substituted by an optionally halogenated C₁₋₆ alkyl group.

Ring B is most preferably a benzene ring substituted by trifluoromethyl at the 3- and 5-positions.

As compound (I), a compound represented by the formula (I)
wherein Ar has an (R) configuration, and 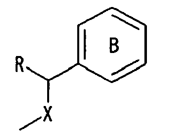 has an (S) configuration is preferable. That is, an optically active compound represented by the formula: 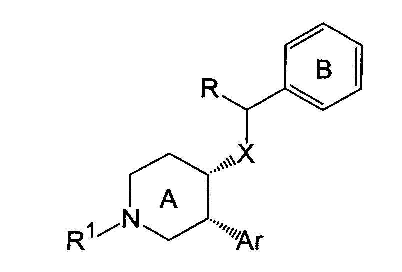 wherein each symbol is as defined above is preferable.

In this case, X is preferably an oxygen atom or NH.

In addition, compound (I) wherein R has an (R) configuration and X is an oxygen atom is also preferable.

Concretely, the following compound is preferable as compound (I).

A compound (I) wherein
Ar is a C₆₋₁₄ aryl group optionally having halogen atoms (e.g., fluorine atom),
R is a methyl group,
R¹ is
(1) a hydrogen atom,
(2) an acyl group represented by the formula: -(C=O)-Ra or - (C=O) NRaRa'
   wherein Ra is
   (I) a hydrogen atom, or
   (II) (a) a hydrocarbon group (e.g., a C₁₋₆ alkyl group (e.g., a methyl group), a C₃₋₆ cycloalkyl group (e.g., a cyclohexyl group, a cyclopropyl group), a C₂₋₆ alkenyl group (e.g., an ethenyl (vinyl) group) or a C₆₋₁₄ aryl group (e.g. , a phenyl group)), or
      (b) a non-aromatic heterocyclic group optionally having one or two oxo groups (e.g., a 4- to 6-membered non-aromatic heterocyclic group containing, besides carbon atoms, 1 to 4 of one or two kinds of hetero atoms selected from the group consisting of a nitrogen atom, an oxygen atom and a sulfur atom (e.g., 1-piperidyl, 4-piperidyl, 1,4-piperazinyl) which optionally has one or two oxo groups),
      each of which optionally having 1 or 2 substituents selected from the group consisting of
      (i) a halogen atom,
      (ii) a formyl group,
      (iii) a C₁₋₆ alkyl group optionally substituted by substituents selected from the group consisting of formyl, C₁₋₆ alkyl-carbonyl (e.g., acetyl), C₁₋₆ alkoxy-carbonyl (e.g., methoxycarbonyl, isopropoxycarbonyl), carbamoyl and mono- or di-C₁₋₆ alkyl-carbamoyl (e.g., dimethylcarbamoyl),
      (iv) an amino group,
      (v) a formylamino group,
      (vi) a C₁₋₆ alkyl-carbonylamino group (e.g., acetylamino, ethylcarbonylamino, propylcarbonylamino, isopropylcarbonylamino, tert-butylcarbonylamino) optionally substituted by substituents selected from the group consisting of (i') C₁₋₆ alkyl (e.g., methyl), (ii') hydroxy, (iii') C₁₋₆ alkoxy (e.g., methoxy), (iv') halogen atom (e.g., chlorine atom), (v') formylamino, (vi') C₁₋₆ alkyl-carbonylamino (e.g., acetylamino) and (vii') a heterocyclic group optionally having one or two oxo groups (e.g., a 5- or 6-membered a non-aromatic or aromatic heterocyclic group containing, besides carbon atoms, 1 to 4 of one or two kinds of hetero atoms selected from the group consisting of a nitrogen atom, an oxygen atom and a sulfur atom (e.g., piperidino, imidazolyl, triazolyl, tetrazolyl, oxazolyl), which optionally has one or two oxo groups), optionally having one or two C₁₋₆ alkyl groups (e.g., methyl),
      (vii) a C₃₋₇ cycloalkyl-carbonylamino group (e.g., cyclopropylcarbonylamino),
      (viii) a C₁₋₆ alkoxy-carbonylamino group (e.g., methoxycarbonylamino, ethoxycarbonylamino, tert-butoxycarbonylamino),
      (ix) an aromatic heterocycle-carbonylamino group (e.g., a 5- or 6-membered aromatic heterocycle (e.g., thienyl, furyl, pyrrolyl)-carbonyl-amino group, wherein the heterocycle contains, besides carbon atoms, 1 to 4 of one or two kinds of hetero atoms selected from the group consisting of a nitrogen atom, an oxygen atom and a sulfur atom),
      (x) an ureido group optionally substituted by one or two C₁₋₆ alkyl groups (e.g., methyl, ethyl, propyl, isopropyl),
      (xi) a C₁₋₆ alkoxy-carbonyl group (e.g., methoxycarbonyl, ethoxycarbonyl),
      (xii) a C₁₋₆ alkyl-carbonyl group (e.g., acetyl, ethylcarbonyl) optionally substituted by substituents selected from the group consisting of hydroxy, formylamino, C₁₋₆ alkyl-carbonylamino (e.g., acetylamino) and an aromatic heterocyclic group optionally having one or two oxo groups (e.g., a 5- or 6-membered aromatic heterocyclic group containing, besides carbon atoms, 1 to 4 of one or two kinds of hetero atoms selected from the group consisting of a nitrogen atom, an oxygen atom and a sulfur atom (e.g., triazolyl, tetrazolyl, pyrazolyl, imidazolyl, pyrrolyl), which optionally has one or two oxo groups),
      (xiii) a heterocycle-carbonyl group optionally having one or two oxo groups (e.g., a 5- or 6-membered aromatic or non-aromatic heterocycle (e.g., imidazolyl, pyrrolidinyl, piperidino)-carbonyl group, wherein the heterocycle contains, besides carbon atoms, 1 to 4 of one or two kinds of hetero atoms selected from the group consisting of a nitrogen atom, an oxygen atom and a sulfur atom, which optionally has one or two oxo groups),
      (xiv) a hydroxy group,
      (xv) a carbamoyl group optionally having C₁₋₆ alkoxy groups (e.g., methoxy, ethoxy),
      (xvi) a mono- or di-C₁₋₆ alkyl-carbamoyl group (e.g., methylcarbamoyl, ethylcarbamoyl, dimethylcarbamoyl),
      (xvii) a C₁₋₆ alkylsulfonylamino group (e.g., methylsulfonylamino, ethylsulfonylamino),
      (xviii) one or two oxo groups,
      (xix) a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., triazolyl, tetrazolyl, imidazolyl, thiazolyl, isothiazolidinyl, oxazolidinyl, dihydrofuryl, pyrrolyl, piperidino, morpholino, pyrrolidinyl, piperazinyl, pyridyl, thienyl, furyl, oxazinyl, oxadiazolyl), which optionally has one or two oxo), optionally having substituents selected from the group consisting of (i') a hydroxy group, (ii') a C₁₋₆ alkyl group (e.g., a methyl group, an ethyl group, an isopropyl group), (iii') a formyl group, (iv') a C₁₋₆ alkyl-carbonyl group (e.g., acetyl), (v') a formylamino group, (vi') a C₁₋₆ alkyl-carbonylamino group (e.g., acetylamino) and (vii') a C₁₋₆ alkoxy-carbonyl group (e.g., tert-butoxycarbonyl),
      (xx) a C₁₋₆ alkylsulfonyl group (e.g., methylsulfonyl),
      (xxi) a cyano group, and
      (xxii) a carboxy group, and
         Ra' is a hydrogen atom or a C₁₋₆ alkyl group, or
(3) a C₆₋₁₄ aryl-C₁₋₆ alkyl group (e.g., a benzyl group),
   X is an oxygen atom, and ring B is a C₆₋₁₄ aryl ring (e.g., benzene ring) having substituents selected from the group consisting of an optionally halogenated C₁₋₆ alkyl group (e.g., trifluoromethyl) and a halogen atom (e.g., fluorine atom).

Furthermore, a compound (I) wherein Ar is a C₆₋₁₄ aryl group optionally having halogen atoms (e.g., fluorine atom),
R¹ is
(1) a hydrogen atom,
(2) a formyl group,
(3) a group represented by the formula -(C=O)-NRb¹Rb²
   wherein Rb¹ and Rb² are the same or different and each is a hydrogen atom or a C₁₋₆ alkyl group (e.g., methyl, ethyl),
(4) a group represented by the formula -(C=O)-(CH₂)m-Rb³
   wherein m is an integer of 1 to 3, Rb³ is a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., triazolyl, tetrazolyl, imidazolyl, dihydrofuryl, pyrrolyl, pyrrolidinyl, piperidino, piperazinyl, pyridyl, thienyl, furyl, thiazolyl, oxadiazolyl), preferably an aromatic heterocyclic group (e.g., triazolyl, tetrazolyl, pyrrolyl, pyridyl, thienyl, furyl, thiazolyl, oxadiazolyl), which optionally has one or two oxo groups), optionally substituted by substituents selected from the group consisting of C₁₋₆ alkyl (e.g., methyl, ethyl), formyl, C₁₋₆ alkyl-carbonyl (e.g., acetyl, ethylcarbonyl), hydroxy, C₁₋₆ alkoxy-carbonyl (e.g., methoxycarbonyl, ethoxycarbonyl, tert-butoxycarbonyl), formylamino and C₁₋₆ alkyl-carbonylamino (e.g., acetylamino, ethylcarbonylamino),
(5) a group represented by the formula -(C=O)-(CH₂)n-NRb⁴Rb⁵
   wherein n is an integer of 1 to 4, Rb⁴ and Rb⁵ are the same and different and each is (a) a hydrogen atom, (b) a formyl group, (c) a C₁₋₆ alkyl-carbonyl group (e.g., acetyl, ethylcarbonyl, propylcarbonyl, isopropylcarbonyl, tert-butylcarbonyl) optionally having halogen atoms (e.g., chlorine atom), (d) a C₁₋₆ alkoxy-carbonyl group (e.g., methoxycarbonyl, ethoxycarbonyl, tert-butoxycarbonyl), (e) a C₁₋₆ alkylsulfonyl group (e.g., methylsulfonyl, ethylsulfonyl) or (f) a mono- or di-C₁₋₆ alkyl-carbamoyl group (e.g., methylcarbamoyl, ethylcarbamoyl, isopropylcarbamoyl, dimethylcarbamoyl, diethylcarbamoyl),
(6) a group represented by the formula: 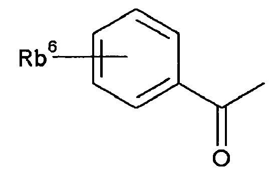 wherein Rb⁶ is a formylamino group or a C₁₋₆ alkyl-carbonylamino group (e.g., acetylamino),
(7) a group represented by the formula: 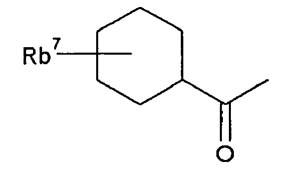 wherein Rb⁷ is
   (i) an amino group,
   (ii) a formylamino group,
   (iii) a C₁₋₆ alkyl-carbonylamino group (e.g., acetylamino, ethylcarbonylamino, propylcarbonylamino, isopropylcarbonylamino) optionally substituted by substituents selected from the group consisting of (a) C₁₋₆ alkyl (e.g., methyl, ethyl), (b) hydroxy, (c) C₁₋₆ alkoxy (e.g., methoxy), (d) a halogen atom (e.g., chlorine atom), (e) formylamino, (f) C₁₋₆ alkyl-carbonylamino (e.g., acetylamino) and (g) a heterocyclic group optionally having one or two oxo groups (e.g., a 5- or 6-membered non-aromatic or aromatic heterocyclic group containing, besides carbon atoms, 1 to 4 of one or two kinds of hetero atoms selected from the group consisting of a nitrogen atom, an oxygen atom and a sulfur atom (e.g., piperidino, imidazolyl, triazolyl, tetrazolyl, oxazolyl), which optionally has one or two oxo groups), optionally having one or two C₁₋₆ alkyl groups (e.g., methyl, ethyl),
   (iv) a C₃₋₇ cycloalkyl-carbonylamino group (e.g., cyclopropylcarbonylamino),
   (v) a C₁₋₆ alkoxy-carbonylamino group (e.g., methoxycarbonylamino, ethoxycarbonylamino, tert-butoxycarbonylamino),
   (vi) a heterocycle-carbonylamino group (e.g., a 5- or 6-membered aromatic heterocycle-carbonylamino group, wherein the heterocycle contains, besides carbon atoms, 1 to 4 of one or two kinds of hetero atoms selected from the group consisting of a nitrogen atom, an oxygen atom and a sulfur atom (e.g., thienylcarbonylamino, furylcarbonylamino, pyrrolylcarbonylamino)),
   (vii) an ureido group optionally substituted by one or two C₁₋₆ alkyl groups (e.g., methyl, ethyl, propyl, isopropyl),
   (viii) a C₁₋₆ alkoxy-carbonyl group (e.g., methoxycarbonyl, ethoxycarbonyl),
   (ix) a carbamoyl group optionally having C₁₋₆ alkoxy groups (e.g., methoxy, ethoxy),
   (x) a mono- or di-C₁₋₆ alkyl-carbamoyl group (e.g., methylcarbamoyl, ethylcarbamoyl, dimethylcarbamoyl),
   (xi) a C₁₋₆ alkylsulfonylamino group (e.g., methylsulfonylamino, ethylsulfonylamino),
   (xii) a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., triazolyl, tetrazolyl, imidazolyl, dihydrofuryl, pyrrolyl, pyrrolidinyl, piperidino), which optionally has one or two oxo groups), or
   (xiii) a heterocycle-carbonyl group (e.g., a 5- or 6-membered aromatic or non-aromatic heterocycle (e.g., pyrrolidinyl, piperidino)-carbonyl group, wherein the heterocycle contains, besides carbon atoms, 1 to 4 of one or two kinds of hetero atoms selected from the group consisting of a nitrogen atom, an oxygen atom and a sulfur atom),
(8) a group represented by the formula: 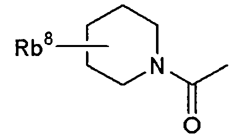 wherein Rb⁸ is (i) a formylamino group, (ii) an alkyl-carbonylamino group (e.g., acetylamino, ethylcarbonylamino) optionally substituted by hydroxy groups, (iii) a C₁₋₆ alkylsulfonylamino group (e.g., methylsulfonylamino, ethylsulfonylamino) or (iv) a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., pyrrolidinyl, piperidino, morpholino, isothiazolidinyl, oxazolidinyl, oxazinyl), which optionally has one or two oxo groups),
(9) a group represented by the formula: 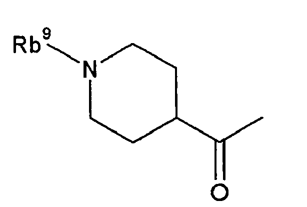 wherein Rb⁹ is
   (i) a hydrogen atom,
   (ii) a C₁₋₆ alkyl group (e.g., methyl) optionally substituted by substituents selected from the group consisting of formyl, C₁₋₆ alkyl-carbonyl (e.g., acetyl), C₁₋₆ alkoxy-carbonyl (e.g., methoxycarbonyl, isopropoxycarbonyl) and mono or di-C₁₋₆ alkyl-carbamoyl (e.g., dimethylcarbamoyl),
   (iii) a formyl group,
   (iv) a C₁₋₆ alkyl-carbonyl group (e.g., acetyl) optionally substituted by substituents selected from the group consisting of (a) formylamino, (b) C₁₋₆ alkyl-carbonylamino (e.g., acetylamino) and (c) a heterocyclic group optionally having one or two oxo groups (e.g., a 5- or 6-membered aromatic heterocyclic group containing, besides carbon atoms, 1 to 4 of one or two kinds of hetero atoms selected from the group consisting of a nitrogen atom, an oxygen atom and a sulfur atom (e.g., triazolyl, tetrazolyl, imidazolyl, pyrazolyl), which optionally has one or two oxo groups),
   (v) a heterocycle-carbonyl group optionally having one or two oxo groups (e.g., a 5- or 6-membered aromatic or non-aromatic heterocycle (e.g., imidazolyl)-carbonyl group, wherein the heterocycle contains, besides carbon atoms, 1 to 4 of one or two kinds of hetero atoms selected from the group consisting of a nitrogen atom, an oxygen atom and a sulfur atom, and optionally has one or two oxo groups),
   (vi) a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., dihydrofuryl), which optionally has one or two oxo groups),
   (vii) a formylamino group,
   (viii) a C₁₋₆ alkyl-carbonylamino group (e.g., acetylamino),
   (ix) a C₁₋₆ alkylsulfonyl group (e.g., methylsulfonyl, ethylsulfonyl),
   (x) a C₁₋₆ alkoxy-carbonyl group (e.g., methoxycarbonyl, ethoxycarbonyl), or
   (xi) a mono- or di-C₁₋₆ alkyl-carbamoyl group (e.g., methylcarbamoyl, ethylcarbamoyl, dimethylcarbamoyl, diethylcarbamoyl),
(10) a group represented by the formula: 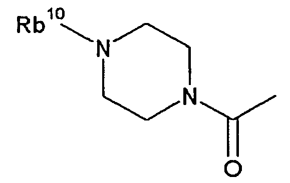 wherein Rb¹⁰ is (i) a formyl group, (ii) a C₁₋₆ alkyl-carbonyl group (e.g., acetyl) or (iii) a heterocyclic group optionally having one or two oxo groups (e.g., a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., pyrrolidinyl, piperidino), which optionally has one or two oxo groups),
(11) a group represented by the formula: 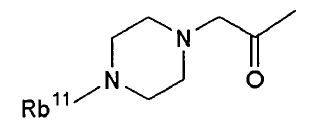 wherein Rb¹¹ is a C₁₋₆ alkyl group (e.g., isopropyl), a formyl group or a C₁₋₆ alkyl-carbonyl group (e.g., acetyl),
(12) a group represented by the formula: 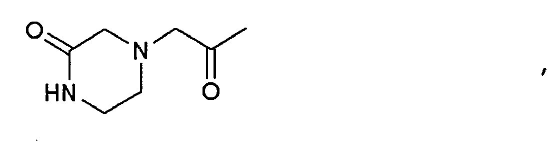
(13) a group represented by the formula: 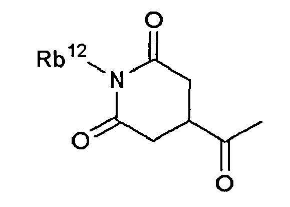 wherein Rb¹² is a hydrogen atom or a C₁₋₆ alkyl group (e.g., methyl),
(14) a group represented by the formula: 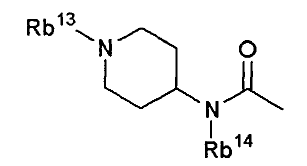 wherein Rb¹³ is
   (i) a hydrogen atom,
   (ii) a formyl group,
   (iii) a C₁₋₆ alkyl-carbonyl group (e.g., acetyl, ethylcarbonyl) optionally substituted by hydroxyl groups,
   (iv) a C₁₋₆ alkoxy-carbonyl group (e.g., methoxycarbonyl, ethoxycarbonyl), or
   (v) a C₁₋₆ alkylsulfonyl group (e.g., methylsulfonyl, ethylsulfonyl), and Rb¹⁴ is a hydrogen atom or a C₁₋₆ alkyl group (e.g., methyl),
(15) a group represented by the formula: 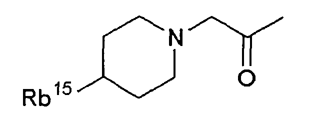 wherein Rb¹⁵ is a hydroxy group, a formylamino group or a C₁₋₆ alkyl-carbonylamino group (e.g., acetylamino),
(16) a group represented by the formula: 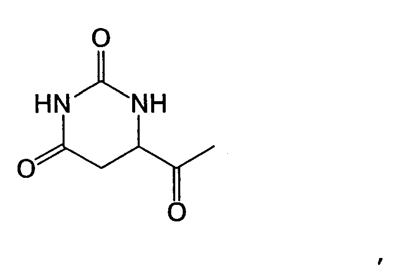
(17) a group represented by the formula: 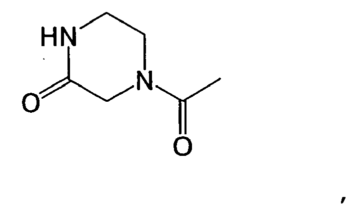
(18) a group represented by the formula: 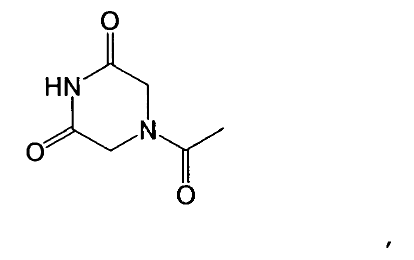
(19) a C₁₋₆ alkyl-carbonyl group optionally substituted by substituents selected from the group consisting of (i) cyano, (ii) hydroxy, (iii) carboxy, (iv) C₁₋₆ alkoxy-carbonyl (e.g., methoxycarbonyl, ethoxycarbonyl), (v) carbamoyl, (vi) C₁₋₆ alkyl-carbamoyl (e.g., methylcarbamoyl, ethylcarbamoyl) and (vii) heterocycle-carbonyl group (e.g., 5- or 6-membered aromatic or non-aromatic heterocycle (e.g., pyrrolidinyl)-carbonyl group, wherein the heterocycle contains, besides carbon atoms, 1 to 4 of one or two kinds of hetero atoms selected from the group consisting of a nitrogen atom, an oxygen atom and a sulfur atom),
(20) a C₃₋₆ cycloalkyl-carbonyl group (e.g., cyclopropylcarbonyl) optionally substituted by carbamoyl groups,
(21) a C₁₋₆ alkenyl-carbonyl group (e.g., ethenyl (vinyl) carbonyl) substituted by heterocyclic groups (e.g., 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom (e.g., pyridyl, thienyl, imidazolyl, pyrrolyl, thiazolyl) optionally substituted by C₁₋₆ alkyl groups (e.g., methyl, ethyl),
(22) a group represented by the formula -(C=O)-(CRb¹⁶Rb¹⁷)-NHRb¹⁸
   wherein Rb¹⁶ is a hydrogen atom or a C₁₋₆ alkyl group (e.g., methyl , ethyl), Rb¹⁷ is a C₁₋₆ alkyl group (e.g., methyl, ethyl) optionally substituted by substituents selected from the group consisting of a carbamoyl group and an aromatic heterocyclic group (e.g., a 5- or 6-membered aromatic heterocycle containing, besides carbon atoms, 1 to 4 of one or two kinds of hetero atoms selected from the group consisting of a nitrogen atom, an oxygen atom and a sulfur atom (e.g., imidazolyl)), and Rb¹⁸ is a hydrogen atom, a C₁₋₆ alkyl-carbonyl group or a C₁₋₆ alkoxy-carbonyl group (e.g., methoxycarbonyl, ethoxycarbonyl, tert-butoxycarbonyl), or
(23) a C₆₋₁₄ aryl-C₁₋₆ alkyl group (e.g., benzyl),
   X is an oxygen atom or NH, and
   ring B is a benzene ring having substituents selected from the group consisting of (i) an optionally halogenated C₁₋₆ alkyl group (e.g., trifluoromethyl), (ii) a halogen atom (e.g., fluorine atom), (iii) a C₁₋₆ alkoxy group (e.g., methoxy) and (iv) a 5- or 6-membered aromatic heterocyclic group (e.g., tetrazole) containing, besides carbon atoms, 1 to 4 hetero atoms selected from the group consisting of a nitrogen atom, an oxygen atom and a sulfur atom, which is optionally substituted by an optionally halogenated C₁₋₆ alkyl group (e.g., trifluoromethyl).

Particularly, a compound (I) wherein X is an oxygen atom, ring B is a benzene ring having substituents selected from the group consisting of an optionally halogenated C₁₋₆ alkyl group (e.g., trifluoromethyl) and a halogen atom (e.g., fluorine atom) is preferable.

Furthermore, as compound (I),
(1) (3R*,4S*)-1-[(1-acetylpiperidin-4-yl)carbonyl]-4-[(1R*)-1-[3,5-bis(trifluoromethyl)phenyl]ethoxy]-3-phenylpiperidine
(2) N-(2-[4-[[(3R*,4S*)-4-[(1R*)-1-[3,5-bis(trifluoromethyl)phenyl]ethoxy]-3-phenylpiperidin-1-yl]carbonyl]piperidin-1-yl]-2-oxoethyl]acetamide
(3) 1-[4-[(3R*,4S*)-4-[(1R*)-1-[3,5-bis(trifluoromethyl)phenyl]ethoxy]-3-phenylpiperidin-1-yl]carbonyl]piperidin-1-yl]acetone
(4) methyl [4-[[(3R*,4S*)-4-[(1R*)-1-[3,5-bis(trifluoromethyl)phenyl]ethoxy]-3-phenylpiperidin-1-yl]carbonyl]piperidin-1-yl]acetate
(5) N-[1-[2-[(3R*,4S*)-4-[(1R*)-1-[3,5-bis(trifluoromethyl)phenyl]ethoxy]-3-phenylpiperidin-1-yl]-2-oxoethyl]piperidin-4-yl]acetamide
(6) 1-[[4-[[(3R*,4S*)-4-[(1R*)-1-[3,5-bis(trifluoromethyl)phenyl]ethoxy]-3-phenylpiperidin-1-yl]carbonyl]piperidin-1-yl]carbonyl]imidazolidin-2-one
(7) 2-[4-[[(3R*,4S*)-4-[(1R*)-1-[3,5-bis(trifluoromethyl)phenyl]ethoxy]-3-phenylpiperidin-1-yl]carbonyl]piperidin-1-yl]-N,N-dimethylacetamide
(8) 1-[2-[(3R*,4S*)-4-[(1R*)-1-[3,5-bis(trifluoromethyl)phenyl]ethoxy]-3-phenylpiperidin-1-yl]-2-oxoethyl]-4-isopropylpiperazine
(9) N-[trans-4-[[(3R*,4S*)-4- [(1R*)-1- [3,5-bis(trifluoromethyl)phenyl]ethoxy]-3-phenylpiperidin-1-yl] carbonyl] cyclohexyl]acetamide
(10) N-[trans-4-[[(3R*,4S*)-4-[(1R*)-1-[3,5-bis(trifluoromethyl)phenyl]ethoxy]-3-phenylpiperidin-1-yl]carbonyl]cyclohexyl]propanamide
(11) N-[trans-4-[[(3R*,4S*)-4-[(1R*)-1-[3,5-bis(trifluoromethyl)phenyl]ethoxy]-3-phenylpiperidin-1-yl]carbonyl]cyclohexyl]-2-methylpropanamide
(12) N-[trans-4-[[(3R*,4S*)-4-[(lR*)-1-[3,5-bis(trifluoromethyl)phenyl]ethoxy]-3-phenylpiperidin-1-yl]carbonyl]cyclohexyl]-N'-methylurea
(13) 3-[4-[[(3R,4S)-4-[(1R)-1-[3,5-bis(trifluoromethyl)phenyl]ethoxy]-3-phenylpiperidin-1-yl]carbonyl]piperidin-1-yl]dihydrofuran-2(3H)-one hydrochloride (retention time: short)
(14) 3-[4-[[(3R,45)-4-[(1R)-1-[3,5-bis(trifluoromethyl)phenyl]ethoxy]-3-phenylpiperidin-1-yl]carbonyl]piperidin-1-yl]dihydrofuran-2(3H)-one hydrochloride (retention time: long)
(15) (3R,4S)-1-[(1-acetylpiperidin-4-yl)carbonyl]-4-[(1R)-1-[3,5-bis(trifluoromethyl)phenyl]ethoxy]-3-phenylpiperidine
(16) N-[2-[4-[[(3R,4S)-4-[(1R)-1-[3,5-bis(trifluoromethyl)phenyl]ethoxy]-3-phenylpiperidin-1-yl]carbonyl]piperidin-1-yl]-2-oxoethyl]acetamide
(17) (3R,4S)-4-[(1R)-1-[3,5-bis(trifluoromethyl)phenyl]ethoxy]-3-phenyl-1-[[1-(1H-tetrazol-1-ylacetyl)piperidin-4-yl]carbonyl]piperidine
(18) 1-[[4-[[(3R,4S)-4-[(1R)-1-[3,5-bis(trifluoromethyl)phenyl]ethoxy]-3-phenylpiperidin-1-yl]carbonyl]piperidin-1-yl]carbonyl]imidazolidin-2-one
(19) N-[trans-4-[[(3R,4S)-4-[(1R)-1-[3,5-bis(trifluoromethyl)phenyl]ethoxy]-3-phenylpiperidin-1-yl]carbonyl]cyclohexyl]acetamide
(20) N-[trans-4-[[(3R,4S)-4-[(1R)-1-[3,5-bis(trifluoromethyl)phenyl]ethoxy]-3-phenylpiperidin-1-yl]carbonyl]cyclohexyl]propanamide
(21) N-[trans-4-[[(3R,4S)-4-[(1R)-1-[3,5-bis(trifluoromethyl)phenyl]ethoxy]-3-phenylpiperidin-1-yl]carbonyl]cyclohexyl]butanamide
(22) N-[trans-4-[[(3R,4S)-4-[(1R)-1-[3,5-bis(trifluoromethyl)phenyl]ethoxy]-3-phenylpiperidin-1-yl]carbonyl]cyclohexyl]-2-methylpropanamide
(23) N-[trans-4-[[(3R,4S)-4-[(1R)-1-[3,5-bis (trifluoromethyl) phenyl] ethoxy]-3-phenylpiperidin-1-yl]carbonyl]cyclohexyl]cyclopropanecarboxamide
(24) N-[trans-4-[[(3R,4S)-4-[(1R)-1-[3,5-bis(trifluoromethyl)phenyl]ethoxy]-3-phenylpiperidin-l-yl]carbonyl]cyclohexyl]-N'-methylurea
(25) N-[trans-4-[[(3R,4S)-4-[(1R)-1-[3,5-bis(trifluoromethyl)phenyl]ethoxy]-3-phenylpiperidin-1-yl]carbonyl]cyclohexyl]methanesulfonamide
(26) N-[trans-4-[[(3R,4S)-4-[(1R)-1-[3,5-bis(trifluoromethyl)phenyl]ethoxy]-3-phenylpiperidin-l-yl]carbonyl]cyclohexyl]thiophene-2-carboxamide
(27) N-[cis-4-[[(3R,4S)-4-[(1R)-1-[3,5-bis(trifluoromethyl)phenyl]ethoxy]-3-phenylpiperidin-1-yl]carbonyl]cyclohexyl]acetamide
(28) N-[trans-4-[[(3R,4S)-4-[(1R)-1-[3,5-bis(trifluoromethyl)phenyl]ethoxy]-3-phenylpiperidin-1-yl]carbonyl]cyclohexyl]-2-(2-oxopiperidin-1-yl)acetamide
(29) N-[trans-4-[[(3R,4S)-4-[(1R)-1-[3,5-bis(trifluoromethyl)phenyl]ethoxy]-3-phenylpiperidin-1-yl]carbonyl]cyclohexyl]-5-chloropentaneamide
(30) methyl trans-4-[[(3R,4S)-4-[(1R)-1-[3,5-bis(trifluoromethyl)phenyl]ethoxy]-3-phenylpiperidin-1-yl]carbonyl]cyclohexylcarbamate
(31) N-[trans-4-[[(3R,4S)-4-[(1R)-1-[3,5-bis(trifluoromethyl)phenyl]ethoxy]-3-phenylpiperidin-1-yl]carbonyl]cyclohexyl]-N'-ethylurea
(32) N-[trans-4-[[(3R,4S)-4-[(1R)-1-[3,5-bis(trifluoromethyl)phenyl]ethoxy]-3-phenylpiperidin-l-yl]carbonyl]cyclohexyl]-N'-isopropylurea
(33) N'-[trans-4-[[(3R,4S)-4-[(lR)-1-[3,5-bis(trifluoromethyl)phenyl]ethoxy]-3-phenylpiperidin-1-yl]carbonyl]cyclohexyl]-N,N-dimethylurea
(34) trans-4-([(3R,4S)-4-[(1R)-1-[3,5-bis(trifluoromethyl)phenyl]ethoxy]-3-phenylpiperidin-l-yl]carbonyl]-N-methylcyclohexanecarboxamide
(35) trans-4-[[(3R,4S)-4-[(1R)-1-[3,5-bis(trifluoromethyl)phenyl]ethoxy]-3-phenylpiperidin-1-yl]carbonyl]-N-ethylcyclohexanecarboxamide
(36) (3R,4S)-N-(1-acetylpiperidin-4-yl)-4-[(1R)-1-[3,5-bis(trifluoromethyl)phenyl]ethoxy]-3-phenylpiperidine-1-carboxamide
(37) (3R,4S)-4-[(1R)-1-[3,5-bis(trifluoromethyl)phenyl]ethoxy]-3-phenyl-N-(1-propionylpiperidin-4-yl)piperidine-1-carboxamide
(38) N-[1-[[(3R,4S)-4-[(1R)-1-[3,5-bis(trifluoromethyl)phenyl]ethoxy]-3-phenylpiperidin-1-yl]carbonyl]piperidin-4-yl]acetamide
(39) N-[1-[[(3R,4S)-4-[(1R)-1-[3,5-bis(trifluoromethyl)phenyl]ethoxy]-3-phenylpiperidin-1-yl]carbonyl]piperidin-4-yl]propanamide
(40) 4-[[(3R,4S)-4-[(1R)-1-[3,5-bis(trifluoromethyl)phenyl]ethoxy]-3-phenylpiperidin-1-yl]carbonyl]piperidine-2,6-dione
(41) 4-[[(3R,4S)-4-[(1R)-1-[3,5-bis(trifluoromethyl)phenyl]ethoxy]-3-phenylpiperidin-1-yl]carbonyl]-1-methylpiperidine-2,6-dione
(42) 5-[2-[(3R,4S)-4-[(1R)-1-[3,5-bis(trifluoromethyl)phenyl]ethoxy]-3-phenylpiperidin-1-yl]-2-oxoethyl]imidazolidine-2,4-dione
(43) (3R,4S)-4-{ (1R)-1-[3,5-bis(trifluoromethyl)phenyl]ethoxy}-3-phenyl-1-{[1-(1H-1,2,4-triazol-1-ylacetyl)piperidin-4-yl]carbonyl}piperidine
(44) (3R,4S)-4-{(1R)-1-[3,5-bis(trifluoromethyl)phenyl]ethoxy}-3-phenyl-1-{[1-(1H-pyrazol-1-ylacetyl) piperidin-4-yl]carbonyl}piperidine
   or a salt thereof and the like are preferably used, and particularly, (3R,4S)-4-[(1R)-1-[3,5-bis(trifluoromethyl)phenyl]ethoxy]-3-phenyl-1-[[1-(1H-tetrazol-1-ylacetyl)piperidin-4-yl]carbonyl]piperidine N-[trans-4-[[(3R,4S)-4-[(1R)-1-[3,5-bis(trifluoromethyl)phenyl]ethoxy]-3-phenylpiperidin-1-yl] carbonyl] cyclohexyl]propanamide 4-[[(3R,4S)-4-[(1R)-1-[3,5-bis(trifluoromethyl)phenyl]ethoxy]-3-phenylpiperidin-1-yl]carbonyl]piperidine-2,6-dione (3R,4S)-4-{(1R)-1-[3,5-bis(trifluoromethyl)phenyl]ethoxy}-3-phenyl-1-{[1-(1H-1,2,4-triazol-1-ylacetyl)piperidin-4-yl]carbonyl}piperidine (3R,4S)-4-{(1R)-1-[3,5-bis(trifluoromethyl)phenyl]ethoxy}-3-phenyl-1-{[1-(1H-pyrazol-1-ylacetyl)piperidin-4-yl]carbonyl}piperidine
   or a salt thereof and the like are preferably used.

The salt of compound (I) includes, for example, a metal salt, an ammonium salt, a salt with an organic base, a salt with an inorganic acid, a salt with an organic acid, a salt with basic or acidic amino acid etc. Suitable examples of the metal salt include an alkali metal salt such as a sodium salt, a potassium salt etc.; an alkaline earth metal salt such as a calcium salt, a magnesium salt, a barium salt etc.; an aluminum salt etc. Suitable examples of the salts with an organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine etc. Suitable examples of the salts with an inorganic acid include salts with hydrochloric acid, hydrobromic acid, nitric acid, sulfuric acid, phosphoric acid etc. Suitable examples of the salts with an organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid etc. Suitable examples of the salts with basic amino acid include salts with arginine, lysine, ornithine etc. Suitable examples of the salts with acidic amino acid include salts with asparaginic acid and glutamic acid etc.

Of these, pharmaceutically acceptable salts are preferred. For example, if the compound has an acidic functional group, preferred are inorganic salts such as an alkali metal salt (e.g., sodium salt, potassium salt etc.), an alkaline earth metal salt (e.g., calcium salt, magnesium salt, barium salt etc.), an ammonium salt etc. If the compound has a basic functional group, preferred are salts with an inorganic acid such as hydrochloric acid, hydrobromic acid, nitric acid, sulfuric acid, phosphoric acid etc., or salts with an organic acid such as acetic acid, phthalic acid, fumaric acid, oxalic acid, tartaric acid, maleic acid, citric acid, succinic acid, methanesulfonic acid, p-toluene sulfonic acid etc.

The prodrug of the compound (I) or a salt thereof of the present invention means a compound which is converted to the compound (I) of the present invention under the physiological condition in the living body by a reaction with an enzyme, a gastric acid, or the like, that is, by enzymatic oxidation, reduction, hydrolysis etc. or by hydrolysis with gastric acid etc.

The prodrug of the compound (I) of the present invention includes a compound wherein the amino group of the compound (I) of the present invention is modified with acyl, alkyl or phosphoryl (e.g., a compound wherein the amino group of the compound (I) of the present invention is modified to eicosanyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl etc.), and the like; a compound wherein the hydroxy group of the compound (I) of the present invention is modified with acyl, alkyl, phosphoryl or boryl (e.g., a compound wherein the hydroxy group of the compound (I) of the present invention is modified with acetyl, palmitoyl, propanoyl, pivaloyl, succinyl, fumaryl, alanyl or dimethylaminomethylcarbonyl etc.) and the like; a compound wherein a carboxyl group of the compound (I) of the present invention is modified to ester or amide (e.g., a compound wherein a carboxyl group of the compound (I) of the present invention is modified to ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester or methylamide etc.) and the like; etc. These compounds can be prepared by a method known *per se* from the compound (I) of the present invention etc.

In addition, the prodrug of the compound (I) of the present invention may be a compound, which is converted into the compound (I) of the present invention under the physiological conditions, as described in "Pharmaceutical Research and Development", Vol. 7 (Drug Design), pp. 163-198 (1990), published by Hirokawa Publishing Co.

Solvate, for example, hydrates of the compound (I) of the present invention and a salt thereof are all included in the scope of the present invention. The compound (I) of the present invention may be labeled with an isotope (e.g., ³H, ¹⁴C, ³⁵S, ¹²⁵I etc.) and the like.

If the compound (I) of the present invention according to the present invention has chiral center, isomers such as an enantiomer or a diastereomer may exist. Such isomers and a mixture thereof are all included in the scope of the present invention. In addition, there can be instances where the conformational isomers are generated in cases, but such isomers or a mixture thereof are also included in compound (I) or a salt thereof of the present invention. Compound (I) is preferably a cis-isomer in view of activity.

A method of preparing compound (I) or a salt thereof of the present invention will be explained in the following.

Compound (I) or a salt thereof of the present invention can be prepared according to a method described in WO 03/101964, specifically, using Method A, Method B or Method C below.

### [Method A]

Compound (I) or a salt thereof of the present invention can be prepared by subjecting a compound represented by the formula: 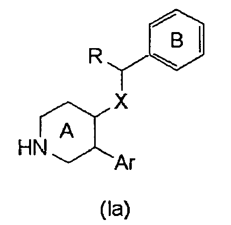 wherein each symbol is as defined above, (hereinafter to be referred to as compound (Ia)) or a salt thereof, to a reaction with a compound represented by the formula

R^{1a}-OH (II)

wherein R^{1a} is a hydrocarbon group optionally having substituents, an acyl group or a heterocyclic group optionally having substituents, which is an alkylating agent or an acylating agent, a salt thereof or a reactive derivative thereof.

The "hydrocarbon group optionally having substituents, the acyl group or the heterocyclic group optionally having substituents" represented by R^{1a} includes, for example, the same group as those referred to herein above for the foregoing group represented by R¹.

The reactive derivative of the compound represented by R^{1a}-OH or a salt thereof includes, for example, a compound represented by the formula:

R^{1a}-L (IIa)

wherein L is a leaving group, and R^{1a} is as defined above, (hereinafter to be simply referred to as a reactive derivative) or a salt thereof.

The leaving group represented by L includes, for example, a hydroxy group, a halogen atom (e.g., a chlorine atom, a bromine atom, an iodine atom etc.), a substituted sulfonyloxy group (e.g., a C₁₋₆ alkylsulfonyloxy group such as methanesulfonyloxy, ethanesulfonyloxy etc.; a C₆₋₁₄ arylsulfonyloxy group such as benzenesulfonyloxy, p-toluenesulfonyloxy etc.; and a C₇₋₁₆ aralkylsulfonyloxy group such as a benzylsulfonyloxy group etc.), acyloxy (acetoxy, benzoyloxy etc.), carbonates, trichloroacetoimidates, oxalates, phosphites (e.g., methyl phosphite etc.), phosphoranes, an oxy group substituted with a hetero ring or an aryl group (succinimide, benzotriazole, quinoline, 4-nitrophenyl etc.), a hetero ring (imidazole etc.) and the like.

The reaction using the above-mentioned reactive derivative as an alkylating agent can be carried out by subjecting the reactive derivative to a reaction, usually in a solvent in the presence of base. The solvent includes, for example, alcohols such as methanol, ethanol, propanol etc., ethers such as dimethoxyethane, dioxane, tetrahydrofuran etc., ketones such as acetone etc., nitriles such as acetonitrile etc., amides such as N,N-dimethylformamide etc., sulfoxides such as dimethyl sulfoxide etc., water and the like, which may be used in a suitable mixture. The base includes, for example, an organic base such as trimethylamine, triethylamine, N-methylmorpholine, pyridine, picoline, N,N-dimethylaniline etc., and an inorganic base such as potassium carbonate, sodium carbonate, potassium hydroxide, sodium hydroxide etc. The amount of the base to be used is, for example, about 1 to about 100 molar equivalents, preferably about 1 to about 10 molar equivalents, per 1 mole of the substrate.

The reactive derivative includes, for example, halides (e.g., chloride, bromide, iodide etc.), sulfuric acid esters, or sulfonic acid esters (e.g., methanesulfonate, p-toluenesulfonate, benzenesulfonate etc.) and the like, and particularly halides. The amount of the reactive derivative to be used is, for example, 1 to 5 molar equivalents, preferably 1 to 3 molar equivalents, per 1 mole of the substrate.

If necessary, the reaction can be facilitated by adding an additive. Such an additive includes, for example, iodides such as sodium iodide, potassium iodide etc. and the amount to be used is about 0.1 to 10 molar equivalents, preferably about 0.1 to 5 molar equivalents, per 1 mole of the substrate.

The reaction temperature is generally -10°C to 200°C, preferably about 0°C to 110°C, and the reaction time is generally 0.5 hr to 48 hr, preferably 0.5 hr to 16 hr.

The reaction using the above-mentioned reactive derivative as an acylating agent depends on the kind of reactive derivative or substrate, but it is usually carried out in a solvent. If necessary, a suitable base may be added to facilitate the reaction. The solvent includes, for example, hydrocarbons such as benzene, toluene etc., ethers such as ethyl ether, dioxane, tetrahydrofuran etc., esters such as ethyl acetate etc., halogenated hydrocarbons such as chloroform, dichloromethane etc., esters such as ethyl acetate etc., amides such as N,N-dimethylformamide etc., aromatic amines such as pyridine etc., water and the like, which may be used in a suitable mixture. In addition, the base includes, for example, alkali metal hydroxides such as sodium hydroxide, potassium hydroxide etc., hydrogen carbonates such as sodium hydrogen carbonate, potassium hydrogen carbonate etc., carbonate such as sodium carbonate, potassium carbonate etc., acetate such as sodium acetate, tertiary amines such as trimethylamine, triethylamine, N-methylmorpholine etc., aromatic amines such as pyridine, picoline, N,N-dimethylaniline etc. and the like. The amount of the base to be used is, for example, about 1 to about 100 molar equivalents, preferably about 1 to about 10 molar equivalents, per 1 mole of the substrate.

The acylating agent includes, for example, carboxylic acid, sulfonic acid, phosphoric acid, carbonic acid or a reactive derivative thereof (e.g., acid halide, acid anhydride, mixed acid anhydride, active ester etc.), isocyanic acid ester, isothiocyanic acid ester and the like.

The amount of such an acylating agent to be used is usually 1 to 10 molar equivalents, preferably 1 to 3 molar equivalents, per 1 mole of the substrate. The reaction temperature is generally -10°C to 150°C, preferably about 0°C to 100°C, and the reaction time is generally 15 min to 24 hr, preferably 30 min to 16 hr.

Compound (Ia) used as the starting material in Method A can be prepared by subjecting compound (Ib or Ic) or a salt thereof obtained by Method B or Method C mentioned below to deacylation or dealkylation.

Such deacylation can be carried out according to a known method. For example, it is usually carried out in the presence of an acid or a base, if necessary, in a solvent that does not adversely influence the reaction though it depends on the kinds of the substrate.

The acid is preferably mineral acids (e.g., hydrochloric acid, hydrobromic acid, sulfuric acid etc.), carboxylic acids (e.g., acetic acid, trifluoroacetic acid, trichloroacetic acid etc.), sulfonic acids (e.g., methanesulfonic acid, toluenesulfonic acid etc.), Lewis acids (aluminum chloride, tin chloride, zinc bromide etc.) and the like. If necessary, it may be used in a mixture of two or more. The amount of the acid varies depending on the kinds of the solvent and other reaction conditions, but it is usually about 0.1 molar equivalents or more, per 1 mole of compound (Ib or Ic), and the acid can be used as a solvent.

The base is preferably an inorganic base (alkali metal hydroxides such as sodium hydroxide, potassium hydroxide etc., alkali metal hydrogen carbonates such as sodium hydrogen carbonate, potassium hydrogen carbonate etc., alkali metal carbonates such as sodium carbonate, potassium carbonate etc., alkoxides such as sodium methoxide, sodium ethoxide etc. and the like), or an organic base (amines such as trimethylamine, triethylamine, diisopropylethylamine etc., cyclic amines such as pyridine, 4-dimethylaminopyridine etc.) and the like, and preferably, sodium hydroxide, potassium hydroxide, sodium ethoxide and the like.

The amount of the base to be used varies depending on the kinds of the solvent and other reaction conditions, but is generally about 0.1 to about 10 molar equivalents, preferably about 0.1 to about 5 molar equivalents, per 1 mole of compound (Ib or Ic).

The solvent that does not adversely influence the reaction includes, for example, alcohols such as methanol, ethanol, propanol, 2-propanol, butanol, isobutanol, tert-butanol; aromatic hydrocarbons such as benzene, toluene, xylene etc.; aliphatic hydrocarbons such as hexane, heptane etc.; halogenated hydrocarbons such as dichloromethane, chloroform etc.; ethers such as diethyl ether, diisopropyl ether, tert-butyl methyl ether, tetrahydrofuran, dioxane, dimethoxyethane etc.; nitriles such as acetonitrile etc.; esters such as ethyl acetate etc.; carboxylic acids such as acetic acid etc.; amides such as N,N-dimethylformamide, dimethylacetamide etc.; sulfoxides such as dimethyl sulfoxide etc.; water and the like. Such a solvent may be used in a mixture of two or more at a suitable ratio.

The reaction temperature is for example, about -50°C to about 200°C, preferably about 0°C to about 100°C, and the reaction time varies depending on the kinds of compound (Ib or Ic) or a salt thereof, the reaction temperature and the like, and it is for example, about 0.5 to about 100 hr, preferably about 0.5 to about 24 hr.

Dealkylation can be carried out by a known method, for example, the method described in Theodora W. Greene, Peter G. M. Wuts, "Protective Groups in Organic Synthesis, 3rd Ed.," (1999) Wiley-Interscience, and the like, or an analogous method thereto. For example, the dealkylation can be carried out by treatment with an acid, a base, ultraviolet radiation, a transition metal catalyst and the like, or by oxidation, reduction or acylation followed by hydrolysis etc., or a combination thereof can be used. 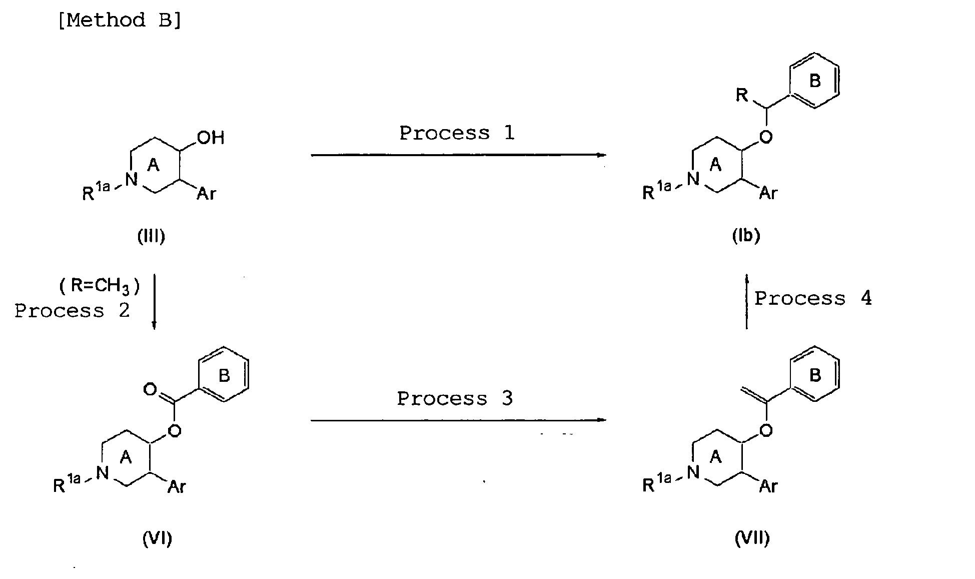 wherein each symbol is as defined above.

A compound represented by the formula (III) or a salt thereof can be prepared according to a method known per se, for example, the method described in WO 03/101964.

### (Process 1)

The present process is a process to prepare compound (Ib) by reacting a compound (III) with a compound represented by the formula: 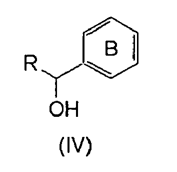 wherein each symbol is as defined above, a salt thereof or a reactive derivative thereof which is an alkylating agent.

The reactive derivative of the compound represented by (IV) or a salt thereof includes, for example, a compound represented by 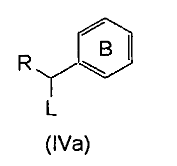 wherein each symbol is as defined above, (hereinafter to be simply referred to as the reactive derivative) or a salt thereof.

The reactive derivative includes, for example, halides (e.g., chloride, bromide, iodide etc.), sulfuric acid esters, or sulfonic acid esters (e.g., methanesulfonate, p-toluenesulfonate, benzenesulfonate etc.), carbonic esters, trichloroacetoimide acid esters, oxalates, phosphites (e.g., methyl phosphite etc.), phosphoranes and the like, and particularly halides or trichloroacetoimide acid esters are preferred. The amount of the reactive derivative to be used is, for example, 1 to 10 molar equivalents, preferably 1 to 5 molar equivalents, per 1 mole of the substrate.

The process can be carried out by reacting the reactive derivative usually in a solvent in the presence of acid or base. The solvent includes, for example, alcohols such as methanol, ethanol, propanol etc., hydrocarbons such as pentane, hexane, cyclohexane, heptane, benzene, toluene, xylene etc., halogenated hydrocarbons such as dichloromethane, chloroform etc., ethers such as dimethoxyethane, dioxane, tetrahydrofuran etc., ketones such as acetone etc., nitriles such as acetonitrile etc., amides such as N,N-dimethylformamide etc., sulfoxides such as dimethyl sulfoxide etc., water and the like, which may be used in a suitable mixture.

The acid includes mineral acids (e.g., hydrochloric acid, hydrobromic acid, sulfuric acid etc.), carboxylic acids (e.g., formic acid, acetic acid, propionic acid, trifluoroacetic acid etc.), sulfonic acids (e.g., methanesulfonic acid, trifluoromehtanesulfonic acid, p-toluenesulfonic acid etc.), Lewis acids (e.g., aluminum chloride, zinc chloride, zinc bromide, boron trifluoride, titanium chloride etc.), and particularly Lewis acids such as boron trifluoride and sulfonic acids such as trifluoromethanesulfonic acid etc are preferred.

The amount of the acid to be used is, for example, 1 to 100 molar equivalents, preferably 1 to 10 molar equivalents, per 1 mole of the substrate.

The base includes, for example, organic amines (e.g., alkylamines such as trimethylamine, triethylamine, diisopropylethylamine, N-methylmorpholine, 1,8-diazabicyclo[5.4.0]undeca-7-ene etc., aromatic amines such as pyridine, N,N-dimethylaniline etc.), alkali metal salts (e.g., sodium hydrogen carbonate, potassium hydrogen carbonate, sodium carbonate, potassium carbonate, sodium hydroxide, potassium hydroxide etc.), metal hydrides (e.g., potassium hydride, sodium hydride etc.), alkali metal alkoxides (e.g., sodium methoxide, sodium ethoxide, sodium tert-butoxide, potassium tert-butoxide etc.) and the like, and preferably, alkali metal salts such as sodium hydroxide etc., metal hydride such as sodium hydride etc. and the like.

The amount of the base to be used is, for example, about 1 to about 100 molar equivalents, preferably about 1 to about 10 molar equivalents, per 1 mole of the substrate.

If necessary, the reaction can be facilitated by adding an additive. Such an additive includes, for example, iodides such as sodium iodide, potassium iodide etc., a phase transfer catalyst such as tetra-n-butylammonium hydrogen sulfate, benzyltriethylammonium chloride etc., molecular sieves (e.g., molecular sieves 3A, 4A and 5A etc.), crown ethers (e.g., 18-crown-6,15-crown-5,12-crown-4 etc.) and the amount to be used is about 0.1 to 10 molar equivalents, preferably about 0.1 to 5 molar equivalents, per 1 mole of the substrate.

The reaction temperature is generally -10°C to 200°C, preferably about 0°C to 110°C, and the reaction time is generally 0.5 hr to 48 hr, preferably 0.5 hr to 16 hr.

### (Process 2)

The present process is a process to prepare compound (VI) by reacting the compound (III) or a salt thereof with a compound represented by the formula: 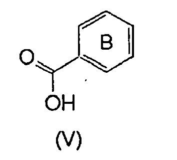 wherein each symbol is as defined above, a salt thereof or a reactive derivative thereof which is an acylating agent.

The reactive derivative of the compound (V) or a salt thereof includes, for example, a compound represented by 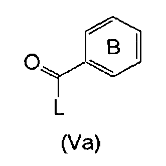 wherein each symbol is as defined above, or a salt thereof. For example, acid halide, acid anhydride, mixed acid anhydride, active ester and the like can be mentioned. Of these, acid halides and acid anhydrides are preferable.

The reaction using the above-mentioned reactive derivative, depends on the kinds of the reactive derivative or the substrate, but it can be usually carried out in a solvent. If necessary, a suitable base may be added to facilitate the reaction. The solvent includes, for example, hydrocarbons such as benzene, toluene etc., ethers such as ethyl ether, dioxane, tetrahydrofuran etc., esters such as ethyl acetate, halogenated hydrocarbons such as chloroform, dichloromethane etc., esters such as ethyl acetate etc., amides such as N,N-dimethylformamide etc., aromatic amines such as pyridine etc., water and the like, which may be used in a suitable mixture. In addition, the base includes, for example, alkali metal hydroxides such as sodium hydroxide, potassium hydroxide etc., hydrogen carbonates such as sodium hydrogen carbonate, potassium hydrogen carbonate etc., carbonates such as sodium carbonate, potassium carbonate etc., acetates such as sodium acetate etc., tertiary amines such as trimethylamine, triethylamine, N-methylmorpholine etc., aromatic amines such as pyridine, picoline, N,N-dimethylaniline etc. and the like. The amount of the base to be used is, for example, about 1 to about 100 molar equivalents, preferably about 1 to about 10 molar equivalents, per 1 mole of the substrate.

The amount of the reactive derivative to be used is usually 1 to 10 molar equivalents, preferably 1 to 3 molar equivalents, per 1 mole of the substrate. The reaction temperature is generally -10°C to 150°C, preferably about 0°C to 100°C, and the reaction time is generally 15 min to 24 hr, preferably 30 min to 16 hr.

### (Process 3)

The present process is a process to prepare compound (VII) by methylenation reaction of the compound (VI) or a salt thereof.

The methylenation reaction can be carried out by a method known per se. For example, the reaction can be carried out by reacting a Tebbe reagent (e.g., Cp₂Ti (Cl) CH₂Al (CH₃)₂, Cp₂Ti=CH₂ etc.), a Wittig reagent, a Grubbs reagent (e.g., TiCl₃/Zn/CH₂Cl₂ etc.) or the like are reacted with a compound represented by compound (VI) or a salt thereof in a solvent inert to the reaction, in the presence of a base. Particularly, use of a Tebbe reagent is preferable.

The amount of the Tebbe reagent to be used is generally 1 to 10 molar equivalents, preferably 1 to 5 molar equivalents, per 1 mol of the substrate.

As the solvent inert to the reaction, for example, aromatic hydrocarbons such as toluene, xylene etc., aliphatic hydrocarbons such as heptane, hexane etc., halogenated hydrocarbons such as chloroform, dichloromethane etc., ethers such as diethyl ether, tetrahydrofuran, dioxane etc., nitriles such as acetonitrile etc., amides such as N,N-dimethylformamide etc., sulfoxides such as dimethyl sulfoxide etc., and the like are used. These solvents may be used in a mixture at an appropriate ratio.

The base to be used includes, for example, tertiary amines such as trimethylamine, triethylamine, N-methylmorpholine etc., aromatic amines such as pyridine, picoline, N,N-dimethylaniline etc. The amount of the base to be used is, for example, about 1 to about 100 molar equivalents, preferably about 1 to about 10 molar equivalents, per 1 mole of the substrate.

The reaction temperature is generally -100°C to 150°C, preferably about -80°C to 50°C, and the reaction time is generally 15 min to 72 hr, preferably 30 min to 48 hr. (Process 4)

The present process is a process to prepare the compound (Ib, R=CH₃) or a salt thereof, by reducing the compound (VII).

The reduction reaction can be carried out by using a method known per se, but is usually carried out by catalytic hydrogenation.

The catalytic hydrogenation can be carried out under a hydrogen atmosphere and in the presence of a catalyst. The catalyst to be used is preferably palladium compounds such as palladium carbon, palladium hydroxide, palladium oxide etc., nickel compounds such as Raney-nickel etc., platinum compounds such as platinum oxide, platinum carbon etc., rhodium compounds such as rhodium acetate etc. and the like, and the amount to be used is about 0.001 to 5 equivalents, preferably about 0.01 to 5 equivalents. The catalytic hydrogenation is usually carried out in a solvent inert to the reaction. Such solvent includes, for example, alcohols such as methanol, ethanol, propanol, butanol etc.; hydrocarbons such as benzene, toluene, xylene etc.; halogenated hydrocarbons such as dichloromethane, chloroform etc.; ethers such as diethyl ether, dioxane, tetrahydrofuran etc.; esters such as ethyl acetate etc.; amides such as N,N-dimethylformamide etc.; carboxylic acids such as acetic acid etc.; water, or a mixture thereof. The hydrogen pressure under which the reaction is carried out, is generally about 1 to 50 atm, preferably about 1 to 10 atm. The reaction temperature is generally about 0°C to 150°C, preferably about 20°C to 100°C, and the reaction time is generally 5 min to 72 hr, preferably 0.5 hr to 40 hr. 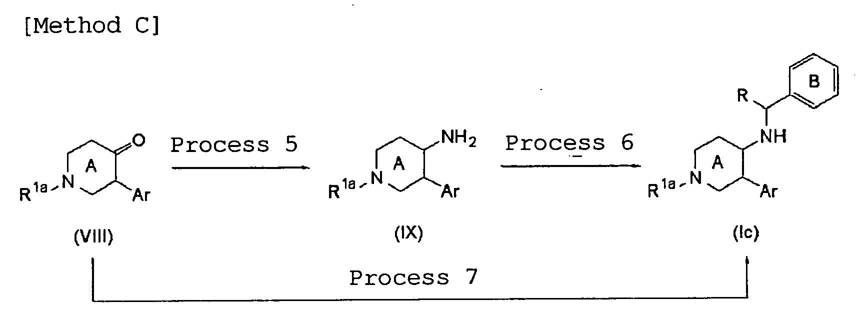 wherein each symbol is as defined above.

### (Process 5)

The present process is a process of converting a ketone of compound (VIII) to imine or oxime, followed by reducing it to thus prepare an amine compound (IX).

Conversion of compound (VIII) into imine or oxime can be carried out by using a known method, for example, according to the method described in WO 03/101964. For example, the reaction can be carried out by using various amines in a solvent inert to the reaction.

The amines include, for example, ammonia such as aqueous ammonia, ammonium chloride, ammonium acetate etc., hydroxyamines such as hydroxyamine, O-methylhydroxyamine, O-benzylhydroxyamine etc., and may be used as a salt form such as hydrochloride, sulfate etc. or as an aqueous solution thereof. The amount to be used of the amine is, for example, about 1 to 50 molar equivalents, preferably about 1 to 10 molar equivalents, per 1 mole of compound (VIII).

The solvent which is inert to the reaction includes, for example, aromatic hydrocarbons such as toluene, xylene etc., aliphatic hydrocarbons such as heptane, hexane etc., halogenated hydrocarbons such as chloroform, dichloromethane etc., ethers such as diethyl ether, tetrahydrofuran, dioxane etc., alcohols such as methanol, ethanol, 2-propanol, butanol, benzyl alcohol etc., nitriles such as acetonitrile etc., amides such as N,N-dimethylformamide etc., sulfoxides such as dimethyl sulfoxide etc., and the like. These solvents may be used in a mixture at a suitable ratio.

If necessary, the reaction can advantageously proceed by adding a catalyst. Such catalyst is preferably mineral acids (e.g., hydrochloric acid, hydrobromic acid, sulfuric acid etc.), carboxylic acids (e.g., formic acid, acetic acid, propionic acid, trifluoroacetic acid etc.), sulfonic acids (e.g., methanesulfonic acid, p-toluenesulfonic acid etc.), Lewis acids (e.g., aluminum chloride, zinc chloride, zinc bromide, boron trifluoride, titanium chloride etc.), acetate (e.g., sodium acetate, potassium acetate etc.), and molecular sieves (e.g., molecular sieves 3A, 4A, 5A, etc). The amount of the catalyst is, for example, about 0.01 to 50 molar equivalents, preferably about 0.1 to 10 molar equivalents, per 1 mole of compound (VIII).

The reaction temperature is generally about 0°C to 200°C, preferably about 20°C to 150°C, and the reaction time is generally 0.5 hr to 48 hr, preferably 0.5 hr to 24 hr.

The reduction of imine or iminium ion can be carried out by a method known per se, for example, a method using metal hydride or a method by catalytic hydrogenation.

The metal hydride as the reducing agent includes, for example, sodium borohydride, lithium borohydride, zinc borohydride, sodium cyanoborohydride, sodium triacetoxyborohydride, lithium cyanoborohydride, aluminum diisobutylhydride, aluminum hydride, lithium aluminum hydride, a borane complex (a borane-THF complex, catechol borane etc.) and the like. The metal hydride includes preferably sodium borohydride, sodium cyanoborohydride, sodium triacetoxyborohydride etc. The amount of the reducing agent to be used is, for example, 1 to 50 molar equivalents, preferably 1 to 10 molar equivalents, per 1 mole of the substrate. The solvent to be used for the reaction includes, for example, aromatic hydrocarbons such as toluene, xylene etc., aliphatic hydrocarbons such as heptane, hexane etc., halogenated hydrocarbons such as chloroform, dichloromethane etc., ethers such as diethyl ether, tetrahydrofuran, dioxane etc., alcohols such as methanol, ethanol, 2-propanol, butanol, benzyl alcohol etc., nitriles such as acetonitrile etc., amides such as N,N-dimethylformamide etc., sulfoxides such as dimethyl sulfoxide etc. and the like. These solvents may be used in a mixture at a suitable ratio. The reaction temperature is generally about -80°C to 80°C, preferably about -40°C to 40°C, and the reaction time is generally 5 min to 48 hr, preferably 1 hr to 24 hr.

The catalytic hydrogenation can be carried out under a hydrogen atmosphere and in the presence of a catalyst. The catalyst to be used is preferably palladium compounds such as palladium carbon, palladium hydroxide, palladium oxide etc., nickel compounds such as Raney-nickel etc., platinum compounds such as platinum oxide, platinum carbon etc., rhodium compounds such as rhodium acetate etc. and the like, and the amount to be used is about 0.001 to 1 equivalent, preferably about 0.01 to 0.5 equivalent. The catalytic hydrogenation is generally carried out in a solvent inert to the reaction. Such solvent includes, for example, alcohols such as methanol, ethanol, propanol, butanol etc.; hydrocarbons such as benzene, toluene, xylene etc.; halogenated hydrocarbons such as dichloromethane, chloroform etc.; ethers such as diethyl ether, dioxane, tetrahydrofuran etc.; esters such as ethyl acetate etc.; amides such as N,N-dimethylformamide etc.; carboxylic acids such as acetic acid etc.; water, or a mixture thereof. The hydrogen pressure under which the reaction is carried out, is generally about 1 to 50 atm, preferably about 1 to 10 atm. The reaction temperature is generally about 0°C to 150°C, preferably about 20°C to 100°C, and the reaction time is generally 5 min to 72 hr, preferably 0.5 hr to 40 hr.

In the present process, the compound (IX) can be also prepared directly from the compound (VIII) while carrying out the reactions of producing and reducing the above-mentioned imine or iminium ion at the same time, without isolating imine or iminium ion which is an intermediate. In this case, pH of the reaction mixture is preferably from about 4 to about 5.

### (Process 6)

In this step, the compound (IX) or a salt thereof is converted to compound (Ic) by subjecting a compound represented by the formula 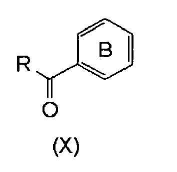 wherein the symbols in the formula are as defined above, or a salt thereof to a reductive alkylation reaction.

The reductive alkylation reaction can be carried out by a method known per se. For example, imine or iminium ion prepared from amine compound (IX) and a ketone compound (X) is subjected to a reduction reaction.

The reaction to prepare imine or iminium ion is generally carried out in a solvent that does not adversely influence the reaction. Such a solvent includes, for example, aromatic hydrocarbons such as toluene, xylene etc., aliphatic hydrocarbons such as heptane, hexane etc., halogenated hydrocarbons such as chloroform, dichloromethane etc., ethers such as diethyl ether, tetrahydrofuran, dioxane etc., alcohols such as methanol, ethanol, 2-propanol, butanol, benzyl alcohol etc., nitriles such as acetonitrile etc., amides such as N,N-dimethylformamide etc., sulfoxides such as dimethyl sulfoxide etc. and the like. Such a solvent may be used in a mixture at a suitable ratio.

If necessary, the reaction can be advantageously carried out by adding a catalyst. Such a catalyst is preferably mineral acids (e.g., hydrochloric acid, hydrobromic acid, sulfuric acid etc.), carboxylic acids (e.g., formic acid, acetic acid, propionic acid, trifluoroacetic acid etc.), sulfonic acids (e.g., methanesulfonic acid, p-toluenesulfonic acid etc.), Lewis acids (e.g., aluminum chloride, zinc chloride, zinc bromide, boron trifluoride, titanium chloride etc.), acetates (e.g. sodium acetate, potassium acetate etc.), molecular sieves (molecular sieves 3A, 4A, 5A, etc.) and the like. The amount of the catalyst to be used is, for example, about 0.01 to 50 molar equivalents, preferably about 0.1 to 10 molar equivalents, per 1 mole of compound (IIb).

The reaction temperature is generally about 0°C to 200°C, preferably about 20°C to 150°C, and the reaction time is generally 0.5 hr to 48 hr, preferably 0.5 hr to 24 hr.

The reduction of imine or iminium ion can be carried out by a method known per se, for example, a method using metal hydride or a method by catalytic hydrogenation.

The metal hydride as the reducing agent includes, for example, sodium borohydride, lithium borohydride, zinc borohydride, sodium cyanoborohydride, sodium triacetoxyborohydride, lithium cyanoborohydride, aluminum dibutylhydride, aluminum hydride, lithium aluminum hydride, a borane complex (a borane-THF complex, catechol borane etc.) and the like. The metal hydride preferably includes sodium borohydride, sodium cyanoborohydride, sodium triacetoxyborohydride etc. The amount of the reducing agent to be used is, for example, 1 to 50 molar equivalents, preferably 1 to 10 molar equivalents, per 1 mole of the substrate. In addition, the reaction solvent includes, for example, aromatic hydrocarbons such as toluene, xylene etc., aliphatic hydrocarbons such as heptane, hexane etc., halogenated hydrocarbons such as chloroform, dichloromethane etc., ethers such as diethyl ether, tetrahydrofuran, dioxane etc., alcohols such as methanol, ethanol, 2-propanol, butanol, benzyl alcohol etc., nitriles such as acetonitrile etc., amides such as N,N-dimethylformamide, etc., sulfoxides such as dimethyl sulfoxide etc. and the like. These solvents may be used in a mixture at a suitable ratio. The reaction temperature is generally about - 80°C to 80°C, preferably about -40°C to 40°C, and the reaction time is generally 5 min to 48 hr, preferably 1 hr to 24 hr.

The catalytic hydrogenation can be carried out under a hydrogen atmosphere and in the presence of a catalyst. The catalyst to be used is preferably palladium compounds such as palladium carbon, palladium hydroxide, palladium oxide etc., nickel compounds such as Raney-nickel etc., platinum compounds such as platinum oxide, platinum carbon etc., rhodium compounds such as rhodium acetate etc. and the like, and the amount to be used is about 0.001 to 1 equivalent, preferably about 0.01 to 0.5 equivalent. The catalytic hydrogenation is generally carried out in a solvent inert to the reaction. Such solvent includes, for example, alcohols such as methanol, ethanol, propanol, butanol etc.; hydrocarbons such as benzene, toluene, xylene etc.; halogenated hydrocarbons such as dichloromethane, chloroform etc.; ethers such as diethyl ether, dioxane, tetrahydrofuran etc.; esters such as ethyl acetate etc.; amides such as N,N-dimethylformamide etc.; carboxylic acids such as acetic acid etc.; water, or a mixture thereof. The hydrogen pressure under which the reaction is carried out, is generally about 1 to 50 atm, preferably about 1 to 10 atm. The reaction temperature is generally about 0°C to 150°C, preferably about 20°C to 100°C, and the reaction time is generally 5 min to 72 hr, preferably 0.5 hr to 40 hr.

In the present process, compound (Ic) can be also prepared directly from compound (IX) while carrying out the reactions to prepare imine or iminium ion and to reduce the product at the same time, without isolating imine or iminium ion which is an intermediate. In this case, pH of the reaction mixture is preferably from about 4 to about 5.

### (Process 7)

The present process is a process for converting compound (VIII) into compound (Ic) by reductive aminationThe present reaction can be carried out by the known method per se, for example, by reacting compound (VIII) with a compound represented by the formula: 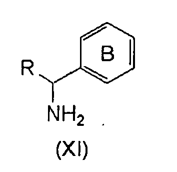 wherein each symbol is as defined above, a salt thereof or a reactive derivative thereof, and reducing the prepared imine or iminium ion.

The reaction to prepare imine or iminium ion and to reduce the product can be carried out by the same method described in the reductive alkylation of Process 6 in Method C.

In the present process, compound (Ic) can be also prepared directly from compound (VIII) while carrying out the reaction to prepare imine or iminium ion and to reduce the product at the same time, without isolating imine or iminium ion which.is an intermediate. In this case, pH of the reaction mixture is preferably from about 4 to about 5.

Compounds (Ia, Ib and Ic) obtained by the methods described in the above-mentioned Method A, Method B and Method C, can be further subjected to known reactions including condensation reactions such as various acylation, alkylation, or oxidation, reduction etc. to prepare further derivatives. Such reactions can be carried out according to a method known per se.

In each of the reactions for the synthesis of the objective compounds and the starting materials, when the starting compounds have an amino group, a carboxyl group or a hydroxy group as a substituent, such groups may be protected with the protecting groups which are generally used in peptide chemistry etc. In such a case, if necessary, such protecting groups can be removed to obtain the objective compounds after the reactions.

Such a protecting group includes, for example, protecting groups described in "Protective Groups in Organic Synthesis, 3rd Ed. (1999)", edited by Theodora W. Greene, Peter G. M. Wuts, published by Wiley-Interscience.

The protecting group for the amino group includes, for example, a formyl group, a C₁₋₆ alkyl-carbonyl group (e.g., an acetyl group, a propionyl group etc.), a phenylcarbonyl group, a C₁₋₆ alkyl-oxycarbonyl group (e.g., a methoxycarbonyl group, an ethoxycarbonyl group etc.), an aryloxycarbonyl group (e.g., a phenyloxycarbonyl group etc.), a C₇₋₁₀ aralkyl-carbonyl group (e.g., a benzyloxycarbonyl group etc.), a benzyl group, a benzhydryl group, a trityl group, a phthaloyl etc., each of which may have substituents. Such substituent includes, for example, a halogen atom (e.g., a fluorine atom, a chlorine atom, a bromine atom, an iodine atom etc.), a C₁₋₆ alkyl-carbonyl group (e.g., an acetyl group, a propionyl group, a butylcarbonyl group etc.), a nitro group and the like. The number of substituents is 1 to 3.

A protecting group for the carboxyl group includes, for example, a C₁₋₆ alkyl group (e.g., a methyl group, an ethyl group, a n-propyl group, an i-propyl group, a n-butyl group, a tert-butyl group etc.), a phenyl group, a trityl group, a silyl group and the like, each of which may have substituents. Such substituent includes, for example, a halogen atom (a fluorine atom, a chlorine atom, a bromine atom, an iodine atom), a formyl group, a C₁₋₆ alkyl-carbonyl group (e.g., an acetyl group, a propionyl group, a butylcarbonyl group etc.), a nitro group and the like. The number of substituent is 1 to 3.

The protecting group for the hydroxy group includes, for example, a C₁₋₆ alkyl group (e.g., a methyl group, an ethyl group, a n-propyl group, an i-propyl group, a n-butyl group, a tert-butyl group etc.), a phenyl group, a C₇₋₁₀ aralkyl group (e.g., a benzyl group etc.), a formyl group, a C₁₋₆ alkyl-carbonyl group (e.g., an acetyl group, a propionyl group etc.), an aryloxycarbonyl group (e.g., a phenyloxycarbonyl group etc.), a C₇₋₁₀ aralkyl-carbonyl group (e.g., a benzyloxycarbonyl group etc.), a pyranyl group, a furanyl group, a silyl group and the like, each of which may have substituents. Such a substituent includes, for example, a halogen atom (a fluorine atom, a chlorine atom, a bromine atom, an iodine atom etc.), a C₁₋₆ alkyl group, a phenyl group, a C₇₋₁₀ aralkyl group, a nitro group and the like. The number of substituents is 1 to 4.

Such protecting groups can be removed by a known deprotection method or the method described in "Protective Groups in Organic Synthesis, 3rd Ed. (1999)", edited by Theodora W. Greene, Peter G. M. Wuts, published by Wiley-Interscience, or an analogous method thereto. For example, treatment with an acid, a base, reduction, ultraviolet radiation, hydrazine, phenylhydrazine, sodium N-methyldithiocarbamate, tetrabutylammonium fluoride, palladium acetate or the like, can be used.

When compound (I) is obtained in a free formin the above-mentioned method, a salt with for example, inorganic acids (e.g., hydrochloric acid, sulfuric acid, hydrobromic acid etc.), organic acids (e.g., methanesulfonic acid, benzenesulfonic acid, toluenesulfonic acid, oxalic acid, fumaric acid, maleic acid, tartaric acid etc.), inorganic bases (e.g., alkali metals such as sodium, potassium etc., alkaline earth metals such as calcium, magnesium etc., aluminum, ammonium, and the like), or organic bases (e.g., trimethylamine, triethylamine, pyridine, picoline, ethanolamine, diethanolamine, triethanolamine, dicyclohexylamine, N,N'-dibenzylethylenediamine etc.) and the like can be prepared in a routine manner. When compound (I) is obtained in the form of a salt, the compound can be converted to a free compound or another salt in a routine manner.

In addition, when the starting compound may form a salt in each of the above-mentioned reactions, the compound may be used as a salt. Such salt includes, for example, those exemplified as a salt of compound (I).

Compound (I) of the present invention thus prepared by such methods, can be isolated and purified by a typical separation means such as recrystallization, distillation, chromatography etc.

When compound (I) contains an optical isomer, a stereoisomer, a regioisomer or a rotamer, these are also included in compound (I), and can be obtained as a single product according to synthesis and separation methods known per se (for example, concentration, solvent extraction, column chromatography, recrystallization etc.). For example, when compound (I) has an optical isomer, the optical isomer resolved from this compound is also included in compound (I).

The optical isomer can be prepared by a method known per se. To be specific, an optically active synthetic intermediate is used, or the final racemate product is subjected to optical resolution according to a conventional method to give an optical isomer.

The method of optical resolution may be a method known per se, such as a fractional recrystallization method, a chiral column method, a diastereomer method etc.

### 1) Fractional recrystallization method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine etc.) is formed, which is separated by a fractional recrystallization method, and if desired, a free optical isomer is obtained by a neutralization step.

### 2) Chiral column method

A method wherein a racemate or a salt thereof is applied to a column for separation of an optical isomer (a chiral column) to allow separation. In the case of a liquid chromatography, for example, a mixture of the optical isomers is applied to a chiral column such as ENANTIO-OVM (manufactured by Tosoh Corporation), CHIRAL series (manufactured by Daicel Chemical Industries, Ltd.) and the like, and developed with water, various buffers (e.g., phosphate buffer) and organic solvents (e.g., ethanol, methanol, isopropanol, acetonitrile, trifluoroacetic acid, diethylamine etc.) solely or in admixture to separate the optical isomer. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (e.g., a fractional recrystallization method, a chromatography method etc.) and the like, and is subjected to a chemical treatment such as hydrolysis and the like to separate an optically active reagent moiety, whereby an optical isomer is obtained. For example, when compound (I) contains hydroxy, or primary or secondary amino group within a molecule, the compound and an optically active organic acid (e.g., MTPA [α-methoxy-α-(trifluoromethyl)phenylacetic acid], (-)-menthoxyacetic acid etc.) and the like are subjected to condensation reaction to give diastereomers of the ester compound or in the amide compound, respectively. When compound (I) has a carboxylic acid group, this compound and an optically active amine or an alcohol reagent are subjected to condensation reaction to give diastereomers of the ester compound or the amide compound, respectively. The separated diastereomer is converted to an optical isomer of the original compound by acid hydrolysis or base hydrolysis.

Compound (I) or a salt thereof may be in the form of a crystal.

The crystal of compound (I) or a salt thereof (hereinafter, it may be referred to as crystal of the present invention) can be prepared by crystallization of compound (I) or a salt thereof by a crystallization method known per se.

Examples of the crystallization method include a method of crystallization from a solution, a method of crystallization from vapor, a method of crystallization from the melts and the like.

The "crystallization from a solution" is typically a method of shifting a non-saturated state to supersaturated state by varying factors involved in solubility of compounds (solvent composition, pH, temperature, ionic strength, redox state etc.) or the amount of solvent. To be specific, for example, a concentration method, a cold removing method, a reaction method (a diffusion method, an electrolysis method), a hydrothermal growth method, a flux method and the like can be mentioned. Examples of the solvent to be used include aromatic hydrocarbons (e.g., benzene, toluene, xylene etc.), halogenated hydrocarbons (e.g., dichloromethane, chloroform etc.), saturated hydrocarbons (e.g., hexane, heptane, cyclohexane etc.), ethers (e.g., diethyl ether, diisopropyl ether, tetrahydrofuran, dioxane etc.), nitriles (e.g., acetonitrile etc.), ketones (e.g., acetone etc.), sulfoxides (e.g., dimethyl sulfoxide etc.), acid amides (e.g., N,N-dimethylformamide etc.), esters (e.g., ethyl acetate etc.), alcohols (e.g., methanol, ethanol, isopropyl alcohol etc.), water and the like. These solvents are used alone or in a combination of two or more at a suitable ratio (e.g., 1:1 to 1:100 (a volume ratio)).

The "crystallization from vapor" is, for example, a vaporization method (a sealed tube method, a gas stream method), a gas phase reaction method, a chemical transportation method and the like.

The "crystallization from the melts" is, for example, a normal freezing method (a Czockralski method, a temperature gradient method and a Bridgman method), a zone melting method (a zone leveling method and a floating zone method), a special growth method (a VLS method and a liquid phase epitaxy method) and the like.

Preferable examples of the crystallization method include a method of dissolving compound (I) or a salt thereof in a suitable solvent (e.g., alcohols such as methanol, ethanol etc. and the like) at a temperature of 20 to 120°C, and cooling the resulting solution to a temperature not higher than the temperature of dissolution (e.g., 0 to 50°C, preferably 0 to 20°C) and the like.

The thus obtained crystals of the present invention can be isolated, for example, by filtration and the like.

In the present specification, the specific rotation ([α]_{D}) means that measured using, for example, polarimeter (JASCO Corporation (JASCO), P-1030 polarimeter (No.AP-2)) and the like.

In the present specification, the melting point means that measured using, for example, a micromelting point apparatus (Yanako, MP-500D) or a DSC (differential scanning calorimetry) device (SEIKO, EXSTAR 6000) and the like.

In the present specification, the peak by powder X-ray diffraction means that measured using, for example, RINT2100 (Rigaku Corporation) etc. with a Cu-Kα1 ray (tube voltage: 40 KV; tube current: 50 mA) as a ray source.

In general, the melting points and the peak by powder X-ray diffraction may vary depending on the measurement apparatuses, the measurement conditions and the like. The crystal in the present specification may show different values from the melting point or the peak by powder X-ray diffraction described in the present specification, as long as it is within each of a general error range.

The crystal of the present invention is superior in physicochemical properties (e.g., melting point, solubility, stability etc.) and biological properties (e.g., pharmacokinetics (absorption, distribution, metabolism, excretion), efficacy expression etc.), and thus it is extremely useful as a pharmaceutical composition.

Compound (I) or a salt or a prodrug thereof of the present invention (hereinafter, it may be briefly referred to as the compound of the present invention) has excellent antagonistic action for a tachykinin receptor, particularly Substance P receptor antagonistic action, neurokinin A receptor antagonistic action, in addition to inhibitory action for the increased permeability of blood vessel of a trachea induced by capsaicin. The compound of the present invention has low toxicity and thus it is safe.

Accordingly, the compounds of the present invention having excellent antagonistic actions for Substance P receptors and neurokinin A receptors etc. can be used as a safe pharmaceutical composition for preventing and treating the following diseases related to Substance P in mammals (e.g., mice, rats, hamsters, rabbits, cats, dogs, bovines, sheep, monkeys, humans etc.).
(1) Lower urinary tract abnormalities [for example, overactive bladder, interstitial cystitis, abnormal urination [for example, urinary frequency, urinary incontinence, urinary urgency due to overactive bladder, hypotonic bladder accompanied by overactive bladder), pelvic visceral pain etc.]
(2) Digestive organ diseases [for example, irritable bowel syndrome, ulcerative colitis syndrome, Crohn's disease, diseases caused by a spiral urease-positive gram-negative bacterium (e.g., *Helicobacter pylori* etc.) (e.g., gastritis, gastric ulcer etc.), gastric cancer, postgastrostomy disorder, dyspepsia, esophageal ulcer, pancreatitis, polyp of the colon, cholelithiasis, hemorrhoids, peptic ulcer, situational ileitis, vomiting, nausea etc.]
(3) Inflammatory or allergic diseases [for example, inflammatory bowel disease, allergic rhinitis, conjunctivitis, gastrointestinal allergy, pollinosis, anaphylaxis, dermatitis, herpes, psoriasis, bronchitis, expectoration, retinopathy, postoperative and posttraumatic inflammation, regression of puffiness, pharyngitis, cystitis, meningitidis, inflammatory ophthalmic diseases etc.]
(4) Osteoarthropathy diseases [for example, rheumatoid arthritis (chronic rheumatoid arthritis), arthritis deformans, rheumatoid myelitis, osteoporosis, abnormal growth of cells, bone fracture, bone refracture, osteomalacia, osteopenia, osseous Behcet's disease, rigid myelitis, articular tissue destruction by gonarthrosis deformans and similar diseases thereto etc.]
(5) Respiratory diseases [for example, cold syndrome, pneumonia, asthma, pulmonary hypertension, pulmonary thrombi/pulmonary obliteration, pulmonary sarcoidosis, pulmonary tuberculosis, interstitial pneumonia, silicosis, adult tachypnea syndrome, chronic obliterative pulmonary diseases, cough etc.]
(6) Infectious diseases [HIV infectious diseases, virus infectious diseases due to cytomegalo virus, influenza virus, herpes virus and the like, rickettsia infectious diseases, bacterial infectious diseases, sexually-transmitted diseases, carinii pneumonia, *helicobacter pylori* infectious disease, systemic fungal infectious diseases, tuberculosis, invasive staphylococcal infectious diseases, acute viral encephalitis, acute bacterial meningitidis, AIDS encephalitis, septicemia, sepsis, sepsis gravis, septic shock, endotoxin shock, toxic shock syndromes etc.]
(7) Cancers [for example, primary, metastatic or recurrent breast cancer, prostatic cancer, pancreatic cancer, gastric cancer, lung cancer, colorectal cancer (colon cancer, rectal cancer, anal cancer), esophagus cancer, duodenal cancer, head and neck cancer (tongue cancer, pharynx cancer, larynx cancer), brain tumor, neurinoma, non-small cell lung cancer, small cell lung cancer, hepatic cancer, renal cancer, colic cancer, uterine cancer (cancer of the uterine body, uterine cervical cancer), ovarian cancer, bladder cancer, skin cancer, hemangioma, malignant lymphoma, malignant melanoma, thyroid cancer, bone tumor, hemangioma, angiofibroma, retinosarcoma, penis cancer, pediatric solid cancer, Kaposi's sarcoma, Kaposi's sarcoma caused by AIDS, tumor of the maxillary sinus, fibrous histiocytoma, smooth muscle sarcoma, rhabdomyosarcoma, liposarcoma, fibroid tumors of the uterus, osteoblastoma, osteosarcoma, chondrosarcoma, carcinomatous mesothelial tumor, tumors such as leukemia, Hodgkin's disease etc.]
(8) Central nerve diseases [for example, neurodegenerative diseases (e.g., Alzheimer's disease, Down's disease, Parkinson's disease, Creutzfeldt-Jakob's disease, amyotrophic lateral sclerosis (ALS), Huntington chorea, diabetic neuropathy, multiple sclerosis etc.), mental diseases (e.g., schizophrenia, depression, mania, anxiety neurosis, obsessive-compulsive neurosis, panic disorder, epilepsy, alcohol dependence, anxiety symptom, anxious mental state etc.), central and peripheral nerve disorders (e.g., head trauma, spinal cord injury, brain edema, disorders of sensory function, abnormality of sensory function, disorders of autonomic nervous function and abnormality of autonomic nervous function, whiplash injury etc.), memory disorders (e.g., senile dementia, amnesia, cerebrovascular dementia etc.), cerebrovascular disorders (e.g., disorders and aftereffect and/or complication from intracerebral hemorrhage, brain infarction, etc, asymptomatic cerebro-vascular accident, transient cerebral ischemic attack, hypertensive encephalopathia, blood-brain barrier disorder etc.), recurrence and aftereffect of cerebro-vascular accident (neural symptoms, mental symptoms, subjective symptoms, disorders of daily living activities etc.), post-cerebrovascular occlusion central hypofunction; disorder or abnormality of cerebral circulation and/or autoregulation of renal circulation]
(9) Circulatory diseases [for example, acute coronary artery syndromes (e.g., acute cardiac infarction, unstable angina etc.), peripheral arterial obstruction, Raynaud's disease; Buerger disease; restenosis after coronary-artery intervention (percutaneous transluminal coronary angioplasty (PTCA), directional coronary atherectomy (DCA), stenting etc.), restenosis after coronary-artery bypass operation, restenosis after intervention (angioplasty, atherectomy, stenting etc.) or bypass operation in other peripheral artery, ischemic cardiac diseases (e.g., cardiac infarction, angina etc.), myocarditis, intermittent claudication, lacunar infarction, arteriosclerosis (e.g., atherosclerosis etc.), cardiac failure (acute cardiac failure, chronic cardiac failure accompanied by congestion), arrhythmia, progress of atherosclerotic plaque, thrombosis, hypertension, hypertensive tinnitus; hypotension etc.]
(10) Pains [e.g., migraine, neuralgia etc.]
(11) Autoimmune diseases [for example, collagen disease, systemic lupus erythematosus, scleroderma, polyarteritis, myasthenia gravis, multiple sclerosis, Sjogren's syndrome, Behcet's disease etc.]
(12) Hepatic diseases [e.g., hepatitis (including chronic hepatitis), cirrhosis, interstitial hepatic diseases etc.]
(13) Pancreatic diseases [e.g., pancreatitis (including chronic pancreatitis) etc.]
(14) Renal diseases [e.g., nephritis, glomerulonephritis, glomerulosclerosis, renal failure, thrombotic microangiopathy, dialysis complications, organ disorders including nephropathia by radiation, diabetic nephropathia etc.]
(15) Metabolic diseases [e.g., diabetic diseases (insulin-dependent diabetes, diabetic complications, diabetic retinopathy, diabetic microangiopathy, diabetic neuropathy etc.); glucose tolerance abnormality, obesity, prostatomegaly, sexual dysfunction etc.]
(16) Endocrine diseases [e.g., Addison's disease, Cushing's syndrome, melanocytoma, primary aldosteronism etc.]
(17) Other diseases
   (A) Transplant rejection [e.g., posttransplantational rejection, posttransplantational polycythemia, hypertension, organ disorder and/or vascular hypertrophy, graft-versus-host disease etc.]
   (B) Abnormality in characteristic of blood and/or blood components [e.g., enhancement in platelet aggregation, abnormality of erythrocyte deformability, enhancement in leukocyte adhesiveness, increase in blood viscosity, polycythemia, vascular peliosis, autoimmune hemolytic anemia, disseminated intravascular coagulation syndrome (DIC), multiple myelopathy etc.]
   (C) Gynecologic diseases [e.g., climacteric disorder, gestational toxicosis, endometriosis, hysteromyoma, ovarian disease, mammary disease etc.]
   (D) Dermatic diseases [e.g., keloid, angioma, psoriasis, pruritus etc.]
   (E) Ophthalmic diseases [e.g., glaucoma, ocular hypertension disease etc.]
   (F) Otolaryngological diseases [e.g., Menuel syndrome, tinnitus, gustation disorder, dizziness, disequilibrium, dysphagia etc.]
   (G) Diseases due to environmental and/or occupational factors (e.g., radiation disorder, disorders by ultraviolet ray·infrared ray·laser ray, altitude sickness etc.)
   (H) Ataxia
   (I) Chronic fatigue syndrome

The compounds of the present invention are particularly useful as a tachykinin receptor antagonist and as an agent for ameliorating abnormality of lower urinary tract functions such as urinary frequency, urinary incontinence etc., and even as a therapeutic drug for treating gastrointestinal diseases such as irritable bowel disease, ulcerative colitis and the like.

Furthermore, MK-869 having the following chemical structural formula (5-[[(2R,3S)-2-[(1R)-1-[3,5-bis(trifluoromethyl)phenyl]ethoxy]-3-(4-fluorophenyl)-4-morpholinyl]methyl]-1,2-dihydro-3H-1,2,4-triazol-3-one; (Aprepitant) has bladder capacity increasing activity, and is therefore useful as an agent for the prophylaxis or treatment of the above-mentioned diseases.

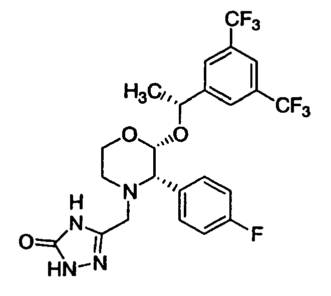

Pharmaceutical preparations comprising the compound of the present invention or the above-mentioned MK-869 may be in any solid forms of powders, granules, tablets, capsules, suppositories etc., and in any liquid forms of syrups, emulsions, injections, suspensions etc.

The pharmaceutical preparations of the present invention can be produced by any conventional methods, for example, blending, kneading, granulation, tabletting, coating, sterilization, emulsification etc., in accordance with the forms of the preparations to be produced. For the production of such pharmaceutical preparations, for example, each of the items in General Principles for pharmaceutical preparations in the Japanese Pharmacopeia, can be made reference to. In addition, the pharmaceutical preparations of the present invention may be formulated into a sustained release preparation containing active ingredients and biodegradable polymer compounds. The sustained release preparation can be produced according to the method described in JP-A-9-263545.

In the pharmaceutical preparations of the present invention, the content of the compound or a salt thereof in the present invention varies depending on the forms of the preparations, but is generally about 0.01 to 100% by weight, preferably about 0.1 to 50% by weight, more preferably 0.5 to 20% by weight, relative to the total weight of each preparation.

When the compound of the present invention is used in the above-mentioned pharmaceutical preparations, it may be used alone, or in admixture with a suitable, pharmaceutically acceptable carrier, for example, excipients (e.g., starch, lactose, sucrose, calcium carbonate, calcium phosphate etc.), binders (e.g., starch, arabic gum, carboxymethyl cellulose, hydroxypropyl cellulose, crystalline cellulose, alginic acid, gelatin, polyvinyl pyrrolidone etc.), lubricants (e.g., stearic acid, magnesium stearate, calcium stearate, talc etc.), disintegrants (e.g., calcium carboxymethylcellulose, talc etc.), diluents (e.g., water for injection, physiological saline etc.) and if desired, with the additives (e.g., a stabilizer, a preservative, a colorant, a fragrance, a dissolution aid, an emulsifier, a buffer, an isotonic agent etc.) and the like, by ordinary methods. It can be formulated into the solid preparations such as powders, fine granules, granules, tablets, capsules etc., or into the liquid preparations such as injections etc., and can be administered orally or parenterally.

While the content of a pharmaceutically acceptable carrier in the pharmaceutical composition of the preparation of the present invention varies depending on the form of the preparation, it is generally about 0-99.99 wt%, preferably about 1-90 wt%, more preferably about 10-90 wt%, relative to the whole preparation.

The dose of the pharmaceutical preparation of the present invention varies depending on the kinds of the compound of the present invention or a pharmaceutically acceptable salt thereof, the administration route, the condition and the age of patients etc. For example, the dose for oral administration of the pharmaceutical preparation to an adult patient suffering from abnormal urination is generally from about 0.005 to 50 mg/kg body/day, preferably from about 0.05 to 10 mg/kg body/day, more preferably from about 0.2 to 4 mg/kg body/day, based on the compound of the present invention, which may be administered once a day or in two or three divided portions a day.

The dose when the pharmaceutical composition of the present invention is a sustained release preparation varies depending on the kinds and the content of compound (I) or a salt thereof, the formulation, the duration time of drug release, the animals to be administered (e.g., mammals such as humans, rats, mice, cats, dogs, rabbits, bovines, swines etc.), and the object of administration. For example, when it is parenterally administered, preferably about 0.1 to about 100 mg of compound (I) or a salt thereof is released from the preparation for 1 week.

The compound of the present invention can be used in a mixture or combination with other pharmaceutically active ingredients at a suitable ratio.

Combination of the compound of the present invention with other pharmaceutically active ingredients can give the following excellent effects:
(1) a dose can be reduced as compared with separate administration of the compound of the present invention or other pharmaceutically active ingredients. More specifically, when the compound of the present invention is combined with anticholinergic agents or NK-2 receptor antagonists, the dose can be reduced as compared with separate administration of anticholinergic agents or NK-2 receptor antagonists, and therefore, side effects such as dry mouth can be reduced;
(2) according to symptoms of patient (mild symptoms, severe symptoms etc.), a drug to be combined with the compound of the present invention can be selected;
(3) by choosing other pharmaceutically active ingredients which have different mechanism of action from that of the compound of the present invention, the therapeutic period can be designed longer;
(4) by choosing other pharmaceutically active ingredients which have different mechanism of action from that of the compound of the present invention, continuation of therapeutic effects can be obtained; and
(5) by combining the compound of the present invention and other pharmaceutically active ingredients, excellent effects such as synergic effects can be obtained.

A drug which is mixed or combined with the compound of the present invention (hereinafter, briefly referred to as combination drugs) includes the following:

### (1) Agent for treating diabetes

Insulin preparations (e.g., animal insulin preparations extracted from the bovine or swine pancreas; human insulin preparations synthesized by a genetic engineering technique using *Escherichia coli* or a yeast; insulin zinc; protamine zinc insulin; a fragment or a derivative of insulin (e.g., INS-1 etc.), agents for potentiating insulin sensitivity (e.g., pioglitazone hydrochloride, troglitazone, rosiglitazone or its maleate, JTT-501, MCC-555, YM-440, GI-262570, KRP-297, FK-614, CS-011 etc.), α-glucosidase inhibitors (e.g., voglibose, acarbose, miglitol, emiglitate etc.), biguanides (e.g., phenformin, metformin, buformin etc.), sulfonylureas (e.g., tolbutamide, glibenclamide, gliclazide, chlorpropamide, tolazamide, acetohexamide, glyclopyramide, glimepiride etc.) and other insulin secretagogues (e.g., repaglinide, senaglinide, mitiglinide or its calcium salt hydrate, GLP-1, nateglinide etc.), dipeptidylpeptidase IV inhibitors (e.g., NVP-DPP-278, PT-100, P32/98 etc.), β3 agonists (e.g., CL-316243, SR-58611-A, UL-TG-307, AJ-9677, AZ40140 etc.), amylin agonists (e.g., pramlintide etc.), phosphotyrosine phosphatase inhibitors (e.g., vanadic acid etc.), gluconeogenesis inhibitors (e.g., glycogen phosphorylase inhibitors, glucose-6-phosphatase inhibitors, glucagon antagonists etc.), SGLT (sodium-glucose cotransporter) inhibitors (e.g., T-1095 etc.) and the like.

### (2) Agent for treating diabetic complications

Aldose reductase inhibitors (e.g., tolrestat, epalrestat, zenarestat, zopolrestat, fidarestat (SNK-860), minalrestat (ARI-509), CT-112 etc.), neurotrophic factors (e.g., NGF, NT-3 etc.), AGE inhibitors (e.g., ALT-945, pimagedine, pyratoxathine, N-phenacylthiazolium bromide (ALT-766), EXO-226 etc.), active oxygen scavengers (e.g., thioctic acid etc.), cerebral vasodilators (e.g., tiapuride etc.) and the like.

### (3) Antihyperlipidemic agent

Statin compounds inhibiting cholesterol synthesis (e.g., pravastatin, simvastatin, lovastatin, atorvastatin, fluvastatin, cerivastatin or their salt (e.g., sodium salt etc.) and the like), squalene synthase inhibitors or fibrate compounds having triglyceride lowering action (e.g., bezafibrate, clofibrate, simfibrate, clinofibrate etc.) and the like.

### (4) Hypotensive agent

Angiotensin converting enzyme inhibitors (e.g., captopril, enalapril, delapril etc.), angiotensin II antagonists (e.g., losartan, candesartan cilexetil etc.), calcium antagonists (e.g., manidipine, nifedipine, amlodipine, efonidipine, nicardipine etc.), clonidine, and the like.

### (5) Antiobesity agent

Antiobesity drugs acting on the central nervous system (e.g. dexfenfluramine, fenfluramine, phentermine, sibutramine, anfepramone, dexamphetamine, mazindol, phenylpropanolamine, clobenzorex etc.), pancreatic lipase inhibitors (e.g. orlistat etc.), β₃ agonists (e.g. CL-316243, SR-58611-A, UL-TG-307, AJ-9677, AZ40140 etc.), anorectic peptides (e.g. leptin, CNTF (Ciliary Neurotrophic Factor) etc.), cholecystokinin agonists (e.g. lintitript, FPL-15849 etc.).

### (6) Diuretic agent

Xanthine derivatives (e.g., theobromine sodium salicylate, theobromine calcium salicylate etc.), thiazide preparations (e.g., ethiazide, cyclopenthiazide, trichlormethiazide, hydrochlorothiazide, hydroflumethiazide, benzylhydrochlorothiazide, penflutizide, polythiazide, methyclothiazide etc.), antialdosterone preparations (e.g., spironolactone, triamterene etc.), carbonic anhydrase inhibitors (e.g., acetazolamide etc.), chlorobenzenesulfonamide preparations (e.g., chlorthalidone, mefruside, indapamide etc.), azosemide, isosorbide, ethacrynic acid, piretanide, bumetanide, furosemide etc.

### (7) Chemotherapeutic agent

Alkylating agents (e.g., cyclophosphamide, ifosfamide etc.), metabolic antagonists (e.g., methotrexate, 5-fluorouracil etc.), antitumor antibiotics (e.g., mitomycin, adriamycin etc.), plant-derived antitumor agents (e.g., vincristine, vindesine, taxol etc.), cisplatin, carboplatin, etoposide etc. Among these, 5-fluorouracil derivatives such as Furtulon and Neo-Furtulon are preferred.

### (8) Immunotherapeutic agent

Microorganism- or bacterium-derived components (e.g., muramyl dipeptide derivatives, Picibanil etc.), immunopotentiator polysaccharides (e.g., lentinan, schizophyllan, krestin etc.), genetically engineered cytokines (e.g., interferons, interleukins (IL) etc.), colony stimulating factors (e.g., granulocyte colony stimulating factor, erythropoietin etc.) and the like. Among these, interleukins such as IL-1, IL-2, IL-12 etc. are preferred.

### (9) Therapeutic agent recognized to ameliorate cachexia in animal models or clinical practice

Progesterone derivatives (e.g., megestrol acetate) [Journal of Clinical Oncology, vol. 12, pp. 213-225, 1994], metoclopramide pharmaceuticals, tetrahydrocannabinol pharmaceuticals (the above reference is applied to both), fat metabolism ameliorating agents (e.g., eicosapentanoic acid) [British Journal of Cancer, vol. 68, pp. 314-318, 1993], growth hormones, IGF-1, and antibodies to the cachexia-inducing factors such as TNF-α, LIF, IL-6 and oncostatin M.

### (10) Antiinflammatory agent

Steroids (e.g., dexamethasone etc.), sodium hyaluronate, cyclooxygenase inhibitors (e.g., indomethacin, ketoprofen, loxoprofen, meloxicam, ampiroxicam, celecoxib, rofecoxib etc.) and the like.

### (11) Miscellaneous

Glycosylation inhibitors (e.g., ALT-711 etc.), nerve regeneration promoting drugs (e.g., Y-128, VX853, prosaptide etc.), drugs acting on the central nervous system (e.g., antidepressants such as desipramine, amitriptyline, imipramine, fluoxetine, paroxetine, doxepin etc.), anticonvulsants (e.g., lamotrigine, carbamazepine), antiarrhythmic drugs (e.g., mexiletine), acetylcholine receptor ligands (e.g., ABT-594), endothelin receptor antagonists (e.g., ABT-627), monoamine uptake inhibitors (e.g., tramadol), indoleamine uptake inhibitors (e.g., fluoxetine, paroxetine), narcotic analgesics (e.g., morphine), opioid receptor complete agonist (e.g., pentazocine), opioid receptor partial agonist (e.g., buprenorphine, axomadol), γ-aminobutyric acid (GABA) receptor agonists, GABA uptake inhibitors (e.g., tiagabine), α₂ receptor agonists (e.g., clonidine), local analgesics (e.g., capsaicin), protein kinase C inhibitors (e.g., LY-333531), antianxiety drugs (e.g., benzodiazepines), phosphodiesterase inhibitors (e.g., sildenafil), dopamine receptor agonists (e.g., apomorphine), dopamine receptor antagonists (e.g., haloperidol), serotonin receptor agonists (e.g., tandospirone citrate, sumatryptan), serotonin receptor antagonists (e.g., cyproheptadine hydrochloride, ondansetron), serotonin uptake inhibitors (e.g., fluvoxamine maleate, fluoxetine, paroxetine), serotonin noradrenalin uptake inhibitor (e.g., duloxetine, venlafaxine), hypnotics (e.g., triazolam, zolpidem), anticholinergic agents, α₁ receptor blocking agents (e.g., tamsulosin, alfuzosin,silodosin), muscle relaxants (e.g., baclofen etc.), potassium channel openers (e.g., nicorandil), calcium channel blocking agents (e.g., nifedipine, gabapentin), agents for preventing and/or treating Alzheimer's disease (e.g., donepezil, rivastigmine, galanthamine), agents for treating Parkinson's disease (e.g., L-dopa), agents for preventing and/or treating multiple sclerosis (e.g., interferon β-1a), histamine H₁ receptor inhibitors (e.g., promethazine hydrochloride), proton pump inhibitors (e.g., lansoprazole, omeprazole), antithrombotic agents (e.g., aspirin, cilostazol), NK-2 receptor antagonists, agents of treating HIV infection (saquinavir, zidovudine, lamivudine, nevirapine), agents of treating chronic obstructive pulmonary diseases (salmeterol, thiotropium bromide, cilomilast) etc.

Anticholinergic agents include, for example, atropine, scopolamine, homatropine, tropicamide, cyclopentolate, butylscopolamine bromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, trospium chloride or a salt thereof (e.g., atropine sulfate, scopolamine hydrobromide, homatropine hydrobromide, cyclopentolate hydrochloride, flavoxate hydrochloride, pirenzepine hydrochloride, trihexyphenidyl hydrochloride, oxybutynin chloride, tolterodine tartrate etc.), preferably, oxybutynin, propiverine, darifenacin, tolterodine, temiverine, trospium chloride or a salt thereof (e.g., oxybutynin chloride, tolterodine tartrate etc.). In addition, acetylcholinesterase inhibitors (e.g., distigmine etc.) and the like can be used.

NK-2 receptor antagonists include, for example, a piperidine derivative such as GR159897, GR149861, SR48968 (saredutant), SR144190, YM35375, YM38336, ZD7944, L-743986, MDL105212A, ZD6021, MDL105172A, SCH205528, SCH62373, R-113281 etc., a perhydroisoindole derivative such as RPR-106145 etc., a quinoline derivative such as SB-414240 etc., a pyrrolopyrimidine derivative such as ZM-253270 etc., a pseudopeptide derivative such as MEN11420 (nepadutant), SCH217048, L-659877, PD-147714 (CAM-2291), MEN10376, S16474 etc., and others such as GR100679, DNK333, GR94800, UK-224671, MEN10376, MEN10627, or a salt thereof, and the like.

The pharmaceutical composition comprising a mixture or combination of the compound of the present invention and the concomitant drugs may be formulated into
(1) a single formulation as a pharmaceutical composition containing the compound of the present invention and the concomitant drugs, or
(2) a formulation comprising the compound of the present invention and the concomitant drugs which are separately formulated. Hereinafter, it is generally briefly referred to as the combination preparation of the present invention.

The combination preparation of the present invention can be formulated by mixing the compound of the present invention and active ingredients of the concomitant drugs separately or at the same time as itself or with pharmaceutically acceptable carriers in the same manner as in the method of producing the pharmaceutical preparation comprising the compound of the present invention.

In the concomitant agent of the present invention simultaneously containing the compound of the present invention and a concomitant drug, the contents of the compound of the present invention and the concomitant drug vary depending on the forms of the preparation, but are usually about 0.01 to 99.9 wt%, preferably about 0.1 to 50 wt%, and further preferably about 0.5 to 20 wt%, respectively, relative to the total preparation.

In the concomitant agent of the present invention simultaneously containing the compound of the present invention and a concomitant drug, while the content of the pharmaceutically acceptable carrier varies depending on the form of the preparation, it is generally about 0 to 99.98 wt%, preferably about 1 to 90 wt%, more preferably about 10 to 90 wt%, relative to the whole preparation.

In the concomitant agent of the present invention separately containing the compound of the present invention and a concomitant drug, while the contents of the compound of the present invention and concomitant drug vary depending on the form of the preparation, it is generally about 0.01 to 99.9 wt%, preferably about 0.1 to 50 wt%, more preferably about 0.5 to 20 wt%, respectively, relative to the whole preparation.

In the concomitant agent of the present invention separately containing the compound of the present invention and concomitant drug, while the content of the pharmaceutically acceptable carrier varies depending on the form of the preparation, it is generally about 0 to 99.99 wt%, preferably about 1 to 90 wt%, more preferably about 10 to 90 wt%, relative to the whole preparation.

The dose of the compound or the combination preparation of the present invention may be set within the range such that it causes no problems of side effects.

The daily dose of the combination preparation of the present invention varies depending on the severity of symptoms, age, sex, body weight and sensitivity of the subject to be administered, time and interval of administration, property, formulation and kinds of pharmaceutical preparation, kinds of active ingredients etc., and is not particularly limited. While the dose of the compound of the present invention and the concomitant drug is not particularly limited as long as the dose does not problematically pose side effects, the daily dosage of the compound of the present invention is generally about 0.005 to 50 mg, preferably about 0.05 to 10 mg, and more preferably about 0.2 to 4 mg, per 1 kg body weight, which may be administered once a day or in two or three divided portions a day.

In administering the combination preparation of the present invention, the compound of the present invention and the combination drugs may be administered at the same time or, the combination drugs may be administered before administering the compound of the present invention, and vice versa. In case of staggered administration, the time interval varies depending on the active ingredients to be administered, a formulation and an administration route. For example, if the combination drugs are administered first, the compound of the present invention may be administered 1 minute to 3 days, preferably 10 min to 1 day, more preferably 15 min to 1 hr after administering the combination drugs. If the compound of the present invention is administered first, the combination drugs may be administered 1 minute to 1 day, preferably 10 min to 6 hr, more preferably 15 min to 1 hr after administering the compound of the present invention.

### Examples

The present invention is further described in detail in with reference to Reference Examples, Examples, Preparative Examples and Experimental Examples which are not intended to restrict the invention and may be modified without departing from the scope of the invention.

Elution in the column chromatography in the following Reference Examples and Examples was conducted under observation by TLC (thin layer chromatography), unless otherwise specifically indicated. In the TLC observation, 60F254, TLC plates, produced by Merck & Co., Inc. was used, and the solvent employed as an elution solvent in the column chromatography was used as an eluent. For the detection, a UV detector was used. As silica gel for the column chromatography, Silica Gel 60 (70 to 230 mesh) produced by Merck & Co., Inc. was used. The "room temperature" referred herein means temperature generally from about 10°C to 35°C. For drying extract, sodium sulfate or magnesium sulfate was used.

The abbreviations in Examples and Reference Examples mean the following.
LC: liquid chromatography
MS: mass spectrometry
M: molecular weight of the compound
ESI: electrospray ionization
Rt: retention time
Boc: tert-butyloxycarbonyl
*: relative configuration
N: normal concentration
NMR: nuclear magnetic resonance
Hz: hertz
J: coupling constant
m: multiplet
q: quartet
t: triplet
d: doublet
s: singlet
br: broad
dt: double triplet
brs: broad singlet
like: similar to -
DMF: N,N-dimethylformamide
THF: tetrahydrofuran
DMSO: dimethyl sulfoxide
IPE: diisopropyl ether
Et₂O: diethyl ether
MeOH: methyl alcohol
EtOH: ethyl alcohol
DMA: N,N-dimethylacetamide
HOBt·H₂O: 1-hydroxybenzotriazole hydrate
WS·CHCl: 1-ethyl-3-(dimethylaminopropyl)carbodiimide hydrochloride
DEPC: diethyl cyanophosphate
Et₃N: triethylamine
iPr₂NEt: ethyldiisopropylamine
Boc₂O: di-tert-butyl bicarbonate
CDI: N,N'-carbonyldiimidazole
DBU: 1,8-diazabicyclo[5.4.0]-7-undecene

LC-MS in Examples and Reference Examples were measured under the following conditions.

### Analysis by LC-MS

Instrument: Waters LC-MS system
HPLC system: Agilent HP1100
MS system: Micromass ZMD
HPLC conditions
Column: CAPCELL PAK C18UG120, S-3 µm, 1.5×35 mm (Shiseido)
Solvents: Solution A; water containing 0.05% trifluoroacetic acid, Solution B; acetonitrile containing 0.05% trifluoroacetic acid
Gradient cycles: 0.00 min (Solution A/Solution B =90/10), 2.00 min (Solution A/Solution B =5/95), 2.75 min (Solution A/Solution B =5/95), 2.76 min (Solution A/Solution B =90/10), 3.60 min.

### (Solution A/Solution B =90/10)

Injection volume: 2 µL, Flow rate: 0.5 mL/min, Detection method: UV 220 nm
MS conditions
Ionization method: ESI
Analysis by LC
Instrument: Shimadzu Corporation CLASS-VP system
HPLC conditions
Columns: Inertsil ODS-2, CAPCELL PAK C18UG120, 5 µm, 4.6×150 mm (GL Sciences Inc.)
Solvents: Solution A; water containing 0.1% trifluoroacetic acid, Solution B; acetonitrile containing 0.1% trifluoroacetic acid
Gradient cycles: 0.00 min (Solution A/Solution B =70/30), 15.00 min (Solution A/Solution B =15/85), 15.01 min (Solution A/Solution B =5/95), 20.00 min (Solution A/Solution B =5/95), 20.01 min (Solution A/Solution B =70/30), 25.00 min. (Solution A/Solution B =70/30)
Injection volume: 10 µL, Flow rate: 1.0 mL/min, Detection method: UV 220 nm

Purification by preparative HPLC in Examples and Reference Examples was carried out under the following conditions.

Instrument: High Throughput Purification System, Gilson Company, Inc.
Column: YMC CombiPrep ODS-AS-5 µm, 50x20 mm
Solvents: Solution A; 0.1% trifluoroacetic acid-containing water, Solution B; 0.1% trifluoroacetic acid-containing acetonitrile
Gradient cycle: 0.00 minute (Solution A/Solution B=95/5), 1.00 minute (Solution A/Solution B=95/5), 5.20 min (Solution A/Solution B=5/95), 6.40 min (Solution A/Solution B=5/95), 6.50 min (Solution A/Solution B=95/5), 6.60 min (Solution A/Solution B=95/5)
Flow rate: 25 ml/min, Detection method: UV 220 nm

### Example 1

(3R*,4S*)-4-[(1R*)-1-[3,5-bis(trifluoromethyl)phenyl]ethoxy]-3-phenylpiperidine hydrochloride

### (Step 1)

1-[3,5-Bis(trifluoromethyl)phenyl]ethanol (5.00 g) was dissolved in Et₂O (100 ml), sodium hydride (155 mg) was added at room temperature and the mixture was stirred for 30 min. Trichloroacetonitrile (3.36 g) was added to the reaction mixture and stirred at room temperature for 4 hr, and then water was added. The organic layer was separated and washed with brine and dried, and the solvent was evaporated under reduced pressure. The obtained residue was purified by silica gel column chromatography (ethyl acetate:hexane=1:20) to give 1-[3,5-bis(trifluoromethyl)phenyl]ethyl 2,2,2-trichloroethaneimidate (7.5 g) as a pale-yellow oil.
¹H-NMR(CDCl₃): δ 1.70(3H,d,J=6.8Hz),6.04-6.11(1H,m),7.83(1H,s), 7.88(2H,s),8.40(1H,s)

### (Step 2)

tert-Butyl (3R*,4S*)-4-hydroxy-3-phenylpiperidine-1-carboxylate (555 mg) synthesized according to a known method (WO03/101964) and the compound obtained in Step 1 (1.21 g) were dissolved in CH₂Cl₂ (20 ml) and cyclohexane (40 ml), and molecular sieves 4A (5 g) was added. The mixture was stirred for 5 min. Trifluoromethanesulfonic acid (300 mg) was added at 5-10°C and the mixture was stirred at room temperature for 15 hr. The reaction mixture was poured into saturated aqueous sodium hydrogen carbonate and ethyl acetate, stirred, filtered through celite and partitioned. The obtained organic layer was washed with brine and dried, and the solvent was evaporated under reduced pressure. The residue was dissolved in MeOH (20 ml) and THF (20 ml), Et₃N (0.41 g) and then Boc₂O (0.44 g) were added at room temperature, and the mixture was stirred at 40°C for 30 min. The solvent was evaporated, and the residue was dissolved in ethyl acetate, and washed with water, 3% aqueous KHSO₄ solution and brine. After drying and solvent evaporation, the obtained residue was purified by silica gel column chromatography (ethyl acetate: hexane=1:10) to give tert-butyl (3R*,4S*)-4-[(1S*)-1-[3,5-bis(trifluoromethyl)phenyl]ethoxy]-3-phenylpiperidine-1-carboxylate (0.23 g) as a less polar compound and tert-butyl (3R*,4S*)-4-[(1R*)-1-[3,5-bis(trifluoromethyl)phenyl]ethoxy]-3-phenylpiperidine-1-carboxylate (0.27 g) as a more polar compound, each as a colorless oil.

### (Step 3)

tert-Butyl (3R*,4S*)-4-[(1R*)-1-[3,5-bis(trifluoromethyl)phenyl]ethoxy]-3-phenylpiperidine-1-carboxylate (0.27 g) obtained in Step 2 was dissolved in MeOH (15 ml), 4N hydrogen chloride/ethyl acetate solution (1.5 mL) was added and the mixture was stirred at 65°C for 30 min. The solvent was evaporated under reduced pressure to give the title compound (230 mg) as a white powder.
MS (ESI+) : 418 (M-HCl+H)
¹H-NMR(DMSO-d₆): δ 1.35(3H,d,J=6.4Hz),1.85-1.95(1H,m),2.28-2.33(1H,m),3.06-3.26(4H,m),3.44-3.53(1H,m),3.62(1H,s), 4.72(1H,q,J=6.4Hz),7.11-7.22(5H,m),7.47(2H,s),7.85(1H,s), 9.07 (2H,br) .
¹³C-NMR(DMSO-d₆): δ 24.07,24.97,37.82,41.25,42.07,71.10,72.55, 121.03(³J_{C-F}=3Hz),123.00(¹J_{C-F}=273Hz),126.65,126.81,127.37,127.87, 129.94(²J_{C-F}=32Hz),138.69,146.25

### Example 2

(3R*,4S*)-4-[(1S*)-1-[3,5-bis(trifluoromethyl)phenyl]ethoxy]-3-phenylpiperidine hydrochloride

tert-Butyl (3R*,45*)-4-[(1S*)-1-[3,5-bis(trifluoromethyl)phenyl]ethoxy]-3-phenylpiperidine-1-carboxylate (0.23 g) obtained in Step 2 of Example 1 was dissolved in MeOH (15 ml), 4N hydrogen chloride/ethyl acetate solution (1.5 mL) was added and the mixture was stirred at 65°C for 30 min. The solvent was evaporated under reduced pressure to give the title compound (200 mg) as a white powder.
MS (ESI+) : 418 (M-HCl+H)
¹H-NMR(DMSO-d6) : δ 0.95(3H,d,J=6.2Hz),1.68-1.74(1H,m),1.83-1.94(1H,m),2.87-2.96(1H,m),3.11-3.26(3H,m),3.47-3.55(1H,m), 3.96(1H,s),4.41(1H,q,J=6.3Hz),7.31-7.41(5H,m),7.93(2H,s), 8.02(1H,s),8.88(2H,br)
¹³C-NMR(DMSO-d₆) : δ
22.12,26.52,37.86,41.41,42.22,52.55,74.59,121.04
(³J_{C-F=}3Hz),123.21(¹J_{C-F}=273Hz),126.50,127.00,127.78,128.09,130.15
(²J_{C}-_{F}=33Hz),139.25,147.82

### Example 3

(3R*,4S*)-4-[(1R*)-1-[3,5-bis(trifluoromethyl)phenyl]ethoxy]-N-methyl-3-phenylpiperidine-1-carboxamide

The compound obtained in Example 1 (70 mg) was dissolved in acetonitrile (5 mL), and iPr₂NEt (30 mg) was added. After stirring for 5 min, methylisocyanate (27 mg) was added at room temperature and the mixture was stirred for 24 hr. Water was added to the reaction mixture, and the mixture was extracted with ethyl acetate. The organic layer was washed with 0.1 N hydrochloric acid, saturated aqueous sodium hydrogen carbonate and brine and dried, and the solvent was evaporated under reduced pressure. The residue was purified by silica gel column chromatography (CHCl₃:MeOH=20:1) to give the title compound (38 mg) as a white powder.
MS (ESI+) : 475 (M+H)

### Example 4

(3R*,4S*)-4-[(1S*)-1-[3,5-bis(trifluoromethyl)phenyl]ethoxy]-N-methyl-3-phenylpiperidine-1-carboxamide

The compound was synthesized by the reaction and work-up in the same manner as in Example 3 and using the compound obtained in Example 2.
MS (ESI+) : 475 (M+H)

### Example 5

(3R*,4S*)-4-[(1R*)-1-[3,5-bis(trifluoromethyl)phenyl]ethoxy]-N-ethyl-3-phenylpiperidine-1-carboxamide

The compound was synthesized by the reaction and work-up in the same manner as in Example 3 and using the compound obtained in Example 1 and ethylisocyanate.
MS (ESI+) : :489 (M+H)

### Example 6

N-[2-[(3R*,4S*)-4-[(1R*)-1-[3,5-bis(trifluoromethyl)phenyl]ethoxy]-3-phenylpiperidin-1-yl]-2-oxoethyl]acetamide

To a solution of the compound obtained in Example 1 (100 mg) in DMF (5 mL) were added N-acetylglycine (39 mg), WSC·HCl (85 mg) and HOBt·H₂O (68 mg), and iPr₂NEt (143 mg) was added dropwise at room temperature. After stirring for 15 hr, water was added to the reaction mixture, and the product was extracted with ethyl acetate. The organic layer was washed with water, 3% KHSO₄ aqueous solution and brine and dried, and the solvent was evaporated under reduced pressure. The obtained residue was purified by silica gel column chromatography (CHCl₃:MeOH=20:1) to give the title compound (100 mg) as a pale-yellow oil.
MS (ESI+) : 517 (M+H)

### Example 7

(3R*,4S*)-4-[(1R*)-1-[3,5-bis(trifluoromethyl)phenyl]ethoxy]-3-phenyl-1-(piperidine-4-carbonyl)piperidine hydrochloride

To a solution of the compound obtained in Example 1 (730 mg) in DMF (10 mL) were added 1-(tert-butoxycarbonyl)piperidine-4-carboxylic acid (560 mg), WSC·HCl (617 mg) and HOBt·H₂O (493 mg), and iPr₂NEt (1.04 g) was added dropwise at room temperature. After stirring for 15 hr, water was added to the reaction mixture, and the product was extracted with ethyl acetate. The organic layer was washed with water, 3% KHSO₄ aqueous solution and brine and dried, and the solvent was evaporated under reduced pressure. The residue was purified by silica gel column chromatography (ethyl acetate:hexane=1:1). The thus-obtained oil was dissolved in MeOH (20 ml), 4N hydrogen chloride/ethyl acetate solution (2 ml) was added and the mixture was stirred at 65°C for 30 min. The solvent was evaporated under reduced pressure to give the title compound (230 mg) as a white powder. MS (ESI+) : 529 (M-HCl+H)

### Example 8

(3R*,4S*)-4-[(1S*)-1-[3,5-bis(trifluoromethyl)phenyl]ethoxy]-3-phenyl-1-(piperidine-4-carbonyl)piperidine hydrochloride

The compound was synthesized by the reaction and work-up in the same manner as in Example 7 and using the compound obtained in Example 2. MS (ESI+) : 529 (M-HCl+H)

### Example 9

(3R*,4S*)-1-[(1-acetylpiperidin-4-yl)carbonyl]-4-[(1R*)-1-[3,5-bis(trifluoromethyl)phenyl]ethoxy]-3-phenylpiperidine

The compound was synthesized by the reaction and work-up in the same manner as in Example 6 and using the compound obtained in Example 7 and acetic acid.
MS (ESI+) :571 (M+H)

### Example 10

N-[2-[4-[[(3R*,4S*)-4-[(1R*)-1-[3,5-bis(trifluoromethyl)phenyl]ethoxy]-3-phenylpiperidin-1-yl]carbonyl]piperidin-1-yl]-2-oxoethyl]acetamide

The compound was synthesized by the reaction and work-up in the same manner as in Example 6 and using the compound obtained in Example 7.
MS (ESI+) : 628 (M+H)

### Example 11

1-[4-[[(3R*,4S*)-4-[(1R*)-1-[3,5-bis(trifluoromethyl)phenyl]ethoxy]-3-phenylpiperidin-1-yl]carbonyl]piperidin-1-yl]acetone hydrochloride

To a solution of the compound obtained in Example 7 (100 mg) in DMF (5 ml) was added iPr₂NEt (230 mg), and 1-bromoacetone (73 mg) was added dropwise. After stirring for 3 hr, water was added to the reaction mixture, and the product was extracted with ethyl acetate. The organic layer was washed with brine and dried, and the solvent was evaporated under reduced pressure. The obtained residue purified by silica gel column chromatography (CHCl₃:MeOH=20:1), and converted to hydrochloride to give the title compound (26 mg) as pale-yellow amorphous.
MS (ESI+) : 585 (M-HCl+H)

### Example 12

Methyl [4-[[(3R*,4S*)-4-[(1R*)-1-[3,5-bis(trifluoromethyl)phenyl]ethoxy]-3-phenylpiperidin-1-yl]carbonyl]piperidin-1-yl]acetate hydrochloride

The compound was synthesized by the reaction and work-up in the same manner as in Example 11 and using the compound obtained in Example 7 and methyl bromoacetate.
MS(ESI+):601(M-HCl+H)

### Example 13

Isopropyl [4-[[(3R*,4S*)-4-[(1R*)-1-[3,5-bis(trifluoromethyl)phenyl]ethoxy]-3-phenylpiperidin-1-yl]carbonyl]piperidin-1-yl]acetate hydrochloride

The compound was synthesized by the reaction and work-up in the same manner as in Example 11 and using the compound obtained in Example 7 and isopropyl bromoacetate.
MS (ESI+) : 629 (M-HCl+H)

### Example 14

1-Acetyl-4-[2-[(3R*,4S*)-4-[(1R*)-1-[3,5-bis(trifluoromethyl)phenyl]ethoxy]-3-phenylpiperidin-1-yl]-2-oxoethyl]piperazine hydrochloride

To a solution of the compound obtained in Example 1 (70 mg) in DMF (5 mL) was added iPr₂NEt (100 mg), and chloroacetyl chloride (21 mg) was added dropwise at room temperature. After stirring for 15 min, sodium iodide (70 mg) and 1-acetylpiperazine (99 mg) were added to the reaction mixture, and the mixture was stirred at 40°C for 5 hr. Water was added to the reaction mixture, and the product was extracted with ethyl acetate. The organic layer was washed with brine and dried, and the solvent was evaporated under reduced pressure. The obtained residue was purified by silica gel column chromatography (CHCl₃:MeOH=20:1) and treated with hydrochloric acid to give the title compound (35 mg) as a colorless amorphous form.
MS (ESI+) : 586 (M-HCl+H)

### Example 15

4-[2-[(3R*,45*)-4-[(1R*)-1-[3,5-Bis(trifluoromethyl)phenyl]ethoxy]-3-phenylpiperidin-1-yl]-2-oxoethyl]piperazin-2-one hydrochloride

The compound was synthesized by the reaction and work-up in the same manner as in Example 14 and using the compound obtained in Example 1 and piperazin-2-one.
MS (ESI+) : 558 (M-HCl+H)

### Example 16

N-[1-[2-[(3R*,4S*)-4-[(1R*)-1-[3,5-Bis(trifluoromethyl)phenyl]ethoxy]-3-phenylpiperidin-1-yl]-2-oxoethyl]piperidin-4-yl]acetamide hydrochloride

The compound was synthesized by the reaction and work-up in the same manner as in Example 14 and using the compound obtained in Example 1 and N-(piperidin-4-yl)acetamide.
MS (ESI+) : 600 (M-HCl+H)

### Example 17

1-[[4-[[(3R*,45*)-4-(1R*)-1-[3,5-Bis(trifluoromethyl)phenyl]ethoxy]-3-phenylpiperidin-1-yl]carbonyl]piperidin-1-yl]carbonyl]imidazolidin-2-one

To a solution of the compound obtained in Example 7 (90 mg) in DMF (5 mL) was added Et₃N (161 mg), 2-oxoimidazolidinecarbonyl chloride (48 mg) was added at room temperature. After stirring for 30 min, water was added to the reaction mixture, and the product was extracted with ethyl acetate. The organic layer was washed with water, 3% KHSO₄ aqueous solution, saturated aqueous sodium hydrogen carbonate and brine and dried, and the solvent was evaporated under reduced pressure. The obtained residue was crystallized from IPE/hexane to give the title compound (65 mg) as a white powder.
MS(ESI+):641(M+H)

### Example 18

2-[4-[[(3R*,4S*)-4-[(1R*)-1-[3,5-Bis(trifluoromethyl)phenyl]ethoxy]-3-phenylpiperidin-1-yl]carbonyl]piperidin-1-yl]-N,N-dimethylacetamide hydrochloride

The compound was synthesized by the reaction and work-up in the same manner as in Example 11 and using the compound obtained in Example 7 and 2-chloro-N,N-dimethylacetamide.
MS (ESI+) : 614 (M-HCl+H)

### Example 19

1-[2-[(3R*,4S*)-4-[(1R*)-1-[3,5-Bis(trifluoromethyl)phenyl]ethoxy]-3-phenylpiperidin-1-yl]-2-oxoethyl]-4-isopropylpiperazine hydrochloride

The compound was synthesized by the reaction and work-up in the same manner as in the method described in Example 14 and using the compound obtained in Example 1 and 1-isopropylpiperazine.
MS (ESI+) : 586 (M-2HCl+H)

### Example 20

1-[2-[(3R*,4S*)-4-[(1R*)-1-[3,5-Bis(trifluoromethyl)phenyl]ethoxy]-3-phenylpiperidin-1-yl]-2-oxoethyl]piperidin-4-ol hydrochloride

The compound was synthesized by the reaction and work-up in the same manner as in the method described in Example 14 and using the compound obtained in Example 1 and piperidin-4-ol.
MS (ESI+) : 559 (M-HCl+H)

### Example 21

1-Acetyl-4-[[(3R*,4S*)-4-[(1R*)-1-[3,5-bis(trifluoromethyl)phenyl]ethoxy]-3-phenylpiperidin-1-yl]carbonyl]piperazine

To a solution of the compound obtained in Example 1 (80 mg) in DMF (5 ml) was added K₂CO₃ (74 mg), and 4-nitrophenyl 4-acetylpiperazine-1-carboxylate (52 mg) synthesized by a known method (WO03/101964) was added at room temperature. After stirring at 135°C for 4 hr, water was added to the reaction mixture, and the product was extracted with ethyl acetate. The organic layer was washed with water, 3% KHSO₄ aqueous solution and brine and dried, and the solvent was evaporated under reduced pressure. The obtained residue was purified by silica gel column chromatography (CHCl₃:MeOH=20:1) to give the title compound (50 mg) as a colorless amorphous.
MS (ESI+) : 572 (M+H)

### Example 22

N-[trans-4-[[(3R*,4S*)-4-[(1R*)-1-[3,5-Bis(trifluoromethyl)phenyl]ethoxy]-3-phenylpiperidin-1-yl]carbonyl]cyclohexyl]acetamide

### (Step 1)

To a solution of the compound obtained in Example 1 (100 mg) in DMF (5 mL) were added trans-4-[(tert-butoxycarbonyl)amino]cyclohexanecarboxylic acid (81 mg), WSC·HCl (85 mg) and HOBt·H₂O (63 mg), and iPr₂NEt (143 mg) was added dropwise at room temperature. After stirring for 12 hr, water was added to the reaction mixture, and the precipitated product was collected by filtration to give tert-butyl [trans-4-[[(3R*,4S*)-4-[(1R*)-1-[3,5-bis(trifluoromethyl)phenyl]ethoxy]-3-phenylpiperidin-1-yl]carbonyl]cyclohexyl]carbamate (140 mg) as a white powder.
MS (ESI+) : 643 (M+H)

### (Step 2)

The compound obtained in Step 1 (120 mg) was dissolved in MeOH (20 mL), 4N hydrogen chloride/ethyl acetate solution (2 mL) was added and the mixture was stirred at 65°C for 30 min. The solvent was evaporated under reduced pressure to give trans-4-[[(3R*,4S*)-4-[(1R*)-1-[3,5-bis(trifluoromethyl)phenyl]ethoxy]-3-phenylpiperidin-1-yl]carbonyl]cyclohexylamine hydrochloride (108 mg) as a colorless amorphous.
MS (ESI+) : 543 (M-HCl+H)

### (Step 3)

To a solution of the compound obtained in Step 2 (108 mg) in DMF (5 mL) was added Et₃N (189 mg), and acetyl chloride (44 mg) was added dropwise at room temperature. After stirring for 1 hr, water was added to the reaction mixture, and the product was extracted with ethyl acetate. The organic layer was washed with water, 3% KHSO₄ aqueous solution and brine and dried, and the solvent was evaporated under reduced pressure. The obtained residue was purified by silica gel column chromatography (CHCl₃:MeOH=20:1) and crystallized from IPE to give the title compound (67 mg) as a white powder.
MS (ESI+) : 585 (M+H)

### Example 23

N-[trans-4-[[(3R*,4S*)-4-[(1R*)-1-[3,5-Bis(trifluoromethyl)phenyl]ethoxy]-3-phenylpiperidin-1-yl]carbonyl]cyclohexyl]propanamide

The compound was synthesized by the reaction and work-up in the same manner as in the method described in Step 3 of Example 22 and using the compound obtained in Step 2 of Example 22 and propionyl chloride.
MS (ESI+) : 599 (M+H)

### Example 24

N-[trans-4-[[(3R*,4S*)-4-[(1R*)-1-[3,5-Bis(trifluoromethyl)phenyl]ethoxy]-3-phenylpiperidin-1-yl]carbonyl]cyclohexyl]-2-methoxyacetamide

To a solution of the compound obtained in Step 2 of Example 22 (72 mg) in DMF (5 mL) were added methoxyacetic acid (23 mg), WSC·HCl (72 mg) and HOBt-H₂O (58 mg), and iPr₂NEt (81 mg) was added dropwise at room temperature. After stirring for 3 hr, water was added to the reaction mixture, and the product was extracted with ethyl acetate. The organic layer was washed with water, 3% KHSO₄ aqueous solution and brine and dried, and the solvent was evaporated under reduced pressure. The obtained residue was purified by silica gel column chromatography (CHCl₃:MeOH=20:1) and crystallized from IPE to give the title compound (30 mg) as a white powder.
MS (ESI+) : 615 (M+H)

### Example 25

N-[trans-4-[[(3R*,4S*)-4-(1R*)-1-[3,5-Bis(trifluoromethyl)phenyl]ethoxy]-3-phenylpiperidin-1-yl]carbonyl]cyclohexyl]-2-methylpropanamide

The compound was synthesized by the reaction and work-up in the same manner as in the method described in Step 3 of Example 22 and using the compound obtained in Step 2 of Example 22 and isobutyryl chloride.
MS(ESI+):613(M+H)

### Example 26

N²-Acetyl-N¹-[trans-4-(3R*,4S*)-4-[(1R*)-1-(3,5-bis(trifluoromethyl)phenyl]ethoxy]-3-phenylpiperidin-1-yl]carbonyl]cyclohexyl]glycinamide

The compound was synthesized by the reaction and work-up in the same manner as in the method described in Example 24 and using the compound obtained in Step 2 of Example 22 and N-acetylglycine.
MS (ESI+) : 642 (M+H)

### Example 27

N-[trans-4-[[(3R*,4S*)-4-[(1R*)-1-[3,5-Bis(trifluoromethyl)phenyl]ethoxy]-3-phenylpiperidin-1-yl]carbonyl]cyclohexyl]-N'-methylurea

The compound was synthesized by the reaction and work-up in the same manner as in the method described in Example 3 and using the compound obtained in Step 2 of Example 22 and methylisocyanate.
MS (ESI+) : 600 (M+H)

### Example 28

N'-[trans-4-[[(3R*,4S*)-4-[(1R*)-1-[3,5-Bis(trifluoromethyl)phenyl]ethoxy]-3-phenylpiperidin-1-yl]carbonyl]cyclohexyl]-N,N-dimethylurea

The compound was synthesized by the reaction and work-up in the same manner as in the method described in Example 17 and using the compound obtained in Step 2 of Example 22 and N,N-dimethylcarbamoyl chloride.
MS(ESI+):614(M+H)

### Example 29

Methyltrans-4-[[(3R^{*},4S^{*})-4-[(1R^{*})-1-[3,5-Bis(trifluoromethyl)phenyl]ethoxy]-3-phenylpiperidin-1-yl]carbonyl]cyclohexylcarbamate

The compound was synthesized by the reaction and work-up in the same manner as in the method described in Example 17 and using the compound obtained in Step 2 of Example 22 and methyl chloroformate.
MS (ESI+) : 601 (M+H)

### Example 30

(3R*,4S*)-1-[(1-Acetylpiperidin-4-yl)carbonyl]-4-[(1R*)-1-[3,5-bis(trifluoromethyl)phenyl]ethoxy]-3-(4-fluorophenyl)piperidine

### (Step 1)

(3R*,4S*)-4-[(1R*)-1-[3,5-Bis(trifluoromethyl)phenyl]ethoxy]-3-(4-fluorophenyl)piperidine hydrochloride was synthesized by the reaction and work-up in the same manner as in the method described in Step 2 and Step 3 of Example 1 and using tert-butyl (3R*,4S*)-3-(4-fluorophenyl)-4-hydroxypiperidine-1-carboxylate synthesized by a known method (WO03/101964).

### (Step 2)

The title compound was synthesized by the reaction and work-up in the same manner as in the method described in Example 7 and Example 9 and using the compound obtained in Step 1.
MS (ESI+) : 589 (M+H)

### Example 31

N-[trans-4-[[(3R*,4S*)-4-[(1R*)-1-[3,5-Bis(trifluoromethyl)phenyl]ethoxy]-3-(4-fluorophenyl)piperidin-1-yl]carbonyl]cyclohexyl]acetamide

The compound was synthesized by the reaction and work-up in the same manner as in the method described in Example 22 and using the compound obtained in Step 1 of Example 30.
MS (ESI+) : 603 (M+H)

The compounds described in Example 1 to Example 31 are as shown below (Tables 1 and 2).

**Table 1**

| | | | | | | | |
|---|---|---|---|---|---|---|---|
| 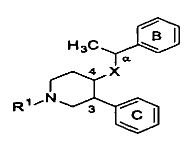 | | | | | | | |

| Ex. No. | stereochemistry | R¹ | X | 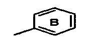 | 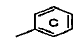 | additive | MS (ESI) |
|---|---|---|---|---|---|---|---|
| 1 | (3R*,4S*), α-R* | H | O | 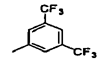 | 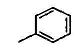 | HCl | 418 |
| 2 | (3R*,4S*), α-S* | H | O | 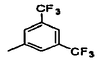 | 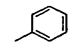 | HCl | 418 |
| 3 | (3R*,4S*), α-R* | CH₃NHCO | O | 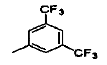 | 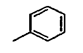 | | 475 |
| 4 | (3R*,4S*), α-S* | CH₃NHCO | O | 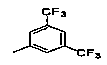 | 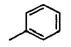 | | 475 |
| 5 | (3R*,4S*), α-R* | C₂H₅NHCO | O | 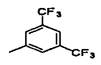 | 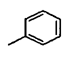 | | 489 |
| 6 | (3R*,4S*), α-R* | 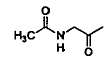 | O | 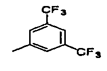 | 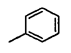 | | 517 |
| 7 | (3R*,4S*), α-R* | 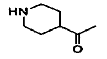 | O | 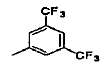 | 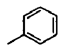 | HCl | 529 |
| 8 | (3R*,4S*), α-S* | 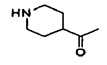 | O | 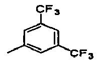 | 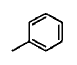 | HCl | 529 |
| 9 | (3R*,4S*), α-R* | 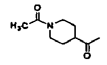 | O | 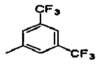 | 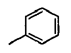 | | 571 |
| 10 | (3R*,4S*), α-R* | 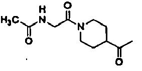 | O | 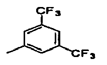 | 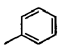 | | 628 |
| 11 | (3R*,4S*), α-R* | 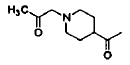 | O | 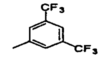 | 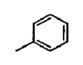 | HCl | 585 |
| 12 | (3R*,4S*), α-R* | 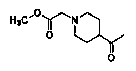 | O | 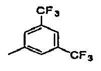 | 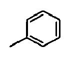 | HCl | 601 |
| 13 | (3R*,4S*), α-R* | 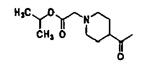 | O | 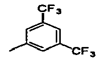 | 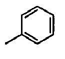 | HCl | 629 |
| 14 | (3R*,4S*), α-R* | 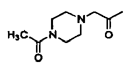 | O | 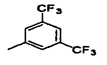 | 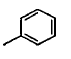 | HCl | 586 |
| 15 | (3R*,4S*), α-R* | 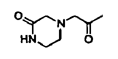 | O | 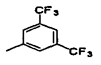 | 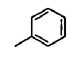 | HCl | 558 |
| 16 | (3R*,4S*), α-R* | 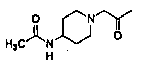 | O | 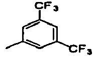 | 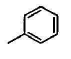 | HCl | 600 |

**Table 2**

| | | | | | | | |
|---|---|---|---|---|---|---|---|
| 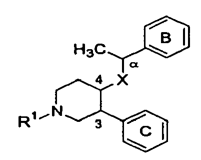 | | | | | | | |

| Ex. No. | stereochemistry | R¹ | X | 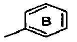 | 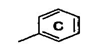 | additive | MS (ESI) |
|---|---|---|---|---|---|---|---|
| 17 | (3R*,4S*), α-R* | 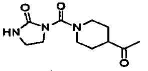 | O | 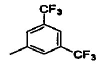 | 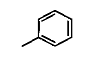 | | 641 |
| 18 | (3R*,4S*), α-R* | 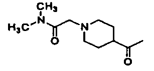 | O | 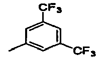 | 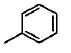 | HCl | 614 |
| 19 | (3R*,4S*), α-R* | 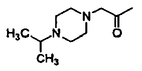 | O | 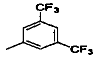 | 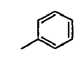 | 2HCl | 586 |
| 20 | (3R*,4S*), α-R* | 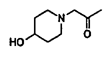 | O | 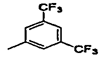 | 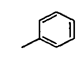 | HCl | 559 |
| 21 | (3R*,4S*), α-R* | 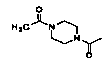 | O | 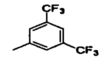 | 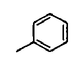 | | 572 |
| 22 | (3R*,4S*), α-R* | 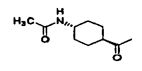 | O | 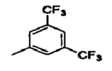 | 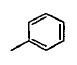 | | 585 |
| 23 | (3R*,4S*), α-R* | 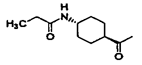 | O | 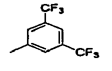 | 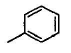 | | 599 |
| 24 | (3R*,4S*), α-R* | 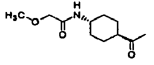 | O | 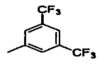 | 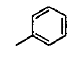 | | 615 |
| 25 | (3R*,4S*), α-R* | 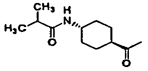 | O | 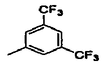 | 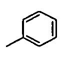 | | 613 |
| 26 | (3R*,4S*), α-R* | 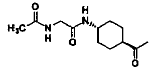 | O | 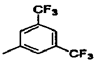 | 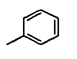 | | 642 |
| 27 | (3R*,4S*), α-R* | 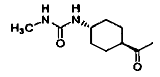 | O | 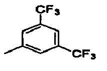 | 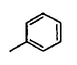 | | 600 |
| 28 | (3R*,4S*), α-R* | 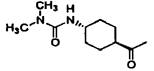 | O | 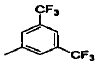 | 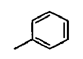 | | 614 |
| 29 | (3R*,4S*), α-R* | 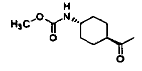 | O | 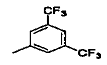 | 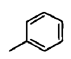 | | 601 |
| 30 | (3R*,4S*), α-R* | 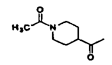 | O | 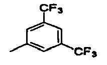 | 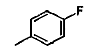 | | 589 |
| 31 | (3R*,4S*), α-R* | 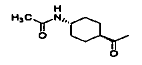 | O | 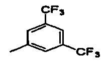 | 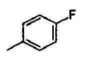 | | 603 |

### Example 32

(3R,4S)-4-[(1R)-1-[3,5-Bis(trifluoromethyl)phenyl]ethoxy]-3-phenylpiperidine hydrochloride

### [Method 1]

The compound was synthesized by the reaction and work-up in the same manner as in the method described in Step 2 and Step 3 of Example 1 and using tert-butyl (3R,4S)-4-hydroxy-3-phenylpiperidine-1-carboxylate synthesized by a known method (WO03/101964).
MS (ESI+) : 418 (M-HCl+H)

### [Method 2]

### (Step 1)

To a solution of tert-butyl (3R,4S)-4-hydroxy-3-phenylpiperidine-1-carboxylate (3.34 g) synthesized by a known method (WO03/101964) and 4-dimethylaminopyridine (1.47 g) in pyridine (30 mL) and CH₂Cl₂ (5 mL) was added 3,5-bis(trifluoromethyl)benzoyl chloride (5.0 g) at 0°C, and the mixture was stirred at room temperature for 14 hr. Water was added to the reaction mixture, and the product was extracted with ethyl acetate. The organic layer was washed with 10% aqueous citric acid solution and brine and dried, and the solvent was evaporated under reduced pressure. The obtained residue was purified by silica gel column chromatography (ethyl acetate:hexane=1:1) to give tert-butyl (3R,4S)-4-[[3,5-bis(trifluoromethyl)benzoyl]oxy]-3-phenyl-1-piperidinecarboxylate (6.4 g) as a colorless oil.
¹H-NMR(CDCl₃): δ 1.49(9H,s),1.95-2.20(2H,m),3.10-3.25(2H,m),3.50-3.80(1H,br),4.00-4.50(2H,m),5.51-5.53(1H,m),7.18-7.35(5H,m),8.03(1H,s),8.31(2H,s)

### (Step 2)

To a solution of the compound obtained in Step 1 (1.86 g) in toluene (8 mL), THF (2 mL) and pyridine (2 mL) was added Tebbe reagent (0.5 M toluene solution, 25 mL) at -78°C and the mixture was stirred at room temperature for 48 hr. The reaction mixture was cooled to 0°C, 2N aqueous sodium hydroxide solution (20 mL) was added and insoluble materials were filtered off. The filtrate was extracted with ethyl acetate, washed with water and brine and dried, and the solvent was evaporated under reduced pressure. The obtained residue was dissolved in acetonitrile (10 mL), Et₃N (0.75 mL) and Boc₂O (1.2 g) were added at room temperature and the mixture was stirred for 14 hr. Water was added to the reaction mixture, and the product was extracted with ethyl acetate. The organic layer was washed with 10% aqueous citric acid solution and brine and dried, and the solvent was evaporated under reduced pressure. The obtained residue was purified by silica gel column chromatography (ethyl acetate:hexane=5:95) to give tert-butyl (3R,4S)-4-[[1-[3,5-bis(trifluoromethyl)phenyl]vinyl]oxy]-3-phenylpiperidine-1-carboxylate (1.1 g) as a pale-yellow oil.
¹H-NMR(CDCl₃): δ 1.48(9H,s),1.75-1.90(1H,m),2.20-2.30(1H,m),3.05-3.20(2H,m),3.50-3.75(1H,br),3.90-4.50(2H,m),4.30(1H,d,J=3.6Hz),4.65(1H,m),4.71(1H,d,J=3.6Hz),7.20 -7.34(5H,m),7.76(1H,s)7.84(2H,s)

### (Step 3)

A solution of the compound obtained in Step 2 (25 mg) and 10% palladium carbon (250 mg) in ethanol (5 mL) was stirred under a hydrogen atmosphere (1 atm) at 70°C for 30 min. The catalyst was filtered off, and the filtrate was concentrated under reduced pressure. The obtained residue was purified by silica gel column chromatography (ethyl acetate·hexane=1:10) to give a more polar compound, tert-butyl (3R,4S)-4-[(1R)-1-[3,5-bis(trifluoromethyl)phenyl]ethoxy]-3-phenylpiperidine-1-carboxylate (20 mg) and a less polar compound, tert-butyl (3R,4S)-4-[(1S)-1-[3,5-bis(trifluoromethyl)phenyl]ethoxy]-3-phenylpiperidine-1-carboxylate (5 mg), each as a pale-yellow oil.

### (Step 4)

The title compound was synthesized by the reaction and work-up in the same manner as in the method described in Step 3 of Example 1 and using tert-butyl (3R,4S)-4-[(1R)-1-[3,5-bis(trifluoromethyl)phenyl]ethoxy]-3-phenylpiperidine-1-carboxylate obtained in Step 3.
MS (ESI+) : 418 (M-HCl+H)
melting point: :169-171°C
[α]_{D}²⁵ +52.5° (c 1.0,MeOH)
¹H-NMR(CDCl₃):δ 1.38(3H,d,J=6.3Hz),2.30-2.35(2H,m),3.25-3.50(4H,m),3.60-3.75(2H,m),4.35(1H,q,J=6.3Hz),7.01-7.05(2H,m),7.16-7.26(5H,m),7.64(1H,s),9.00-10.40(2H,br)

### Example 33

(3R,4S)-4-[(1S)-1-[3,5-Bis(trifluoromethyl)phenyl]ethoxy]-3-phenylpiperidine hydrochloride

The compound was synthesized by the reaction and work-up in the same manner as in the method described in Step 3 of Example 1 and using tert-butyl (3R,4S)-4-[(1S)-1-[3,5-bis(trifluoromethyl)phenyl]ethoxy]-3-phenylpiperidine-1-carboxylate obtained in Step 3 in method 2 of Example 32.
MS (ESI+) : 418 (M-HCl+H)
melting point: :150-152°C
[α]_{D}²⁵ +30.9° (c 1.0,MeOH)
¹H-NMR(DMSO-d₆) : δ 0.95(3H,d,J=6.5Hz),1.72(1H,d like),1.93(1H,t like),2.91(1H,dt,J=13.3 and 2.5Hz),3.12(1H,d like),3.27(1H,d like),3.29(1H,d,like),3.50(1H,t,J=13.4Hz),3.97(1H,s like),4.41(1H,q,J=6.5Hz),7.32(1H,t like),7.35(2H,d like),7.39(2H,t like),7.93(2H,s),8.01(1H,s),9.19(2H,brs)

### Example 34

3-[4-[[(3R,4S)-4-[(1R)-1-[3,5-Bis(trifluoromethyl)phenyl]ethoxy]-3-phenylpiperidin-1-yl]carbonyl]piperidin-1-yl]dihydrofuran-2(3H)-one hydrochloride (retention time: short)

### (Step 1)

(3R,4S)-4-[(1R)-1-[3,5-Bis(trifluoromethyl)phenyl]ethoxy]-3-phenyl-1-(piperidin-4-ylcarbonyl)piperidine hydrochloride was synthesized by the reaction and work-up of the compound obtained in Example 32 in the same manner as in the method described in Example 7.
MS(ESI+):529(M-HCl+H)

### (Step 2)

The diastereo mixture obtained by the reaction and work-up in the same manner as in the method described in Example 11 and using the compound obtained in Step 1 and α-bromo-γ-butyrolactone was purified by chiral column chromatography. The preparative fraction having a shorter retention time was concentrated and treated with hydrochloric acid to give the title compound (44 mg).
MS (ESI+) : 613 (M-HCl+H)
purification conditions by chiral column chromatography
column: Kromasil 5CHI-TBB 20 mmIDx250 mmL
solvent: hexane/ethanol=98/2
flow rate: 13 mL/min
temperature: 20°C
detection method: UV 220 nm

### Example 35

3-[4-[[(3R,4S)-4-[(1R)-1-[3,5-Bis(trifluoromethyl)phenyl]ethoxy]-3-phenylpiperidin-1-yl]carbonyl]piperidin-1-yl]dihydrofuran-2(3H)-one hydrochloride (retention time: long)

The title compound (25 mg) was obtained by concentrating the preparative fraction with a longer retention time among those described in Step 2 of Example 34 and treating with hydrochloric acid.
MS (ESI+) : 613 (M-HCl+H)
purification conditions by chiral column chromatography
column: Kromasil 5CHI-TBB 20 mmIDx250 mmL
solvent: hexane/ethanol=98/2
flow rate: 13 mL/min
temperature: 20°C
detection method: UV 220 nm

### Example 36

(3R,4S)-1-[(1-Acetylpiperidin-4-yl)carbonyl]-4-[(1R)-1-[3,5-bis(trifluoromethyl)phenyl]ethoxy]-3-phenylpiperidine

The compound was synthesized by the reaction and work-up in the same manner as in the method described in Example 9 and using the compound obtained in Step 1 of Example 34.
MS (ESI+) : 571 (M+H)

### Example 37

(3R,4S)-1-[(1-Acetylpiperidin-4-yl)carbonyl]-4-[(1S)-1-[3,5-bis(trifluoromethyl)phenyl]ethoxy]-3-phenylpiperidine

The compound was synthesized by the reaction and work-up in the same manner as in the methods described in Example 7 and Example 9 using the compound obtained in Example 33.
MS (ESI+) : 571 (M+H)

### Example 38

N-[2-[4-[[(3R,4S)-4-[(1R)-1-[3,5-Bis(trifluoromethyl)phenyl]ethoxy]-3-phenylpiperidin-1-yl]carbonyl]piperidin-1-yl]-2-oxoethyl]acetamide

The compound was synthesized by the reaction and work-up in the same manner as in the method described in Example 10 and using the compound obtained in Step 1 of Example 34.
MS (ESI+) : 628 (M+H)

### Example 39

(3R,4S)-4-[(1R)-1-[3,5-Bis(trifluoromethyl)phenyl]ethoxy]-3-phenyl-1-[[1-(1H-tetrazol-1-ylacetyl)piperidin-4-yl]carbonyl]piperidine

The compound was synthesized by the reaction and work-up in the same manner as in the method described in Example 10 and using the compound obtained in Step 1 of Example 34 and (tetrazol-1-yl)acetic acid.
MS (ESI+) : 639 (M+H)
¹H-NMR(CDCl₃): δ 1.35-1.46(3H,m),1.51-2.21(7H,m),2.48-4.02(9H,m),4.28-4.69(3H,m),5.19-5.50(1H,m),7.01-7.34(7H,m),7.65(1H,s),8.83-8.88(1H,m)
[α]_{D}²⁵ +75.2° (c 1.0,MeOH)

### Example 40

1-[[4-[[(3R,4S)-4-[(1R)-1-[3,5-Bis (trifluoromethyl) phenyl] ethoxy]-3-phenylpiperidin-1-yl]carbonyl] piperidin-1-yl] carbonyl] imidazolidin-2-one

The compound was synthesized by the reaction and work-up in the same manner as in the method described in Example 17 and using the compound obtained in Step 1 of Example 34.
MS(ESI+):641(M+H)

### Example 41

trans-4-[[(3R,4S)-4-[(1R)-1-[3,5-Bis(trifluoromethyl)phenyl]ethoxy]-3-phenylpiperidin-1-yl]carbonyl]cyclohexylamine hydrochloride

The compound was synthesized by the reaction and work-up in the same manner as in the method described in Step 1 and Step 2 of Example 22 and using the compound obtained in Example 32.
MS (ESI+) : 543 (M-HCl+H)

### Example 42

N-[trans-4-[[(3R,4S)-4-[(1R)-1-[3,5-Bis(trifluoromethyl)phenyl]ethoxy]-3-phenylpiperidin-1-yl]carbonyl]cyclohexyl]acetamide

The compound was synthesized by the reaction and work-up in the same manner as in Step 3 of Example 22 and using the compound obtained in Example 41.
MS (ESI+) : 585 (M+H)
[α]_{D}²⁵ +74.9° (c 1.0, MeOH)

### Example 43

N-[trans-4-[[(3R,4S)-4-[(1R)-1-[3,5-Bis(trifluoromethyl)phenyl]ethoxy]-3-phenylpiperidin-1-yl]carbonyl]cyclohexyl]propanamide

The compound was synthesized by the reaction and work-up in the same manner as in Step 3 of Example 22 and using the compound obtained in Example 41 and propionyl chloride.
MS (ESI+) : 599 (M+H)
[α]_{D}²⁵ +80.7° (c 1.0,MeOH)
¹H-NMR(CDCl₃): δ 1.00-1.30(5H,m),1.35-1.45(3H,m),1.55-1.90(5H,m),2.00-2.25(5H,m),2.35-3.91(7H,m),4.30-4.50(1H,m), 4.55-4.70(1H,m),5.13-5.24(1H,m),7.04-7.30(7H,m),7.63(1H,s)

### Example 44

N-[trans-4-[[(3R,4S)-4-[(1R)-1-[3,5-Bis(trifluoromethyl)phenyl]ethoxy]-3-phenylpiperidin-1-yl]carbonyl]cyclohexyl]butanamide

The compound was synthesized by the reaction and work-up in the same manner as in Step 3 of Example 22 and using the compound obtained in Example 41 and butyryl chloride.
MS(ESI+):613(M+H)
[α]_{D}²⁵ +76.1° (c 1.0,MeOH)

### Example 45

N-[trans-4-[[(3R,4S)-4-[(1R)-1-[3,5-Bis(trifluoromethyl)phenyl]ethoxy]-3-phenylpiperidin-1-yl] carbonyl] cyclohexyl]-2-methylpropanamide

The compound was synthesized by the reaction and work-up in the same manner as in Step 3 of Example 22 and using the compound obtained in Example 41 and isobutyryl chloride.
MS (ESI+) : 613 (M+H)

### Example 46

N-[trans-4-[[(3R,4S)-4-[(lR)-1-[3,5-Bis(trifluoromethyl)phenyl]ethoxy]-3-phenylpiperidin-1-yl]carbonyl]cyclohexyl]cyclopropanecarboxamide

The compound was synthesized by the reaction and work-up in the same manner as in Step 3 of Example 22 and using the compound obtained in Example 41 and cyclopropanecarbonyl chloride.
MS (ESI+) : 611 (M+H)

### Example 47

N-[trans-4-[[(3R,4S)-4-[(lR)-l-[3,5-Bis(trifluoromethyl)phenyl]ethoxy]-3-phenylpiperidin-1-yl]carbonyl]cyclohexyl]-N'-methylurea

The compound was synthesized by the reaction and work-up in the same manner as in Example 3 and using the compound obtained in Example 41.
MS(ESI+):600(M+H)
[α]_{D}²⁵ +77.7° (c 1.0,MeOH)

### Example 48

N-[trans-4-[[(3R,4S)-4-[(1R)-1-[3,5-Bis(trifluoromethyl)phenyl]ethoxy]-3-phenylpiperidin-1-yl]carbonyl]cyclohexyl]-2-furamide

The compound was synthesized by the reaction and work-up in the same manner as in the method described in Example 6 and using the compound obtained in Example 41 and furan-2-carboxylic acid.
MS (ESI+) : 637 (M+H)
[α]_{D}²⁵ +75.3° (c 1.0,MeOH)

### Example 49

N-[trans-4-[[(3R,4S)-4-[(1R)-1-[3,5-Bis(trifluoromethyl)phenyl]ethoxy]-3-phenylpiperidin-1-yl]carbonyl]cyclohexyl]methanesulfonamide

The compound was synthesized by the reaction and work-up in the same manner as in Step 3 of Example 22 and using the compound obtained in Example 41 and methanesulfonyl chloride.
MS (ESI+) : 621 (M+H)
[α]_{D}²⁵ +75.7° (c 1.0,MeOH)

### Example 50

N-[trans-4-[[(3R,4S)-4-[(1R)-1-[3,5-Bis(trifluoromethyl)phenyl]ethoxy]-3-phenylpiperidin-1-yl]carbonyl]cyclohexyl]ethanesulfonamide

The compound was synthesized by the reaction and work-up in the same manner as in Step 3 of Example 22 and using the compound obtained in Example 41 and ethanesulfonyl chloride.
MS (ESI+) : 635 (M+H)
[α]_{D}²⁵ +68.1° (c 1.0,MeOH)

### Example 51

N-[trans-4-[[(3R,4S)-4-[(1R)-1-[3,5-Bis(trifluoromethyl)phenyl]ethoxy]-3-phenylpiperidin-1-yl]carbonyl]cyclohexyl]thiophene-2-carboxamide

The compound was synthesized by the reaction and work-up in the same manner as in the method described in Example 6 and using the compound obtained in Example 41 and thiophene-2-carboxylic acid.
MS (ESI+) : 653 (M+H)
[α]_{D}²⁵ +75.7° (c 1.0,MeOH)

### Example 52

N-[trans-4-[[(3R,4S)-4-[(1R)-1-[3,5-Bis(trifluoromethyl)phenyl]ethoxy]-3-phenylpiperidin-1-yl]carbonyl]cyclohexyl]-1H-pyrrole-2-carboxamide

The compound was synthesized by the reaction and work-up in the same manner as in the method described in Example 6 and using the compound obtained in Example 41 and 1H-pyrrole-2-carboxylic acid.
MS (ESI+) : 636 (M+H)
[α]_{D}²⁵ +76.2° (c 1.0,MeOH)

### Example 53

1-[trans-4-[[(3R,4S)-4-[(1R)-1-[3,5-Bis(trifluoromethyl)phenyl]ethoxy]-3-phenylpiperidin-1-yl]carbonyl]cyclohexyl]-1H-pyrrole-2,5-dione

A solution of the compound obtained in Example 41 (202 mg), maleic anhydride (59 mg) and Et₃N (0.1 mL) in toluene (5 mL) was stirred at 90°C for 24 hr, water was added to the reaction mixture, and the product was extracted with ethyl acetate. The organic layer was washed with brine and dried, and the solvent was evaporated under reduced pressure. The obtained residue was dissolved in acetic anhydride (3 mL), and Et₃N (0.1 mL) was added. After stirring at 100°C for 4 hr, water was added to the reaction mixture, and the product was extracted with ethyl acetate. The organic layer was washed with brine and dried, and the solvent was evaporated under reduced pressure. The obtained residue was purified by silica gel column chromatography (ethyl acetate:hexane =1:4) to give the title compound (121 mg) as a colorless amorphous.
MS (ESI+) : 623 (M+H)

### Example 54

N-[cis-4-[[(3R,4S)-4-[(1R)-1-[3,5-Bis(trifluoromethyl)phenyl]ethoxy]-3-phenylpiperidin-1-yl]carbonyl]cyclohexyl]acetamide

The compound was synthesized by the reaction and work-up in the same manner as in the method described in Example 22 and using the compound obtained in Example 32 and cis-4-[(tert-butoxycarbonyl)amino]cyclohexanecarboxylic acid.
MS (ESI+) : 585 (M+H)

### Example 55

N-[trans-4-[[(3R,4S)-4-[(1R)-1-[3,5-Bis(trifluoromethyl)phenyl]ethoxy]-3-phenylpiperidin-1-yl]carbonyl]cyclohexyl]-2-(5-oxo-4,5-dihydro-1H-1,2,4-triazol-3-yl)acetamide

The compound was synthesized by the reaction and work-up in the same manner as in the method described in Example 6 and using the compound obtained in Example 41 and (5-oxo-4,5-dihydro-1H-1,2,4-triazol-3-yl) acetic acid synthesized by a known method (Australian Journal of Chemistry, Vol. 32, page 161 (1979)).
MS (ESI+) : 668 (M+H)

### Example 56

N-[trans-4-[[(3R,4S)-4-[(1R)-1-[3,5-Bis(trifluoromethyl)phenyl]ethoxy]-3-phenylpiperidin-l-yl]carbonyl]cyclohexyl]-2-(2,5-dioxoimidazolidin-4-yl)acetamide

The compound was synthesized by the reaction and work-up in the same manner as in the method described in Example 6 and using the compound obtained in Example 41 and (2,5-dioxoimidazolidin-4-yl)acetic acid synthesized by a known method (Journal of the American Chemical Society, vol. 69, page 1382 (1947)).
MS (ESI+) : 683 (M+H)

### Example 57

N-[trans-4-[[(3R,4S)-4-[(1R)-1-[3,5-Bis(trifluoromethyl)phenyl]ethoxy]-3-phenylpiperidin-1-yl]carbonyl]cyclohexyl]-2-(2-oxopiperidin-1-yl)acetamide

The compound was synthesized by the reaction and work-up in the same manner as in the method described in Example 6 and using the compound obtained in Example 41 and (2-oxopiperidin-1-yl)acetic acid synthesized by a known method (Heterocycles, vol. 55 (No. 10), page 1843 (200)).
MS (ESI+) : 682 (M+H)

### Example 58

N-[trans-4-[[(3R,4S)-4-[(1R)-1-[3,5-Bis(trifluoromethyl)phenyl]ethoxy]-3-phenylpiperidin-1-yl]carbonyl]cyclohexyl]-2-(1H-tetrazol-1-yl)acetamide

The compound was synthesized by the reaction and work-up in the same manner as in the method described in Example 6 and using the compound obtained in Example 41 and (1H-tetrazol-1-yl)acetic acid.
MS (ESI+) : 653 (M+H)

### Example 59

N-[trans-4-[[(3R,4S)-4-[(1R)-1-[3,5-Bis(trifluoromethyl)phenyl]ethoxy]-3-phenylpiperidin-1-yl]carbonyl]cyclohexyl]-5-chloropentanamide

The compound was synthesized by the reaction and work-up in the same manner as in Step 3 of Example 22 and using the compound obtained in Example 41 and 5-chlorovaleryl chloride.
MS (ESI+) : 662 (M+H)

### Example 60

N-[trans-4-[[(3R,4S)-4-[(1R)-1-[3,5-Bis(trifluoromethyl)phenyl]ethoxy]-3-phenylpiperidin-1-yl]carbonyl]cyclohexyl]-2-(2,5-dioxoimidazolidin-1-yl)acetamide

The compound was synthesized by the reaction and work-up in the same manner as in the method described in Example 6 and using the compound obtained in Example 41 and (2,5-dioxoimidazolidin-1-yl)acetic acid synthesized by a known method (Journal of the Chemical Society, Perkin Transactions 1, Vol. 12, 3175 (1988)).
MS (ESI+) : 683 (M+H)

### Example 61

Methyl trans-4-[[(3R,4S)-4-[(1R)-1-[3,5-bis(trifluoromethyl)phenyl]ethoxy]-3-phenylpiperidin-1-yl]carbonyl]cyclohexylcarbamate

The compound was synthesized by the reaction and work-up in the same manner as in Step 3 of Example 22 and using the compound obtained in Example 41 and ethyl chloroformate.
MS(ESI+):601(M+H)
[α]_{D}²⁵ +71.2° (c 1.0,MeOH)

### Example 62

Ethyl trans-4-[[(3R,4S)-4-[(1R)-1-[3,5-bis(trifluoromethyl)phenyl]ethoxy]-3-phenylpiperidin-1-yl]carbonyl]cyclohexylcarbamate

The compound was synthesized by the reaction and work-up in the same manner as in Step 3 of Example 22 and using the compound obtained in Example 41 and ethyl chloroformate.
MS (ESI+) : 615 (M+H)
[α]_{D}²⁵ +70.7° (c 1.0,MeOH)

### Example 63

N-[trans-4-[[(3R,4S)-4-[(1R)-1-[3,5-Bis(trifluoromethyl)phenyl]ethoxy]-3-phenylpiperidin-1-yl]carbonyl]cyclohexyl]-N'-ethylurea

The compound was synthesized by the reaction and work-up in the same manner as in Example 3 and using the compound obtained in Example 41 and ethyl isocyanate.
MS (ESI+) : 614 (M+H)

### Example 64

N-[trans-4-[[(3R,4S)-4-[(1R)-1-[3,5-Bis(trifluoromethyl)phenyl]ethoxy]-3-phenylpiperidin-1-yl]carbonyl]cyclohexyl]-N'-propylurea

The compound was synthesized by the reaction and work-up in the same manner as in Example 3 and using the compound obtained in Example 41 and propyl isocyanate.
MS (ESI+) : 628 (M+H)

### Example 65

N-[trans-4-[[(3R, 4S)-4-[(1R)-1-[3,5-Bis(trifluoromethyl)phenyl]ethoxy]-3-phenylpiperidin-1-yl]carbonyl]cyclohexyl]-N'-isopropylurea

The compound was synthesized by the reaction and work-up in the same manner as in Example 3 and using the compound obtained in Example 41 and isopropyl isocyanate.
MS (ESI+) : 628 (M+H)

### Example 66

N'-[trans-4-[[(3R,4S)-4-[(1R)-1-[3,5-Bis(trifluoromethyl)phenyl]ethoxy]-3-phenylpiperidin-1-yl]carbonyl]cyclohexyl]-N,N-dimethylurea

The compound was synthesized by the reaction and work-up in the same manner as in the method described in Example 17 and using the compound obtained in Example 41 and N,N-dimethylcarbamoyl chloride.
MS (ESI+) : 614 (M+H)

### Example 67

Methyl trans-4-[[(3R,4S)-4-[(1R)-1-(3,5-bis(trifluoromethyl)phenyl]ethoxy]-3-phenylpiperidin-1-yl]carbonyl]cyclohexanecarboxylate

The compound was synthesized by the reaction and work-up in the same manner as in the method described in Example 6 and using the compound obtained in Example 41 and monomethyl trans-cyclohexane-1,4-dicarboxylate synthesized by a known method (Journal of Medicinal Chemistry, Vol. 47 (9), 2318, 2004).
MS (ESI+) : 586 (M+H)

### Example 68

trans-4-[[(3R,4S)-4-[(1R)-1-[3,5-Bis(trifluoromethyl)phenyl]ethoxy]-3-phenylpiperidin-1-yl]carbonyl]-N-methylcyclohexanecarboxamide

### (Step 1)

To a solution of the compound obtained in Example 67 (92 mg) in MeOH (3 mL) was added 1N aqueous sodium hydroxide solution (0.3 mL), and the mixture was stirred at 50°C for 24 hr. Water was added to the reaction mixture, and the mixture was neutralized with 1N hydrochloric acid. The product was extracted with ethyl acetate. The organic layer was washed with brine and dried, and the solvent was evaporated under reduced pressure to give crude trans-4-[[(3R,4S)-4-[(1R)-1-[3,5-bis(trifluoromethyl)phenyl]ethoxy]-3-phenylpiperidin-1-yl]carbonyl]cyclohexanecarboxylic acid.

### (Step 2)

To a solution of the compound obtained in Step 1 in THF (5 ml) were added 40% aqueous methylamine solution (0.032 mL) and DEPC (0.050 mL), and the mixture was stirred at room temperature for 24 hr. Water was added to the reaction mixture, and the product was extracted with ethyl acetate. The organic layer was washed with brine and dried, and the solvent was evaporated under reduced pressure. The obtained residue was purified by silica gel column chromatography (ethyl acetate:hexane =1:1) to give the title compound (58 mg) as a white powder.
MS (ESI+) : 585 (M+H)

### Example 69

trans-4-[[(3R,4S)-4-[(1R)-1-[3,5-Bis(trifluoromethyl)phenyl]ethoxy]-3-phenylpiperidin-1-yl]carbonyl]-N-ethylcyclohexanecarboxamide

The compound was synthesized by the reaction and work-up in the same manner as in Step 2 of Example 68 and using the compound obtained in Step 1 of Example 68 and 70% aqueous ethylamine solution.
MS (ESI+) : 599 (M+H)

### Example 70

Ethyl 4-[[[(3R,4S)-4-[(1R)-1-[3,5-bis(trifluoromethyl)phenyl]ethoxy]-3-phenylpiperidin-1-yl]carbonyl]amino]piperidine-1-carboxylate

### (Step 1)

To a solution of ethyl 4-amino-1-piperidinecarboxylate (1.7 g) and Et₃N (2.8 mL) in CH₂Cl₂ (20 mL) was added 4-nitrophenyl chloroformate (2.4 g) at 0°C, and the mixture was stirred at room temperature for 2 hr. Water was added to the reaction mixture, and the product was extracted with ethyl acetate. The organic layer was washed with brine and dried, and the solvent was evaporated under reduced pressure. The obtained residue was purified by silica gel column chromatography (ethyl acetate:hexane =1:1) to give ethyl 4-[[(4-nitrophenoxy)carbonyl]amino]piperidine-1-carboxylate (1.5 g) as a white powder.
¹H-NMR(CDCl₃):δ 1.27(3H,t,J=6.0Hz),1.44(2H,dq,J=3.0,12.0Hz),1.98-2.09(2H,m),2.94(2H,t,J=12.0Hz),3.67-3.83(1H,m),4.06-4.27(2H,m),4.15(2H,q,J=6.0Hz),5.07(1H,d,J=9.0Hz),7.28-7.36(2H,m),8.21-8.30(2H,m)

### (Step 2)

To a solution of the compound obtained in Example 32 (166 mg) and Et₃N (0.8 mL) in THF (5 mL) was added the compound obtained in Step 1 (120 mg), and the mixture was stirred at room temperature for 72 hr. Water was added to the reaction mixture, and the product was extracted with ethyl acetate. The organic layer was washed with brine and dried, and the solvent was evaporated under reduced pressure. The obtained residue was purified by preparative HPLC to give the title compound (180 mg) as a white powder.
MS (ESI+) : 616 (M+H)
[α]_{D}²⁵ +68.4° (c 1.0, MeOH)

### Example 71

(3R,4S)-N-(1-acetylpiperidin-4-yl)-4-[(1R)-1-[3,5-bis(trifluoromethyl)phenyl]ethoxy]-3-phenylpiperidine-1-carboxamide

### (Step 1)

To a solution of the compound obtained in Example 32 (505 mg) and K₂CO₃ (240 mg) in DMF (10 mL) was added tert-butyl 4-(4-nitrophenoxycarbonylamino)piperidine-1-carboxylate (380 mg) synthesized by a known method (WO03/101964), and the mixture was stirred at 100°C for 14 hr. Water was added to the reaction mixture, and the product was extracted with ethyl acetate. The organic layer was washed with brine and dried, and the solvent was evaporated under reduced pressure to give crude tert-butyl 4-[[[(3R,4S)-4-[(1R)-1-[3,5-bis(trifluoromethyl)phenyl]ethoxy]-3-phenylpiperidin-1-yl]carbonyl]amino]piperidine-1-carboxylate as an oil.

### (Step 2)

A solution of the compound obtained in Step 1 in 4N hydrogen chloride/ethyl acetate solution (2 mL) was stirred at room temperature for 14 hr. The solvent was evaporated under reduced pressure to give crude (3R,4S)-4-[(1R)-1-[3,5-bis(trifluoromethyl)phenyl]ethoxy]-3-phenyl-N-piperidin-4-ylpiperidine-1-carboxamide hydrochloride as an oil.

### (Step 3)

The title compound was obtained as a white powder by the reaction and work-up in the same manner as in Step 3 of Example 22 and using the compound obtained in Step 2.
MS (ESI+) : 586 (M+H)

### Example 72

(3R,4S)-4-[(1R)-1-[3,5-Bis(trifluoromethyl)phenyl]ethoxy]-3-phenyl-N-(1-propionylpiperidin-4-yl)piperidine-1-carboxamide

The compound was synthesized by the reaction and work-up in the same manner as in Step 3 of Example 22 and using the compound obtained in Step 2 of Example 71 and propionyl chloride.
MS (ESI+) : 600 (M+H)

### Example 73

N-[1-[[(3R,4S)-4-[(1R)-1-[3,5-bis(trifluoromethyl)phenyl]ethoxy]-3-phenylpiperidin-1-yl]carbonyl]piperidin-4-yl]acetamide

### (Step 1)

To a solution of 4-Boc-aminopiperidine (2.0 g) and Et₃N (2.8 mL) in CH₂Cl₂ (20 mL) was added 4-nitrophenyl chloroformate (2.4 g) at 0°C, and the mixture was stirred at room temperature for 2 hr. Water was added to the reaction mixture, and the product was extracted with ethyl acetate. The organic layer was washed with brine and dried, and the solvent was evaporated under reduced pressure. The obtained residue was purified by silica gel column chromatography (ethyl acetate:hexane=1:1) to give 4-nitrophenyl 4-[(tert-butoxycarbonyl)amino]piperidine-1-carboxylate (1.5 g) as a white powder.
¹H-NMR(CDCl₃):δ 1.35-1.53(11H,m),1.98-2.13(2H,br),3.09(2H,td,J=13.2,39.3Hz),3.61-3.78(1H,m),4.11-4.28(2H,m),4.43-4.54(1H,m),7.25-7.30(2H,m),8.20-8.27(2H,m)

### (Step 2)

Crude tert-butyl [1-[(3R,4S)-4-[(1R)-1-[3,5-bis(trifluoromethyl)phenyl]ethoxy]-3-phenylpiperidine-1-carbonyl]piperidin-4-yl]carbamate was obtained as an oil by the reaction and work-up in the same manner as in Step 1 of Example 71 and using the compound obtained in Step 1 and the compound obtained in Example 32.

### (Step 3)

Crude 1-[[(3R,4S)-4-[(1R)-1-[3,5-bis(trifluoromethyl)phenyl]ethoxy]-3-phenylpiperidin-1-yl]carbonyl]piperidine-4-amine hydrochloride was obtained as a white powder by the reaction and work-up in the same manner as in Step 2 of Example 71 and using the compound obtained in Step 2.

### (Step 4)

The title compound was obtained as a white powder by the reaction and work-up in the same manner as in Step 3 of Example 22 and using the compound obtained in Step 3.
MS (ESI+) : 586 (M+H)
[α]_{D}²⁵ +69.1° (c 1.0,MeOH)

### Example 74

N-[1-[[(3R,4S)-4-[(1R)-1-[3,5-bis(trifluoromethyl)phenyl]ethoxy]-3-phenylpiperidin-1-yl]carbonyl]piperidin-4-yl]propanamide

The title compound was obtained as a white powder by the reaction and work-up in the same manner as in Step 3 of Example 22 and using the compound obtained in Step 3 of Example 73 and propanoyl chloride.
MS(ESI+):600(M+H)

### Example 75

N-[4-[[(3R,4S)-4-[(1R)-1-[3,5-Bis(trifluoromethyl)phenyl]ethoxy]-3-phenylpiperidin-1-yl]carbonyl]phenyl]acetamide

### (Step 1)

Crude tert-butyl [4-[(3R,4S)-4-[(1R)-1-[3,5-bis(trifluoromethyl)phenyl]ethoxy]-3-phenylpiperidine-1-carbonyl]phenyl]carbamate was obtained as an oil obtained by the reaction and work-up in the same manner as in the method described in Example 6 and using the compound obtained in Example 32 and 4-tert-butoxycarbonylaminobenzoic acid.

### (Step 2)

Crude (4-aminophenyl)-[(3R,4S)-4-[(1R)-1-(3,5-bis(trifluoromethyl)phenyl]ethoxy)-3-phenylpiperidin-1-yl]methanone hydrochloride was obtained as a white powder by the reaction and work-up in the same manner as in Step 2 of Example 71 and using the compound obtained in Step 1.

### (Step 3)

The title compound was obtained as a white powder by the reaction and work-up in the same manner as in Step 3 of Example 22 and using the compound obtained in Step 2.
MS (ESI+) : 579 (M+H)

### Example 76

4-[[(3R,4S)-4-[(1R)-1-[3,5-Bis(trifluoromethyl)phenyl]ethoxy]-3-phenylpiperidin-1-yl]carbonyl]piperidine-2,6-dione

The compound was synthesized by the reaction and work-up in the same manner as in the method described in Example 6 and using the compound obtained in Example 32 and 2,6-dioxopiperidine-4-carboxylic acid synthesized by a known method (US2874158).
MS (ESI+) : 557 (M+H)
¹H-NMR(CDCl₃):δ 1.36-1.44(3H,m),1.54-1.83(1H,m),2.08-2.24(1H,m),2.56-2.93(5H,m),3.03-4.02(5H,m),4.40(1H,dq, J=6.0 and 27.0Hz),4.51-4.65(1H,m),7.02-7.16(2H,m),7.17-7.31(5H,m),7.65(1H,s),7.88-8.03(1H,m)
[α]_{D}²⁵ +74.8° (c 1.0, MeOH)

### Example 77

4-[[(3R,4S)-4-[(1R)-1-[3,5-Bis(trifluoromethyl)phenyl]ethoxy]-3-phenylpiperidin-1-yl]carbonyl]-1-methylpiperidine-2,6-dione

To a solution of the compound obtained in Example 76 (79 mg) and Et₃N (0.040 mL) in acetonitrile (5 mL) was added methyl iodide (0.036 mL), and stirred at 80°C for 14 hr. Water was added to the reaction mixture, and the product was extracted with ethyl acetate. The organic layer was washed with brine and dried, and the solvent was evaporated under reduced pressure. The obtained residue was purified by preparative HPLC to give the title compound (74 mg) as an oil.
MS (ESI+) : :571 (M+H)

### Example 78

5-[2-[(3R,4S)-4-[(1R)-1-[3,5-Bis(trifluoromethyl)phenyl]ethoxy]-3-phenylpiperidin-1-yl]-2-oxoethyl]imidazolidine-2,4-dione

The compound was synthesized by the reaction and work-up in the same manner as in the method described in Example 6 and using the compound obtained in Example 32 and (2,5-dioxoimidazolidin-4-yl)acetic acid synthesized by a known method (Journal of the American Chemical Society, Vol. 69, page 1382, 1947).
MS (ESI+) : 558 (M+H)

### Example 79

(3R,4S)-4-[(1R)-1-[3,5-Bis(trifluoromethyl)phenyl]ethoxy]-3-phenyl-1-(1H-tetrazol-1-ylacetyl)piperidine

The compound was synthesized by the reaction and work-up in the same manner as in the method described in Example 6 and using the compound obtained in Example 32 and 1H-1,2,3,4-tetrazole-1-acetic acid.
MS (ESI+) : 528 (M+H)

### Example 80

5-[2-[(3R,4S)-4-[(1R)-1-[3,5-Bis(trifluoromethyl)phenyl]ethoxy]-3-phenylpiperidin-1-yl]-2-oxoethyl]-2,4-dihydro-3H-1,2,4-triazol-3-one

The compound was synthesized by the reaction and work-up in the same manner as in the method described in Example 6 and using the compound obtained in Example 32 and (5-oxo-2,5-dihydro-1H-1,2,4-triazol-3-yl)acetic acid synthesized by a known method (Australian Journal of Chemistry, vol. 32, page 161 (1979).
MS (ESI+) : 543 (M+H)

### Example 81

(3R,4S)-4-[(1R)-1-[3,5-Bis(trifluoromethyl)phenyl]ethoxy]-3-phenyl-1-(1H-1,2,4-triazol-1-ylacetyl) piperidine

The compound was synthesized by the reaction and work-up in the same manner as in the method described in Example 6 and using the compound obtained in Example 32 and ([1,2,4]triazol-1-yl)acetic acid.
MS (ESI+) : 527 (M+H)

### Example 82

(3R,4S)-N-[(1R)-1-[2-Methoxy-5-[5-(trifluoromethyl)-1H-tetrazol-1-yl]phenyl]ethyl]-3-phenylpiperidine-4-amine hydrochloride, and (3R,4S)-N-[(1S)-1-[2-methoxy-5-[5-(trifluoromethyl)-1H-tetrazol-1-yl]phenyl]ethyl]-3-phenylpiperidine-4-amine hydrochloride (diastereo mixture)

### (Step 1)

To a solution of 2-methoxy-5-[5-(trifluoromethyl)-1H-tetrazol-1-yl]benzaldehyde (3.5 g) synthesized by a known method (J. Labelled Cpd. Radiopharm., Vol. 43, pp. 29-45 (2000)) in Et₂O (15 mL) and THF (25 mL) was added 1M methyl magnesium bromide/Et₂O solution (40 mL) at -78°C, and the mixture was stirred at room temperature for 2 hr. Water was added to the reaction mixture, and the product was extracted with ethyl acetate. The organic layer was washed with brine and dried, and the solvent was evaporated under reduced pressure. The obtained residue was purified by silica gel column chromatography (ethyl acetate:hexane =1:2) to give 1-[2-methoxy-5-[5-(trifluoromethyl)-1H-tetrazol-1-yl]phenyl]ethanol (2.9 g) as a colorless oil.
MS(ESI+):289(M+H)

### (Step 2)

To a solution of the compound obtained in Step 1 (2.9 g) in CH₂Cl₂ (100 mL) were added molecular sieves 4A (5 g) and PDC (pyridinium dichlomate) (7.6 g), and the mixture was stirred at room temperature for 14 hr. Ethyl acetate was added to the reaction solution and the mixture was filtered through celite. The filtrate was concentrated under reduced pressure. The obtained residue was purified by silica gel column chromatography (ethyl acetate:hexane =1:2) to give 1-[2-methoxy-5-[5-(trifluoromethyl)-1H-tetrazol-1-yl]phenyl]ethanone (1.7 g) as a white powder.
MS (ESI+) : 287 (M+H)

### (Step 3)

To a solution of the compound obtained in Step 2 (0.68 g), tert-butyl (3R,4S)-4-amino-3-phenylpiperidine-1-carboxylate (1.3 g) synthesized by a known method (WO03/101964) and Et₃N (1.0 mL) in CH₂Cl₂ (40 mL) was added 1.5 M titanium tetrachloride/CH₂Cl₂ (0.8 mL) solution at 0°C, and the mixture was stirred at room temperature for 1 hr. The reaction solution was cooled to 0°C, a solution of NaBH₃CN (0.45 g) in MeOH (8 mL) was added and the mixture was stirred at room temperature for 30 min. Water was added to the reaction mixture, and the product was extracted with ethyl acetate. The organic layer was washed with brine and dried, and the solvent was evaporated under reduced pressure. The obtained residue was purified by silica gel column chromatography (ethyl acetate:hexane =1:2) to give a mixture (0.51 g) of tert-butyl (3R,4S)-4-[[(1R)-1-[2-methoxy-5-[5-(trifluoromethyl)-1H-tetrazol-1-yl]phenyl]ethyl]amino]-3-phenylpiperidine-1-carboxylate and tert-butyl (3R,4S)-4-[[(1S)-1-[2-methoxy-5-[5-(trifluoromethyl)-1H-tetrazol-1-yl]phenyl]ethyl]amino]-3-phenylpiperidine-1-carboxylate as a colorless oil.
¹H-NMR (CDCl₃) : δ
1.01(3H×1/2,d,J=9.0Hz),1.20(3H×1/2,d,J=9.0Hz),1.40-1.75(1H,m),1.44(9H,s),1.84-1.90(1H,m),2.84-3.04(2H,m),3.50 4.18(5H,m),3.73(3H×1/2,s),3.89(3H×1/2,s),6.87-7.36(8H,m)

### (Step 4)

The compound obtained in Step 3 (0.15 g) was dissolved in MeOH (2 mL), 4N hydrogen chloride/ethyl acetate solution (0.3 mL) was added and the mixture was stirred at 65°C for 30 min. The solvent was evaporated under reduced pressure to give the title compound (0.14 g, diastereo mixture) as a white powder.
MS (ESI+) : 447 (M-HC1+H)

### Example 83

(3R,4S)-1-[(1-Acetylpiperidin-4-yl)carbonyl]-N-[1-[2-methoxy-5-[5-(trifluoromethyl)-1H-tetrazol-1-yl]phenyl]ethyl]-3-phenylpiperidine-4-amine (retention time: short)

To a solution of the compound obtained in Example 82 (150 mg), 1-acetylpiperidine-4-carboxylic acid (80 mg), HOBt·H₂O (68 mg) and Et₃N (87 µL) in DMF (4 mL) was added WSC·HCl (90 mg), and the mixture was stirred at room temperature for 14 hr. Water was added to the reaction mixture, and the product was extracted with ethyl acetate. The organic layer was washed with water, 3% KHSO₄ aqueous solution and brine and dried, and the solvent was evaporated under reduced pressure. The obtained residue was purified by silica gel column chromatography (ethyl acetate:hexane=5:1), and the obtained diastereo mixture was purified by chiral column chromatography. The preparative fraction having a short retention time was concentrated to give the title compound (45 mg) as a colorless amorphous.
MS(ESI+):600(M+H)
[α]_{D}²⁵ -12.7° (c 0.2, MeOH)
purification conditions by chiral column chromatography column: CHIRALPAK AD 50 mmIDx5OO mmL
solvent: hexane/ethanol=85/15
flow rate: 80 mL/min
temperature: 30°C
detection method: UV 254 nm

### Example 84

(3R,4S)-1-[(1-Acetylpiperidin-4-yl)carbonyl]-N-[1-[2-methoxy-5-[5-(trifluoromethyl)-1H-tetrazol-1-yl]phenyl]ethyl]-3-phenylpiperidine-4-amine (retention time: long)

The preparative fraction having a long retention time described in Example 83 was concentrated to give the title compound (100 mg).
MS (ESI+) : 600 (M+H)
[α]_{D}²⁵ -41.9° (c 0.2,MeOH)
purification conditions by chiral column chromatography column: CHIRALPAK AD 50 mmIDx500 mmL
solvent: hexane/ethanol=85/15
flow rate: 80 mL/min
temperature: 30°C
detection method: UV 254 nm

### Example 85

N-[[2-[(3R,4S)-4-[1-[2-Methoxy-5-(5-trifluoromethyl)-1H-tetrazol-1-yl]phenyl]ethylamino]-3-phenylpiperidin-1-yl]-2-oxoethyl]acetamide (retention time: short)

A diastereo mixture was obtained by the reaction and work-up in the same manner as in the method described in Example 6 and using the compound obtained in Example 82. The compound was purified by chiral column chromatography. The preparative fraction having a short retention time was concentrated to give the title compound (37 mg) as a colorless amorphous.
MS (ESI+) : 546 (M+H)
[α]_{D}²⁵ +50.4°(c 0.14, MeOH)
purification conditions by chiral column chromatography column: YMC-Pack ODS-A 30 mmIDx250 mmL
solvent: acetonitrile/50mM KH₂PO₄=50/50 (pH 8)
flow rate: 20 mL/min
temperature: 30°C
detection method: UV 254 nm

### Example 86

N-[[2-[(3R,4S)-4-[1-[2-Methoxy-5-(5-trifluoromethyl)-1H-tetrazol-1-yl]phenyl]ethylamino]-3-phenylpiperidin-1-yl]-2-oxoethyl]acetamide (retention time: long)

The preparative fraction having a long retention time described in Example 85 was concentrated to give the title compound (35 mg).
MS (ESI+) : 546 (M+H)
[α]_{D}²⁵ +8.1° (c 0.17,MeOH)
purification conditions by chiral column chromatography column: YMC-Pack ODS-A 30 mmID×250 mmL
solvent: acetonitrile/50 mM KH₂P0₄=50/50 (pH 8)
flow rate: 20 mL/min
temperature: 30°C
detection method: UV 254 nm

The compounds described in Example 32 to Example 86 are as follows (Tables 3-6).

**Table 3**

| | | | | | | | |
|---|---|---|---|---|---|---|---|
| 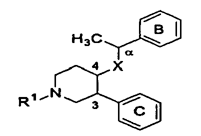 | | | | | | | |

| Ex. No. | stereochemistry | R¹ | X | 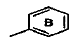 | 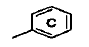 | aditive | MS (ESI) |
|---|---|---|---|---|---|---|---|
| 32 | (3R,4S), α-R | H | O | 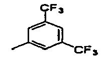 | 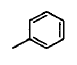 | HCl | 418 |
| 33 | (3R,4S), α-S | H | O | 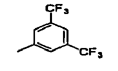 | 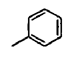 | HCl | 418 |
| 34 | (3R,4S), α-R smaller Rt | 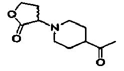 | O | 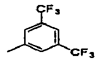 | 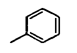 | HCl | 613 |
| 35 | (3R,4S), α-R larger Rt | 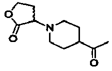 | O | 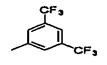 | 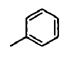 | HCl | 613 |
| 36 | (3R,4S), α-R | 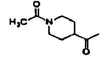 | O | 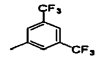 | 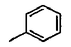 | | 571 |
| 37 | (3R,4S), α-S | 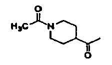 | O | 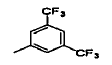 | 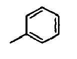 | | 571 |
| 38 | (3R,4S), α-R | 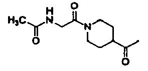 | O | 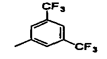 | 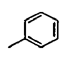 | | 628 |
| 39 | (3R,4S), α-R | 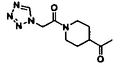 | O | 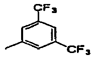 | 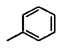 | | 639 |
| 40 | (3R,4S), α-R | 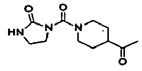 | O | 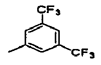 | 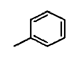 | | 641 |
| 41 | (3R,4S). α-R | 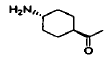 | O | 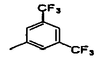 | 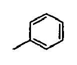 | HCl | 543 |
| 42 | (3R,4S), α-R | 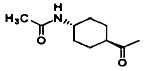 | O | 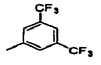 | 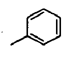 | | 585 |
| 43 | (3R,4S), α-R | 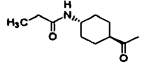 | O | 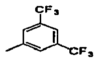 | 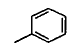 | | 599 |
| 44 | (3R,4S), α-R | 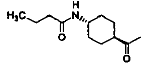 | O | 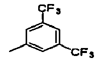 | 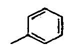 | | 613 |
| 45 | (3R,4S), α-R | 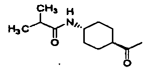 | O | 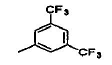 | 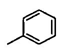 | | 613 |
| 46 | (3R,4S), α-R | 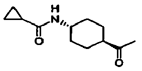 | O | 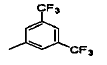 | 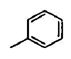 | | 611 |
| 47 | (3R,4S), α-R | 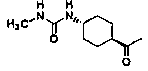 | O | 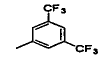 | 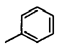 | | 600 |
| 48 | (3R,4S), α-R | 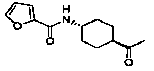 | O | 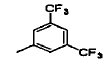 | 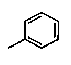 | | 637 |

**Table 4**

| | | | | | | | |
|---|---|---|---|---|---|---|---|
| 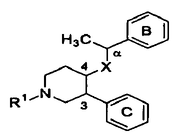 | | | | | | | |

| Ex. No. | Stereochemistry | R¹ | X | 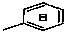 | 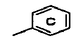 | additive | MS (ESI) |
|---|---|---|---|---|---|---|---|
| 49 | (3R,4S), α-R | 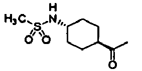 | O | 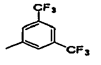 | 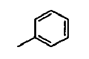 | | 621 |
| 50 | (3R,4S), α-R | 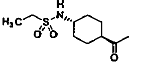 | O | 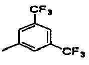 | 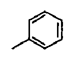 | | 635 |
| 51 | (3R,4S), α-R | 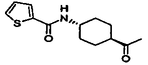 | O | 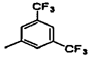 | 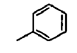 | | 653 |
| 52 | (3R,4S), α-R | 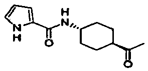 | O | 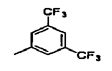 | 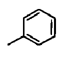 | | 636 |
| 53 | (3R,4S), α-R | 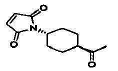 | O | 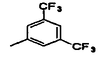 | 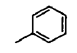 | | 623 |
| 54 | (3R,4S), α-R | 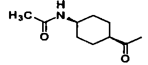 | O | 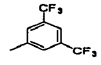 | 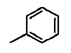 | | 585 |
| 55 | (3R,4S), α-R | 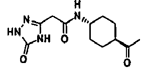 | O | 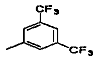 | 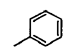 | | 668 |
| 56 | (3R,4S), α-R | 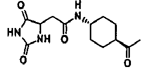 | O | 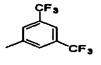 | 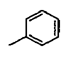 | | 683 |
| 57 | (3R,4S), α-R | 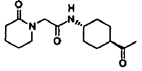 | O | 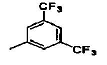 | 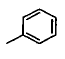 | | 682 |
| 58 | (3R,4S), α-R | 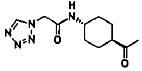 | O | 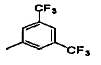 | 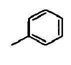 | | 653 |
| 59 | (3R,4S), α-R | 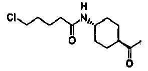 | O | 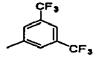 | 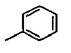 | | 662 |
| 60 | (3R,4S), α-R | 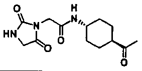 | O | 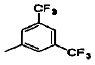 | 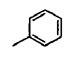 | | 683 |
| 61 | (3R,4S), α-R | 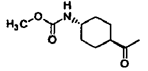 | O | 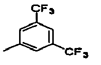 | 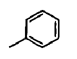 | | 601 |
| 62 | (3R,4S), α-R | 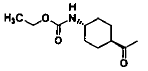 | O | 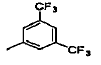 | 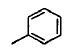 | | 615 |
| 63 | (3R,4S), α-R | 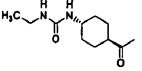 | O | 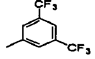 | 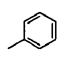 | | 614 |
| 64 | (3R,4S), α-R | 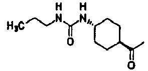 | O | 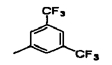 | 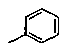 | | 628 |
| 65 | (3R,4S), α-R | 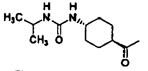 | O | 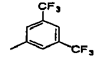 | 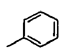 | | 628 |

**Table 5**

| | | | | | | |
|---|---|---|---|---|---|---|
| 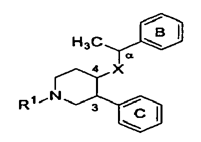 | | | | | | |

| Ex. No. | Stereochemistry | R¹ | X | 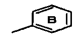 | 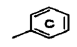 | additive MS (ESI) |
|---|---|---|---|---|---|---|
| 66 | (3R,4S), α-R | 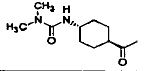 | O | 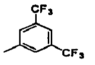 | 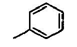 | 614 |
| 67 | (3R,4S), α-R | 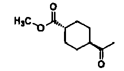 | O | 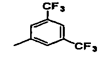 | 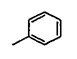 | 586 |
| 68 | (3R,4S), α-R | 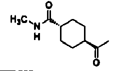 | O | 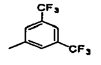 | 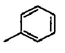 | 585 |
| 69 | (3R,4S), α-R | 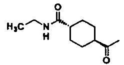 | O | 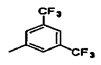 | 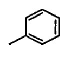 | 599 |
| 70 | (3R,4S), α-R | 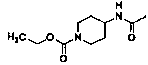 | O | 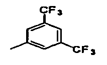 | 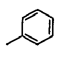 | 616 |
| 71 | (3R,4S), α-R | 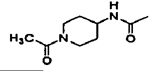 | O | 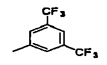 | 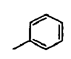 | 586 |
| 72 | (3R,4S), α-R | 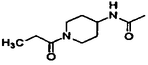 | O | 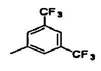 | 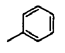 | 600 |
| 73 | (3R,4S), α-R | 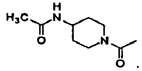 | O | 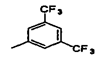 | 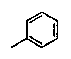 | 586 |
| 74 | (3R,4S), α-R | 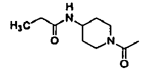 | O | 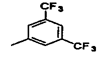 | 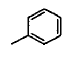 | 600 |
| 75 | (3R,4S), α-R | 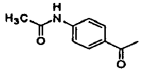 | O | 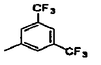 | 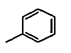 | 579 |
| 76 | (3R,4S), α-R | 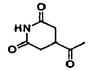 | O | 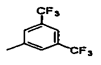 | 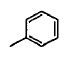 | 557 |
| 77 | (3R,4S), α-R | 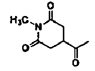 | O | 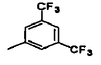 | 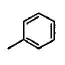 | 571 |
| 78 | (3R,4S), α-R | 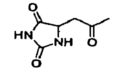 | O | 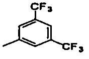 | 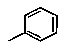 | 558 |
| 79 | (3R,4S), α-R | 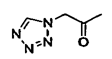 | O | 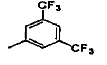 | 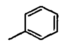 | 528 |
| 80 | (3R,4S), α-R | 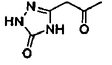 | O | 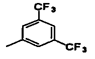 | 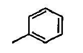 | 543 |
| 81 | (3R,4S), α-R | 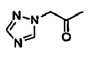 | O | 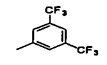 | 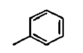 | 527 |

**Table 6**

| | | | | | | | |
|---|---|---|---|---|---|---|---|
| 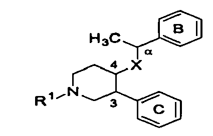 | | | | | | | |

| Ex. No. | Stereochemistry | R¹ | X | 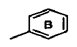 | 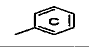 | additive | MS (ESI) |
|---|---|---|---|---|---|---|---|
| 82 | (3R,4S), α-RS | H | NH | 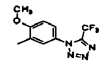 | 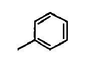 | HCl | 447 |
| 83 | (3R,4S), smaller Rt | 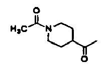 | NH | 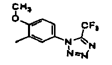 | 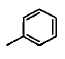 | | 600 |
| 84 | (3R,4S), larger Rt | 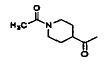 | NH | 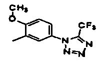 | 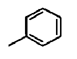 | | 600 |
| 85 | (3R,4S), smaller Rt | 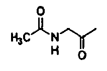 | NH | 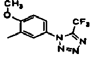 | 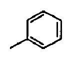 | | 546 |
| 86 | (3R,4S), larger Rt | 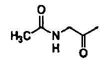 | NH | 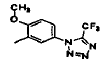 | 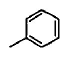 | | 546 |

### Example 87

1-{1-[((3R,4S)-4-{(1R)-1-[3,5-Bis(trifluoromethyl)phenyl]ethoxy}-3-phenylpiperidin-1-yl)carbonyl]piperidin-4-yl}pyrrolidin-2-one

### (Step 1)

To a solution of the compound obtained in Example 185 (1.0 g) and Et₃N (0.48 mL) in CH₂Cl₂ (15 mL) was added 4-nitrophenyl chloroformate (0.41 g) at 0°C, and the mixture was stirred at room temperature for 2 hr. Water was added to the reaction mixture, and the product was extracted with ethyl ace tate. The organic layer was washed with saturated aqueous ammonium chloride solution and brine and dried, and the solvent was evaporated under reduced pressure. The obtained residue was purified by silica gel column chromatography (ethyl acetate:hexane=1:4) to give 4-nitrophenyl (3R,4S)-4-{(1R)-1-[3,5-bis(trifluoromethyl)phenyl]ethoxy}-3-phenylpiperidine-1-carboxylate (1.07 g) as a colorless oil.

### (Step 2)

To a solution of the compound obtained in Step 1 (0.15 g) and K₂CO₃ (0.071 g) in DMF (5 mL) was added 1-piperidin-4-ylpyrrolidin-2-one (0.087 g) synthesized by a known method (JP/59065071), and the mixture was stirred at 140°C for 4 hr. Water was added to the reaction mixture, and the product was extracted with ethyl acetate. The organic layer was washed with saturated aqueous ammonium chloride solution and brine and dried, and the solvent was evaporated under reduced pressure. The obtained residue was purified by silica gel column chromatography (ethyl acetate:hexane:methanol=2:1:0.1) to give the title compound (0.13 g) as a pale-yellow oil.
MS (ESI+) : 612 (M+H)

### Example 88

1-{4-[((3R,4S)-4-{(1R)-1-[3,5-Bis(trifluoromethyl)phenyl]ethoxy}-3-phenylpiperidin-1-yl)carbonyl]piperazin-4-yl}pyrrolidin-2-one

The compound was synthesized by the reaction and work-up in the same manner as in Step 2 of Example 87 and using the compound obtained in Step 1 of Example 87 and 1-piperazin-1-ylpyrrolidin-2-one.
MS(ESI+):613(M+H)

### Example 89

1-{4-[((3R,4S)-4-{(1R)-1-[3,5-Bis(trifluoromethyl)phenyl]ethoxy}-3-phenylpiperidin-1-yl)carbonyl]piperazin-1-yl}piperidin-2-one

The compound was synthesized by the reaction and work-up in the same manner as in Step 2 of Example 87 and using the compound obtained in Step 1 of Example 87 and 1-piperazin-1-ylpiperidin-2-one.
MS (ESI+) : 627 (M+H)

### Example 90

4-{1-[((3R,4S)-4-{(1R)-1-[3,5-Bis(trifluoromethyl)phenyl]ethoxy}-3-phenylpiperidin-1-yl)carbonyl]piperidin-4-yl}morpholin-3-one

The compound was synthesized by the reaction and work-up in the same manner as in Step 2 of Example 87 and using the compound obtained in Step 1 of Example 87 and 4-piperazin-4-ylmorpholin-3-one synthesized by a known method (WO2005/012297).
MS (ESI+) : 628 (M+H)

### Example 91

(3R,4S)-4-{(1R)-1-[3,5-Bis(trifluoromethyl)phenyl]ethoxy}-1-{[4-(1,1-dioxidoisothiazolidin-2-yl)piperidin-1-yl]carbonyl}-3-phenylpiperidine

The compound was synthesized by the reaction and work-up in the same manner as in Step 2 of Example 87 and using the compound obtained in Step 1 of Example 87 and 4-(1,1-dioxidoisothiazolidin-2-yl)piperidine synthesized by a known method (WO2004/037810).
MS (ESI+) : 648 (M+H)

### Example 92

3-{1-[((3R,4S)-4-{(1R)-1-[3,5-Bis(trifluoromethyl)phenyl]ethoxy}-3-phenylpiperidin-1-yl)carbonyl]piperidin-4-yl}-1,3-oxazolidin-2-one

The compound was synthesized by the reaction and work-up in the same manner as in Step 2 of Example 87 and using the compound obtained in Step 1 of Example 87 and 3-piperidin-4-yl-1,3-oxazolidin-2-one synthesized by a known method (Bioorg. Med. Chem. Lett., 11, 18, 2001, 2475-2480).
MS (ESI+) : 614 (M+H)

### Example 93

3-{1-[((3R,4S)-4-{(1R)-1-[3,5-Bis(trifluoromethyl)phenyl]ethoxy}-3-phenylpiperidin-1-yl)carbonyl]piperidin-4-yl}-1,3-oxazinan-2-one

The compound was synthesized by the reaction and work-up in the same manner as in Step 2 of Example 87 and using the compound obtained in Step 1 of Example 87 and 3-piperidin-4-yl-1,3-oxazinan-2-one synthesized by a known method (WO2000/039114).
MS (ESI+) : 628 (M+H)

### Example 94

N-{1-[((3R,4S)-4-{(1R)-1-[3,5-Bis(trifluoromethyl)phenyl]ethoxy}-3-phenylpiperidin-1-yl)carbonyl]piperidin-4-yl}methanesulfonamide

To a solution of the compound obtained in Step 3 of Example 73 (0.10 g) and Et₃N (0.048 mL) in THF (5 mL) was added methanesulfonyl chloride (0.054 mL) at 0°C, and the mixture was stirred at room temperature for 14 hr. Water was added to the reaction mixture, and the product was extracted with ethyl acetate. The organic layer was washed with saturated aqueous ammonium chloride solution and brine and dried, and the solvent was evaporated under reduced pressure. The obtained residue was purified by preparative HPLC to give the title compound (0.082 g) as white crystals.
MS (ESI+) : 622 (M+H)

### Example 95

N-{1-[((3R,4S)-4-{(1R)-1-[3,5-Bis(trifluoromethyl)phenyl]ethoxy}-3-phenylpiperidin-1-yl)carbonyl]piperidin-4-yl}-2-hydroxyacetamide

To a solution of the compound obtained in Step 3 of Example 73 (0.18 g), hydroxyacetic acid (0.031 g), HOBt·H₂O (0.073 g) and Et₃N (0.088 mL) in THF (5 mL) was added WSC·HCl (0.096 g), and the mixture was stirred at room temperature for 14 hr. Water was added to the reaction mixture, and the product was extracted with ethyl acetate. The organic layer was washed with saturated aqueous ammonium chloride solution and brine and dried, and the solvent was evaporated under reduced pressure. The obtained residue was purified by preparative HPLC to give the title compound (0.095 g) as a colorless oil.
MS (ESI+) : 602 (M+H)

### Example 96

N-{1-[((3R,4S)-4-{(1R)-1-[3,5-Bis(trifluoromethyl)phenyl]ethoxy}-3-phenylpiperidin-1-yl)carbonyl]piperidin-4-yl}-3-hydroxypropanamide

The compound was synthesized by the reaction and work-up in the same manner as in the method described in Example 95 and using the compound obtained in Step 3 of Example 73 and 3-hydroxypropanoic acid.
MS (ESI+) : 616 (M+H)

### Example 97

4-[((3R,4S)-4-{(1R)-1-[3,5-Bis(trifluoromethyl)phenyl]ethoxy}-3-phenylpiperidin-1-yl)carbonyl]piperazin-2-one

### (Step 1)

To a solution of piperazin-2-one (2.05 g) and Et₃N (6.96 mL) in CH₂Cl₂ (25 mL) was added 4-nitrophenyl chloroformate (6.04 g) at 0°C, and the mixture was stirred at room temperature for 2 hr. Water was added to the reaction mixture, and the product was extracted with ethyl acetate. The organic layer was washed with saturated aqueous ammonium chloride solution and brine and dried, and the solvent was evaporated under reduced pressure. The obtained residue was crystallized from EtOH to give 4-nitrophenyl 3-oxopiperazine-1-carboxylate (4.2 g) as a white powder.
¹H-NMR(CDCl₃) : δ 3.46-3.60(2H,m),3.86(2H,dt,J=27.0and 6.0Hz),4.30(2H,d,J=27.0Hz)6.56(1H,d,J=30.0Hz),7.33(2H,d,J=12.0Hz ),8.24-8.33(2H,m)

### (Step 2)

The title compound was synthesized by the reaction and work-up in the same manner as in the method described in step 2 of Example 87 and using the compound obtained in Step 1 and the compound obtained in Example 185.
MS (ESI+) : 544 (M+H)

### Example 98

4-[((3R,4S)-4-{(1R)-1-[3,5-Bis (trifluoromethyl)phenyl]ethoxy}-3-phenylpiperidin-1-yl)carbonyl]piperazine-2,6-dione

### (Step 1)

N-(2-Amino-2-oxoethyl)-N-[(4-nitrophenoxy)carbonyl]glycine methyl ester

The title compound was synthesized by the reaction and work-up in the same manner as in the method described in step 1 of Example 91 and using N-(2-amino-2-oxoethyl)glycine methyl ester (1.0 g) synthesized by a known method (WO/9600391).

### (Step 2)

The title compound was synthesized by the reaction and work-up in the same manner as in the method described in step 2 of Example 87 and using the compound obtained in Step 1 and the compound obtained in Example 185.
MS (ESI+) : 558 (M+H)

### Example 99

(3R,4S)-4-{(1R)-1-[3,5-Bis(trifluoromethyl)phenyl]ethoxy}-N-[1-(methylsulfonyl)piperidin-4-yl]-3-phenylpiperidine-1-carboxamide

The compound was synthesized by the reaction and work-up in the same manner as in the method described in Example 94 and using the compound obtained in Step 2 of Example 71.
MS (ESI+) : 622 (M+H)

### Example 100

(3R,4S)-4-{(1R)-1-[3,5-Bis(trifluoromethyl)phenyl]ethoxy}-N-(1-lactoylpiperidin-4-yl)-3-phenylpiperidine-1-carboxamide

The compound was synthesized by the reaction and work-up in the same manner as in the method described in Example 95 and using the compound obtained in Step 2 of Example 71 and 2-hydroxypropanoic acid.
MS (ESI+) : 616 (M+H)

### Example 101

(3R,4S)-4-{(1R)-1-[3,5-Bis(trifluoromethyl)phenyl]ethoxy}-N-[1-(3-hydroxypropanoyl)piperidin-4-yl]-3-phenylpiperidine-1-carboxamide

The compound was synthesized by the reaction and work-up in the same manner as in the method described in Example 95 and using the compound obtained in Step 2 of Example 71 and 3-hydroxypropanoic acid.
MS(ESI+):616(M+H)

### Example 102

1-(trans-4-[((3R,45)-4-{(1R)-1-[3,5-Bis(trifluoromethyl)phenyl]ethoxy}-3-phenylpiperidin-1-yl)carbonyl]cyclohexyl}piperidin-2-one

### (Step 1)

To a solution of the compound obtained in Example 41 (0.077 g) and Et₃N (0.037 mL) in DMA (5 mL) was added 4-chlorobutanoyl chloride (0.051 mL), and the mixture was stirred at room temperature for 14 hr. Water was added to the reaction mixture, and the product was extracted with ethyl acetate. The organic layer was washed with saturated aqueous ammonium chloride solution and brine and dried, and the solvent was evaporated under reduced pressure. The obtained residue was purified by preparative HPLC to give N-{trans-4-[((3R,4S)-4-{(1R)-1-[3,5-bis(trifluoromethyl)phenyl]ethoxy}-3-phenylpiperidin-1-yl)carbonyl]cyclohexyl}-5-chloropentanamide (0.061 g) as a colorless oil.

### (Step 2)

To a solution of the compound obtained in Step 1 (0.091 g) in DMF (5 mL) was added KOt-Bu (0.037 g) and the mixture was stirred at 70°C for 3 hr. Water was added to the reaction mixture, and the product was extracted with ethyl acetate. The organic layer was washed with saturated aqueous ammonium chloride solution and brine and dried, and the solvent was evaporated under reduced pressure. The obtained residue was purified by preparative HPLC to give the title compound (0.026 g) as a white powder.
MS (ESI+) : 625 (M+H)

### Example 103

N-{trans-4-[((3R,4S)-4-{(1R)-1-[3,5-Bis(trifluoromethyl)phenyl]ethoxy}-3-phenylpiperidin-1-yl)carbonyl]cyclohexyl}-N-methylacetamide

To a solution of the compound obtained in Example 42 (0.088 g) and methyl iodide (0.040 mL) in DMF (5 mL) was added KOt-Bu (0.080 g), and the mixture was stirred at 70°C for 14 hr. Water was added to the reaction mixture, and the product was extracted with ethyl acetate. The organic layer was washed with saturated aqueous ammonium chloride solution and brine and dried, and the solvent was evaporated under reduced pressure. The obtained residue was purified by preparative HPLC to give the title compound (0.057 g) as a colorless amorphous.
MS (ESI+) : 599 (M+H)

### Example 104

N-{trans-4-[((3R,4S)-4-{(1R)-1-[3,5-Bis(trifluoromethyl)phenyl]ethoxy}-3-phenylpiperidin-1-yl)carbonyl]cyclohexyl}-2-(5,5-dimethyl-2,4-dioxo-1,3-oxazolidin-3-yl)acetamide

To a solution of the compound obtained in Example 41 (0.16 g), (5,5-dimethyl-2,4-dioxo-1,3-oxazolidin-3-yl)acetic acid (0.058 g) synthesized by a known method (J. Am. Chem. Soc., 70, 1948, 1021) and Et₃N (0.077 mL) in THF (5 mL) was added DEPC (0.086 mL) and the mixture was stirred at room temperature for 14 hr. Water was added to the reaction mixture, and the product was extracted with ethyl acetate. The organic layer was washed with saturated aqueous ammonium chloride solution and brine and dried, and the solvent was evaporated under reduced pressure. The obtained residue was purified by preparative HPLC to give the title compound (0.165 g) as a colorless amorphous.
MS (ESI+) : 712 (M+H)

### Example 105

1-{trans-4-[((3R,4S)-4-{(1R)-1-[3,5-Bis(trifluoromethyl)phenyl]ethoxy}-3-phenylpiperidin-1-yl)carbonyl]cyclohexyl}pyrrolidin-2-one

The compound was synthesized by the reaction and work-up in the same manner as in the method described in Example 102 and using the compound obtained in Example 41 and 3-chloropropanoyl chloride.
MS (ESI+) : 611 (M+H)

### Example 106

N-{trans-4-[((3R,4S)-4-{(1R)-1-[3,5-Bis(trifluoromethyl)phenyl]ethoxy}-3-phenylpiperidin-1-yl)carbonyl]cyclohexyl}-2-hydroxyacetamide

The compound was synthesized by the reaction and work-up in the same manner as in the method described in Example 104 and using the compound obtained in Example 41 and 2-hydroxyacetic acid.
MS (ESI+) : 601 (M+H)

### Example 107

N-{trans-4-[((3R,4S)-4-{(1R)-1-[3,5-Bis(trifluoromethyl)phenyl]ethoxy}-3-phenylpiperidin-1-yl)carbonyl]cyclohexyl}-N-methylpropanamide

The compound was synthesized by the reaction and work-up in the same manner as in the method described in Example 103 and using the compound obtained in Example 43 and methyl iodide.
MS (ESI+) : 613 (M+H)

### Example 108

N-{trans-4-[((3R,4S)-4-{(1R)-1-[3,5-Bis(trifluoromethyl)phenyl]ethoxy}-3-phenylpiperidin-1-yl)carbonyl]cyclohexyl}-3-hydroxypropanamide

The compound was synthesized by the reaction and work-up in the same manner as in the method described in Example 104 and using the compound obtained in Example 41 and 3-hydroxypropanoic acid.
MS (ESI+) : 615 (M+H)

### Example 109

N-{cis-4-[((3R,4S)-4-{(1R)-1-[3,5-Bis(trifluoromethyl)phenyl]ethoxy}-3-phenylpiperidin-1-yl)carbonyl]cyclohexyl}propanamide

The compound was synthesized by the reaction and work-up in the same manner as in the method described in Steps 1 to 3 of Example 22 and using the compound obtained in Example 41, cis-4-[[tert-butoxycarbonyl)amino]cyclohexanecarboxylic acid and propionyl chloride.
MS (ESI+) : 599 (M+H)

### Example 110

N-{trans-4-[((3R,4S)-4-{(1R)-1-[3,5-bis(trifluoromethyl)phenyl]ethoxy}-3-phenylpiperidin-1-yl)carbonyl]cyclohexyl}urea

To a solution of the compound obtained in Example 41 (0.153 g) and Et₃N (0.074 mL) in THF (5 mL) was added CDI (0.051 g), and the mixture was stirred at room temperature for 1 hr. Then, 28% aqueous ammonia (0.20 mL) was added and the mixture was stirred at room temperature for 14 hr. Water was added to the reaction mixture, and the product was extracted with ethyl acetate. The organic layer was washed with saturated aqueous ammonium chloride solution and brine and dried, and the solvent was evaporated under reduced pressure. The obtained residue was purified by preparative HPLC to give the title compound (0.040 g) as a white powder.
MS (ESI+) : 586 (M+H)

### Example 111

trans-4-[((3R,4S)-4-{(1R)-1-[3,5-Bis(trifluoromethyl)phenyl]ethoxy}-3-phenylpiperidin-1-yl)carbonyl]-N,N-dimethylcyclohexanecarboxamide

The title compound was synthesized by the reaction and work-up in the same manner as in the method described in Example 104 and using the compound obtained in Step 1 of Example 68 and dimethylamine.
MS (ESI+) : 599 (M+H)

### Example 112

(3R,4S)-4-{(1R)-1-[3,5-Bis(trifluoromethyl)phenyl]ethoxy}-3-phenyl-1-{[trans-4-(pyrrolidin-1-ylcarbonyl)cyclohexyl]carbonyl}piperidine

The compound was synthesized by the reaction and work-up in the same manner as in the method described in Example 104 and using the compound obtained in Step 1 of Example 68 and pyrrolidine.
MS (ESI+) : 625 (M+H)

### Example 113

(3R,4S)-4-{(1R)-1-[3,5-Bis(trifluoromethyl)phenyl]ethoxy}-3-phenyl-1-{[trans-4-(piperidin-1-ylcarbonyl)cyclohexyl]carbonyl}piperidine

The compound was synthesized by the reaction and work-up in the same manner as in the method described in Example 104 and using the compound obtained in Step 1 of Example 68 and piperidine.
MS (ESI+) : 639 (M+H)

### Example 114

trans-4-[((3R,4S)-4-{(1R)-1-[3,5-Bis(trifluoromethyl)phenyl]ethoxy}-3-phenylpiperidin-1-yl)carbonyl]cyclohexanecarboxamide

The compound was synthesized by the reaction and work-up in the same manner as in the method described in Example 95 and using the compound obtained in Step 1 of Example 68 and 28% ammonia/ethanol solution.
MS (ESI+) : 571 (M+H)

### Example 115

trans-4-[((3R,4S)-4-{(1R)-1-[3,5-Bis(trifluoromethyl)phenyl]ethoxy}-3-phenylpiperidin-1-yl)carbonyl]-N-methoxycyclohexanecarboxamide

The compound was synthesized by the reaction and work-up in the same manner as in the method described in Example 104 and using the compound obtained in Step 1 of Example 68 and O-methylhydroxylamine.
MS (ESI+) : 601 (M+H)

### Example 116

trans-4-[((3R,4S)-4-{(1R)-1-[3,5-Bis(trifluoromethyl)phenyl]ethoxy}-3-phenylpiperidin-1-yl)carbonyl]-N-ethoxycyclohexanecarboxamide

The compound was synthesized by the reaction and work-up in the same manner as in the method described in Example 104 and using the compound obtained in Step 1 of Example 68 and O-ethylhydroxylamine.
MS (ESI+) : 615 (M+H)

### Example 117

(3R,4S)-4-{(1R)-1-[3,5-Bis(trifluoromethyl)phenyl]ethoxy}-3-phenyl-1-(piperidin-4-ylcarbonyl)piperidine hydrochloride

The compound was synthesized by the reaction and work-up in the same manner as in Example 7 and using the compound obtained in Example 185.
MS (ESI+) : 529 (M-HC1+H)

### Example 118

(3R,4S)-4-{(1R)-1-[3,5-Bis(trifluoromethyl)phenyl]ethoxy}-1-{[1-(methylsulfonyl)piperidin-4-yl]carbonyl}-3-phenylpiperidine

The compound was synthesized by the reaction and work-up in the same manner as in Example 94 and using the compound obtained in Example 117.
MS (ESI+) : 607 (M+H)

### Example 119

(3R,4S)-4-{(1R)-1-[3,5-Bis(trifluoromethyl)phenyl]ethoxy}-3-phenyl-1-{[1-(1H-1,2,4-triazol-1-ylacetyl)piperidin-4-yl]carbonyl}piperidine

The compound was synthesized by the reaction and work-up in the same manner as in the method described in Example 104 and using the compound obtained in Example 117 and 1H-1,2,4-triazol-1-yl acetic acid.
MS(ESI+):638(M+H)
¹H-NMR(CDCl₃): δ 1.36-1.46(3H,m),1.62-2.01(5H,m),2.07-2.21(1H,m), 2.68-3.43(5H,m),3.49-4.06(4H,m),4.28-4.70(3H,m), 4.94-5.20(2H,m),7.01-7.34(7H,m),7.65(1H,s),7.96(1H,d,J=6.0Hz), 8.24(1H,d,J=6.0Hz)
[α]_{D}²⁵ +72.5° (c 1.0, MeOH)

### Example 120

5-(2-{4-[((3R,4S)-4-{(1R)-1-[3,5-Bis (trifluoromethyl)phenyl] ethoxy}-3-phenylpiperidin-1-yl)carbonyl]piperidin-1-yl}-2-oxoethyl)-2,4-dihydro-3H-1,2,4-triazol-3-one

The compound was synthesized by the reaction and work-up in the same manner as in the method described in Example 104 and using the compound obtained in Example 117 and 5-oxo-4,5-dihydro-1H-1,2,4-triazole-3-carboxylic acid.
MS (ESI+) : 654 (M+H)

### Example 121

(3R,4S)-4-{(1R)-1-[3,5-Bis(trifluoromethyl)phenyl]ethoxy}-3-phenyl-1-{[1-(1H-tetrazol-5-ylacetyl)piperidin-4-yl]carbonyl}piperidine

The compound was synthesized by the reaction and work-up in the same manner as in the method described in Example 104 and using the compound obtained in Example 117 and 1H-tetrazol-5-ylacetic acid.
MS (ESI+) : 639 (M+H)

### Example 122

(3R,4S)-4-{(1R)-1-[3,5-Bis(trifluoromethyl)phenyl]ethoxy}-3-phenyl-1-{[1-(1H-pyrazol-1-ylacetyl)piperidin-4-yl]carbonyl}piperidine

The compound was synthesized by the reaction and work-up in the same manner as in the method described in Example 104 and using the compound obtained in Example 117 and 1H-pyrazol-1-ylacetic acid.
MS (ESI+) : 637 (M+H)
¹H-NMR(CDCl₃): δ 1.35-1.44(3H,m),1.60-1.85(5H,m),2.06-2.18(1H,m),2.61-4.09(9H,m),4.28-4.70(3H,m),4.90-5.14(2H,m),6.29-6.36(1H,m),7.01-7.32(7H,m),7.48-7.57(2H,m),7.64(1H,s)
[α]_{D}²⁵ +75.0°(c 1.0, MeOH)

### Example 123

5-(2-{4-[((3R,4S)-4-{(lR)-1-[3,5-Bis(trifluoromethyl)phenyl]ethoxy}-3-phenylpiperidin-1-yl)carbonyl]piperidin-1-yl}-2-oxoethyl)imidazolidine-2,4-dione

The compound was synthesized by the reaction and work-up in the same manner as in the method described in Example 104 and using the compound obtained in Example 117 and (2,5-dioxoimidazolidin-4-yl)acetic acid.
MS (ESI+) : 669 (M+H)

### Example 124

(6R)-6-[((3R,4S)-4-{(R)-1-[3,5-Bis(trifluoromethyl)phenyl]ethoxy}-3-phenylpiperidin-1-yl)carbonyl]dihydropyrimidine-2,4(1H,3H)-dione

To a solution of the compound obtained in Example 185 (0.504 g), N²-(tert-butoxycarbonyl)-D-asparagine (0.239 g), HOBt· H₂O (0.200 g) and Et₃N (0.238 mL) in THF (10 ml) was added WSC· HCl (0.248 g), and the mixture was stirred at room temperature for 14 hr. Water was added to the reaction mixture, and the product was extracted with ethyl acetate. The organic layer was washed with saturated aqueous ammonium chloride solution and brine and dried, and the solvent was evaporated under reduced pressure. The obtained residue was treated with 1% trifluoroacetic acid/acetonitrile solution, and purified by preparative HPLC to give the title compound (0.121 g) as a colorless oil.
MS (ESI+) : 558 (M+H)

### Example 125

2-((3R,4S)-4-{(1R)-1-[3,5-Bis(trifluoromethyl)phenyl]ethoxy}-3-phenylpiperidin-1-yl)-2-oxoethanol

The compound was synthesized by the reaction and work-up in the same manner as in Example 95 and using the compound obtained in Example 185.
MS (ESI+) : 476 (M+H)

### Example 126

1-((3R,4S)-4-{(1R)-1-[3,5-Bis(trifluoromethyl)phenyl]ethoxy}-3-phenylpiperidin-1-yl)-1-oxopropan-2-ol

The compound was synthesized by the reaction and work-up in the same manner as in the method described in Example 95 and using the compound obtained in Example 185 and 2-hydroxypropanoic acid.
MS (ESI+) : 490 (M+H)

### Example 127

(2S)-1-((3R,4S)-4-{(1R)-1-[3,5-Bis(trifluoromethyl)phenyl]ethoxy}-3-phenylpiperidin-1-yl)-1-oxopropan-2-ol

The compound was synthesized by the reaction and work-up in the same manner as in the method described in Example 95 and using the compound obtained in Example 185 and (2S)-2-hydroxypropanoic acid.
MS (ESI+) : 490 (M+H)

### Example 128

1-((3R,4S)-4-{(1R)-1-[3,5-Bis(trifluoromethyl)phenyl]ethoxy}-3-phenylpiperidin-1-yl)-2-methyl-1-oxopropan-2-ol

The compound was synthesized by the reaction and work-up in the same manner as in the method described in Example 95 and using the compound obtained in Example 185 and 2-hydroxy-2-methylpropanoic acid.
MS (ESI+) : 504 (M+H)

### Example 129

3-((3R,4S)-4-{(1R)-1-[3,5-Bis(trifluoromethyl)phenyl]ethoxy}-3-phenylpiperidin-1-yl)-3-oxopropan-1-ol

The compound was synthesized by the reaction and work-up in the same manner as in the method described in Example 95 and using the compound obtained in Example 185 and 3-hydroxypropanoic acid.
MS (ESI+) : 490 (M+H)

### Example 130

tert-Butyl [2-((3R,4S)-4-{(1R)-1-[3,5-bis(trifluoromethyl)phenyl]ethoxy}-3-phenylpiperidin-1-yl)-2-oxoethyl]carbamate

The compound was synthesized by the reaction and work-up in the same manner as in the method described in Example 95 and using the compound obtained in Example 185 and N-(tert-butoxycarbonyl)glycine.
MS (ESI+) : 575 (M+H)

### Example 131

N-[2-((3R,4S)-4-{(1R)-1-[3,5-Bis(trifluoromethyl)phenyl]ethoxy}-3-phenylpiperidin-1-yl)-2-oxoethyl]methanesulfonamide

### (Step 1)

[2-((3R,4S)-4-{(1R)-1-[3,5-Bis(trifluoromethyl)phenyl]ethoxy}-3-phenylpiperidin-1-yl)-2-oxoethyl]amine hydrochloride was synthesized by the reaction and work-up in the same manner as in the method described in step 3 of Example 1 and using the compound obtained in Example 130.

### (Step 2)

The title compound was synthesized by the reaction and work-up in the same manner as in the method described in Example 99 and using the compound obtained in Step 1.
MS (ESI+) : 553 (M+H)

### Example 132

N-[2-((3R,4S)-4-{(1R)-1-[3,5-Bis(trifluoromethyl)phenyl]ethoxy}-3-phenylpiperidin-1-yl)-2-oxoethyl]acetamide

The title compound was synthesized by the reaction and work-up in the same manner as in the method described in Example 6 and using the compound obtained in Example 185.
MS (ESI+) : 517 (M+H)

### Example 133

tert-Butyl [2-((3R,4S)-4-{(1R)-1-[3,5-bis(trifluoromethyl)phenyl]ethoxy}-3-phenylpiperidin-1-yl)-1,1-dimethyl-2-oxoethyl]carbamate

The title compound was synthesized by the reaction and work-up in the same manner as in the method described in Example 6 and using the compound obtained in Example 185 and N-(tert-butoxycarbonyl)-2-methylalanine.
MS (ESI+) : 603 (M+H)

### Example 134

1-((3R,4S)-4-{(1R)-1-[3,5-Bis(trifluoromethyl)phenyl]ethoxy}-3-phenylpiperidin-1-yl)-2-methyl-1-oxopropane-2-amine hydrochloride

The title compound was synthesized by the reaction and work-up in the same manner as in the method described in step 3 of Example 1 and using the compound obtained in Example 133.
MS (ESI+) : 503 (M-HCl+H)

### Example 135

N-[(1S)-2-((3R,4S)-4-{(1R)-1-[3,5-Bis(trifluoromethyl)phenyl]ethoxy}-3-phenylpiperidin-1-yl)-1-(1H-imidazol-5-ylmethyl)-2-oxoethyl]acetamide

The title compound was synthesized by the reaction and work-up in the same manner as in the method described in Example 104 and using the compound obtained in Example 185 and (3S)-3-(acetylamino)-4-(1H-imidazole-5-yl)butanoic acid monohydrate.
MS (ESI+) : 597 (M+H)

### Example 136

tert-Butyl {(1R)-3-amino-1-[2-((3R,4S)-4-{(1R)-1-[3,5-bis(trifluoromethyl)phenoxy]ethoxy}-3-phenylpiperidin-1-yl)-2-oxoethyl]-3-oxopropyl}carbamate

The title compound was synthesized by the reaction and work-up in the same manner as in the method described in Example 6 and using the compound obtained in Example 185 and (3R)-5-amino-3-[(tert-butoxycarbonyl)amino]-5-oxopentanoic acid.
MS (ESI+) :632 (M+H)

### Example 137

(3R)-3-(Acetylamino)-4-((3R,4S)-4-{(1R)-1-[3,5-bis(trifluoromethyl)phenyl]ethoxy}-3-phenylpiperidin-1-yl)-4-oxobutanamide

The title compound was synthesized by the reaction and work-up in the same manner as in the method described in Example 104 and using the compound obtained in Example 185 and (3R)-3-(acetylamino)-5-amino-5-oxopentanoic acid.
MS (ESI+) : 574 (M+H)

### Example 138

(3S)-3-(Acetylamino)-4-((3R,4S)-4-{(1R)-1-[3,5-bis(trifluoromethyl)phenyl]ethoxy}-3-phenylpiperidin-1-yl)-4-oxobutanamide

The title compound was synthesized by the reaction and work-up in the same manner as in the method described in Example 104 and using the compound obtained in Example 185 and (3S)-3-(acetylamino)-5-amino-5-oxopentanoic acid.
MS (ESI+) : 574 (M+H)

### Example 139

tert-Butyl [3-((3R,4S)-4-{(1R)-1-[3,5-bis(trifluoromethyl)phenyl]ethoxy}-3-phenylpiperidin-1-yl)-3-oxopropyl]carbamate

The title compound was synthesized by the reaction and work-up in the same manner as in the method described in Example 6 and using the compound obtained in Example 185 and N-(tert-butoxycarbonyl)-β-alanine.
MS (ESI+) : 589 (M+H)

### Example 140

3-((3R,4S)-4-{(1R)-1-[3,5-Bis(trifluoromethyl)phenyl]ethoxy}-3-phenylpiperidin-1-yl)-3-oxopropane-1-amine hydrochloride

The title compound was synthesized by the reaction and work-up in the same manner as in the method described in step 3 of Example 1 and using the compound obtained in Example 139.
MS (ESI+) : 489 (M-HCl+H)

### Example 141

N-[3-((3R,4S)-4-{(1R)-1-[3,5-Bis(trifluoromethyl)phenyl]ethoxy}-3-phenylpiperidin-1-yl)-3-oxopropyl]acetamide

The title compound was synthesized by the reaction and work-up in the same manner as in the method described in Example 6 and using the compound obtained in Example 185 and N-acetyl-β-alanine.
MS (ESI+) : 531 (M+H)

### Example 142

N-[3-((3R,4S)-4-{(1R)-1-[3,5-Bis(trifluoromethyl)phenyl]ethoxy}-3-phenylpiperidin-1-yl)-3-oxopropyl]propanamide

The compound was synthesized by the reaction and work-up in the same manner as in Step 3 of Example 22 and using the compound obtained in Example 140 and propionyl chloride.
MS (ESI+) : 545 (M+H)

### Example 143

3-[3-((3R,4S)-4-{(1R)-1-[3,5-Bis(trifluoromethyl)phenyl]ethoxy}-3-phenylpiperidin-1-yl)-3-oxopropyl]-1,3-thiazolidine-2,4-dione

### (Step 1)

The title compound was synthesized by the reaction and work-up in the same manner as in the method described in Example 104 and using the compound obtained in Example 185 and 3-(2,4-dioxo-1,3-thiazolidin-3-yl)propanoic acid synthesized by a known method (Egyptian Journal of Chemistry (1972), 15(1), 11-21).
MS (ESI+) : 589 (M+H)

### Example 144

N-[3-((3R,4S)-4-{(1R)-1-[3,5-Bis(trifluoromethyl)phenyl]ethoxy}-3-phenylpiperidin-1-yl)-3-oxopropyl]methanesulfonamide

The compound was synthesized by the reaction and work-up in the same manner as in Step 3 of Example 22 and using the compound obtained in Example 140 and methanesulfonyl chloride.
MS (ESI+) : 567 (M+H)

### Example 145

1-[3-((3R,4S)-4-{(1R)-1-[3,5-Bis(trifluoromethyl)phenyl]ethoxy}-3-phenylpiperidin-1-yl)-3-oxopropyl]pyrrolidine-2,5-dione

The title compound was synthesized by the reaction and work-up in the same manner as in Example 104 and using the compound obtained in Example 185 and 3-(2,5-dioxopyrrolidin-1-yl)propanoic acid synthesized by a known method (J. Am. Chem. Soc., 71, 1948, 1251).
MS (ESI+) : 571 (M+H)

### Example 146

1-[3-((3R,4S)-4-{(1R)-1-[3,5-Bis(trifluoromethyl)phenyl]ethoxy}-3-phenylpiperidin-1-yl)-3-oxopropyl]piperidine-2,6-dione

The title compound was synthesized by the reaction and work-up in the same manner as in Example 104 and using the compound obtained in Example 185 and 3-(2,6-dioxopiperidin-1-yl)propanoic acid synthesized by a known method (Pharmaceutical Research (1984), (6), 267-9).
MS (ESI+) : 585 (M+H)

### Example 147

N-[3-((3R,4S)-4-{(1R)-1-[3,5-Bis(trifluoromethyl)phenyl]ethoxy}-3-phenylpiperidin-1-yl)-3-oxopropyl]-N-isopropylurea

The title compound was synthesized by the reaction and work-up in the same manner as in Example 3 and using the compound obtained in Example 140 and isopropyl isocyanate.
MS (ESI+) : 574 (M+H)

### Example 148

N-[3-((3R,4S)-4-{(1R)-1-[3,5-Bis(trifluoromethyl)phenyl]ethoxy}-3-phenylpiperidin-1-yl)-3-oxopropyl]-2,2-dimethylpropanamide

The title compound was synthesized by the reaction and work-up in the same manner as in Example 3 and using the compound obtained in Example 140 and pivaloyl chloride.
MS (ESI+) : 573 (M+H)

### Example 149

N-[3-((3R,4S)-4-{(lR)-l-[3,5-Bis(trifluoromethyl)phenyl]ethoxy}-3-phenylpiperidin-1-yl)-3-oxopropyl]-4-chlorobutanamide

The compound was synthesized by the reaction and work-up in the same manner as in Step 1 of Example 102 and using the compound obtained in Example 140 and 3-chloropropanoyl chloride.
MS(ESI+) : 594 (M+H)

### Example 150

1-[3-((3R, 4S)-4-{(1R)-1-[3,5-Bis(trifluoromethyl)phenyl]ethoxy}-3-phenylpiperidin-1-yl)-3-oxopropyl]pyrrolidin-2-one

The compound was synthesized by the reaction and work-up in the same manner as in Step 2 of Example 102 and using the compound obtained in Example 149.
MS (ESI+) : 557 (M+H)

### Example 151

N'-[3-((3R,4S)-4-{(1R)-1-[3,5-Bis(trifluoromethyl)phenyl]ethoxy}-3-phenylpiperidin-1-yl)-3-oxopropyl]-N,N-dimethylurea

The compound was synthesized by the reaction and work-up in the same manner as in the method described in Example 3 and using the compound obtained in Example 140 and N,N-dimethylcarbamate chloride.
MS (ESI+) : 560 (M+H)

### Example 152

tert-Butyl [4-((3R,45)-4-{(1R)-1-[3,5-bis(trifluoromethyl)phenyl]ethoxy}-3-phenylpiperidin-1-yl)-4-oxobutyl]carbamate

The title compound was synthesized by the reaction and work-up in the same manner as in Example 104 and using the compound obtained in Example 185 and 4-[(tert-butoxycarbonyl)amino]butanoic acid.
MS (ESI+) : 603 (M+H)

### Example 153

4-((3R,4S)-4-{(1R)-1-[3,5-Bis(trifluoromethyl)phenyl]ethoxy}-3-phenylpiperidin-1-yl)-4-oxobutane-l-amine hydrochloride

The title compound was synthesized by the reaction and work-up in the same manner as in step 3 of Example 1 and using the compound obtained in Example 152.
MS (ESI+) : 503 (M-HCl+H)

### Example 154

N-[4-((3R,4S)-4-{(1R)-1-[3,5-Bis(trifluoromethyl)phenyl]ethoxy}-3-phenylpiperidin-1-yl)-4-oxobutyl]acetamide

The title compound was synthesized by the reaction and work-up in the same manner as in step 3 of Example 22 and using the compound obtained in Example 153.
MS (ESI+) : 545 (M+H)

### Example 155

N-[4-((3R,4S)-4-{(1R)-1-[3,5-Bis(trifluoromethyl)phenyl]ethoxy}-3-phenylpiperidin-1-yl)-4-oxobutyl] propanamide

The title compound was synthesized by the reaction and work-up in the same manner as in step 3 of Example 22 and using the compound obtained in Example 153 and propionyl chloride.
MS (ESI+) : 559 (M+H)

### Example 156

N-[4-((3R,4S)-4-{(1R)-1-[3,5-Bis(trifluoromethyl)phenyl)ethoxy}-3-phenylpiperidin-1-yl)-4-oxobutyl]methanesulfonamide

The compound was synthesized by the reaction and work-up in the same manner as in Step 3 of Example 22 and using the compound obtained in Example 153 and methanesulfonyl chloride.
MS (ESI+) : 581 (M+H)

### Example 157

tert-Butyl 2-[3-((3R,4S)-4-{(1R)-1-[3,5-bis(trifluoromethyl)phenyl]ethoxy}-3-phenylpiperidin-1-yl)-3-oxopropyl]pyrrolidine-1-carboxylate

The title compound was synthesized by the reaction and work-up in the same manner as in Example 104 and using the compound obtained in Example 185 and 3-[1-(tert-butoxycarbonyl)pyrrolidin-2-yl]propanoic acid.
MS (ESI+) : 643 (M+H)

### Example 158

(3R,4S)-1-[3-(1-Acetylpyrrolidin-2-yl)propanoyl]-4-{(1R)-1-[3,5-bis(trifluoromethyl)phenyl]ethoxy}-3-phenylpiperidine

### (Step 1)

(3R,4S)-4-{(1R)-1-[3,5-Bis(trifluoromethyl)phenyl]ethoxy}-3-phenyl-1-(3-pyrrolidin-2-ylpropanoyl)piperidine was synthesized by the reaction and work-up in the same manner as in step 3 of Example 1 and using the compound obtained in Example 157.

### (Step 2)

The compound was synthesized by the reaction and work-up in the same manner as in Step 3 of Example 22 and using the compound obtained in Step 1.
MS (ESI+) : 585 (M+H)

### Example 159

tert-Butyl [5-((3R,4S)-4-{(1R)-1-[3,5-bis(trifluoromethyl)phenyl]ethoxy}-3-phenylpiperidin-1-yl)-5-oxopentyl]carbamate

The title compound was synthesized by the reaction and work-up in the same manner as in Example 104 and using the compound obtained in Example 185 and 4-[(tert-butoxycarbonyl)amino]pentanoic acid.
MS (ESI+) : 617 (M+H)

### Example 160

5-((3R,4S)-4-{(1R)-1-[3,5-Bis(trifluoromethyl)phenyl]ethoxy}-3-phenylpiperidin-1-yl)-5-oxopentane-1-amine hydrochloride

The title compound was synthesized by the reaction and work-up in the same manner as in Step 3 of Example 1 and using the compound obtained in Example 159.
MS (ESI+) : 517 (M-HCl+H)

### Example 161

N-[5-((3R,4S)-4-{(1R)-1-[3,5-Bis(trifluoromethyl)phenyl]ethoxy}-3-phenylpiperidin-1-yl)-5-oxopentyl]acetamide

The title compound was synthesized by the reaction and work-up in the same manner as in Step 3 of Example 22 and using the compound obtained in Example 160.
MS (ESI+) : 559 (M+H)

### Example 162

Methyl 4-((3R,4S)-4-{(1R)-1-[3,5-bis(trifluoromethyl)phenyl]ethoxy}-3-phenylpiperidin-1-yl)-4-oxobutanoate

The title compound was synthesized by the reaction and work-up in the same manner as in Example 104 and using the compound obtained in Example 185 and 4-methoxy-4-oxobutanoic acid.
MS (ESI+) : 532 (M+H)

### Example 163

4-((3R,4S)-4-{(1R)-1-[3,5-Bis(trifluoromethyl)phenyl]ethoxy}-3-phenylpiperidin-1-yl)-4-oxobutanoic acid

To a solution of the compound obtained in Example 162 (0.82 g) in MeOH (10 mL) was added 2N potassium hydroxide (10 ml) and the mixture was stirred at 50°C for 14 hr. The reaction mixture was acidified with hydrochloric acid, and the product was extracted with ethyl acetate. The organic layer was washed with brine and dried, and the solvent was evaporated under reduced pressure. The obtained residue was purified by preparative HPLC to give the title compound (0.67 g) as a colorless oil.
MS (ESI+) : 518 (M+H)

### Example 164

4-((3R,4S)-4-{(1R)-1-[3,5-Bis(trifluoromethyl)phenyl]ethoxy}-3-phenylpiperidin-1-yl)-4-oxobutanamide

The title compound was synthesized by the reaction and work-up in the same manner as in Example 6 and using the compound obtained in Example 185 and 4-amino-4-oxobutanoic acid.
MS (ESI+) : 517 (M+H)

### Example 165

4-((3R,4S)-4-{(1R)-1-[3,5-Bis(trifluoromethyl)phenyl]ethoxy}-3-phenylpiperidin-1-yl)-N-methyl-4-oxobutanamide

The title compound was synthesized by the reaction and work-up in the same manner as in Example 104 and using the compound obtained in Example 163 and 40% methylamine/methanol solution.
MS (ESI+) : 531 (M+H)

### Example 166

(3R,4S)-4-{(1R)-1-[3,5-Bis(trifluoromethyl)phenyl]ethoxy}-1-(4-oxo-4-pyrrolidin-1-ylbutanoyl)-3-phenylpiperidine

The title compound was synthesized by the reaction and work-up in the same manner as in Example 104 and using the compound obtained in Example 163 and pyrrolidine.
MS (ESI+) : 571 (M+H)

### Example 167

1-[((3R,4S)-4-{(1lR)-1-[3,5-Bis(trifluoromethyl)phenyl]ethoxy)-3-phenylpiperidin-1-yl)carbonyl]cyclopropanecarboxamide

The title compound was synthesized by the reaction and work-up in the same manner as in Example 6 and using the compound-obtained in Example 185 and 1-(aminocarbonyl)cyclopropanecarboxylic acid.
MS (ESI+) : 529 (M+H)

### Example 168

2-[(1E)-3-((3R,4S)-4-{(1R)-1-[3,5-Bis(trifluoromethyl)phenyl]ethoxy}-3-phenylpiperidin-1-yl)-3-oxoprop-1-ene-1-yl]pyridine

The title compound was synthesized by the reaction and work-up in the same manner as in Example 6 and using the compound obtained in Example 185 and (2E)-3-pyridin-2-ylacrylic acid hydrochloride.
MS (ESI+) : 549 (M+H)

### Example 169

2-[3-((3R,4S)-4-{(1R)-1-[3,5-Bis(trifluoromethyl)phenyl]ethoxy}-3-phenylpiperidin-1-yl)-3-oxopropyl]pyridine

The title compound was synthesized by the reaction and work-up in the same manner as in Example 6 and using the compound obtained in Example 185 and 3-pyridin-2-ylpropanoic acid hydrochloride.
MS (ESI+) : 551 (M+H)

### Example 170

5-[3-((3R,4S)-4-{(1R)-1-[3,5-Bis(trifluoromethyl)phenyl]ethoxy}-3-phenylpiperidin-1-yl)-3-oxopropyl]imidazolidine-2,4-dione

The title compound was synthesized by the reaction and work-up in the same manner as in Example 6 and using the compound obtained in Example 185 and 3-(2,5-dioxoimidazolidin-4-yl)propanoic acid synthesized by a known method (J. Am. Chem. Soc., 66, 1944, 650).
MS (ESI+) : 572 (M+H)

### Example 171

(3R,4S)-4-{(1R)-1-[3,5-Bis(trifluoromethyl)phenyl]ethoxy}-3-phenyl-1-[(2E)-3-(3-thienyl)prop-2-enoyl]piperidine

The title compound was synthesized by the reaction and work-up in the same manner as in Example 104 and using the compound obtained in Example 185 and (2E)-3-(3-thienyl)acrylic acid synthesized by a known method (US6177443).
MS (ESI+) : 554 (M+H)

### Example 172

(3R,4S)-4-{(1R)-1-[3,5-Bis(trifluoromethyl)phenyl]ethoxy}-3-phenyl-1-[3-(3-thienyl)propanoyl]piperidine

The title compound was synthesized by the reaction and work-up in the same manner as in Example 104 and using the compound obtained in Example 185 and 3-(3-thienyl)propanoic acid synthesized by a known method (US6313312).
MS(ESI+):556(M+H)

### Example 173

(3R,4S)-4-{(1R)-1-[3,5-Bis(trifluoromethyl)phenyl]ethoxy}-1-[(2E)-3-(2-ethyl-1H-imidazol-5-yl)prop-2-enoyl]-3-phenylpiperidine

### (Step 1)

(2E)-3-(2-Ethyl-1H-imidazol-5-yl)acrylic acid was synthesized by the reaction and work-up in the same manner as in Step 1 and Step 3 of Example 179 and using 2-ethyl-1H-imidazole-5-carbaldehyde.

### (Step 2)

The title compound was synthesized by the reaction and work-up in the same manner as in Example 104 and using the compound obtained in Step 1 and the compound obtained in Example 185.
MS (ESI+) : 566 (M+H)

### Example 174

(3R,4S)-4-{(1R)-1-[3,5-Bis(trifluoromethyl)phenyl]ethoxy}-1-[3-(2-furyl)propanoyl]-3-phenylpiperidine

The title compound was synthesized by the reaction and work-up in the same manner as in Example 104 and using the compound obtained in Example 185 and 3-(2-furyl)propanoic acid synthesized by a known method (US6710190).
MS (ESI+) : 540 (M+H)

### Example 175

(3R,4S)-4-{ (1R)-1-[3,5-Bis(trifluoromethyl)phenyl]ethoxy}-1-[3-(2-ethyl-1H-imidazol-5-yl)propanoyl]-3-phenylpiperidine

### (Step 1)

3-(2-Ethyl-1H-imidazol-5-yl)propanoic acid was synthesized by the reaction and work-up in the same manner as in Steps 1 to 3 of Example 179 and using 2-ethyl-1H-imidazol-5-carbaldehyde.

### (Step 2)

The title compound was synthesized by the reaction and work-up in the same manner as in Example 104 and using the compound obtained in Step 1 and the compound obtained in Example 185.
MS (ESI+) : 568 (M+H)

### Example 176

(3R,4S)-4-{(1R)-1-[3,5-Bis(trifluoromethyl)phenyl]ethoxy}-3-phenyl-1-[(2E)-3-(1H-pyrrol-2-yl)prop-2-enoyl]piperidine

The title compound was synthesized by the reaction and work-up in the same manner as in Example 104 and using the compound obtained in Example 185 and (2E)-3-(1H-pyrrol-2-yl)acrylic acid synthesized by a known method (Chem. Pharm. Bull., 37, 3, 1989, 684-687).
MS (ESI+) : 537 (M+H)

### Example 177

(3R,4S)-4-{(1R)-1-[3,5-Bis(trifluoromethyl)phenyl]ethoxy}-3-phenyl-1-[(2E)-3-(1,3-thiazol-2-yl)prop-2-enoyl]piperidine

The title compound was synthesized by the reaction and work-up in the same manner as in Example 104 and using the compound obtained in Example 185 and (2E)-3-(1,3-thiazol-2-yl)acrylic acid synthesized by a known method (US4154834).
MS (ESI+) : 555 (M+H)

### Example 178

(3R,4S)-4-{(1R)-1-[3,5-Bis(trifluoromethyl)phenyl]ethoxy}-3-phenyl-1-[3-(1,3-thiazol-2-yl)propanoyl]piperidine

The title compound was synthesized by the reaction and work-up in the same manner as in Example 104 and using the compound obtained in Example 185 and 3-(1,3-thiazol-2-yl)propanoic acid synthesized by a known method (US2002/77329).
MS (ESI+) : 557 (M+H)

### Example 179

(3R,4S)-4-{(1R)-1-[3,5-Bis(trifluoromethyl)phenyl]ethoxy}-1-[3-(1-methyl-1H-imidazol-2-yl)propanoyl]-3-phenylpiperidine

### (Step 1)

To a solution of ethyl diethylphosphonoacetate (6.5 mL) in THF (70 mL) was added sodium hydride (1.97 g, 60% in oil) at 0°C, and the mixture was stirred for 30 min. Then, a solution of 1-methyl-1H-imidazole-2-carbaldehyde (3.0 g) in THF (70 mL) was added and the mixture was stirred at 0°C for 2 hr. The reaction mixture was poured into water, and the product was extracted with ethyl acetate. The organic layer was washed with brine and dried, and the solvent was evaporated under reduced pressure to give crude methyl (2E)-3-(1-methyl-1H-imidazol-2-yl)acrylate (4.6 g) as a colorless oil.
¹H-NMR(CDCl₃): δ 1.33(3H,t,J=6.0Hz),3.76(3H,s), 4.27(2H,q,J=6,OHz),6.81(1H,d,J=15.0Hz),6.97(1H,s),7.15(1H,s), 7.52(1H,d,J=15.Hz)

### (Step 2)

To a solution of the crude product (4.6 g) obtained in Step 1 in MeOH (150 mL) was added 10% palladium carbon (1.5 g), and the mixture was stirred at room temperature for 24 hr under a hydrogen atmosphere (1 atm). The catalyst was filtered off, and the filtrate was concentrated under reduced pressure to give crude 3-(1-methyl-1H-imidazol-2-yl)propanoic acid (2.6 g) as a colorless oil.

### (Step 3)

To a solution of the compound (2.6 g) obtained in Step 2 in MeOH (30 mL) was added 2N aqueous potassium hydroxide solution (8 mL), and the mixture was stirred at room temperature for 14 hr. The reaction mixture was acidified with hydrochloric acid and the product was extracted with ethyl acetate. The organic layer was washed with brine and dried, and the solvent was evaporated under reduced pressure to give crude 3-(1-methyl-1H-imidazol-2-yl)acrylic acid (1.2 g) as a colorless oil.

### (Step 4)

The title compound was synthesized by the reaction and work-up in the same manner as in Example 104 and using the compound obtained in Step 3 and the compound obtained in Example 185.
MS (ESI+) : 554 (M+H)

### Example 180

3-((3R,4S)-4-{(1R)-1-[3,5-Bis(trifluoromethyl)phenyl]ethoxy}-3-phenylpiperidin-1-yl)-3-oxopropanenitrile

The title compound was synthesized by the reaction and work-up in the same manner as in Example 104 and using the compound obtained in Example 185 and cyanoacetic acid.
MS (ESI+) : 485 (M+H)

### Example 181

4-((3R,4S)-4-{(1R)-1-[3,5-Bis(trifluoromethyl)phenyl]ethoxy}-3-phenylpiperidin-1-yl)-4-oxobutanenitrile

The title compound was synthesized by the reaction and work-up in the same manner as in Example 104 and using the compound obtained in Example 185 and 3-cyanopropanoic acid.
MS (ESI+) : 499 (M+H)

### Example 182

3-[3-((3R,4S)-4-{ (1R)-1-[3,5-Bis(trifluoromethyl)phenyl]ethoxy}-3-phenylpiperidin-1-yl)-3-oxopropyl]-1,2,4-oxadiazol-5(4H)-one

To a solution of the compound obtained in Example 181 (0.35 g) in DMSO (5 mL) were added 50% aqueous hydroxyamine solution (5 mL) and sodium hydrogen carbonate (0.075 g), and the mixture was stirred at 70°C for 3 hr. Water was added to the reaction mixture, and the product was extracted with ethyl acetate. The organic layer was washed with brine and dried, and the solvent was evaporated under reduced pressure. To a solution of the obtained residue in THF (5 mL) were added CDI (0.126 g) and DBU (0.11 mL), and the mixture was stirred at room temperature for 1 hr. Diluted hydrochloric acid was added to the reaction mixture and the product was extracted with ethyl acetate. The organic layer was washed with brine and dried, and the solvent was evaporated under reduced pressure. The residue was purified by preparative HPLC to give the title compound (0.18 g) as a colorless oil.
MS (ESI+) : 558 (M+H)

### Example 183

3-[2-((3R,4S)-4-{(1R)-1-[3,5-Bis(trifluoromethyl)phenyl]ethoxy}-3-phenylpiperidin-1-yl)-2-oxoethyl]-1,2,4-oxadiazol-5(4H)-one

The title compound was synthesized by the reaction and work-up in the same manner as in Example 182 and using the compound obtained in Example 180.
MS (ESI+) : 544 (M+H)

### Example 184

(3R,4S)-4-{(1R)-1-[3,5-Bis(trifluoromethyl)phenyl]ethoxy}-3-phenyl-1-(1H-tetrazol-5-ylacetyl)piperidine

The title compound was synthesized by the reaction and work-up in the same manner as in Example 104 and using the compound obtained in Example 185 and 1H-tetrazol-5-ylacetic acid synthesized by a known method (WO2005/14602).
MS (ESI+) : 528 (M+H)

### Example 185

(3R,4S)-4-{(1R)-1-[3,5-Bis(trifluoromethyl)phenyl]ethoxy}-3-phenylpiperidine p-tosylate

### (Step 1)

To a solution of tert-butyl (3R,4S)-4-hydroxy-3-phenylpiperidine-1-carboxylate (80.0 g) synthesized by a known method (WO03/101964) in MeOH (288 ml) was added 4N hydrogen chloride/ethyl acetate solution (288 ml), and the mixture was stirred at 50°C for 2 hr. The solvent was evaporated under reduced pressure to give a white powder. To a solution of the obtained white powder in acetonitrile (600 ml) was added diisopropylethylamine (193 g) and benzyl bromide (24.9 g) was added at 0°C. The reaction solution was stirred at room temperature for 14 hr. and concentrated under reduced pressure. The residue was dissolved in ethyl acetate and water, and adjusted to pH 7 to 8. The organic layer was separated and the aqueous layer was extracted again with ethyl acetate. The obtained organic layer was washed with brine and dried, and the solvent was evaporated under reduced pressure to give (3R,4S)-1-benzyl-3-phenylpiperidin-4-ol (62.8 g) as a white powder.
MS (ESI+) : 268 (M+H)

### (Step 2)

To a mixed solution of the compound obtained in Step 1 (52.3 g) and the compound obtained in Step 1 of Example 1 (280.6 g) in CH₂Cl₂ (1300 ml) and cyclohexane (650 ml) was added molecular sieves 4A (150 g) and the mixture was stirred for 5 min. Trifluoromethanesulfonic acid (104.6 g) was added at -10°C to 0°C, and the mixture was stirred at room temperature stirred for 14 hr. The reaction mixture was poured into saturated aqueous sodium hydrogen carbonate and ethyl acetate. The mixture was stirred, filtered through celite and partitioned. The obtained organic layer was washed with brine and dried, and the solvent was evaporated under reduced pressure. The precipitated white powder (CCl₃CONH₂) was filtrated off using Et₂O-hexane mixed solution (1:1, v/v). The filtrate was concentrated under reduced pressure and the precipitated white powder (CCl₃CONH₂) was filtrated with Et₂O-hexane mixed solution (1:1, v/v) again (this operation was repeated three times). The thus-obtained residue was purified by silica gel column chromatography (ethyl acetate:hexane=1:10) to give crude (3R,4S)-1-benzyl-4-{1-[3,5-bis(trifluoromethyl)phenyl]ethoxy}-3-phenylpiperidine (144.4 g) as a diastereomer mixture as a pale-yellow oil.
MS (ESI+):508 (M+H)

### (Step 3)

To a solution of the compound obtained in Step 2 (144.0 g) in EtOH (1800 ml) was added 10% Pd(OH)₂ (14.4 g), and the mixture was stirred under hydrogen atmosphere (1atm) at room temperature for 2 days. The catalyst was filtered off, and the filtrate was concentrated under reduced pressure. p-Toluenesulfonic acid monohydrate (37.8 g) was added to the obtained residue and crystallized from a mixed solution of EtOH and IPE to give the title compound (39.1 g) as a white powder (diastereomer excess 86%). The obtained powder was crystallized again from a mixed solution of EtOH and IPE to give the title compound (34.7 g) as a white powder (diastereomer excess not less than 99%).
MS (ESI+) : 418 (M-CH₃C₆H₄SO₃H+H)
melting point: 212-214°C
¹H-NMR(CDCl₃) : δ 1.35(3H,d,J=6.3Hz),2.00-2.20(2H,m),2.34(3H,s), 3.16-3.62(6H,m),4.32(1H,q,J=6.4Hz),6.90-6.94(2H,m),7.14-7.25(7H,m),7.62(1H,s),7.74-7.77(2H,d,J=8.1Hz),8.60-9.60(2H,br)
[α]D²⁵ +41.6° (c 1.0,MeOH)
Measurement of diastereomer excess by LC:
column: Inertsil ODS-24.6 mmID×150 mmL
solvent: 0.1% trifluoroacetic acid containing water, Solution
B; 0.1% trifluoroacetic acid containing acetonitrile gradient cycle: 0.00 min (Solution A/Solution B = 70/30),
15.00 min (Solution A/Solution B = 15/85),
15.01 min (Solution A/Solution B = 5/95),
20.00 min (Solution A/Solution B = 5/95),
20.01 min (Solution A/Solution B = 70/30),
25.00 min (Solution A/Solution B = 70/30) hexane/ethanol=98/2
flow rate: 1.0 mL/min
temperature: 20°C
detection method: UV 220 nm
retention time: 11.4 min (free form of compound of Example 185) 11.6 min (its diastereomer)

### Example 186

(3S,4R)-4-{(1S)-1-[3,5-Bis(trifluoromethyl)phenyl]ethoxy}-3-phenylpiperidine p-tosylate

The title compound (diastereomer excess not less than 99%) was synthesized by the reaction and work-up in the same manner as in Example 185 and using tert-butyl (3S,4R)-4-hydroxy-3-phenylpiperidine-1-carboxylate synthesized by a known method (WO03/101964).
MS (ESI+) : 418 (M-CH₃C₆H₄SO₃H+H)
melting point: 215-217°C
¹H-NMR(CDCl₃) : δ 1.35(3H,d,J=6.3Hz),2.00-2.20(2H,m),2.34(3H,s), 3.16-3.62(6H,m),4.32(1H,q,J=6.4Hz),6.90-6.94(2H,m),7.14-7.25(7H,m),7.62(1H,s),7.74-7.77(2H,d,J=8.1Hz),8.60-9.60(2H,br)
[α]D²⁵ -41.0° (c 1.0, MeOH)

### Measurement of diastereomer excess by LC:

measurement conditions: same as those described in Example 185 retention time: 11.4 min (free form of compound of Example 186) 11.6 min (its diastereomer)

### Example 187

(3S,4R)-4-{(1S)-1-[3,5-Bis(trifluoromethyl)phenyl]ethoxy}-3-phenylpiperidine hydrochloride

The compound obtained in Example 186 (0.40 g) was suspended in a mixed solution of ethyl acetate and water, and 2N aqueous sodium hydroxide solution (1 mL) was added. The organic layer was washed with brine and dried, and the solvent was evaporated under reduced pressure. To a solution of the obtained residue in ethyl acetate was added 4N hydrogen chloride/ethyl acetate solution (0.18 mL), and the mixture was crystallized from IPE to give the title compound (0.27 g) as a white powder (diastereomer excess not less than 99%).
melting point: :169-171°C
[α]_{D}²⁵ -53.9° (c 1.0, MeOH)
¹H-NMR(CDCl₃): δ 1.38(3H,d,J=6.3Hz), 2.30-2.35(2H,m), 3.25-3.50(4H,m), 3.60-3.75(2H,m), 4.35(1H,q,J=6.3Hz), 7.01-7.05(2H,m), 7.16-7.26(5H,m), 7.64(1H,s), 9.00-10.40(2H,br)

### Example 188

tert-Butyl {trans-4-[((3S,4R)-4-{(1S)-1-[3,5-bis(trifluoromethyl)phenyl]ethoxy}-3-phenylpiperidin-1-yl)carbonyl]cyclohexyl}carbamate

The title compound was synthesized by the reaction and work-up in the same manner as in Step 1 of Example 22 and using the compound obtained in Example 186.
MS(ESI+):643(M+H)

### Example 189

trans-4-[((3S,4R)-4-{(1S)-1-[3,5-Bis(trifluoromethyl)phenyl]ethoxy}-3-phenylpiperidin-1-yl)carbonyl]cyclohexanamine hydrochloride

The title compound was synthesized by the reaction and work-up in the same manner as in step 2 of Example 22 and using the compound obtained in Example 188.
MS (ESI+) : 543 (M-HCl+H)

### Example 190

N-{trans-4-[((3S,4R)-4-{(1S)-1-[3,5-Bis(trifluoromethyl)phenyl]ethoxy}-3-phenylpiperidin-1-yl)carbonyl]cyclohexyl}propanamide

The title compound was synthesized by the reaction and work-up in the same manner as in Example 23 and using the compound obtained in Example 189.
MS (ESI+) : 599 (M+H)
[α]_{D}²⁵ -79.4° (c 1.0, MeOH)

### Example 191

(3S,4R)-4-{(1R)-1-[3,5-Bis(trifluoromethyl)phenyl]ethoxy}-3-phenylpiperidine hydrochloride

IPE was added to the residue (9.24 g) of the filtrate obtained by the crystallization step in Example 186, and the precipitated crystals were collected by filtration. The thus-obtained filtrate was concentrated under reduced pressure, and IPE was added to the residue and the precipitated crystals were collected by filtration (this operation was repeated three times). The residue was suspended in a mixed solution of ethyl acetate and water and basified with 2N aqueous sodium hydroxide solution. The organic layer was washed with brine and dried, and the solvent was evaporated under reduced pressure. To a solution of the obtained residue in ethyl acetate was added a monoequivalent amount of 4N hydrogen chloride/ethyl acetate solution and the mixture was stood at 0°C for 2 days. The precipitated crystals were collected by filtration to give the title compound (2.08 g) as a white powder (diastereomer excess 93.0%).
MS(ESI+): 418 (M-HCl+H)
melting point: 150-152°C
[α]_{D}²⁵ -31.4° (c 1.0,MeOH)
¹H-NMR(DMSO-d₆) : δ 0.95(3H,d,J=6.5Hz),1.72(1H,dlike),1.93(1H,t like), 2.91 (1H,dt,J=13.3 and 2.5Hz), 3.12(1H,d like), 3.27(1H,d like), 3.29(1H,d,like), 3.50(1H,t,J=13.4Hz), 3.97(1H,s like), 4.41(1H,q,J=6.5Hz),7.32(1H,tlike),7.35(2H,d like),7.39(2H,t like),7.93(2H,s),8.01(1H,s),9.19(2H,brs)

### Example 192

tert-Butyl {trans-4-[((3S,4R)-4-{(1R)-1-[3,5-bis(trifluoromethyl)phenyl]ethoxy}-3-phenylpiperidin-1-yl)carbonyl]cyclohexyl}carbamate

The title compound was synthesized by the reaction and work-up in the same manner as in step 1 of Example 22 and using the compound obtained in Example 191.
MS (ESI+) : 643 (M+H)

### Example 193

trans-4-[((3S,4R)-4-{(1R)-1-[3,5-Bis(trifluoromethyl)phenyl]ethoxy}-3-phenylpiperidin-1-yl)carbonyl]cyclohexanamine hydrochloride

The title compound was synthesized by the reaction and work-up in the same manner as in step 2 of Example 22 and using the compound obtained in Example 192.
MS (ESI+) : 543 (M-HCl+H)

### Example 194

N-{trans-4-[((3S,4R)-4-{(1R)-1-[3,5-Bis(trifluoromethyl)phenyl]ethoxy}-3-phenylpiperidin-1-yl)carbonyl]cyclohexyl}propanamide

The title compound was synthesized by the reaction and work-up in the same manner as in Example 23 and using the compound obtained in Example 193.
MS (ESI+) : 599 (M+H)
[α]_{D}²⁵ -56.8° (c 1.0,MeOH)

### Example 195

tert-Butyl {trans-4-[((3R,4S)-4-{(1S)-1-[3,5-bis(trifluoromethyl)phenyl]ethoxy}-3-phenylpiperidin-1-yl)carbonyl]cyclohexyl}carbamate

The title compound was synthesized by the reaction and work-up in the same manner as in step 1 of Example 22 and using the compound obtained in Example 33.
MS (ESI+) : 643 (M+H)

### Example 196

trans-4-[((3R,4S)-4-{(1S)-1-[3,5-Bis(trifluoromethyl)phenyl]ethoxy}-3-phenylpiperidin-1-yl)carbonyl]cyclohexanamine hydrochloride

The title compound was synthesized by the reaction and work-up in the same manner as in step 2 of Example 22 and using the compound obtained in Example 195.
MS (ESI+) : 543 (M-HC1+H)

### Example 197

N-{trans-4-[((3R,4S)-4-{(1S)-1-[3,5-Bis(trifluoromethyl)phenyl]ethoxy}-3-phenylpiperidin-1-yl)carbonyl]cyclohexyl}propanamide

The title compound was synthesized by the reaction and work-up in the same manner as in Example 23 and using the compound obtained in Example 196.
MS (ESI+) : 599 (M+H)
[α]_{D}²⁵ +56.7° (c 1.0,MeOH)

### Example 198

(3R,4S)-1-Benzyl-4-{1-[3-methyl-5-(trifluoromethyl)phenyl]ethoxy}-3-phenylpiperidine

### (Step 1)

To a mixed solution of [3-methyl-5-(trifluoromethyl)benzyl]amine (9.45 g) synthesized by a known method (US6337344) in acetic acid (20 ml) and water (6 ml) was added hexamethylenetetramine (7.70 g), and the mixture was stirred at 110°C for 4 hr. 6N Hydrochloric acid (20 ml) was added to the reaction solution, and the mixture was stirred at 110°C for 30 min. The reaction mixture was basified with 2N aqueous sodium hydroxide, and the product was extracted with ethyl acetate. The organic layer was washed with brine and dried, and the solvent was evaporated under reduced pressure. The obtained residue was distilled and purified to give 3-methyl-5-(trifluoromethyl)benzaldehyde (4.90 g) as a colorless oil.
boiling point: 42-45°C (1 mmHg)
¹H-NMR(CDCl₃) : δ 2.52(3H,s),7.69(1H,s like),7.88(1H,s), 7.94(1H,s),10.04(1H,s)

### (Step 2)

To a solution of the compound obtained in Step 1 (26.0 g) in Et₂O (60 mL) was added 3 mol/L methyl magnesium bromide/Et₂O solution (60 mL) at -78°C and the mixture was stirred at 0°C for 30 min. The reaction mixture was poured into aqueous ammonium chloride, and the product was extracted with ethyl acetate. The organic layer was washed with brine and dried, and the solvent was evaporated under reduced pressure. The obtained residue was purified by silica gel column chromatography (ethyl acetate:hexane=1:20) to give 1-[3-methyl-5-(trifluoromethyl)phenyl]ethanol (26.8 g) as a colorless oil. ¹H-NMR(CDCl₃) : δ1.51(3H,d,J=6.6Hz),1.85(1H,d,J=3,6Hz),2.42(3H,s), 4.89-4.97(1H,m), 7.36(1H,s like), 7.37(1H,s like), 7,43(1H,s like)

### (Step 3)

1-[3-Methyl-5-(trifluoromethyl)phenyl]ethyl 2,2,2-trichloroethaneimidate was synthesized by the reaction and work-up in the same manner as in Step 1 of Example 1 and using the compound obtained in Step 2. ¹H-NMR (CDCl₃) : δ1.65 (3H,d,J=7.2Hz) , 2.42 (3H,s) , 5.98 (1H,q,J=6.6Hz), 7.36(1H,s like),7.39(1H,s like),7.48(1H,s like),8.33(1H,brs)

### (Step 4)

The title compound was obtained as a diastereo mixture by the reaction and work-up in the same manner as in Step 2 of Example 185 and using the compound obtained in Step 3 and (3R,4S)-1-benzyl-3-phenylpiperidin-4-ol.
MS (ESI+) : 454 (M+H)

### Example 199

(3R,4S)-4-{(1R)-1-[3-Methyl-5-(trifluoromethyl)phenyl]ethoxy}-3-phenylpiperidine p-tosylate

The title compound (diastereomer excess 98.0%) was obtained by the reaction and work-up in the same manner as in step 3 of Example 185 and using the compound obtained in Example 198.
MS (ESI+) : 364 (M-CH₃C₆H₄SO₃H+H)
melting point: 167-169°C
¹H-NMR(DMSO-d6) : δ 1.29(3H,d,J=6.0Hz),1.70-1.90(1H,m), 2.10(3H,s),2.20-2.40(5H,m),3.05-3.60(5H,m),4.57(1H,d,J=6.0Hz), 6.64(1H,s),6.99(1H,s),7.06-7.40(8H,m),7.48(2H,d,J=8.4Hz), 8.61(2H,brs)
[α]_{D}²⁵ +47.7° (c 1.0,MeOH)
Measurement of diastereomer excess by LC: measurement conditions: same as those described in Example 185 retention time: 10.8 min (free form of compound of Example 199) 11.2 min (its diastereomer)

### Example 200

N-{trans-4-[((3R,4S)-4-{(1R)-1-[3-Methyl-5-(trifluoromethyl)phenyl]ethoxy}-3-phenylpiperidin-1-yl)carbonyl]cyclohexyl}propanamide

The title compound was obtained by the reaction and work-up in the same manner as in Steps 1 and 2 of Example 22 and Example 23 and using the compound obtained in Example 199.
MS (ESI+) : 545 (M+H)

### Example 201

4-[[(3R,4S)-4-{(1R)-1-[3-Methyl-5-(trifluoromethyl)phenyl]ethoxy}-3-phenylpiperidin-1-yl]carbonyl]piperidine-2,6-dione

The title compound was obtained by the reaction and work-up in the same manner as in the method described in Example 76 and using the compound obtained in Example 199.
MS (ESI+) : 503 (M+H)

### Example 202

4-[[(3R,4S)-4-{(1R)-1-[3,5-Bis(trifluoromethyl)phenyl]ethoxy}-3-phenylpiperidin-1-yl]carbonyl]-N-methylpiperidine-1-carboxamide

The title compound was obtained by the reaction and work-up in the same manner as in the method described in Example 3 and using the compound obtained in Step 1 of Example 34 and methylisocyanate.
MS (ESI+) : 586 (M+H)

### Example 203

4-[[(3R,4S)-4-{(1R)-1-[3,5-Bis (trifluoromethyl) phenyl] ethoxy}-3-phenylpiperidin-1-yl] carbonyl] -N-ethylpiperidine-1-carboxamide

The title compound was obtained by the reaction and work-up in the same manner as in the method described in Example 3 and using the compound obtained in Step 1 of Example 34 and ethylisocyanate.
MS (ESI+) : 600 (M+H)

### Example 204

4-[[(3R,4S)-4-{(1R)-1-[3,5-Bis(trifluoromethyl)phenyl]ethoxy}-3-phenylpiperidin-1-yl)carbonyl]-N,N-dimethylpiperidine-1-carboxamide

The title compound was obtained by the reaction and work-up in the same manner as in the method described in Example 3 and using the compound obtained in Step 1 of Example 34 and N,N-dimethylcarbamate chloride.
MS (ESI+) : 600 (M+H)

### Example 205

Methyl 4-[[(3R,4S)-4-{(1R)-1-[3,5-bis(trifluoromethyl)phenyl]ethoxy}-3-phenylpiperidin-1-yl]carbonyl]-1-carboxylate

The title compound was obtained by the reaction and work-up in the same manner as in the method described in Example 3 and using the compound obtained in Step 1 of Example 34 and methyl chloroformate.
MS (ESI+) : 587 (M+H)

### Example 206

Ethyl 4-[[(3R,4S)-4-{(1R)-1-[3,5-bis(trifluoromethyl)phenyl]ethoxy}-3-phenylpiperidin-1-yl]carbonyl]-1-carboxylate

The title compound was obtained by the reaction and work-up in the same manner as in the method described in Example 3 and using the compound obtained in Step 1 of Example 34 and ethyl chloroformate.
MS (ESI+) : 601 (M+H)

### Example 207

(3R,4S)-4-{(1R)-1-[3,5-Bis(trifluoromethyl)phenyl]ethoxy}-1-{[1-(1H-imidazol-1-ylacetyl)piperidin-4-yl]carbonyl}-3-phenylpiperidine

The title compound was obtained by the reaction and work-up in the same manner as in the method described in Example 3 and using the compound obtained in Step 1 of Example 34 and 1H-imidazol-1-ylacetic acid.
MS (ESI+) : 637 (M+H)

### Example 208

(3R,4S)-1-Benzyl-4-{1-[3-fluoro-5-(trifluoromethyl)phenyl]ethoxy}-3-phenylpiperidine

### (Step 1)

To a solution of 1-[3-fluoro-5-(trifluoromethyl)phenyl]ethanone (10.0 g) in MeOH (15 mL) was added sodium borohydride (1.47 g) at 0°C, and the mixture was stirred at room temperature for 3 hr. The reaction mixture was concentrated under reduced pressure and the residue was poured into a mixed solution of 1N hydrochloric acid and tert-butyl methyl ether, and the organic layer was separated. The organic layer was washed with brine and dried, and the solvent was evaporated under reduced pressure. The obtained residue was purified by silica gel column chromatography (ethyl acetate:hexane=1:10) to give 1-[3-fluoro-5-(trifluoromethyl)phenyl]ethanol (10.7 g) as a colorless oil.
¹H-NMR(CDCl₃) : δ 1.51(3H,d,J=6.0Hz),1.96-2.35(1H,m), 4,96(1H,q,J=6.0Hz),7.11-7.34(2H,m),7.43(1H,s)

### (Step 2)

Crude 1-[3-fluoro-5-(trifluoromethyl)phenyl]ethyl 2,2,2-trichloroethaneimidate was obtained as a colorless oil by the reaction and work-up in the same manner as in the method described in Step 1 of Example 1 and using the compound obtained in Step 1.

### (Step 3)

The title compound was obtained by the reaction and work-up in the same manner as in the method described in Step 2 of Example 185 and using the compound obtained in Step 2 and (3R,4S)-1-benzyl-3-phenylpiperidin-4-ol obtained in Step 1 of

### Example 185.

MS (ESI+) : 458 (M+H)

The compounds described in Example 87 to Example 208 are as shown below (Tables 7-13).

**Table 7**

| | | | | | | | |
|---|---|---|---|---|---|---|---|
| 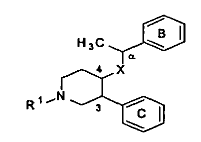 | | | | | | | |

| Ex. No. | stereochemistry | R¹ | X | 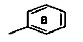 | 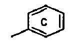 | additive | MS (ESI) |
|---|---|---|---|---|---|---|---|
| | | | | | | | |
| 87 | (3R,4S), α-R | 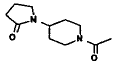 | O | 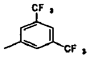 | 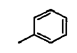 | | 612 |
| 88 | (3R,4S), α-R | 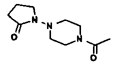 | O | 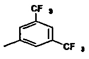 | 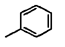 | | 613 |
| 89 | (3R,4S), α-R | 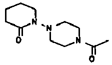 | O | 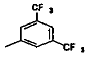 | 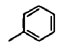 | | 627 |
| 90 | (3R,4S), α-R | 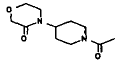 | O | 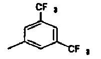 | 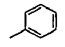 | | 628 |
| 91 | (3R,4S), α-R | 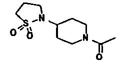 | O | 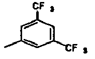 | 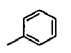 | | 648 |
| 92 | (3R,4S), α-R | 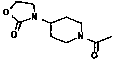 | O | 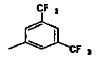 | 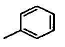 | | 614 |
| 93 | (3R,4S), α-R | 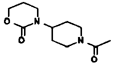 | o | 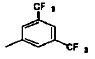 | 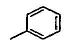 | | 628 |
| 94 | (3R,4S), α-R | 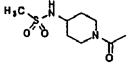 | O | 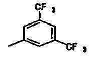 | 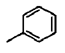 | | 622 |
| 95 | (3R,4S), α-R | 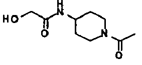 | O | 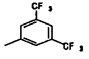 | 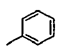 | | 602 |
| 96 | (3R,4S), α-R | 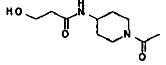 | O | 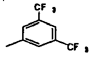 | 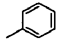 | | 616 |
| 97 | (3R,4S), α-R | 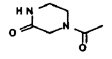 | O | 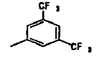 | 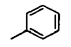 | | 544 |
| 98 | (3R,4S), α-R | 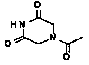 | O | 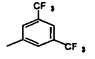 | 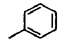 | | 558 |
| 99 | (3R,4S), α-R | 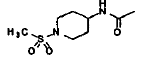 | O | 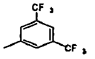 | 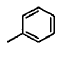 | | 622 |
| 100 | (3R,4S), α-R | 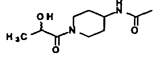 | O | 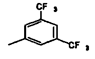 | 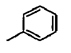 | | 616 |
| 101 | (3R,4S), α-R | 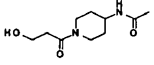 | O | 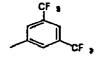 | 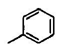 | | 616 |
| 102 | (3R,4S), α-R | 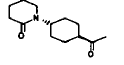 | O | 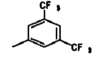 | 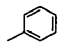 | | 625 |
| 103 | (3R,4S), α-R | 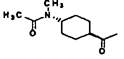 | O | 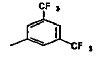 | 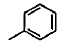 | | 599 |
| 104 | (3R,4S), α-R | 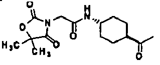 | O | 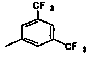 | 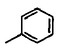 | | 712 |

**Table 8**

| | | | | | | | |
|---|---|---|---|---|---|---|---|
| 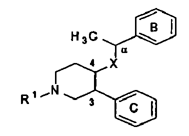 | | | | | | | |

| Ex. No. | stereochemistry | R¹ | X | 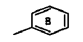 | 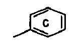 | additive | MS (ESI) |
|---|---|---|---|---|---|---|---|
| 105 | (3R,4S), α-R | 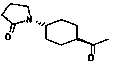 | O | 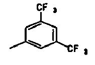 | 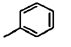 | | 611 |
| 106 | (3R,4S), α-R | 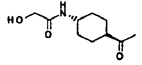 | O | 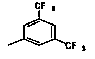 | 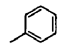 | | 601 |
| 107 | (3R,4S), α-R | 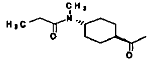 | O | 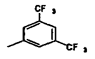 | 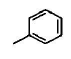 | | 613 |
| 108 | (3R,4S), α-R | 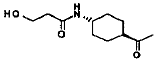 | O | 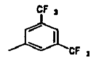 | 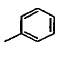 | | 615 |
| 109 | (3R,4S), α-R | 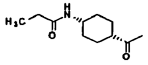 | O | 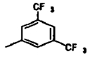 | 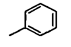 | | 599 |
| 110 | (3R,4S), α-R | 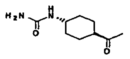 | O | 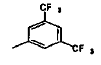 | 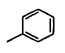 | | 586 |
| 111 | (3R.4S). α-R | 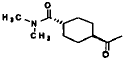 | O | 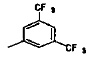 | 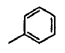 | | 599 |
| 112 | (3R,4S), α-R | 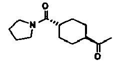 | O | 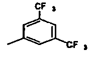 | 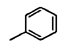 | | 625 |
| 113 | (3R.4S). α-R | 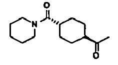 | O | 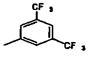 | 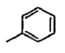 | | 639 |
| 114 | (3R,4S), α-R | 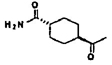 | O | 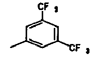 | 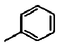 | | 571 |
| 115 | (3R,4S), α-R | 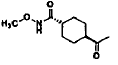 | O | 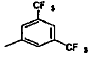 | 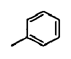 | | 601 |
| 116 | (3R,4S), α-R | 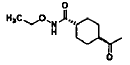 | O | 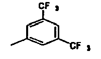 | 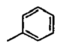 | | 615 |
| 117 | (3R,4S), α-R | 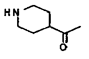 | O | 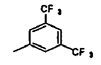 | 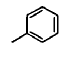 | HCI | 529 |
| 118 | (3R,4S), α-R | 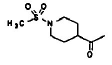 | O | 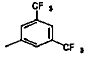 | 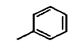 | | 607 |
| 119 | (3R,4S), α-R | 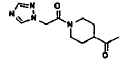 | O | 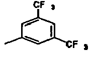 | 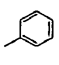 | | 638 |
| 120 | (3R,4S), α-R | 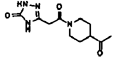 | O | 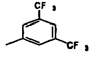 | 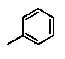 | | 654 |
| 121 | (3R,4S), α-R | 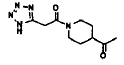 | O | 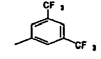 | 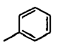 | | 639 |
| 122 | (3R,4S), α-R | 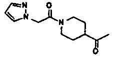 | O | 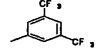 | 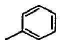 | | 637 |

**Table 9**

| | | | | | | | |
|---|---|---|---|---|---|---|---|
| 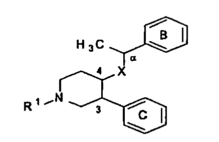 | | | | | | | |

| Ex. No. | stereochemistry | R¹ | X | 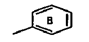 | 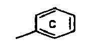 | additive | MS (ESI) |
|---|---|---|---|---|---|---|---|
| 123 | (3R,4S), α-R | 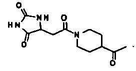 | O | 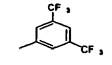 | 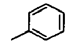 | | 669 |
| 124 | (3R,4S), α-R | 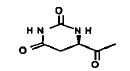 | O | 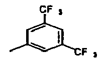 | 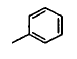 | | 558 |
| 125 | (3R,4S), α-R | 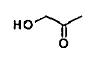 | O | 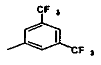 | 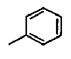 | | 476 |
| 126 | (3R,4S), α-R | 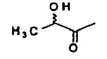 | O | 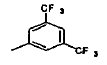 | 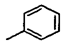 | | 490 |
| 127 | (3R,4S), α-R | 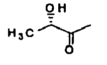 | O | 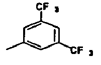 | 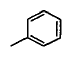 | | 490 |
| 128 | (3R,4S), α-R | 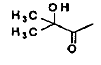 | O | 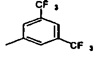 | 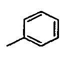 | | 504 |
| 129 | (3R,4S), α-R | 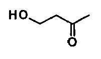 | O | 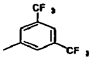 | 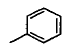 | | 490 |
| 130 | (3R,4S), α-R | 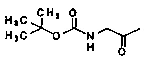 | O | 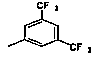 | 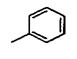 | | 575 |
| 131 | (3R,4S), α-R | 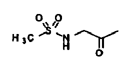 | O | 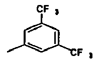 | 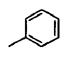 | | 553 |
| 132 | (3R,4S), α-R | 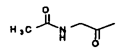 | O | 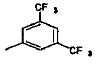 | 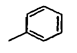 | | 517 |
| 133 | (3R,4S), α-R | 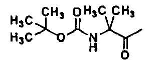 | O | 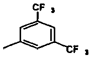 | 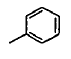 | | 603 |
| 134 | (3R,4S), α-R | 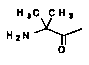 | O | 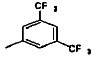 | 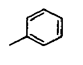 | HCl | 503 |
| 135 | (3R,4S), α-R | 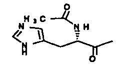 | O | 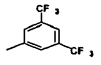 | 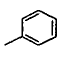 | | 597 |
| 136 | (3R,4S), α-R | 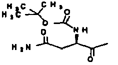 | O | 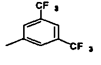 | 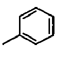 | | 632 |
| 137 | (3R,4S), α-R | 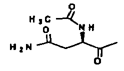 | O | 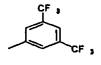 | 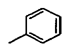 | | 574 |
| 138 | (3R,4S), α-R | 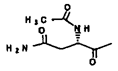 | O | 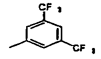 | 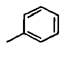 | | 574 |
| 139 | (3R,4S), α-R | 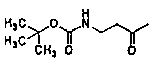 | O | 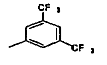 | 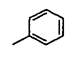 | | 589 |

**Table 10**

| | | | | | | | |
|---|---|---|---|---|---|---|---|
| 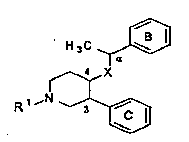 | | | | | | | |

| Ex. No. | stereochemistry | R¹ | X | 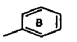 | 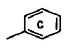 | additive | MS (ESI) |
|---|---|---|---|---|---|---|---|
| 140 | (3R,4S), α-R | 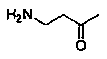 | O | 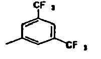 | 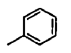 | HCl | 489 |
| 141 | (3R,4S), α-R | 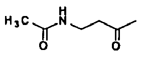 | O | 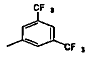 | 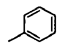 | | 531 |
| 142 | (3R,4S), α-R | 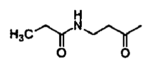 | O | 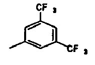 | 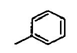 | | 545 |
| 143 | (3R,4S), α-R | 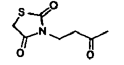 | O | 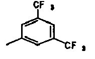 | 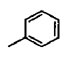 | | 589 |
| 144 | (3R,4S), α-R | 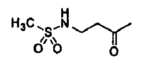 | O | 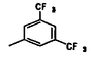 | 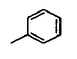 | | 567 |
| 145 | (3R,4S), α-R | 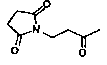 | O | 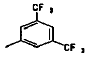 | 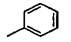 | | 571 |
| 146 | (3R,4S), α-R | 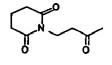 | O | 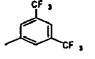 | 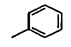 | | 585 |
| 147 | (3R,4S), α-R | 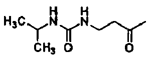 | O | 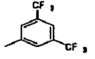 | 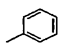 | | 574 |
| 148 | (3R,4S), α-R | 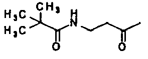 | O | 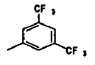 | 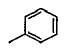 | | 573 |
| 149 | (3R,4S), α-R | 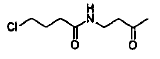 | O | 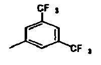 | 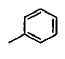 | | 594 |
| 150 | (3R,4S), α-R | 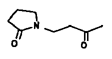 | O | 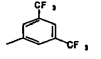 | 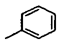 | | 557 |
| 151 | (3R,4S), α-R | 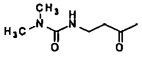 | O | 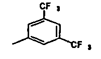 | 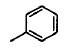 | | 560 |
| 152 | (3R,4S), α-R | 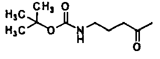 | O | 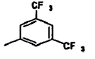 | 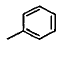 | | 603 |
| 153 | (3R,4S), α-R | 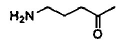 | O | 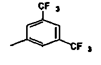 | 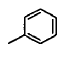 | HCl | 503 |
| 154 | (3R,4S), α-R | 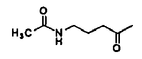 | O | 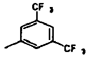 | 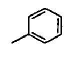 | | 545 |
| 155 | (3R,4S), α-R | 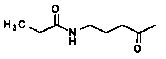 | O | 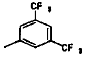 | 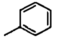 | | 559 |
| 156 | (3R,4S), α-R | 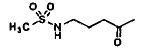 | O | 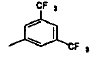 | 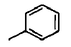 | | 581 |
| 157 | (3R,4S), α-R | 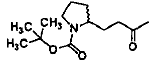 | O | 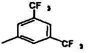 | 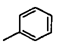 | | 643 |

**Table 11**

| | | | | | | | |
|---|---|---|---|---|---|---|---|
| 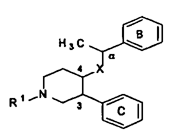 | | | | | | | |

| Ex. No. | stereochemistry | R¹ | X | 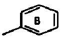 | 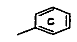 | additive | MS (ESI) |
|---|---|---|---|---|---|---|---|
| 158 | (3R,4S), α-R | 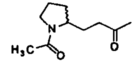 | O | 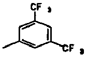 | 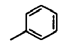 | | 585 |
| 159 | (3R,4S), α-R | 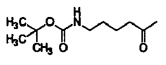 | O | 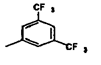 | 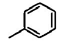 | | 617 |
| 160 | (3R,4S), α-R | 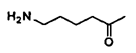 | O | 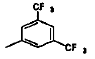 | 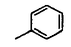 | HCl | 517 |
| 161 | (3R,4S), α-R | 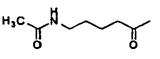 | O | 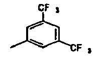 | 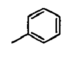 | | 559 |
| 162 | (3R,4S), α-R | 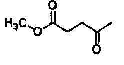 | O | 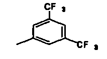 | 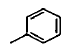 | | 532 |
| 163 | (3R,4S), α-R | 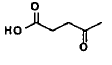 | O | 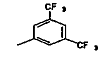 | 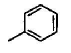 | | 518 |
| 164 | (3R,4S), α-R | 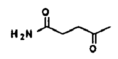 | O | 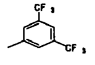 | 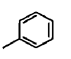 | | 517 |
| 165 | (3R,4S), α-R | 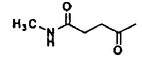 | O | 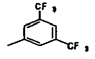 | 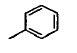 | | 531 |
| 166 | (3R,4S), α-R | 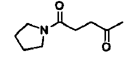 | O | 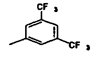 | 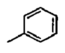 | | 571 |
| 167 | (3R,4S), α-R | 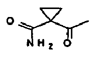 | O | 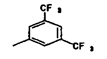 | 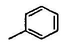 | | 529 |
| 168 | (3R,4S), α-R | 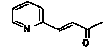 | O | 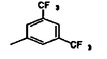 | 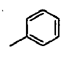 | | 549 |
| 169 | (3R,4S), α-R | 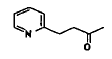 | O | 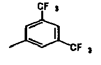 | 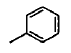 | | 551 |
| 170 | (3R,4S), α-R | 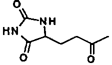 | O | 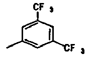 | 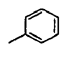 | | 572 |
| 171 | (3R.4S), α-R | 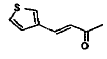 | O | 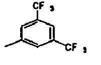 | 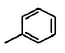 | | 554 |
| 172 | (3R,4S), α-R | 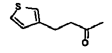 | O | 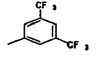 | 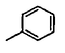 | | 556 |
| 173 | (3R,4S), α-R | 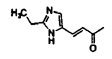 | O | 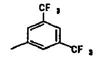 | 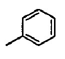 | | 566 |
| 174 | (3R,4S), α-R | 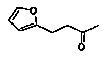 | O | 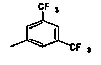 | 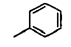 | | 540 |
| 175 | (3R,4S), α-R | 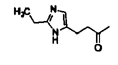 | O | 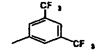 | 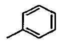 | | 568 |

**Table 12**

| | | | | | | | |
|---|---|---|---|---|---|---|---|
| 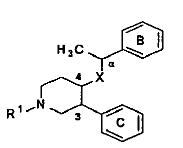 | | | | | | | |

| Ex. No. | stereochemistry | R¹ | X | 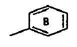 | 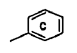 | additive | MS (ESI) |
|---|---|---|---|---|---|---|---|
| 176 | (3R,4S), α-R | 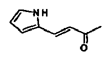 | O | 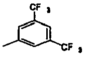 | 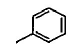 | | 537 |
| 177 | (3R,4S), α-R | 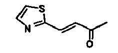 | O | 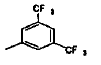 | 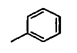 | | 555 |
| 178 | (3R,4S), α-R | 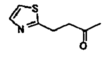 | O | 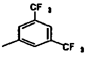 | 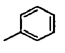 | | 557 |
| 179 | (3R,4S), α-R | 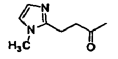 | O | 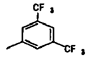 | 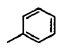 | | 554 |
| 180 | (3R,4S), α-R | 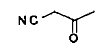 | O | 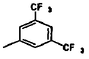 | 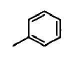 | | 485 |
| 181 | (3R,4S), α-R | 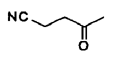 | O | 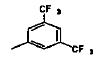 | 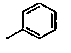 | | 499 |
| 182 | (3R,4S), α-R | 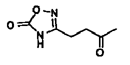 | O | 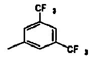 | 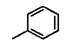 | | 558 |
| 183 | (3R,4S), α-R | 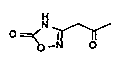 | O | 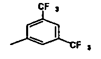 | 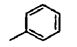 | | 544 |
| 184 | (3R,4S), α-R | 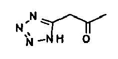 | O | 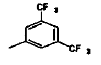 | 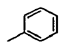 | | 528 |
| 185 | (3R,4S), α-R | H | O | 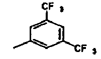 | 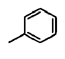 | *p*-TsOH | 418 |
| 186 | (3S,4R), α-S | H | O | 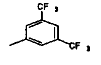 | 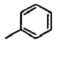 | *p*-TsOH | 418 |
| 187 | (3S,4R), α-S | H | O | 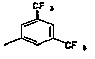 | 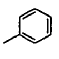 | HCl | 418 |
| 188 | (3S,4R), α-S | 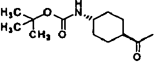 | O | 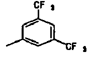 | 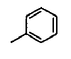 | | 643 |
| 189 | (3S,4R), α-S | 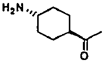 | O | 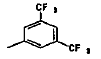 | 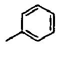 | HCl | 543 |
| 190 | (3S,4R), α-S | 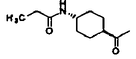 | O | 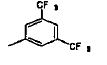 | 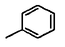 | | 599 |
| 191 | (3S,4R), α-R | H | O | 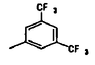 | 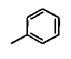 | HCl | 418 |
| 192 | (3S,4R), α-R | 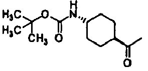 | O | 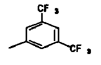 | 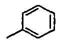 | | 643 |
| 193 | (3S,4R), α-R | 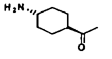 | O | 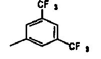 | 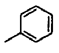 | HCl | 543 |

**Table 13**

| | | | | | | | |
|---|---|---|---|---|---|---|---|
| 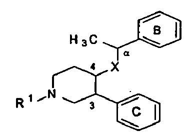 | | | | | | | |

| Ex. No. | stereochemistry | R¹ | X | 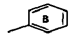 | 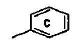 | additive | MS (ESI) |
|---|---|---|---|---|---|---|---|
| 194 | (3S,4R), α-R | 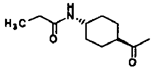 | O | 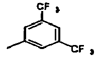 | 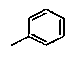 | | 599 |
| 195 | (3R,4S), α-S | 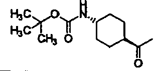 | O | 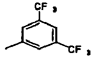 | 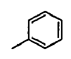 | | 643 |
| 196 | (3R,4S), α-S | 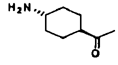 | O | 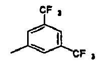 | 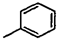 | HCl | 543 |
| 197 | (3R,4S), α-S | 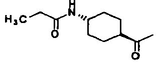 | O | 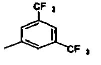 | 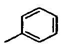 | | 599 |
| 198 | (3R,4S), α-RS | 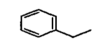 | O | 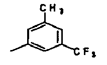 | 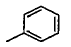 | | 454 |
| 199 | (3R,4S), α-R | H | O | 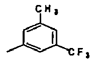 | 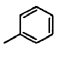 | *p*-TsOH | 364 |
| 200 | (3R,4S), α-R | 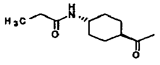 | O | 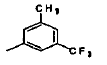 | 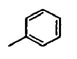 | | 545 |
| 201 | (3R,4S), α-R | 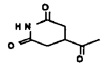 | O | 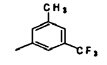 | 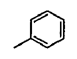 | | 503 |
| 202 | (3R,4S), α-R | 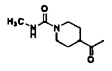 | O | 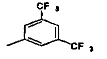 | 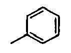 | | 586 |
| 203 | (3R,4S), α-R | 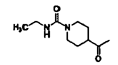 | O | 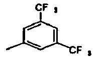 | 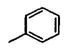 | | 600 |
| 204 | (3R,4S), α-R | 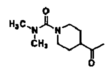 | O | 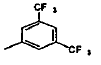 | 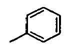 | | 600 |
| 205 | (3R,4S), α-R | 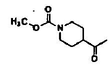 | O | 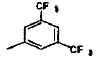 | 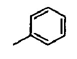 | | 587 |
| 206 | (3R,4S), α-R | 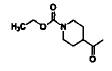 | O | 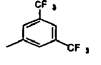 | 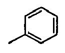 | | 601 |
| 207 | (3R,4S), α-R | 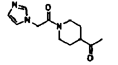 | O | 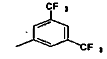 | 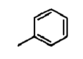 | | 637 |
| 208 | (3R,4S), α-RS | 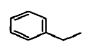 | O | 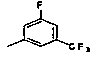 | 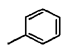 | | 458 |

### Preparative Example 1

| | | |
|---|---|---|
| (1) | Compound of Example 1 | 10 mg |
| (2) | Lactose | 60 mg |
| (3) | Corn starch | 35 mg |
| (4) | Hydroxypropylmethylcellulose | 3 mg |
| (5) | Magnesium stearate | 2 mg |

A mixture of 10 mg of the compound obtained in Example 1, 60 mg of lactose and 35 mg of corn starch is granulated using 0.03 mL of an aqueous solution of 10 wt% hydroxypropylmethylcellulose (3 mg as hydroxypropylmethylcellulose), and then dried at 40°C and sieved. The obtained granules are mixed with 2 mg of magnesium stearate and compressed. The obtained uncoated tablets are sugar-coated with an aqueous suspension of sucrose, titanium dioxide, talc and gum arabic. The thus-coated tablets are glazed with bees wax to obtain finally-coated tablets.

### Preparative Example 2

| | | |
|---|---|---|
| (1) | Compound of Example 1 | 10 mg |
| (2) | Lactose | 70 mg |
| (3) | Corn starch | 50 mg |
| (4) | Soluble starch | 7 mg |
| (5) | Magnesium stearate | 3 mg |

10 mg of the compound obtained in Example 1 and 3 mg of magnesium stearate are granulated with 0.07 mL (7 mg as soluble starch) of an aqueous soluble starch solution, dried, and mixed with 70 mg of lactose and 50 mg of corn starch. The mixture is compressed to obtain tablets.

### Reference Preparative Example 1

| | | |
|---|---|---|
| (1) | Rofecoxib | 5.0 mg |
| (2) | Table salt | 20.0 mg |
| (3) | Distilled water | to 2 mL of total volume |

5.0 mg of rofecoxib and 20.0 mg of table salt are dissolved in distilled water, and water is added to make 2.0 mL of total volume. The solution is filtered, and filled into 2 mL of ampoule under sterile condition. The ampoule is sterilized, and then sealed to obtain a solution for injection.

### Reference Preparative Example 2

| | | |
|---|---|---|
| (1) | Rofecoxib | 50 mg |
| (2) | Lactose | 34 mg |
| (3) | Corn starch | 10.6 mg |
| (4) | Corn starch (paste) | 5 mg |
| (5) | Magnesium stearate | 0.4 mg |
| (6) | Calcium carboxymethylcellulose | 20 mg |
| | total | 120 mg |

The above-mentioned (1) to (6) are mixed according to a conventional method and were tableted by a tablet machine to obtain tablets.

### Preparative Example 3

The formulation prepared in Preparative Example 1 or 2, and the formulation prepared in Reference Preparative Example 1 or 2 are combined.

### Experimental Example 1

Radioligand receptor binding inhibitory activity (Binding inhibitory activity using receptor from human lymphoblast cells (IM-9))

The method of M. A. Cascieri et al., "Molecular Pharmacology 42, p. 458 (1992)" was modified and used. The receptor was prepared from human lymphoblast cells (IM-9). IM-9 cells (2 × 10⁵ cells/mL) were incubated for 3 days (one liter), which was then subjected to centrifuge for 5 min at 500 × G to obtain cell pellets. The obtained pellets were washed once with phosphate buffer (Flow Laboratories, CAT. No. 28-103-05), which were then homogenized using Polytron homogenizer ("Kinematika", Germany) in 30 mL of 50 mM Tris-HCl buffer (pH 7.4) containing 120 mM sodium chloride, 5 mM potassium chloride, 2 µg/mL chymostatin, 40 µg/mL bacitracin, 5 µg/mL phosphoramidon, 0.5 mM phenylmethylsulfonyl fluoride, 1 mM ethylenediamine tetraacetate, which was subjected to centrifuge at 40,000 × G for 20 min. The residue was washed twice with 30 mL of the buffer, which was then preserved frozen (-80°C) as a specimen of the receptor.

The specimen was suspended in a reaction buffer (50 mM Tris-HCl buffer (pH 7.4), 0.02% bovine serum albumin, 1 mM phenylmethylsulfonyl fluoride, 2 µg/mL chymostatin, 40 µg/mL bacitracin and 3 mM manganese chloride) to have protein in the concentration of 0.5 mg/mL of protein and 100 µL portion of the suspension was used in the reaction. After addition of the sample and ¹²⁵I-BHSP (0.46 KBq), the reaction was allowed to proceed in 0.2 mL of reaction buffer at 25°C for 30 min. The amount of nonspecific binding was determined by adding substance P at a final concentration of 2×10⁻⁶ M.

After the reaction, using a cell harvester (290 PHD, Cambridge Technology, Inc, U.S.A.), filtration was carried out through a glass filter (GF/B, Whatman, U.S.A.), which was immersed in 0.1% polyethyleneimine for 24 hr. and dried. After washing three times with 250 µL of 50 mM Tris-HC1 buffer (pH 7.4) containing 0.02% bovine serum albumin, the radioactivity remaining on the filter was determined with a gamma counter.

The antagonistic activity of each compound obtained in Examples was determined in terms of the concentration necessary to cause 50% inhibition (IC₅₀ value) under the above-described conditions, and the results were shown in Table 14.

**Table 14**

| Example No. | IC₅₀ Value (nM) |
|---|---|
| 34 | 0.035 |
| 35 | 0.043 |
| 36 | 0.026 |
| 38 | 0.025 |
| 39 | 0.032 |
| 40 | 0.038 |
| 42 | 0.023 |
| 43 | 0.039 |
| 44 | 0.053 |
| 45 | 0.050 |
| 46 | 0.061 |
| 47 | 0.036 |
| 49 | 0.055 |
| 51 | 0.077 |
| 54 | 0.075 |
| 57 | 0.057 |
| 59 | 0.087 |
| 61 | 0.098 |
| 63 | 0.075 |
| 65 | 0.099 |
| 66 | 0.072 |
| 68 | 0.094 |
| 69 | 0.099 |
| 71 | 0.094 |
| 72 | 0.091 |
| 73 | 0.097 |
| 74 | 0.087 |
| 76 | 0.084 |
| 77 | 0.062 |
| 78 | 0.096 |
| 83 | 0.058 |
| 85 | 0.090 |
| 119 | 0.020 |
| 122 | 0.021 |

The radio ligand means substance P labeled with [¹²⁵I].

From the Table, it has been clarified that the compounds of the present invention have superior antagonistic action for the substance P receptor.

### Experimental Example 2

### Bladder capacity increasing activity of substance P receptor antagonist (bladder capacity increasing action in urethane anesthetized guinea pigs)

A urinary frequency/urine incontinence suppressing effect of a substance having a substance P receptor antagonistic action was shown in terms of the ability to increase bladder capacity of urethane anesthetized male guinea pigs. After emptying the bladder by suction, saline warmed to 39°C is infused into the bladder at a constant rate (0.3 mL/min), until voiding. This procedure was repeated to confirm stable bladder capacity (amount of saline injected before induction of voiding). After confirming a stable response, a compound dissolved in DMSO was intravenously administered and the action was measured. Changes in the bladder capacity after drug administration were measured. The results are shown in Table 15 and Table 16. MK-869 and a substance P receptor antagonist, increased the bladder capacity without affecting the voiding pressure.

**Table 15**

| drug | dose (mg/kg) | number of animals per group | Changes in bladder capacity (vs before administration, %) |
|---|---|---|---|
| DMSO MK-869 | | 6 | 9.1±7.3 |
| | 0.1 | 6 | 14.7±4.8 |
| | 0.3 | 6 | 49.5±10.3* |
| | 1 | 6 | 71.2±16.9** |

| | | | |
|---|---|---|---|
| The data show the mean±S.E.M. (standard error of the mean). * P<0.05, ** P<0.01 (significant difference relative to DMSO administration control group, Dunnett's multiple comparison test, two- tailed test). | | | |

**Table 16**

| drug | dose (mg/kg) | number of animals per group | Changes in bladder capacity (vs before administration, %) |
|---|---|---|---|
| DMSO compounds of Example 43 | | 6 | 2.0±7.6 |
| | 0.01 | 6 | 20.8±5.6 |
| | 0.03 | 6 | 19.3±4.5 |
| | 0.1 | 6 | 39.9±4.9 ^{*} |
| | 0.3 | 6 | 55.2±13.3 ^{**} |
| | 1 | 6 | 74.3±11.1 ^{**} |

| | | | |
|---|---|---|---|
| The data show the mean±S.E.M. *P<0.05, ** P<0.01 (significance relative to DMSO administration control group, Dunnett's multiple comparison test, two- tailed test). | | | |

### Industrial Applicability

The compound of the present invention (I), a salt thereof and a prodrug thereof have potent tachykinin receptor antagonistic action, particularly substance P receptor antagonistic action, are superior in central nervous system penetrating property, show low toxicity and are safe as pharmaceutical agents. Therefore, the compound of the present invention (I) a salt thereof and a prodrug thereof are useful as pharmaceutical agents, such as tachykinin receptor antagonist, agents for the prophylaxis or treatment of lower urinary tract abnormality and the like.

This application is based on a patent application No. 2004-272639 filed in Japan, the contents of which are hereby incorporated by reference. In addition, the references cited herein, including patent document and non-patent document, are hereby incorporated in their entireties by reference, to the extent that they have been disclosed herein.

## Claims

1. A compound represented by the formula: 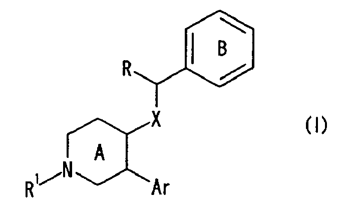 wherein Ar is an aryl group optionally having substituents, R is a C₁₋₆ alkyl group, R¹ is a hydrogen atom, a hydrocarbon group optionally having substituents, an acyl group or a heterocyclic group optionally having substituents, X is an oxygen atom or an imino group optionally having a substituent, ring A is a piperidine ring optionally further having substituents, and ring B is a benzene ring having substituents, or a salt thereof.

2. The compound of claim 1, wherein Ar is a C₆₋₁₄ aryl group optionally having halogen atoms.

3. The compound of claim 1, wherein R is a methyl group.

4. The compound of claim 1, wherein R¹ is a hydrogen atom, a C₆₋₁₄ aryl-C₁₋₆ alkyl group or an acyl group.

5. The compound of claim 1, wherein X is an oxygen atom or NH.

6. The compound of claim 1, wherein Ar has an (R) configuration, and 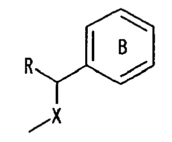 has an (S) configuration.

7. The compound of claim 1, which is an optically active compound represented by the formula: 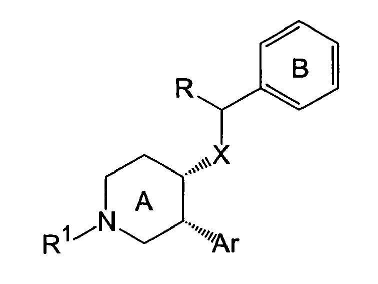 wherein each symbol is as defined in claim 1.

8. The compound of claim 1, wherein X is an oxygen atom.

9. The compound of claim 1, wherein R has an (R) configuration and X is an oxygen atom.

10. The compound of claim 1, wherein ring B is a benzene ring having substituents selected from the group consisting of an optionally halogenated C₁₋₆ alkyl group and a halogen atom.

11. The compound of claim 1, wherein Ar is a C₆₋₁₄ aryl group optionally having halogen atoms,
R is a methyl group, and R¹ is a hydrogen atom, a C₆₋₁₄ aryl-C₁₋₆ alkyl group or an acyl group.

12. The compound of claim 1, wherein Ar is a C₆₋₁₄ aryl group optionally having halogen atoms,
R is a methyl group,
R¹ is
(1) a hydrogen atom,
(2) a formyl group,
(3) a group represented by the formula -(C=O)-NRb¹Rb²
wherein Rb¹ and Rb² are each a hydrogen atom or a C₁₋₆ alkyl group,
(4) a group represented by the formula -(C=O)-(CH₂)m-Rb³
wherein m is an integer of 1 to 3, and Rb³ is a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which is optionally substituted by substituents selected from the group consisting of C₁₋₆ alkyl, formyl, C₁₋₆ alkyl-carbonyl, hydroxy, C₁₋₆ alkoxy-carbonyl, formylamino and C₁₋₆ alkyl-carbonylamino, and optionally has one or two oxo groups,
(5) a group represented by the formula-(C=O)-(CH₂)n-NRb⁴Rb⁵
wherein n is an integer of 1 to 4, Rb⁴ and Rb⁵ are each (a) a hydrogen atom, (b) a formyl group, (c) a C₁₋₆ alkyl-carbonyl group optionally having halogen atoms, (d) a C₁₋₆ alkoxy-carbonyl group, (e) a C₁₋₆ alkylsulfonyl group or (f) a mono- or di-C₁₋₆ alkyl-carbamoyl group,
(6) a group represented by the formula: 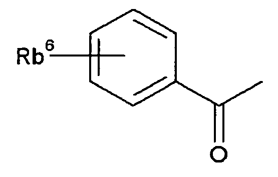 wherein Rb⁶ is formylamino or a C₁₋₆ alkyl-carbonylamino group,
(7) a group represented by the formula: 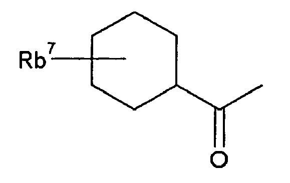 wherein Rb⁷ is
(i) an amino group,
(ii) a formylamino group,
(iii) a C₁₋₆ alkyl-carbonylamino group optionally substituted by substituents selected from the group consisting of (a) C₁₋₆ alkyl, (b) hydroxy, (c) C₁₋₆ alkoxy, (d) a halogen atom, (e) formylamino, (f) C₁₋₆ alkyl-carbonylamino, and (g) a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups and one or two C₁₋₆ alkyl groups,
(iv) a C₃₋₇ cycloalkyl-carbonylamino group,
(v) a C₁₋₆ alkoxy-carbonylamino group,
(vi) a 5- or 6-membered aromatic heterocycle-carbonyl-amino group, wherein the heterocycle contains, besides carbon atoms, 1 to 4 of one or two kinds of hetero atoms selected from the group consisting of a nitrogen atom, an oxygen atom and a sulfur atom,
(vii) an ureido group optionally substituted by one or two C₁₋₆ alkyl groups,
(viii) a C₁₋₆ alkoxy-carbonyl group,
(ix) a carbamoyl group optionally having C₁₋₆ alkoxy groups,
(x) a mono- or di-C₁₋₆ alkyl-carbamoyl group,
(xi) a C₁₋₆ alkylsulfonylamino group,
(xii) a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups, or
(xiii) a 5- or 6-membered aromatic or non-aromatic heterocycle-carbonyl group, wherein the heterocycle contains, besides carbon atoms, 1 to 4 of one or two kinds of hetero atoms selected from the group consisting of a nitrogen atom, an oxygen atom and a sulfur atom,
(8) a group represented by the formula: 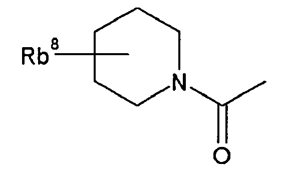 wherein Rb⁸ is (i) a formylamino group, (ii) a C₁₋₆ alkyl-carbonylamino group optionally substituted by hydroxyl groups, (iii) a C₁₋₆ alkylsulfonylamino group, or (iv) a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups,
(9) a group represented by the formula: 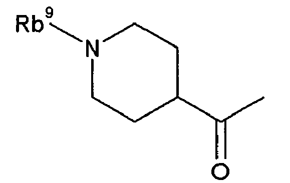 wherein Rb⁹ is
(i) a hydrogen atom,
(ii) a C₁₋₆ alkyl group optionally substituted by substituents selected from the group consisting of formyl, C₁₋₆ alkyl-carbonyl, C₁₋₆ alkoxy-carbonyl, carbamoyl and mono or di-C₁₋₆ alkyl-carbamoyl,
(iii) a formyl group,
(iv) a C₁₋₆ alkyl-carbonyl group optionally substituted by substituents selected from the group consisting of (a) formylamino, (b) C₁₋₆ alkyl-carbonylamino, and (c) a 5- or 6-membered aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups,
(v) a 5- or 6-membered aromatic or non-aromatic heterocycle-carbonyl group, wherein the heterocycle comprises, besides carbon atoms, 1 to 4 of one or two kinds of hetero atoms selected from the group consisting of a nitrogen atom, an oxygen atom and a sulfur atom, which optionally has one or two oxo groups,
(vi) a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups,
(vii) a formylamino group,
(viii) a C₁₋₆ alkyl-carbonylamino group,
(ix) a C₁₋₆ alkylsulfonyl group,
(x) a C₁₋₆ alkoxy-carbonyl group, or
(xi) a mono- or di-C₁₋₆ alkyl-carbamoyl group,
(10) a group represented by the formula: 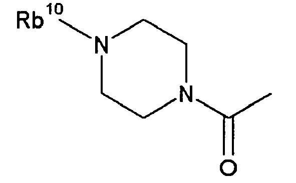 wherein Rb¹⁰ is (i) a formyl group, (ii) a C₁₋₆ alkyl-carbonyl group or (iii) a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which optionally has one or two oxo groups,
(11) a group represented by the formula: 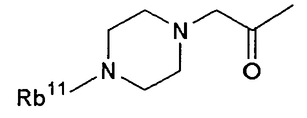 wherein Rb¹¹ is a C₁₋₆ alkyl group, a formyl group, or a C₁₋₆ alkyl-carbonyl group,
(12) a group represented by the formula: 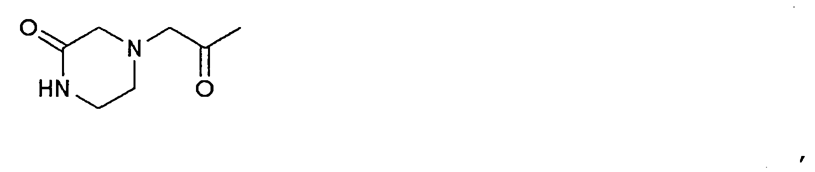
(13) a group represented by the formula: 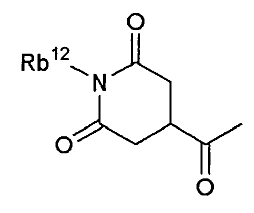 wherein Rb¹² is a hydrogen atom or a C₁₋₆ alkyl group,
(14) a group represented by the formula: 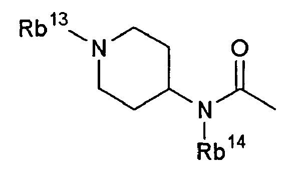 wherein Rb¹³ is
(i) a hydrogen atom,
(ii) a formyl group,
(iii) a C₁₋₆ alkyl-carbonyl group optionally substituted by hydroxyl groups,
(iv) a C₁₋₆ alkoxy-carbonyl group, or
(v) a C₁₋₆ alkylsulfonyl group, and Rb¹⁴ is a hydrogen atom or a C₁₋₆ alkyl group,
(15) a group represented by the formula: 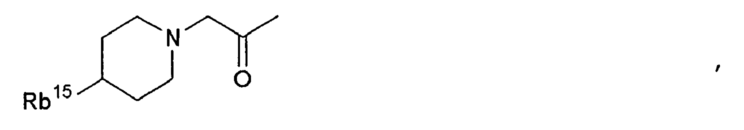 wherein Rb¹⁵ is a hydroxy group, formylamino or a C₁₋₆ alkyl-carbonylamino group,
(16) a group represented by the formula: 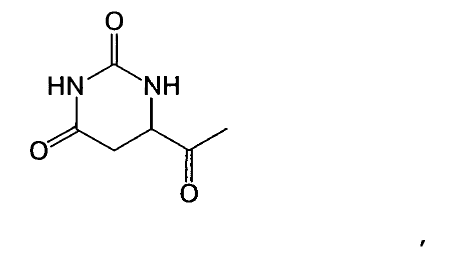
(17) a group represented by the formula: 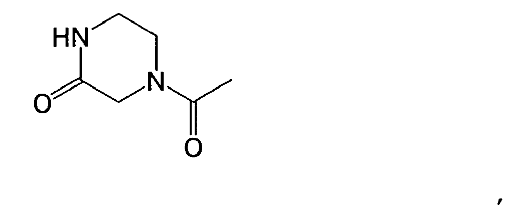
(18) a group represented by the formula: 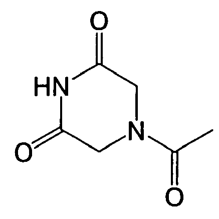
(19) a C₁₋₆ alkyl-carbonyl group optionally substituted by substituents selected from the group consisting of (i) cyano, (ii) hydroxy, (iii) carboxy, (iv) C₁₋₆ alkoxy-carbonyl, (v) carbamoyl, (vi) C₁₋₆ alkyl-carbamoyl and (vii) a 5- or 6-membered aromatic or non-aromatic heterocycle-carbonyl, wherein the heterocycle comprises, besides carbon atoms, 1 to 4 of one or two kinds of hetero atoms selected from the group consisting of a nitrogen atom, an oxygen atom and a sulfur atom,
(20) a C₃₋₆ cycloalkyl-carbonyl group optionally substituted by carbamoyl groups,
(21) a C₁₋₆ alkenyl-carbonyl group substituted by a 5- or 6-membered aromatic or non-aromatic heterocyclic group containing, besides carbon atoms, 1 to 4 of one or two kinds of hetero atoms selected from the group consisting of a nitrogen atom, an oxygen atom and a sulfur atom, which is optionally substituted by C₁₋₆ alkyl groups,
(22) a group represented by the formula-(C=O)-(CRb¹⁶Rb¹⁷)-NHRb¹⁸ wherein Rb¹⁶ is a hydrogen atom or a C₁₋₆ alkyl group, Rb¹⁷ is a C₁₋₆ alkyl group optionally substituted by substituents selected from the group consisting of a carbamoyl group and a 5- or 6-membered aromatic heterocyclic group containing, besides carbon atoms, 1 to 4 of one or two kinds of hetero atoms selected from the group consisting of a nitrogen atom, an oxygen atom and a sulfur atom, and Rb¹⁸ is a hydrogen atom, a C₁₋₆ alkyl-carbonyl group or a C₁₋₆ alkoxy-carbonyl group, or
(23) a C₆₋₁₄ aryl-C₁₋₆ alkyl group,
X is an oxygen atom or NH, and
ring B is a benzene ring having substituents selected from the group consisting of an optionally halogenated C₁₋₆ alkyl group and a halogen atom.

13. (3R,4S)-4-[(1R)-1-[3,5-Bis(trifluoromethyl)phenyl]ethoxy]-3-phenyl-1-[[1-(1H-tetrazol-1-ylacetyl)piperidin-4-yl]carbonyl]piperidine, N-[trans-4-[[(3R,4S)-4-[(1R)-1-[3,5-bis (trifluoromethyl)phenyl] ethoxy] -3-phenylpiperidin-1-yl]carbonyl]cyclohexyl]propanamide, 4-[[(3R,4S)-4-[(1R)-1-[3,5-bis(trifluoromethyl)phenyl]ethoxy]-3-phenylpiperidin-1-yl]carbonyl]piperidine-2,6-dione, (3R,4S)-4-{(1R)-1-[3,5-bis(trifluoromethyl)phenyl]ethoxy}-3-phenyl-1-{[1-(1H-1,2,4-triazol-1-ylacetyl)piperidin-4-yl]carbonyl}piperidine, (3R,4S)-4-{(1R)-1-[3,5-bis(trifluoromethyl)phenyl]ethoxy}-3-phenyl-1-{[1-(1H-pyrazol-1-ylacetyl)piperidin-4-yl]carbonyl}piperidine, or a salt thereof.

14. A prodrug of the compound of claim 1.

15. A pharmaceutical composition comprising the compound of claim 1 or a prodrug thereof.

16. The pharmaceutical composition of claim 15, which is a tachykinin receptor antagonist.

17. The pharmaceutical composition of claim 15, which is an agent for the prophylaxis or treatment of lower urinary tract abnormality, gastrointestinal disease or central nervous system disease.

18. The pharmaceutical composition of claim 15, which is an agent for the prophylaxis or treatment of overactive bladder, irritable bowel syndrome, inflammatory bowel disease, vomiting, nausea, depression, anxiety neurosis, anxiety, pelvic visceral pain or interstitial cystitis.

19. A method for the prophylaxis or treatment of lower urinary tract abnormality, gastrointestinal disease or central nervous system disease in a mammal, which comprises administering an effective amount of the compound of claim 1 or a prodrug thereof.

20. Use of the compound of claim 1 or a prodrug thereof for the production of an agent for the prophylaxis or treatment of lower urinary tract abnormality, gastrointestinal disease or central nervous system disease.
